# EUROPEAN PATENT APPLICATION

(11) **EP 2 182 040 A2**
(43) Date of publication of application: **05.05.2010**
(21) Application number: 09156604.2
(22) Date of filing: 30.03.2009
(51) Int. Cl.: C09K 11/06

(54) **Aromatic compounds and organic electronic device using the same**

(30) Priority: 31.10.2008 KR 20080107606
(71) Applicant: Gracel Display Inc., Seoul 133-833 (KR)
(72) Inventor: Eum, Sung Jin, Gwanak-gu Seoul (KR); Cho, Young Jun, 136-060, Seoul (KR); Kwon, Hyuck Joo, 130-100, Seoul (KR); Kim, Bong Ok, 135-090, Seoul (KR); KIM, Sung Min, 157-886, Seoul (KR); Yoon, Seung Soo, 135-884, Seoul (KR)
(74) Representative: Kent, Venetia Katherine

(57) **Abstract**

Provided are novel compounds for organic electronic material, and organic electronic devices comprising the same. Specifically, the compounds for organic electronic material according to the invention are **characterized in that** they are represented by Chemical Formula (1) or (2): 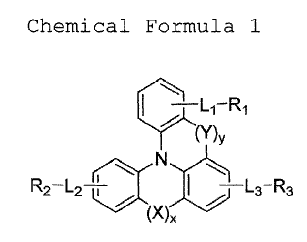 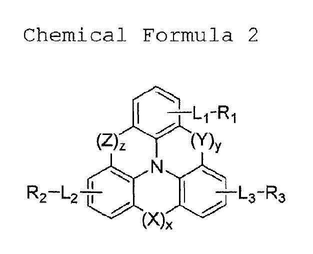 wherein, R₁, R₂ and R₃ cannot be hydrogen all at the same time; excluding the case wherein R₁-L₁- 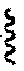 , R₂-L₂- 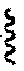 or R₃-L₃- 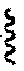 independently represents diphenylamino group ( 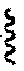 -NPh₂).

## Description

### FIELD OF THE INVENTION

The present invention relates to novel compounds for organic electronic material, and organic electronic devices comprising the same. More specifically, the invention relates to novel compounds for organic electronic material having high efficiency, and organic electronic devices which comprise the compounds in a hole transport layer or a hole injecting layer, or in an electroluminescent layer as the host for phosphor.

### BACKGROUND OF THE INVENTION

Among display devices, electroluminescence devices (EL devices) are self-luminescent display devices showing the advantage of wide angle of view, excellent contrast and rapid response rate. Eastman Kodak developed in 1987 an organic EL device which employs a low molecular weight aromatic diamine and an aluminum complex as material for forming an EL layer, for the first time [Appl. Phys. Lett. 51, 913, 1987].

An organic EL device is a device wherein, when charge is applied to an organic film formed between an electron injection electrode (cathode) and a hole injection electrode (anode), an electron and a hole form a pair and then become extinct with emitting light. A device can be formed on a transparent flexible substrate such as plastics. The device can be operated at a lower voltage (not more than 10 V) with relatively lower power consumption but excellent color purity, as compared to a plasma display panel or an inorganic EL display.

The most important factor to determine the performances such as luminous efficiency, lifetime or the like in an organic EL device is electroluminescent material. Several properties required for such electroluminescent materials include that the material should have high fluorescent quantum yield in solid state and high mobility of electrons and holes, is not easily decomposed during vapor-deposition in vacuo, and forms uniform and stable thin film.

Organic electroluminescent materials can be generally classified into high-molecular materials and low-molecular materials. The low-molecular materials include metal complexes and thoroughly organic electroluminescent materials which do not contain metal, from the aspect of molecular structure. Such electroluminescent materials include chelate complexes such as tris(8-quinolinolato)aluminum complexes, coumarin derivatives, tetraphenylbutadiene derivatives, bis(styrylarylene) derivatives and oxadiazole derivatives. From those materials, it is reported that light emission of visible region from blue to red can be obtained

Three electroluminescent materials (for red, green and blue) are employed to realize a full-colored OLED display. The important issue is to develop red, green and blue electroluminescent materials with high efficiency and long life, in order to enhance the overall feature of the organic electroluminescent (EL) devices. The EL materials are classified into host materials and dopant materials from the aspect of their functions. It is generally known that a device structure having the most excellent EL properties can be fabricated with an EL layer prepared by doping a dopant to a host. Recently, development of organic EL devices with high efficiency and long life comes to the fore as an urgent subject, and particularly urgent is development of a material with far better EL properties as compared to conventional EL materials as considering EL properties required for a medium to large sized OLED panel.

As a host material for phosphorescent light emitting material, 4,4'-N,N'-dicarbazole-biphenyl (CBP) has been most widely known up to the present, and OLED's having high efficiency to which a hole blocking layer (such as BCP and BAlq) had been applied have been developed. Pioneer (Japan) or the like reported OLED's of high performances which were developed by using bis(2-methyl-8-quinolinato)(p-phenylphenolato)aluminum (III) (BAlq) derivatives as the host.

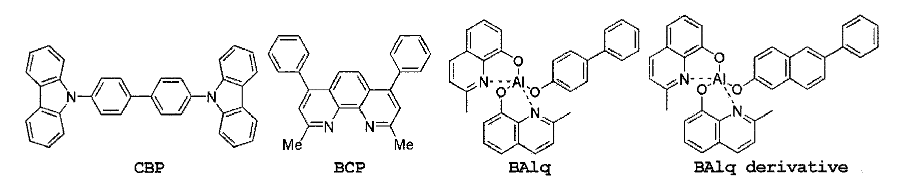

Though the conventional materials are advantageous in view of light emitting property, they have low glass transition temperature and very poor thermal stability, so that the materials tend to be changed during high temperature vapor-deposition in vacuo. In an organic electroluminescent device (OLED), it is defined that power efficiency = (π/voltage) x current efficiency. Thus, the power efficiency is inversely proportional to the voltage, and the power efficiency should be higher in order to obtain lower power consumption of an OLED. In practice, an OLED employing phosphorescent electroluminescent (EL) material shows significantly higher current efficiency (cd/A) than an OLED employing fluorescent EL material. However, in case that a conventional material such as BAlq and CBP as host material of the phosphorescent EL material is employed, no significant advantage can be obtained in terms of power efficiency (lm/w) because of higher operating voltage as compared to an OLED employing a fluorescent material.

Furthermore, there was no satisfactory result in view of life of an OLED, so that development of host material providing better stability and higher performance is still required.

Further, hole injecting/transport materials including copper phthalocyanine (CuPc), 4,4'-bis[N-(1-naphthyl)-N-phenylamino]biphenyl (NPB) and MTDATA have been known.

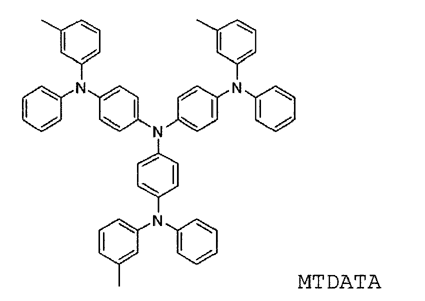

In view of improvement in durability of an organic electroluminescent device, it is reported that compounds having higher non-crystallinity give higher stability of thin film. Glass transition temperature (Tg) is employed as an index for non-crystallinity.

It is recognized that conventional MTDATA does not show high non-crystallinity, having glass transition temperature of 76°C. Thus, no satisfactory property could be obtained in terms of durability of the organic EL device, or luminous efficiency which comes from the hole injecting/transport property.

### SUMMARY OF INVENTION

Thus, the object of the invention is to provide organic novel compounds for organic electronic material having the backbone to give better luminous efficiency and device life with appropriate color coordinate as compared to those of conventional host material of phosphor, with overcoming disadvantages of them.

Another object of the invention is to provide organic electronic device employing the novel compounds for organic electronic material in the hole injecting layer, the hole transport layer or the electroluminescent layer.

Still another object of the invention is to provide organic solar cells comprising the novel compounds for organic electronic material.

The present invention relates to novel compounds for organic electronic material, and organic electronic devices comprising the same. Specifically, the novel compounds for organic electronic material according to the invention are represented by Chemical Formula (1) or Chemical Formula (2): 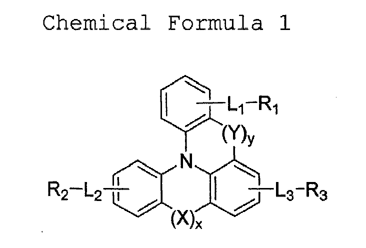 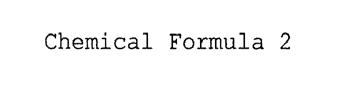 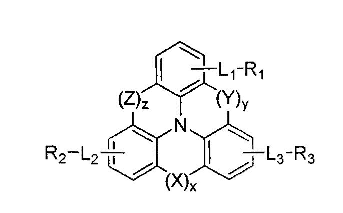 wherein,
L₁, L₂ and L₃ independently represent a chemical bond, (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C6-C60)ar(C1-C60)alkylene, (C1-C60)alkyenethio, (C1-C60)alkylenoxy, (C6-C60)arylenoxy or (C6-C60)arylenethio;
R₁, R₂ and R₃ independently represent hydrogen, deuterium, (C6-C60)aryl, (C3-C60)heteroaryl, 5- or 6-membered heterocycloalkyl containing one or more heteroatom(s) selected from N, O and S, (C3-C60)cycloalkyl, adamantyl, (C7-C60)bicycloalkyl, (C2-C60)alkenyl, (C2-C60)alkynyl, (C1-C60)alkylthio, (C6-C60)aryloxy, (C6-C60)arylthio, NR₁₁R₁₂, or a substituent selected from the following structures (excluding the case wherein R₁, R₂ and R₃ are hydrogen all at the same time): 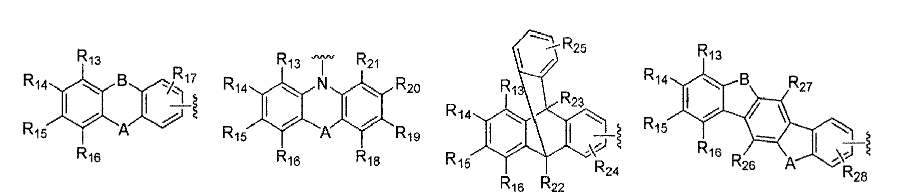 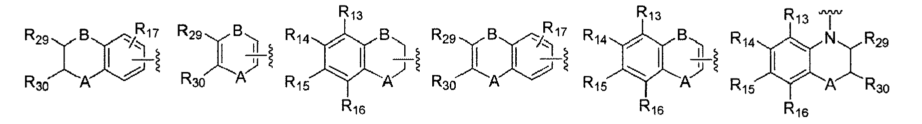 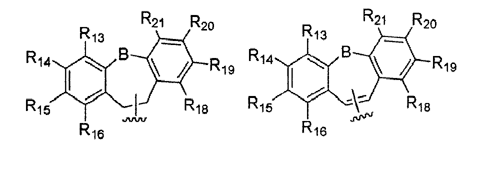
R₁₁ and R₁₂ independently represent (C6-C60)aryl or (C3-C60)heteroaryl, or may be linked via (C3-C60)alkylene or (C3-C60)alkenylene with or without a fused ring to form an alicyclic ring, or a monocyclic or polycyclic aromatic ring;
R₁₃ through R₃₀ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₁₃ through R₂₅ may be linked to an adjacent substituent via (C3-C60)alkylene or (C3-C60)alkenylene with or without a fused ring to form an alicyclic ring, or a monocyclic or polycyclic aromatic ring;
A, B, X, Y and Z independently represent a chemical bond, or -(CR₃₁R₃₂)ₐ-, -N(R₃₃)-, -S-, -O-, -Si(R₃₄)(R₃₅)-, -P(R₃₆)-, - C(=O)-, -B(R₃₇)-, -In(R₃₈)-, -Se-, -Ge(R₃₉)(R₄₀)-, -Sn(R₄₁)(R₄₂)-, -Ga(R₄₃)- or -(R₄₄)C=C(R₄₅)-;
R₃₁ through R₄₅ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenoxy or arylenethio of L₁, L₂ and L₃; the aryl or heteroaryl of R₁, R₂, R₃, R₁₁ and R₁₂; or the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, aralkyl, alkyloxy, alkylthio, aryloxy, arylthio, alkylamino, arylamino, alkoxycarbonyl, alkylcarbonyl or arylcarbonyl of R₁₃ through R₃₀ and R₃₁ through R₄₅ may be further substituted by one or more substituent(s) selected from a group consisting of deuterium, halogen, (C1-C60)alkyl with or without halogen substituent(s), (C6-C60)aryl, (C3-C60)heteroaryl with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
a, x, y and z independently represent an integer from 0 to 4;
but excluding the case wherein R₁-L₁-, R₂-L₂- or R₃-L₃-independently represents diphenylamino group (-NPh₂).

### BRIEF DESCRIPTION OF THE DRAWINGS

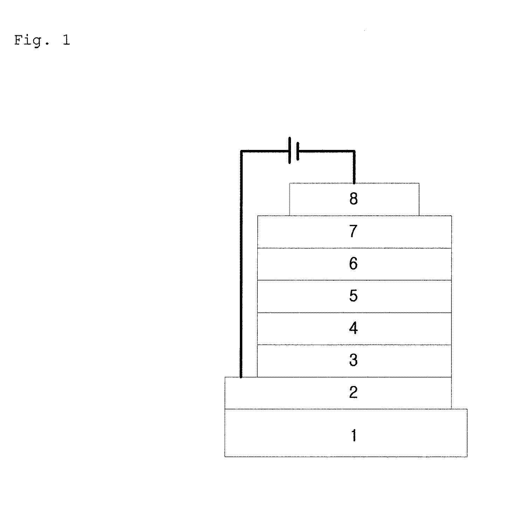

Fig. 1 is a cross-sectional view of an OLED.

### DETAILED DESCRIPTION OF THE INVENTION

Referring now to the Drawings, Fig. 1 illustrates a cross-sectional view of an OLED of the present invention comprising a Glass 1, Transparent electrode 2, Hole injecting layer 3, Hole transport layer 4, Electroluminescent layer 5, Electron transport layer 6, Electron injecting layer 7 and Al cathode 8.

The term "alkyl" and other substituents containing "alkyl" moiety includes linear and branched species.

The term "aryl" described herein means an organic radical derived from aromatic hydrocarbon via elimination of one hydrogen atom. Each ring suitably comprises a monocyclic or fused ring system containing from 4 to 7, preferably from 5 to 6 cyclic atoms. Specific examples include phenyl, naphthyl, biphenyl, anthryl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S for the aromatic cyclic backbone atoms, and carbon atom(s) for remaining aromatic cyclic backbone atoms. The heteroaryl may be 5- or 6-membered monocyclic heteroaryl or a polycyclic heteroaryl which is fused with one or more benzene ring(s), and may be partially saturated. The heteroaryl groups may include divalent aryl groups of which the heteroatoms are oxidized or quarternized to form N-oxides, quaternary salts, or the like. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The substituents comprising "(C1-C60)alkyl" moiety described herein may contain 1 to 60 carbon atoms, 1 to 20 carbon atoms, or 1 to 10 carbon atoms. The substituents comprising "(C6-C60)aryl" moiety may contain 6 to 60 carbon atoms, 6 to 20 carbon atoms, or 6 to 12 carbon atoms. The substituents comprising "(C3-C60)heteroaryl" moiety may contain 3 to 60 carbon atoms, 4 to 20 carbon atoms, or 4 to 12 carbon atoms. The substituents comprising "(C3-C60)cycloalkyl" moiety may contain 3 to 60 carbon atoms, 3 to 20 carbon atoms, or 3 to 7 carbon atoms. The substituents comprising "(C2-C60)alkenyl or alkynyl" moiety may contain 2 to 60 carbon atoms, 2 to 20 carbon atoms, or 2 to 10 carbon atoms.

In Chemical Formula (1) or (2), if R₁-L₁-, R₂-L₂- and R₃-L₃- independently represent diphenylamino group (-NPh₂) (in other word, if L₁, L₂ or L₃ represents chemical bonds, R₁, R₂ or R₃ represent NR₁₁R₁₂, and both R₁₁ and R₁₂ represent phenyl), at least one of phenyl must have a substituent other than hydrogen.

The compounds for organic electronic material according to the invention can be selected from those represented by one of Chemical Formulas (3) to (6): 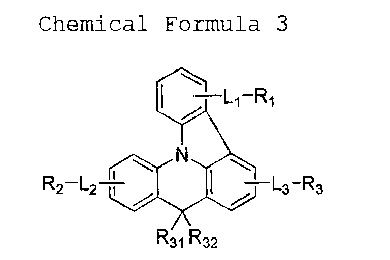 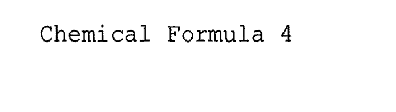 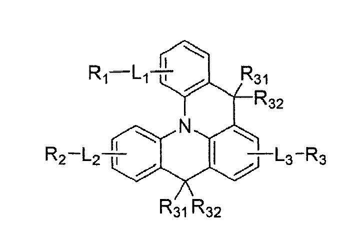 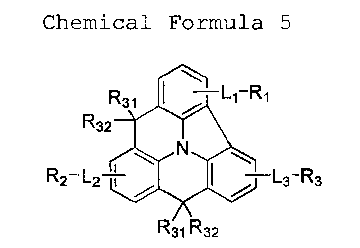 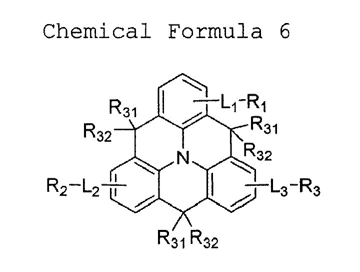 wherein, L₁, L₂, L₃, R₁, R₂, R₃, R₃₁ and R₃₂ are defined as in Chemical Formula (1) or (2).

In the chemical formulas, R₁, R₂ and R₃ independently represent, without restriction, hydrogen, deuterium or NR₁₁R₁₂, or a substituent selected from the following structures: 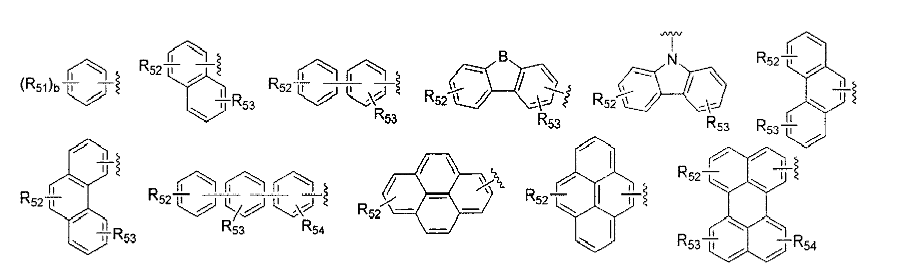 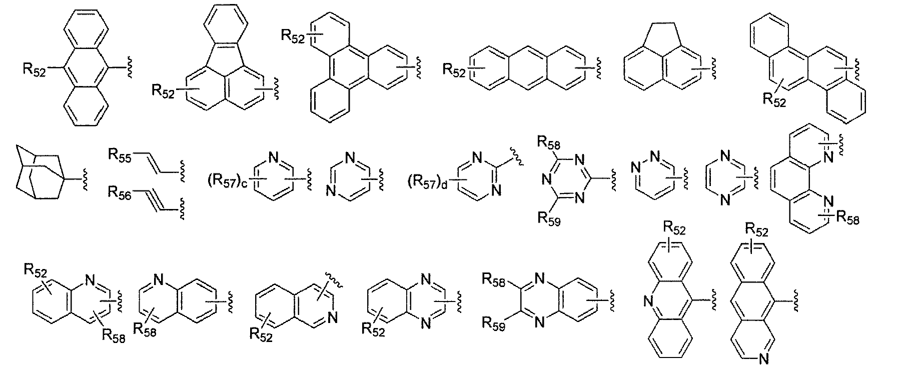 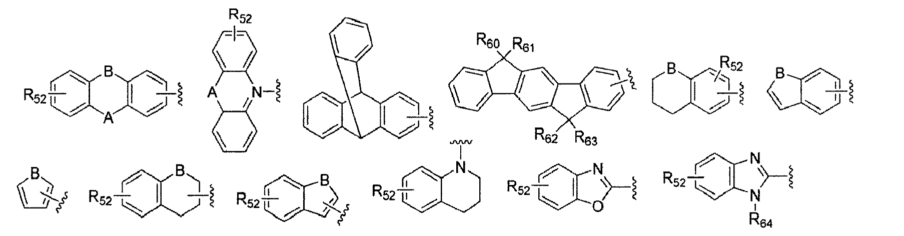 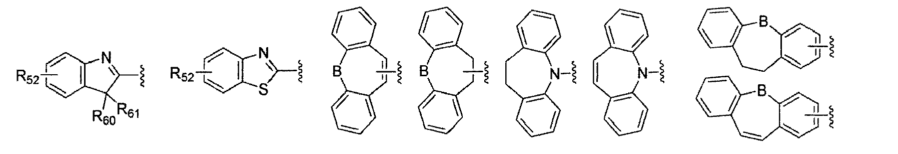
wherein, R₁₁ and R₁₂ are defined as in Chemical Formula (1) or (2);
R₅₁ through R₆₄ independently represent hydrogen, deuterium, halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl;
A and B independently represent -C(R₃₁)(R₃₂)-, -N(R₃₃)-, - S-, -O-, -Si(R₃₄)(R₃₅)-, -P(R₃₆)-, -C(=O)-, -B(R₃₇)-, -In(R₃₈)-, - Se-, -Ge(R₃₉)(R₄₀)-, -Sn(R₄₁)(R₄₂)-, -Ga(R₄₃)- or -(R₄₄)C=C(R₄₅)-;
R₃₁ through R₄₅ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₃₁ through R₄₅ and R₅₁ through R₆₄ may be further substituted by deuterium, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl;
b represents an integer from 1 to 5; c represents an integer from 1 to 4; and d represents an integer from 1 to 3.

More specifically, R₁, R₂ and R₃ can be independently exemplified by the following structures, without restriction:

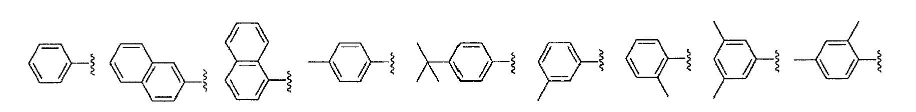

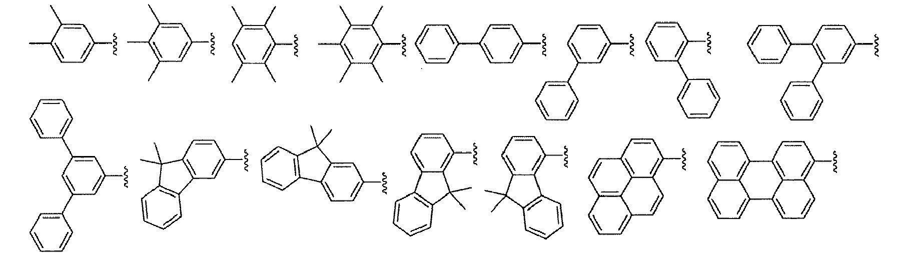

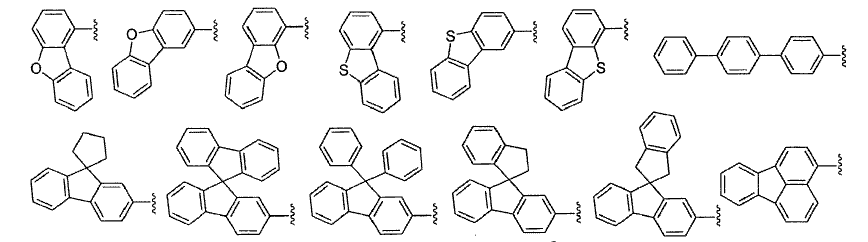

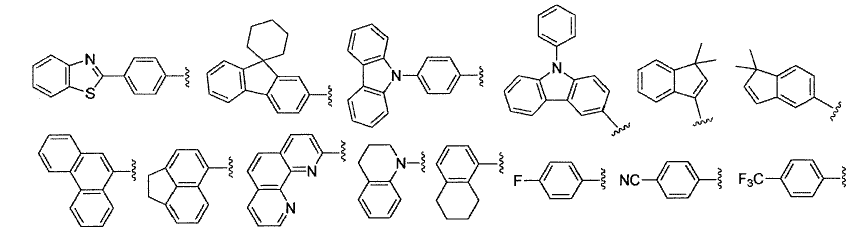

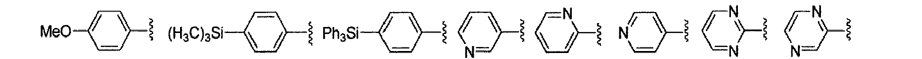

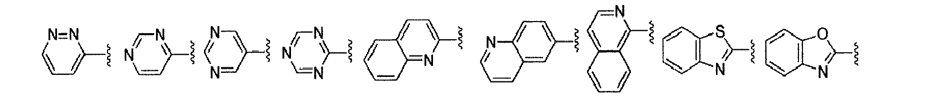

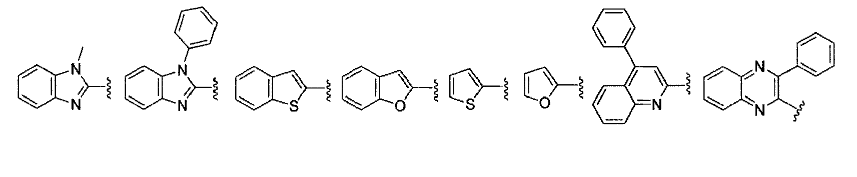

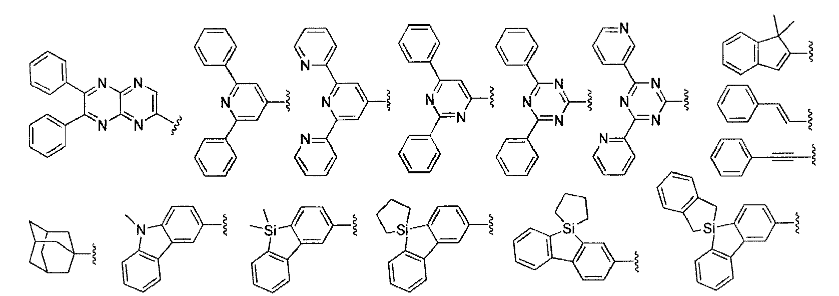

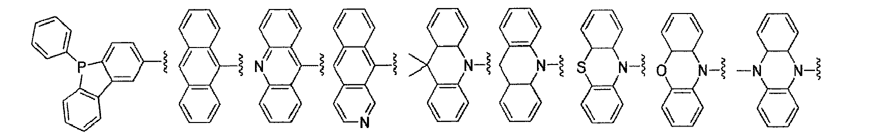

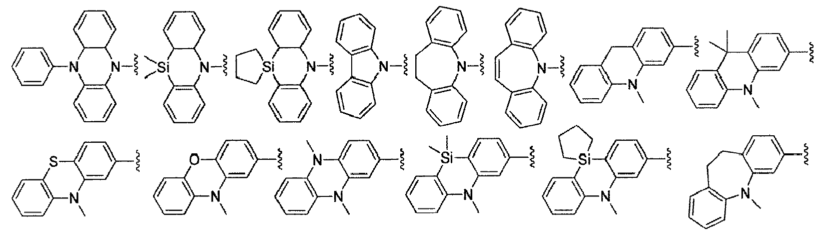

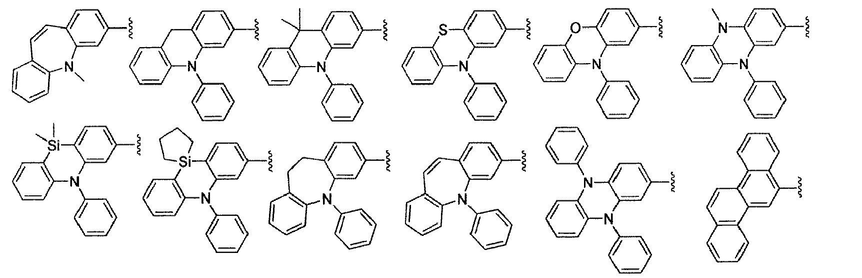

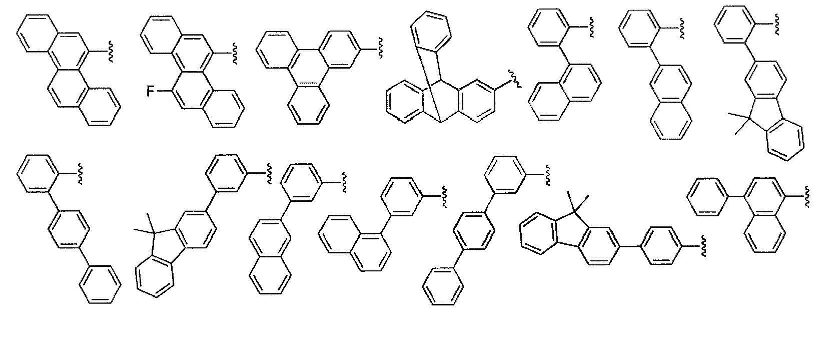

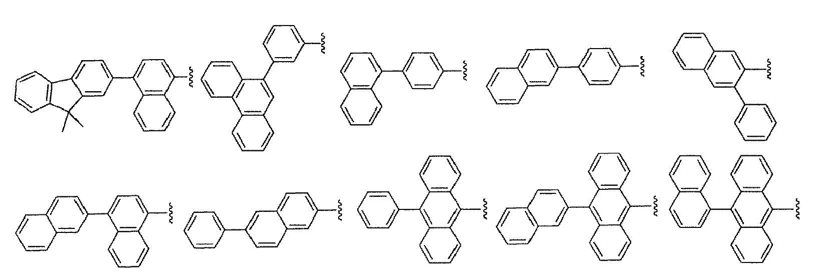

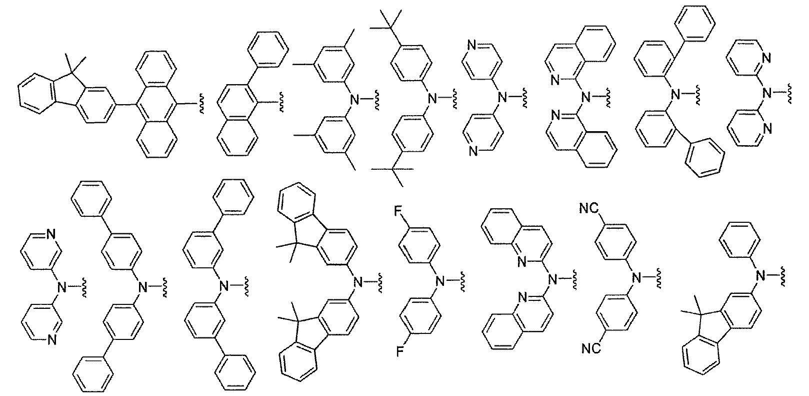

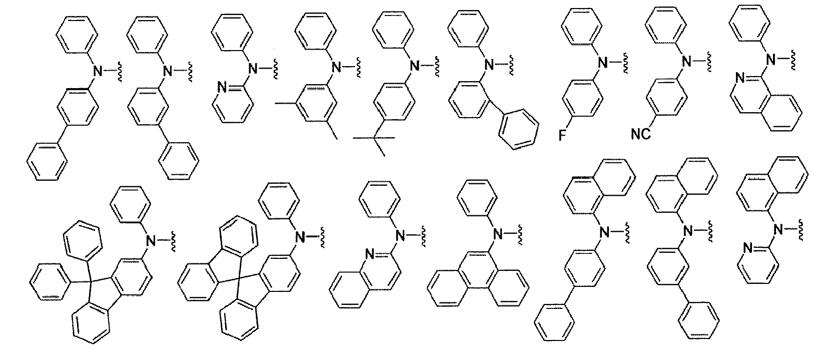

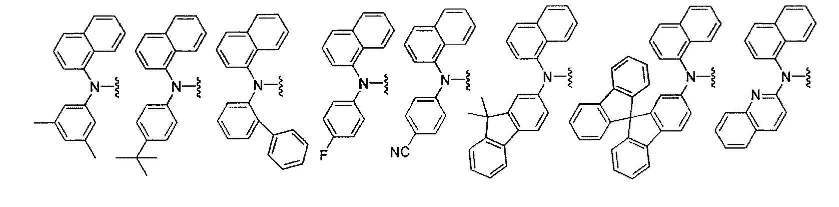

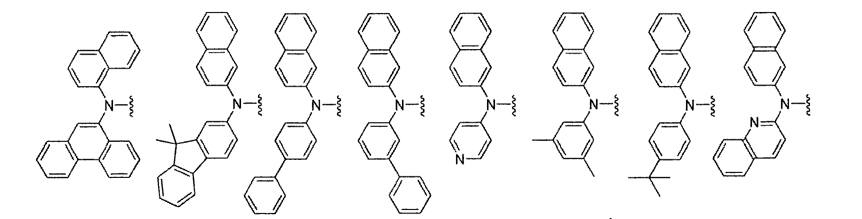

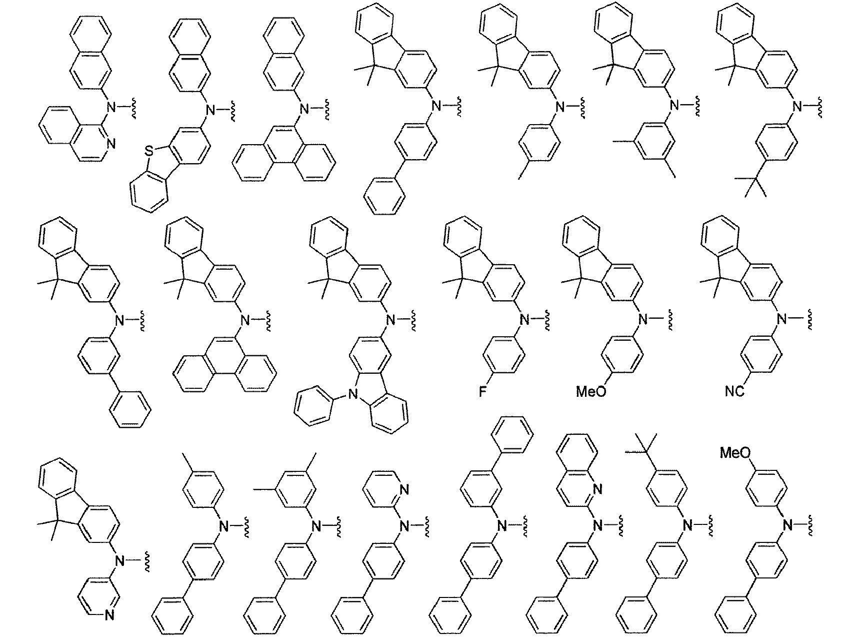

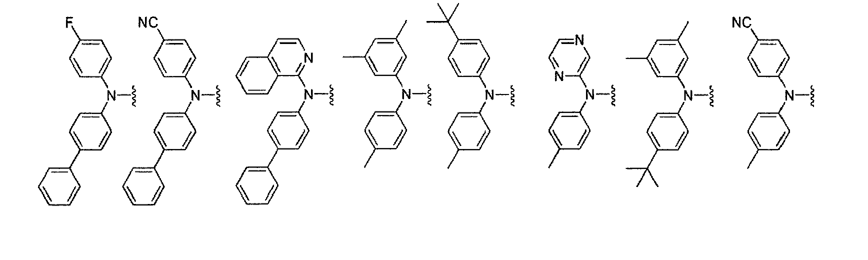

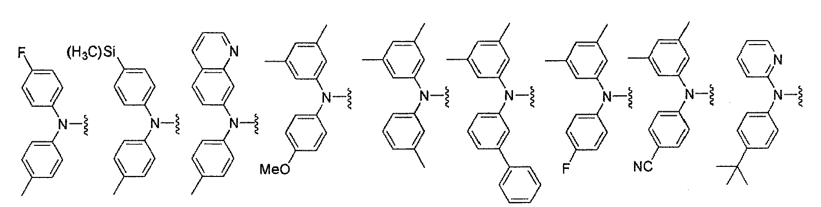

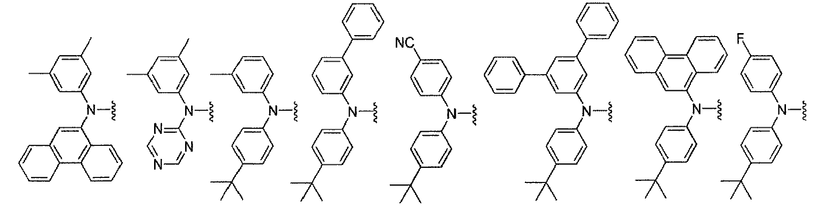

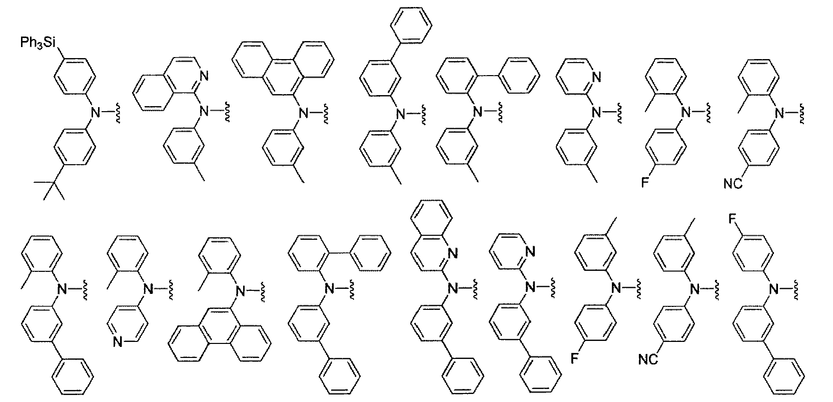

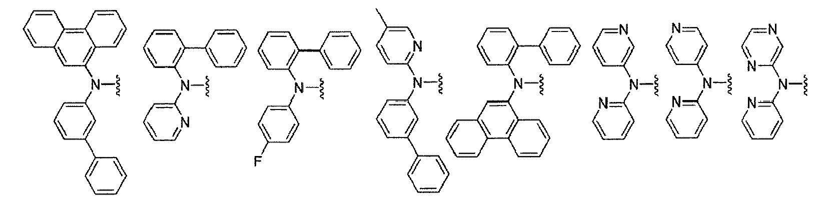

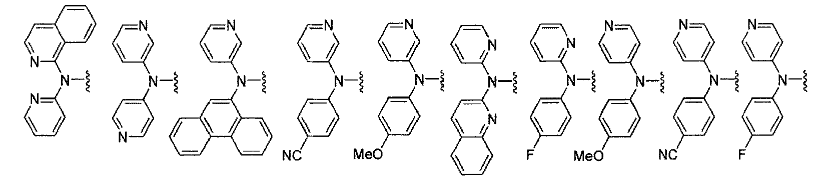

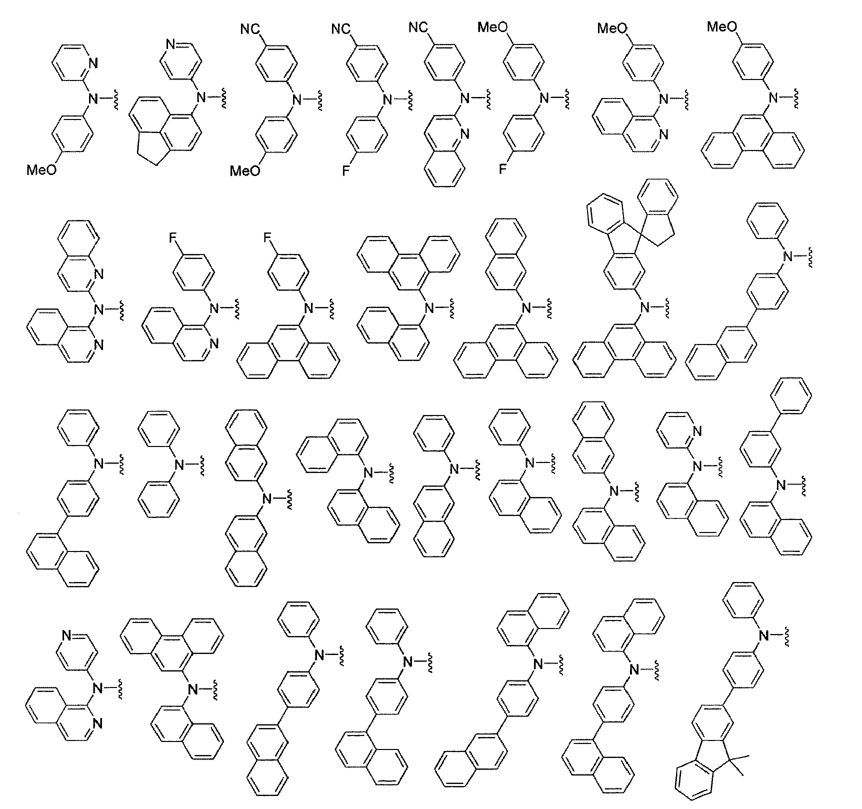

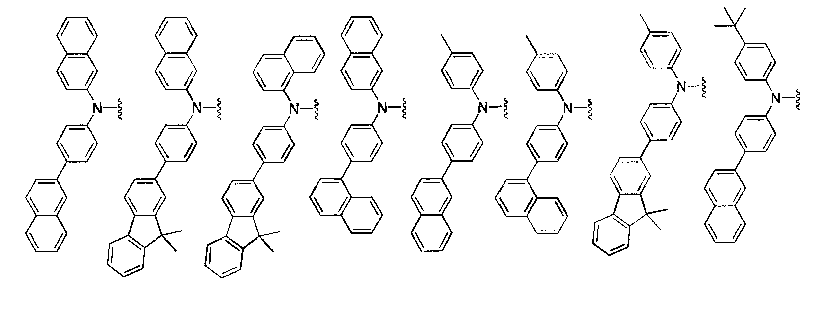

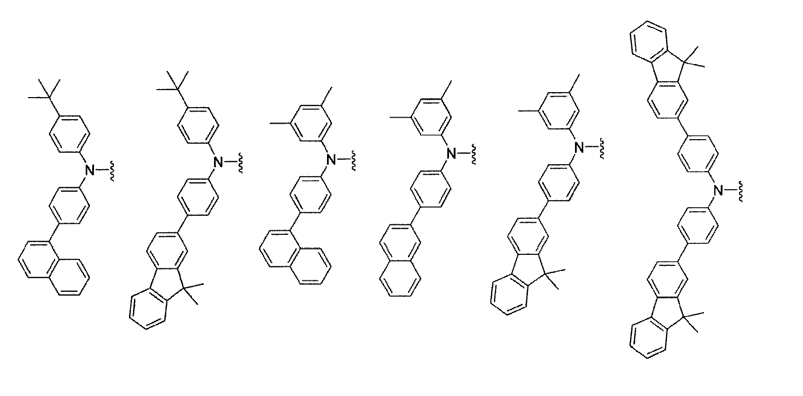

In the chemical formulas, L₁, L₂ and L₃ independently represent a chemical bond, or arylene or heteroarylene selected from the phenylene, naphthylene, biphenylene, fluorenylene, phenanthrylene, anthrylene, fluoranthenylene, triphenylenylene, pyrenylene, chrysenylene, naphthacenylene, perylenylene, spirobifluorenyl, tetrahydronaphthylene, acenaphthenylene, indenylene, pyridylene, bipyridylene, pyrrolylene, furylene, thienylene, imidazolylene, benzimidazolylene, pyrazinylene, pyrimidinylene, pyridazinylene, quinolinylene, triazinylene, benzofurylene, dibenzofurylene, benzothienylene, dibenzothienylene, pyrazolylene, indolylene, carbazolylene, indenocarbazolylene, thiazolylene, oxazolylene, benzothiazolylene, benzoxazolylene, phenanthridinylene, phenanthrolinylene, piperidinylene, quinazolinylene, 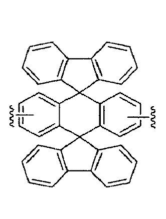 or 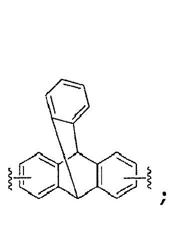 and the arylene or heteroarylene of L₁, L₂ and L₃ may be further substituted by one or more substituent(s) selected from a group consisting of deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, cyano, carbazolyl, (C6-C60)ar(C1-C60)alkyl, (C1-C60)alkyloxy, (C1-C60)alkylthio, (C6-C60)aryloxy, (C6-C60)arylthio, carboxyl, nitro and hydroxyl.

The organic electroluminescent compounds according to the present invention can be more specifically exemplified by the following compounds, but they are not restricted thereto:

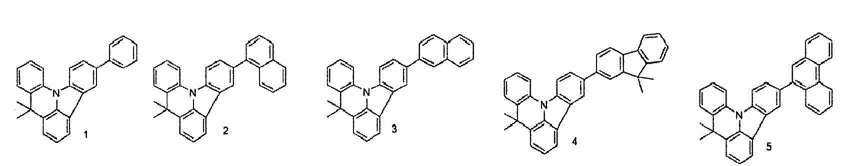

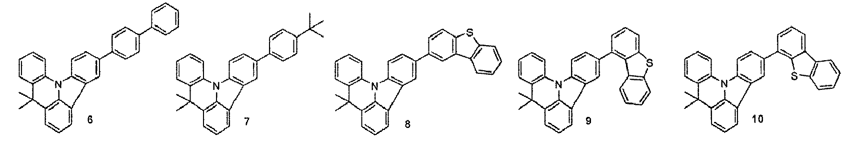

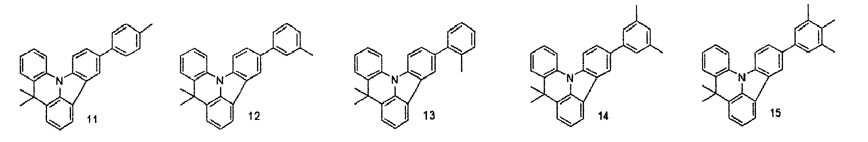

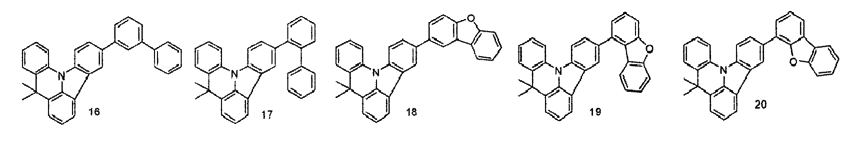

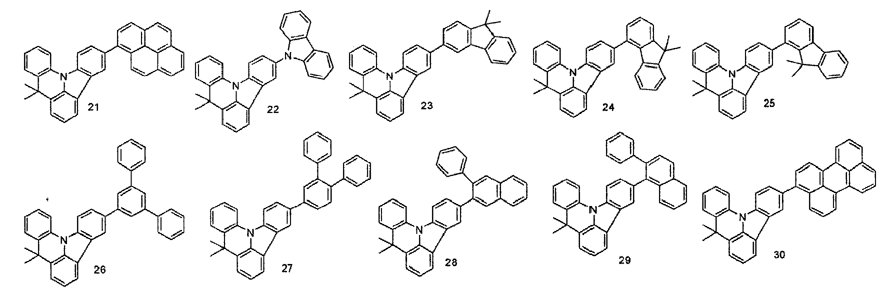

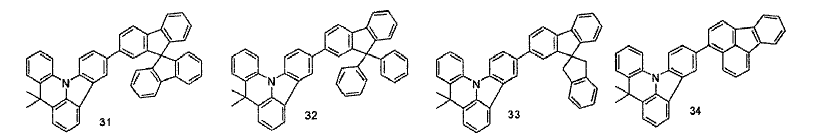

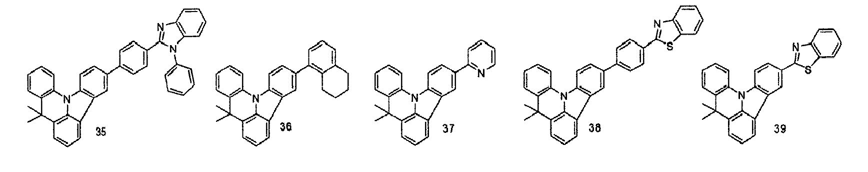

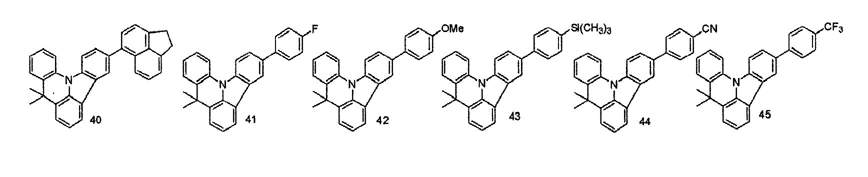

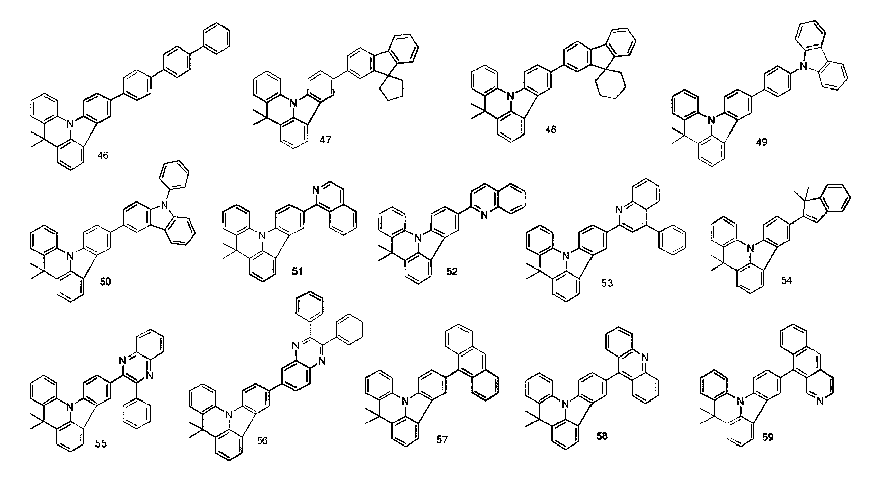

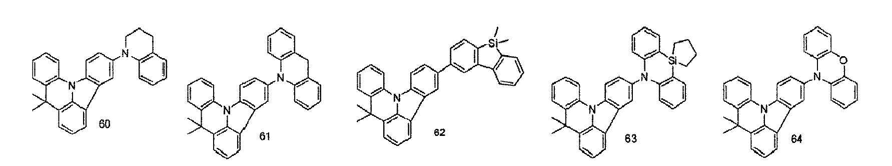

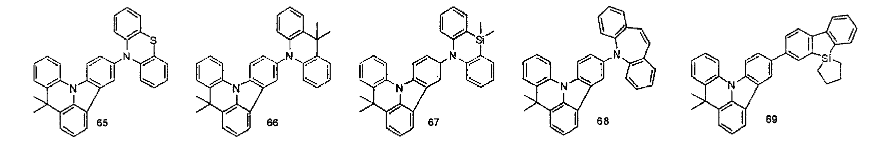

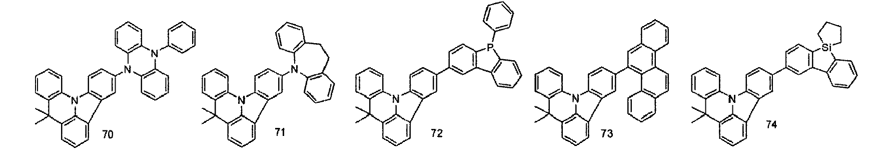

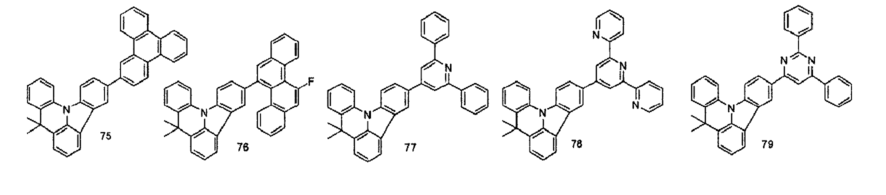

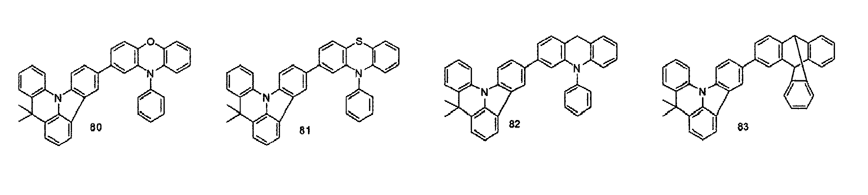

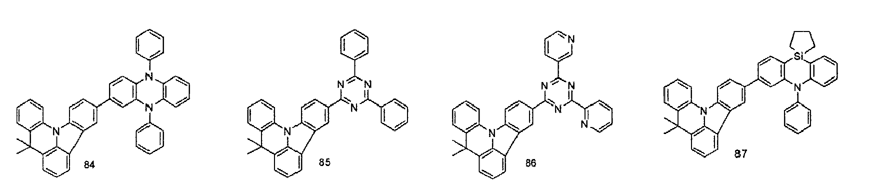

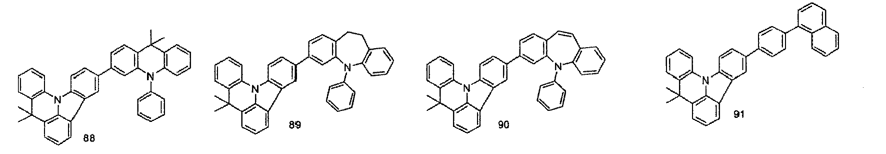

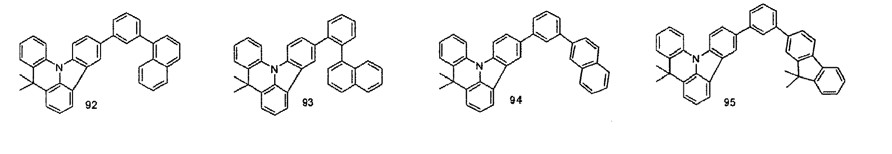

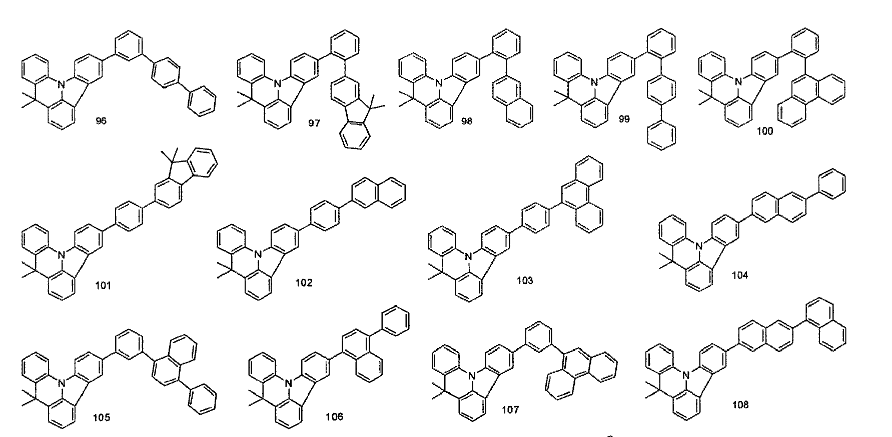

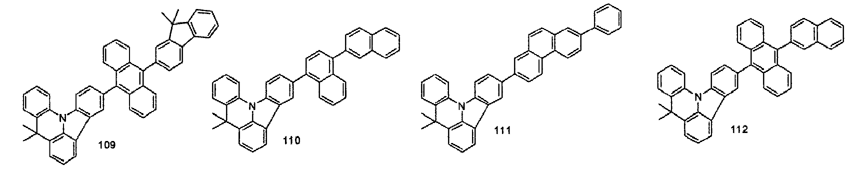

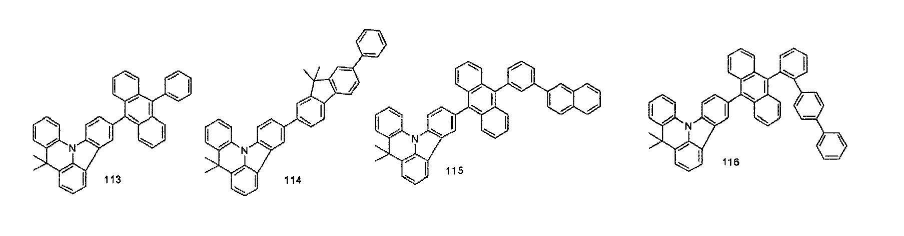

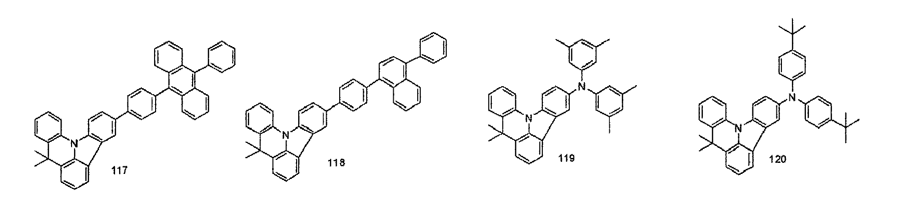

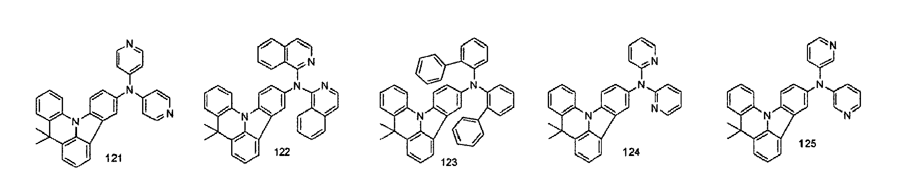

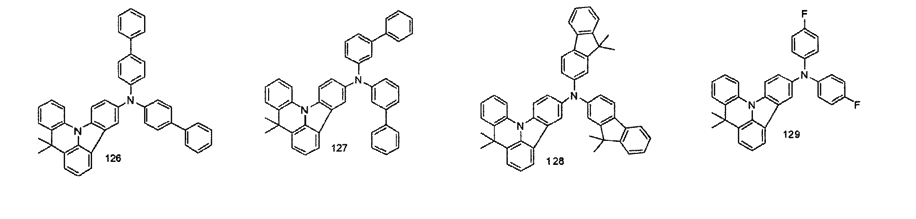

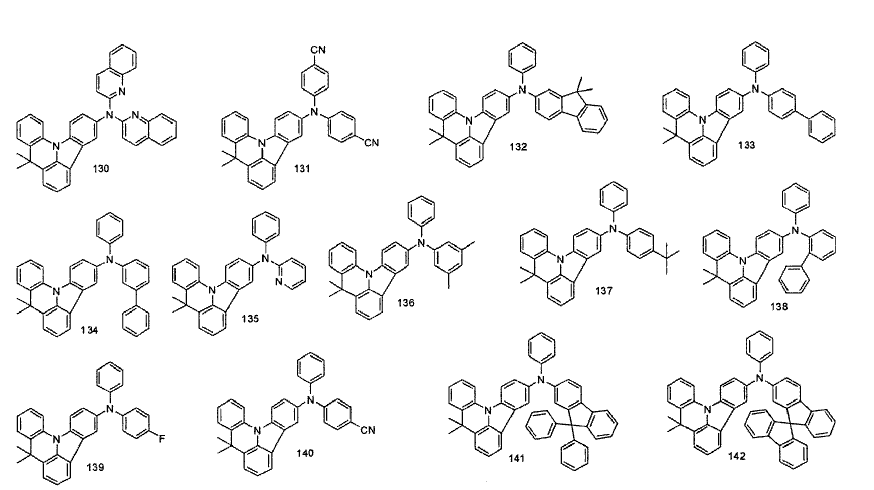

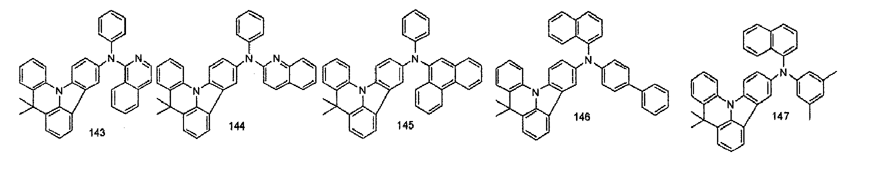

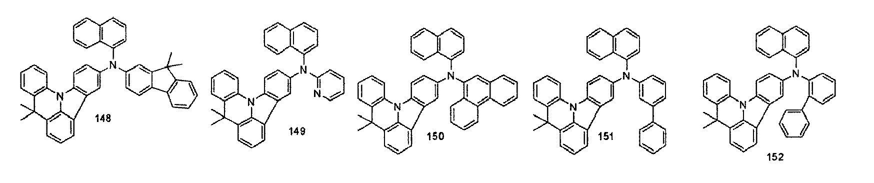

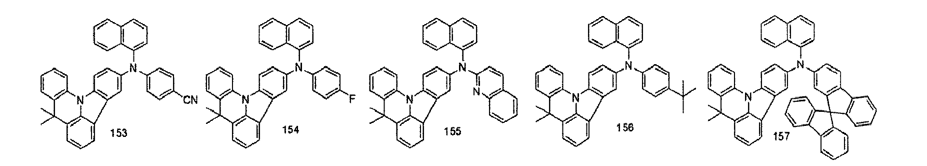

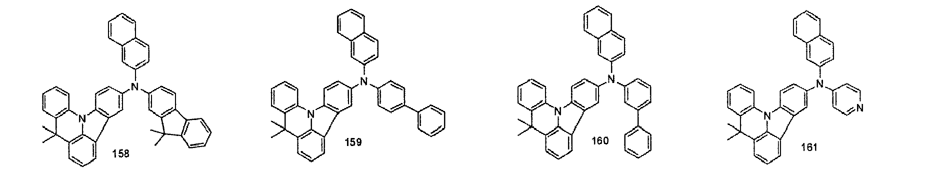

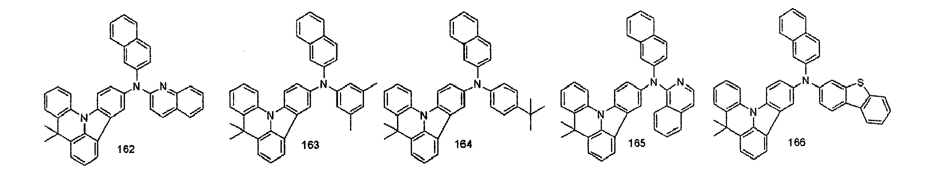

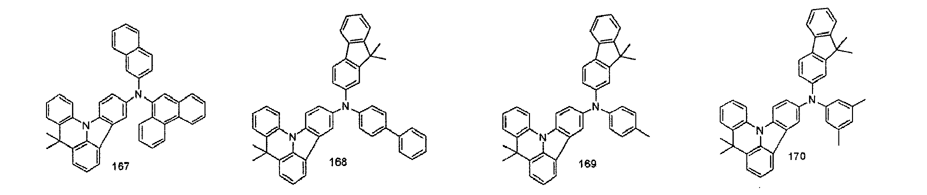

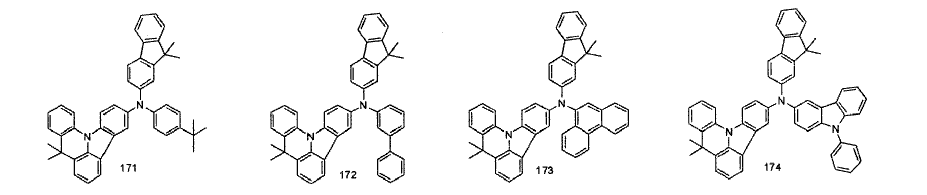

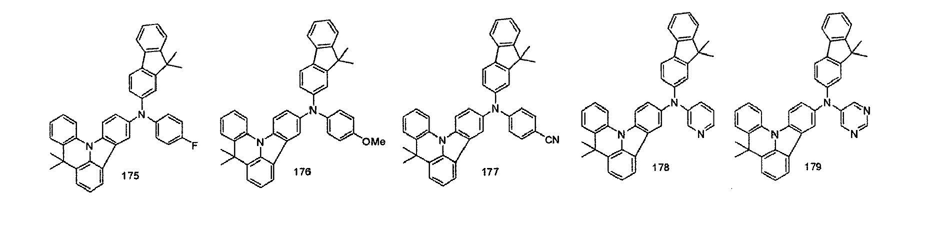

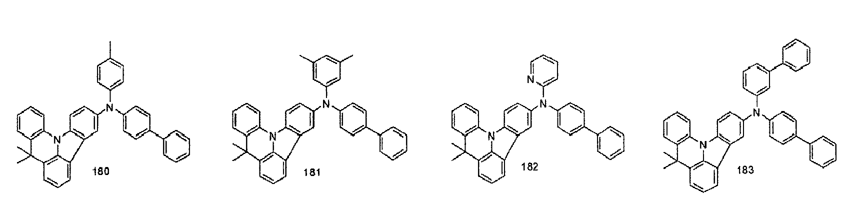

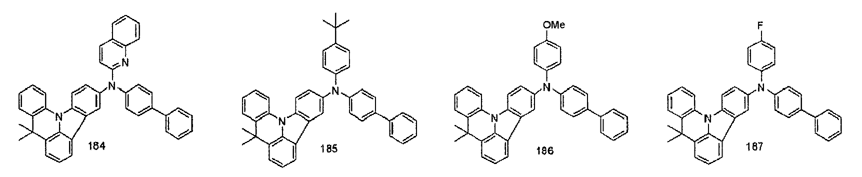

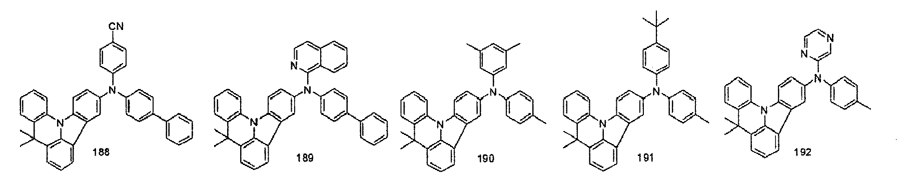

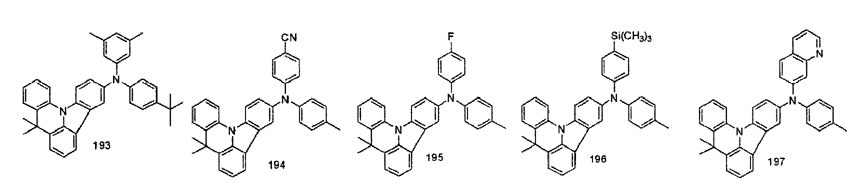

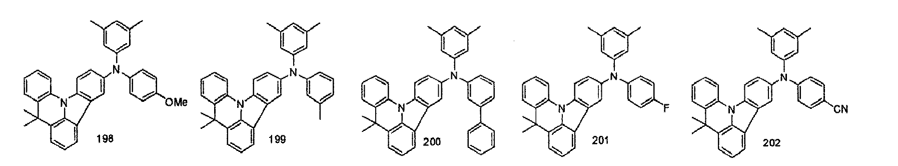

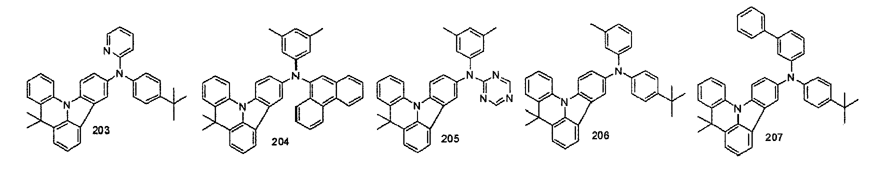

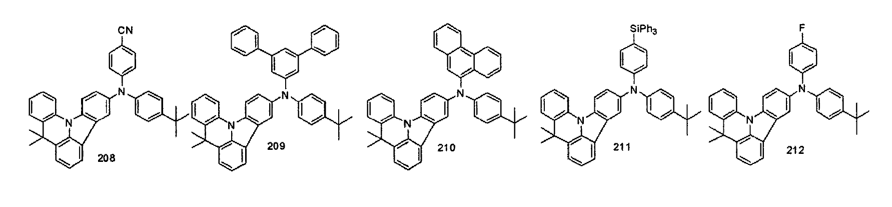

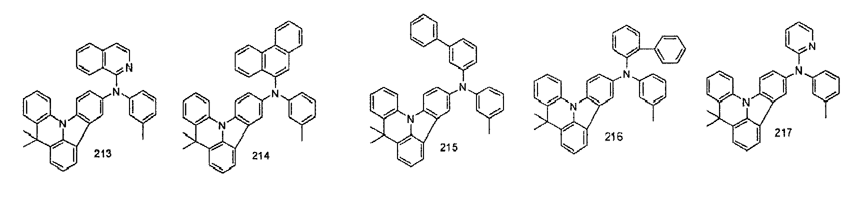

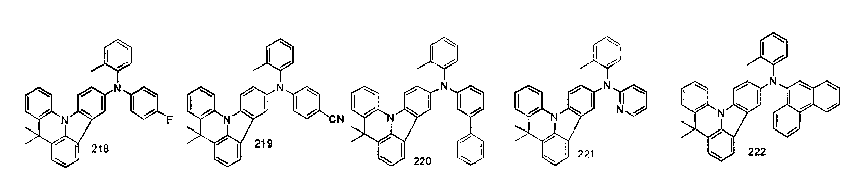

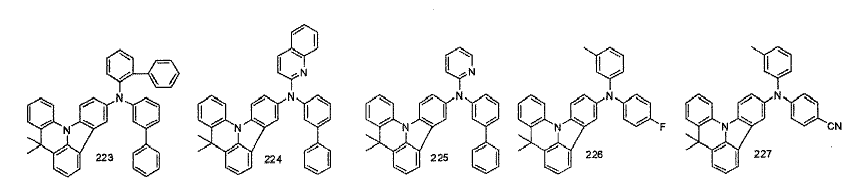

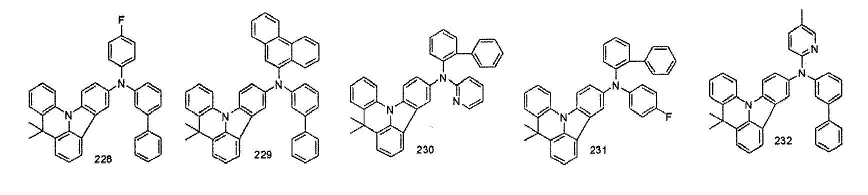

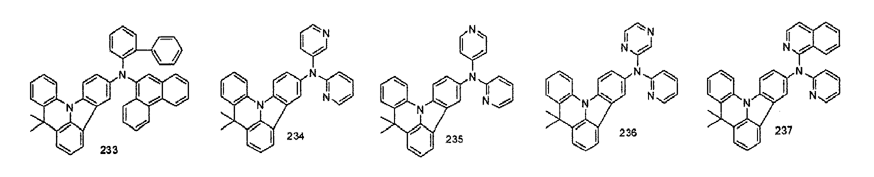

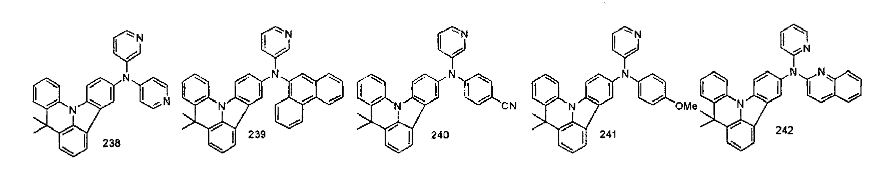

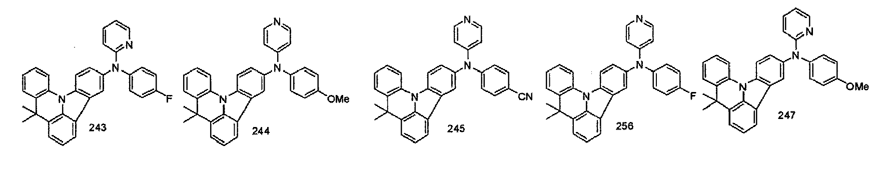

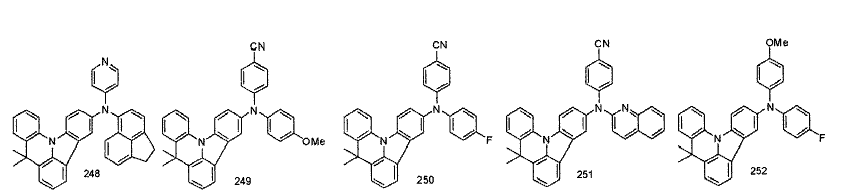

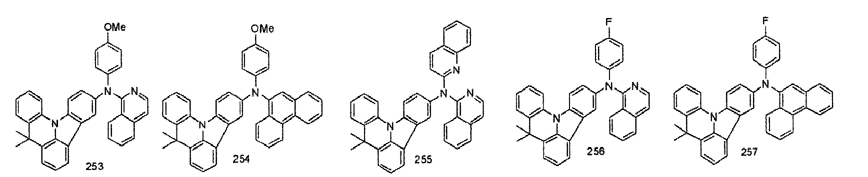

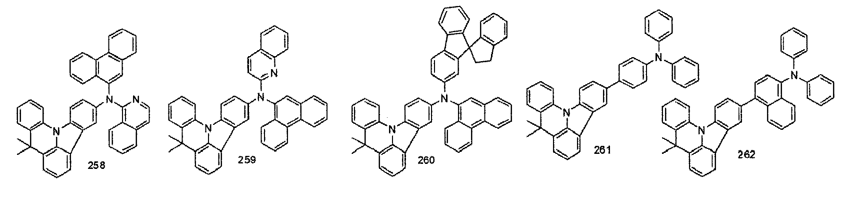

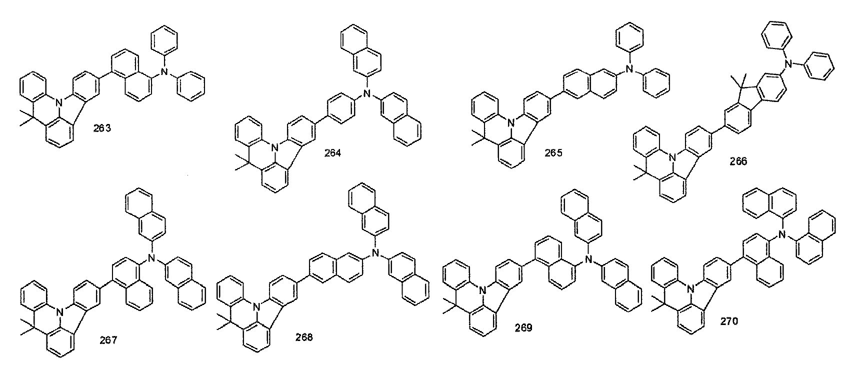

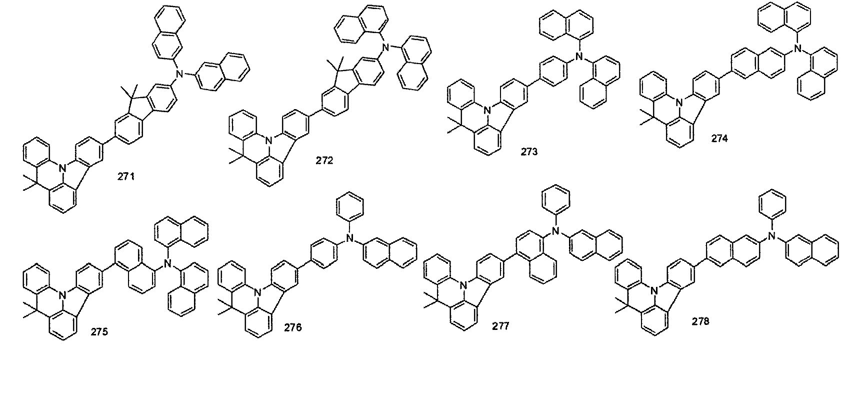

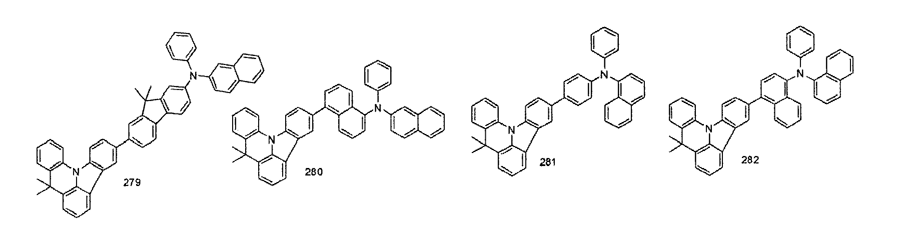

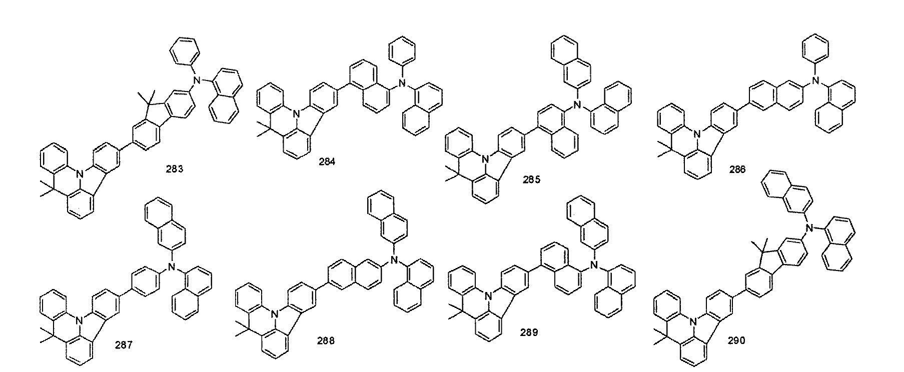

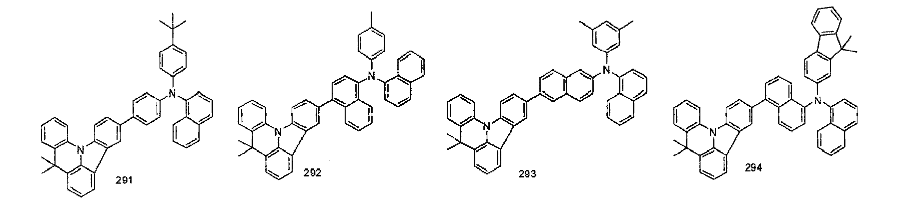

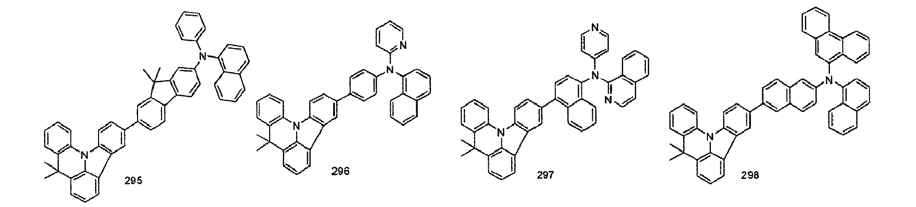

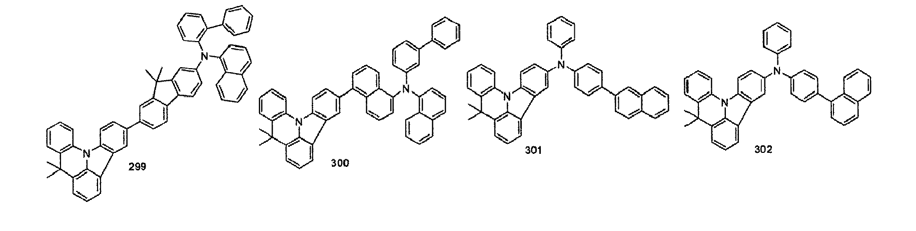

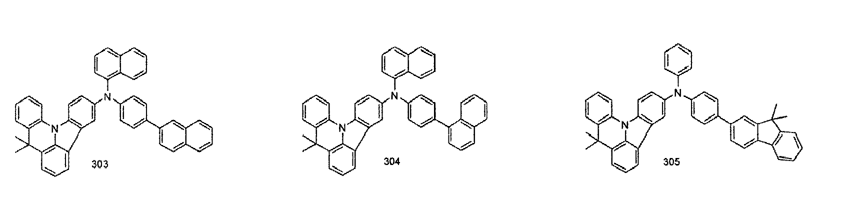

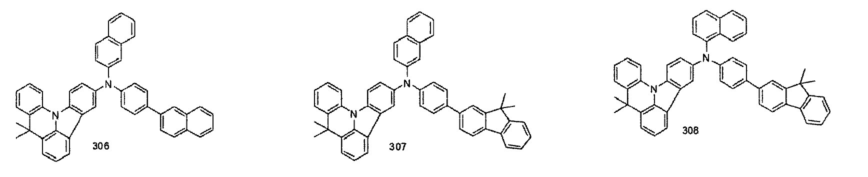

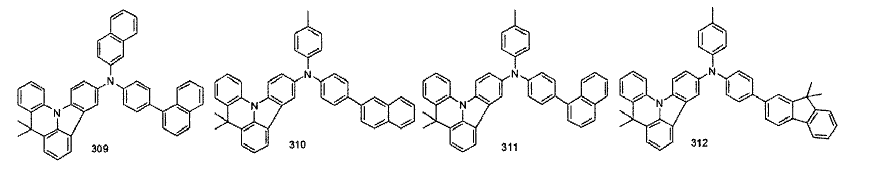

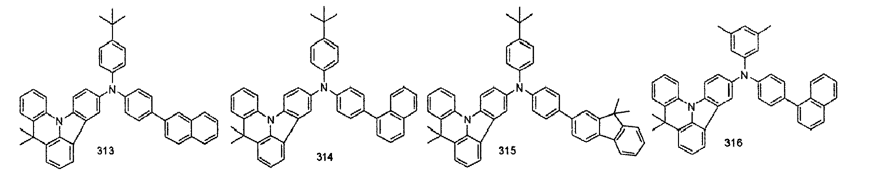

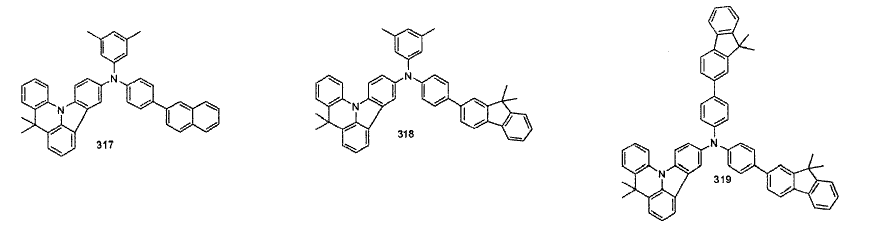

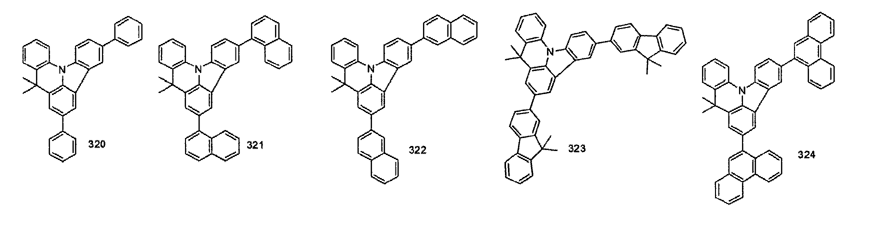

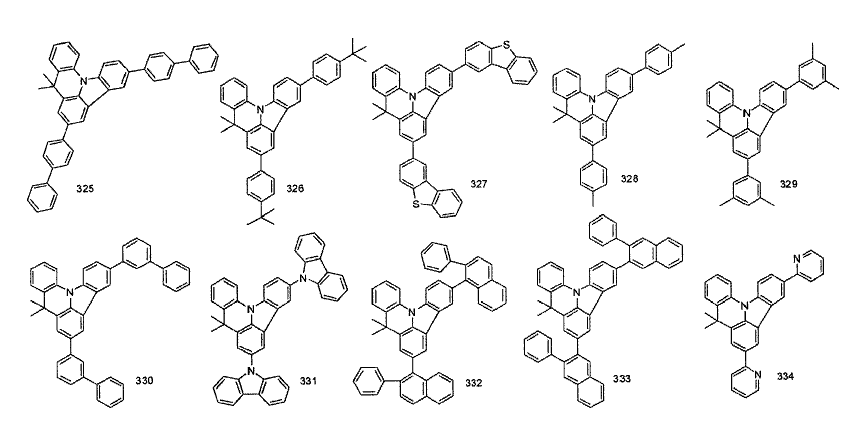

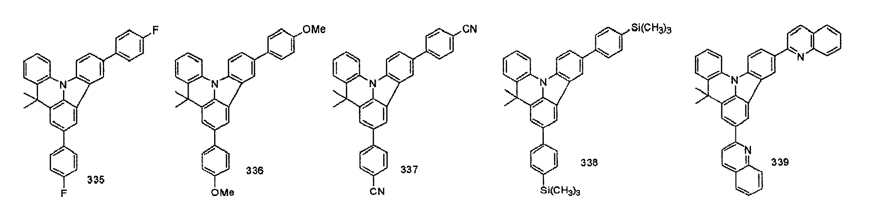

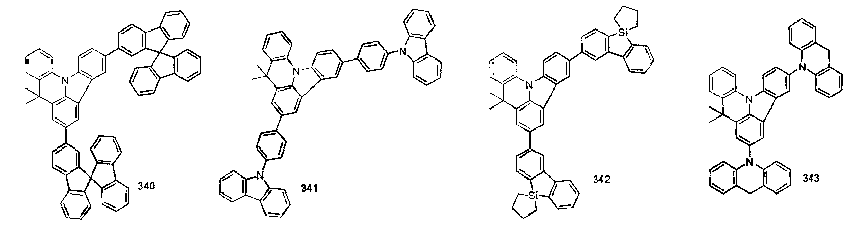

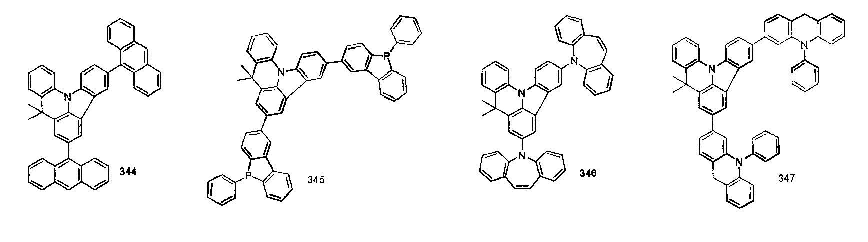

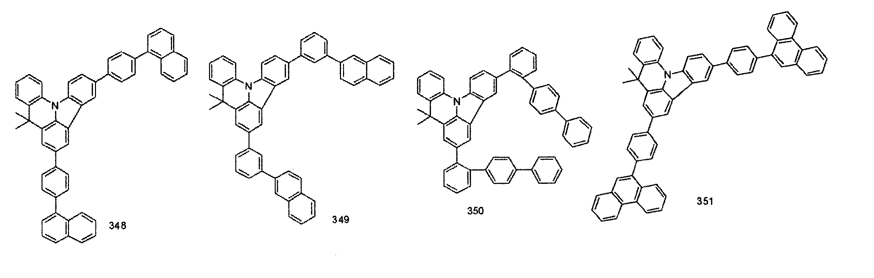

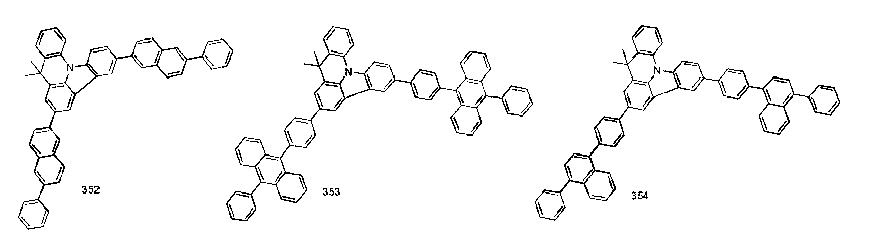

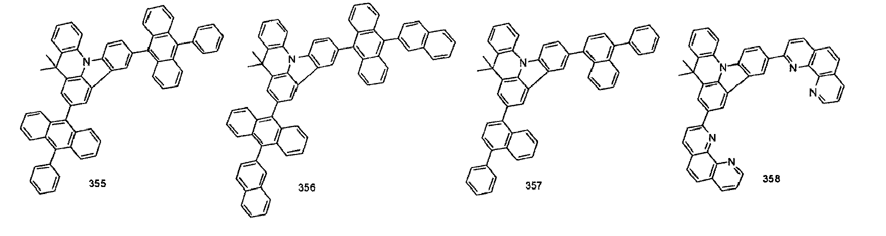

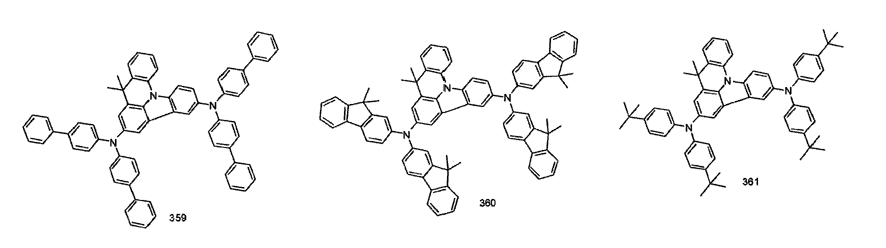

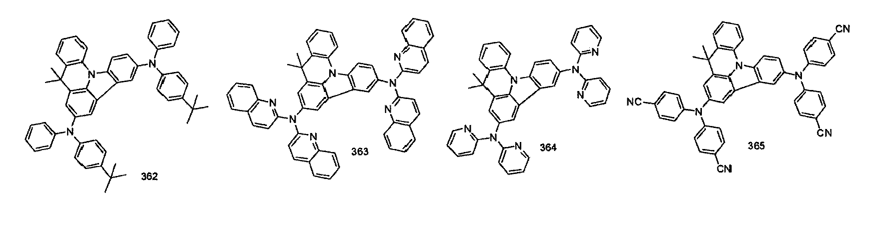

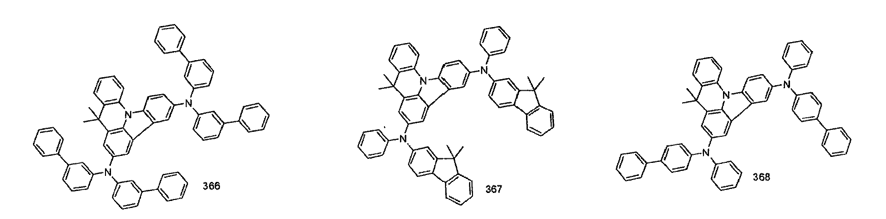

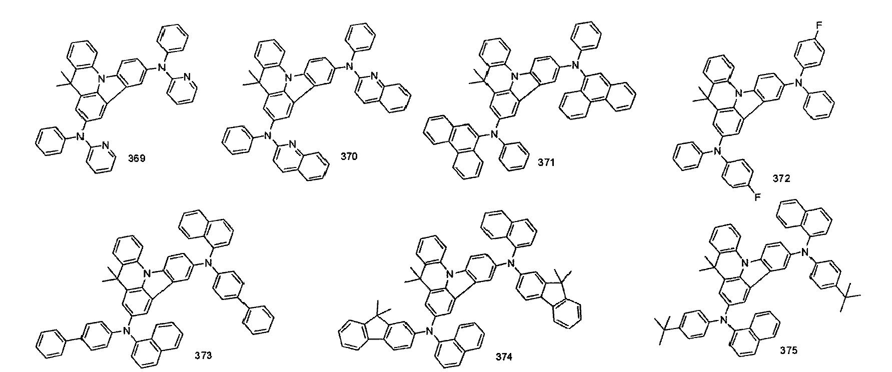

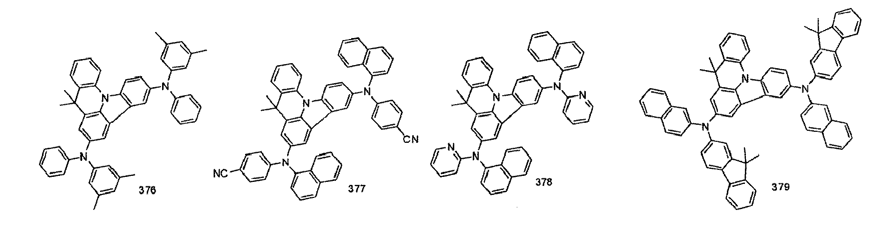

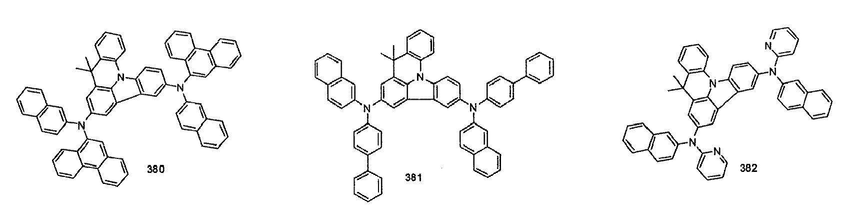

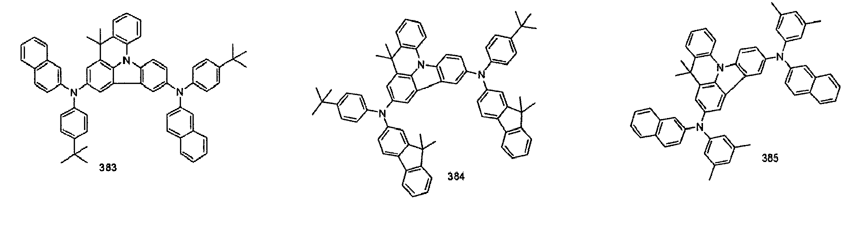

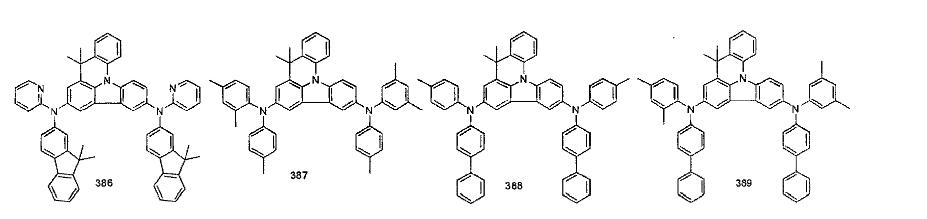

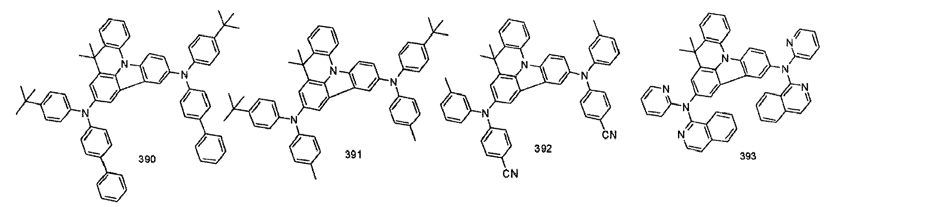

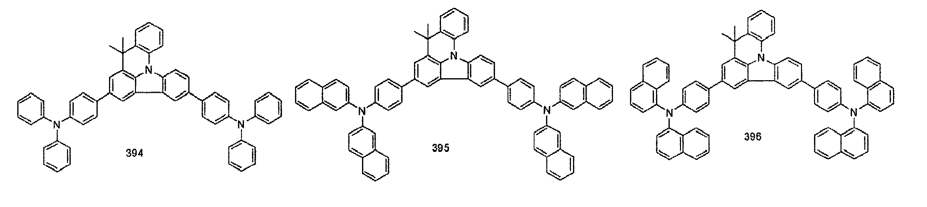

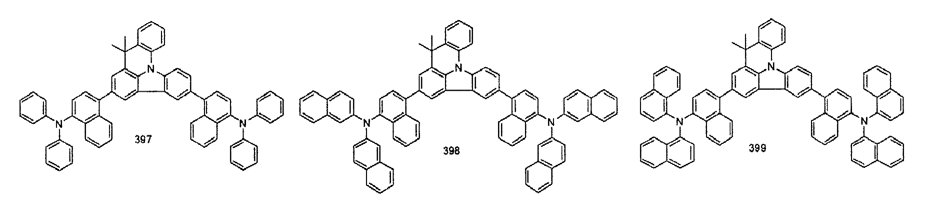

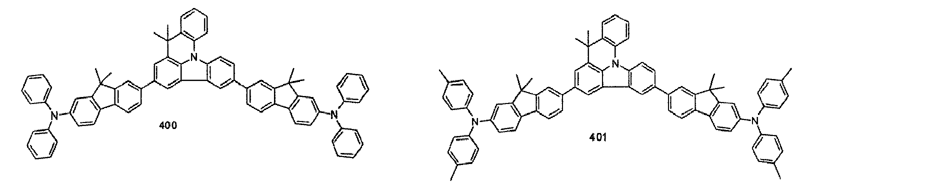

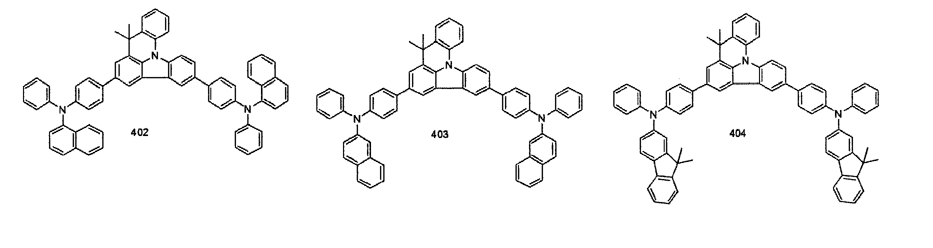

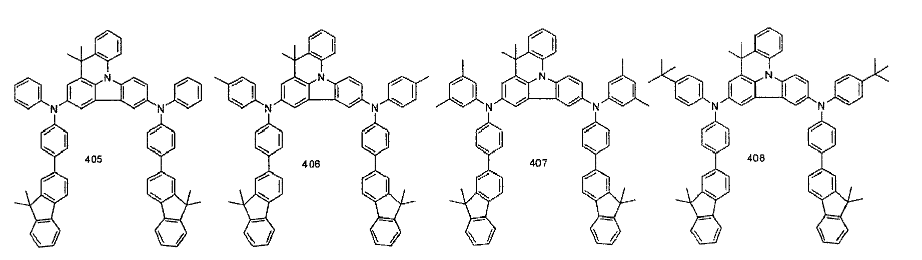

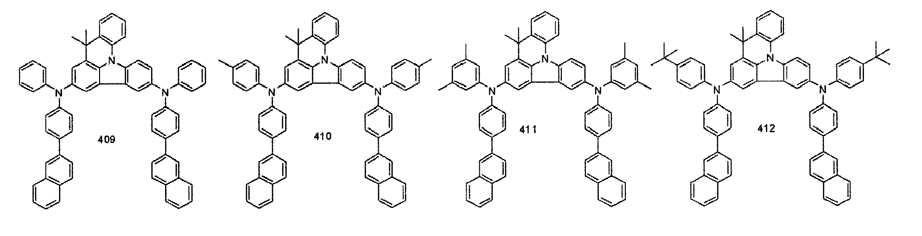

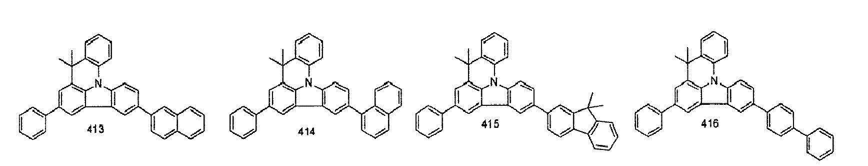

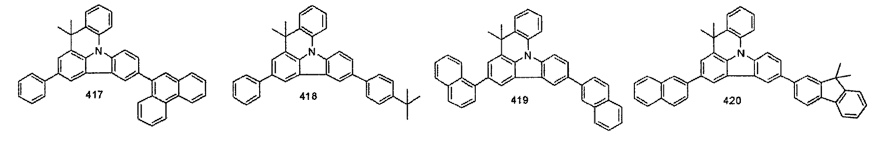

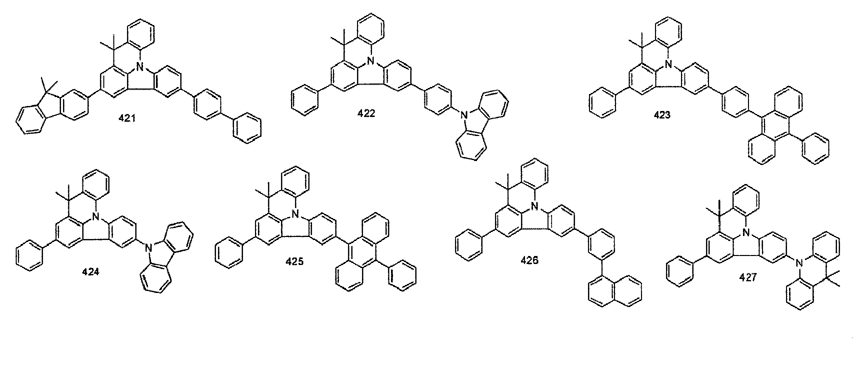

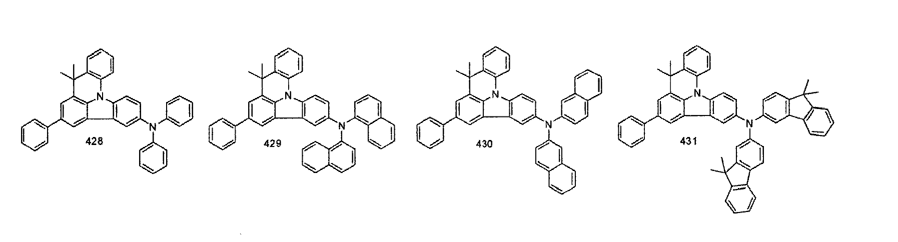

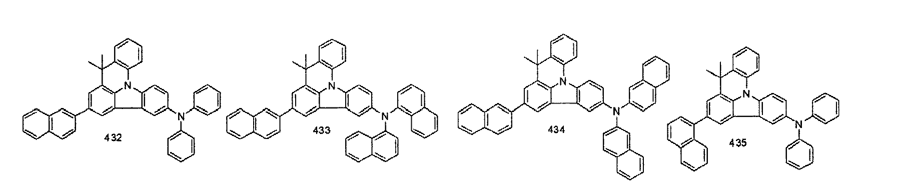

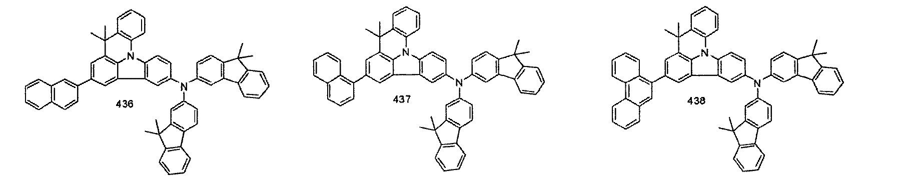

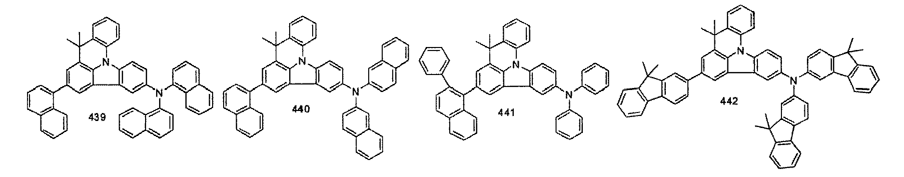

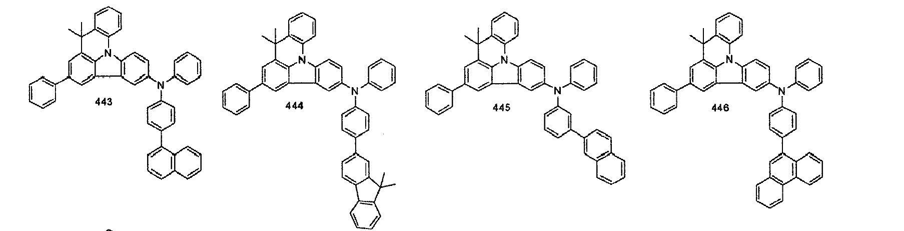

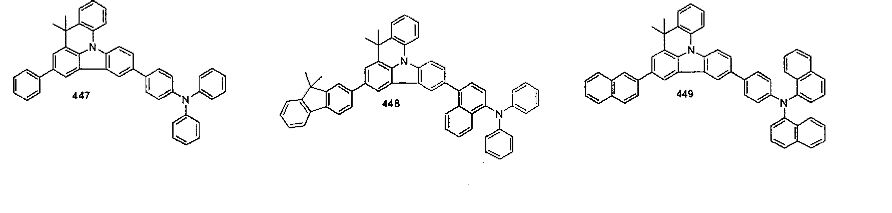

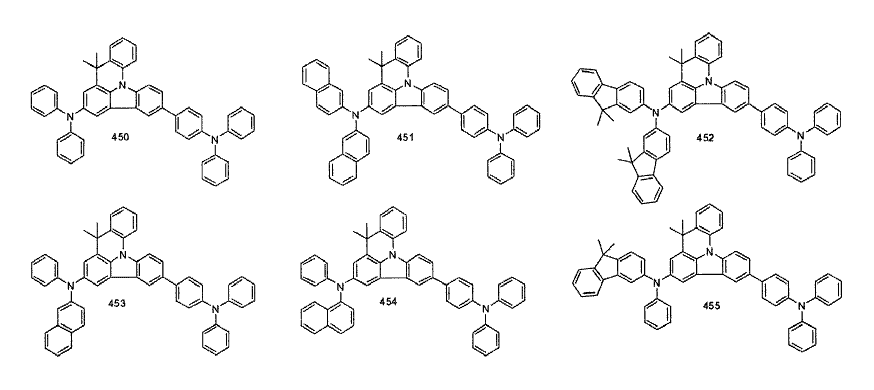

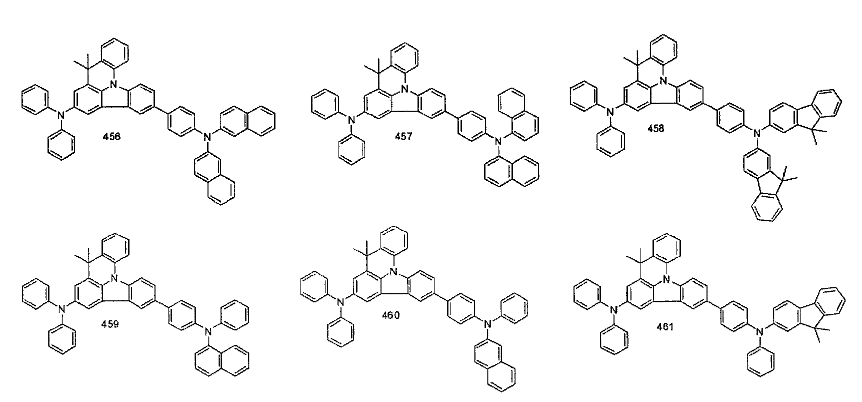

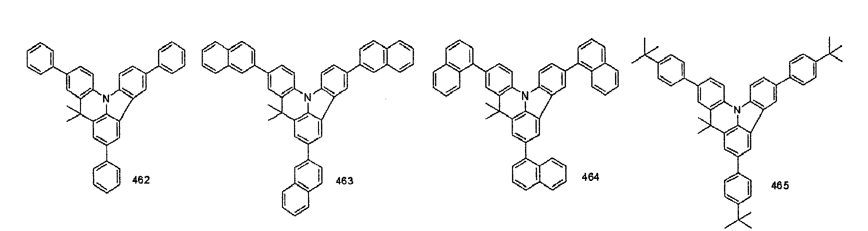

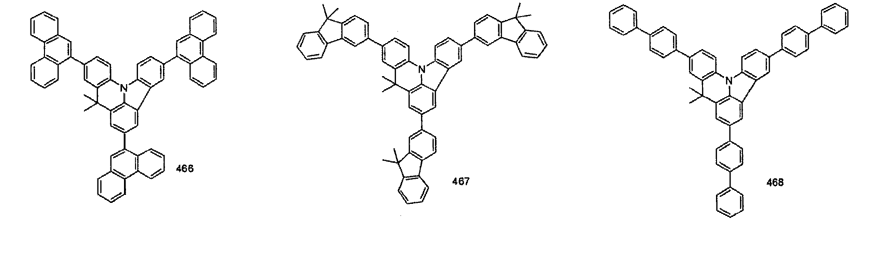

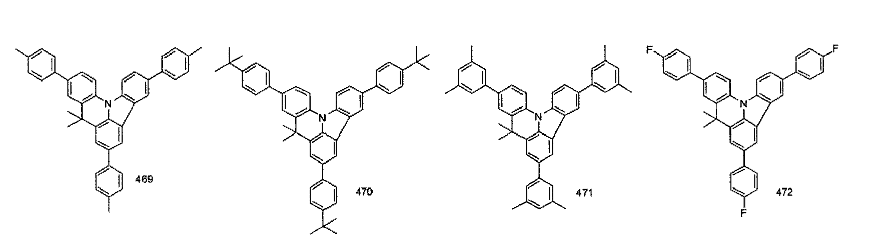

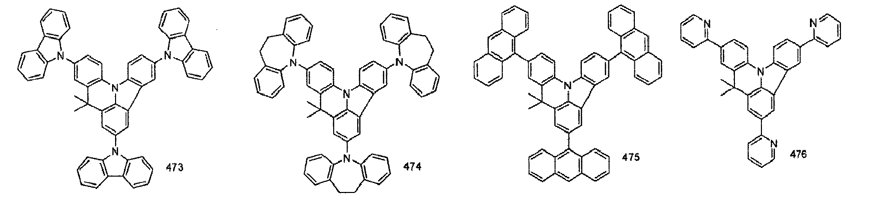

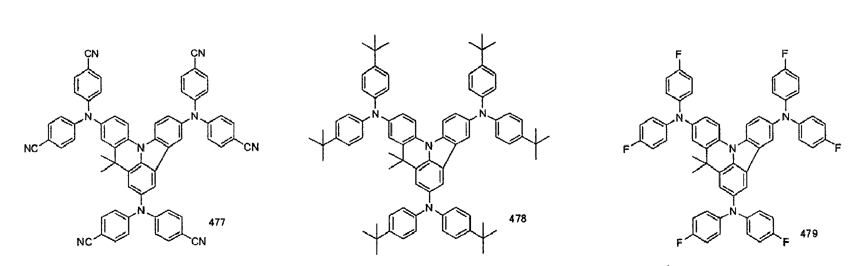

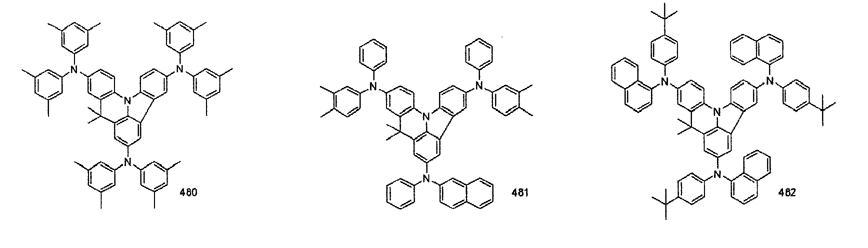

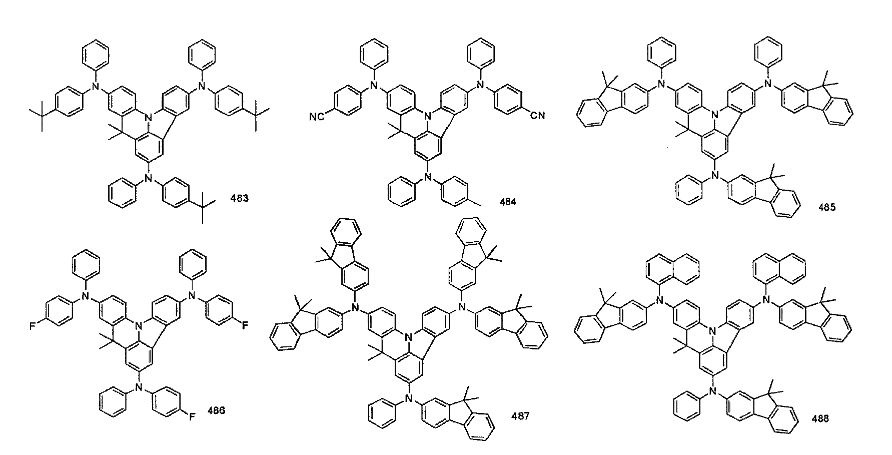

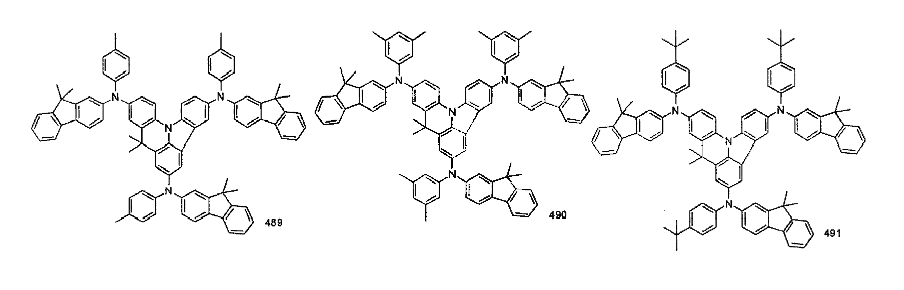

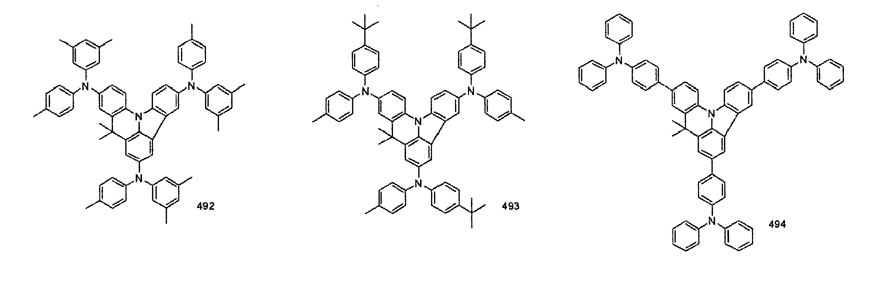

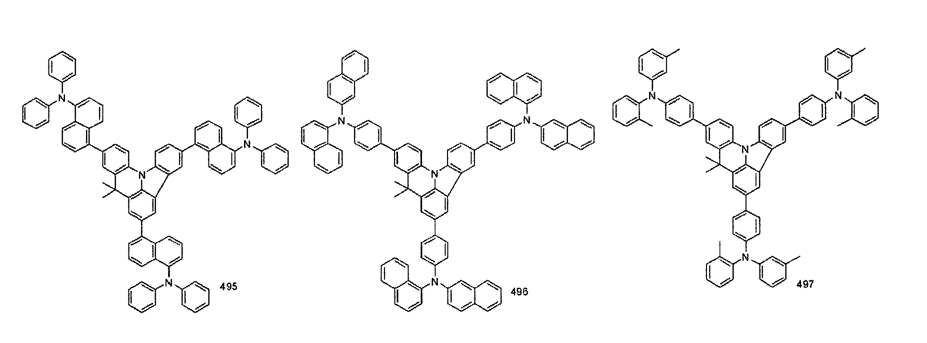

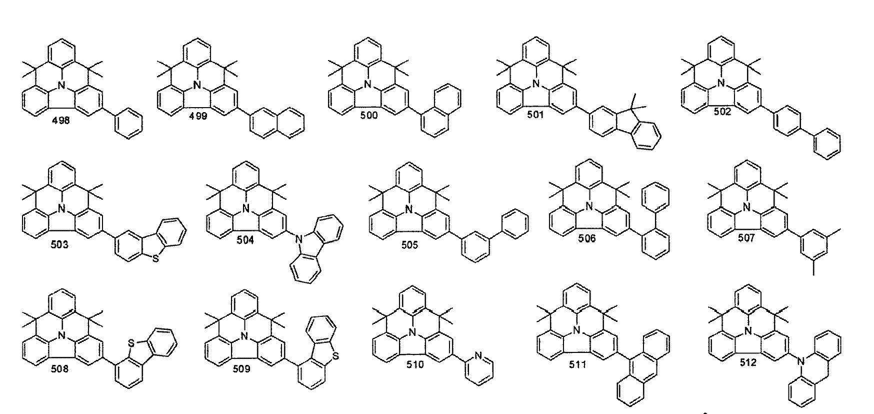

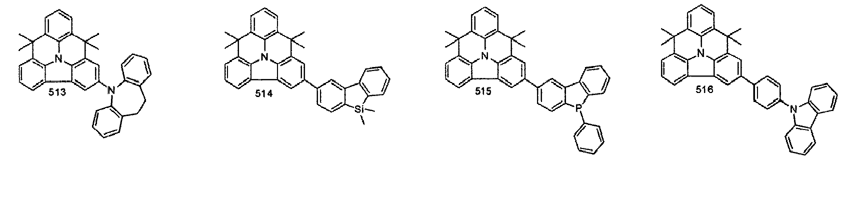

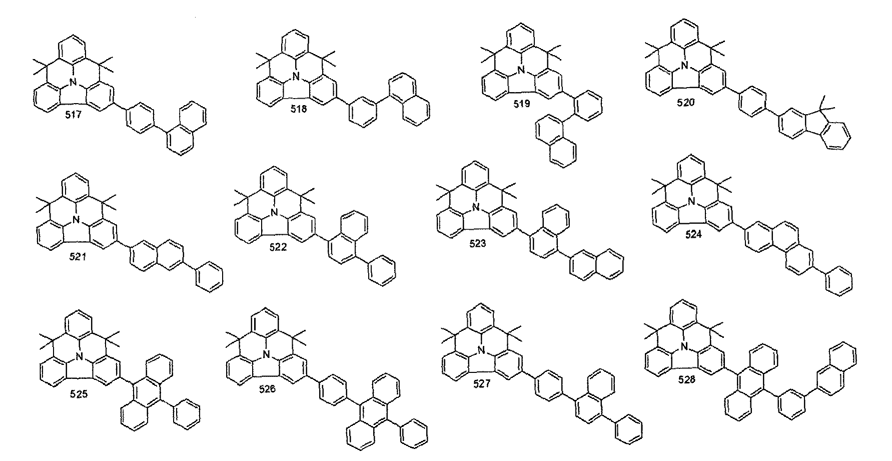

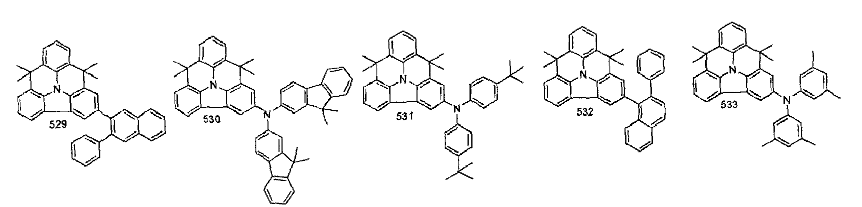

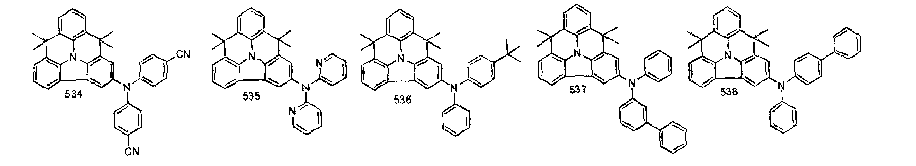

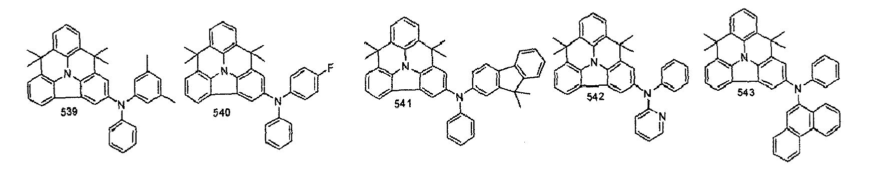

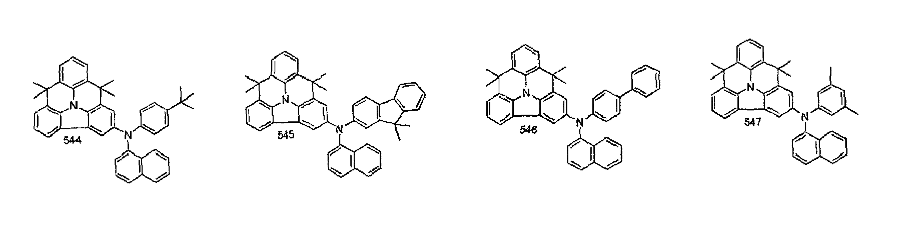

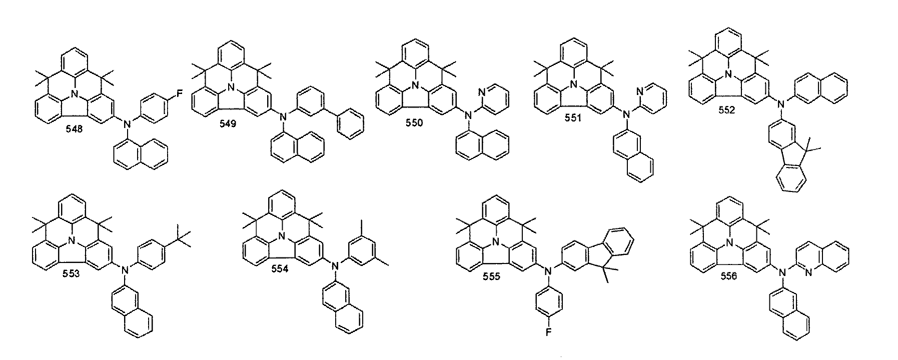

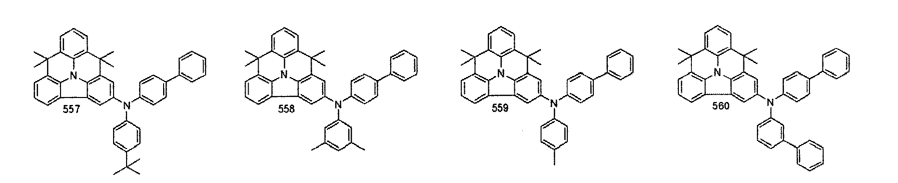

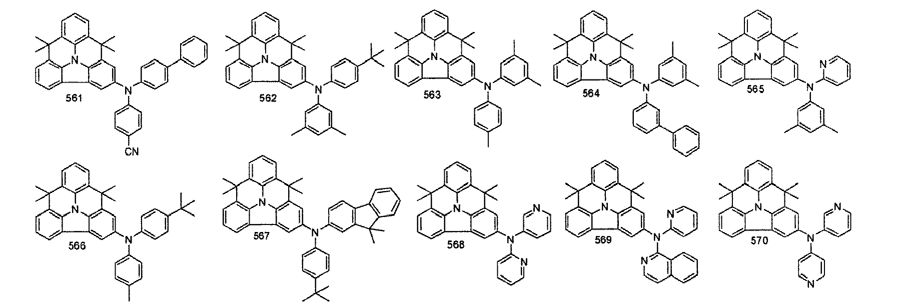

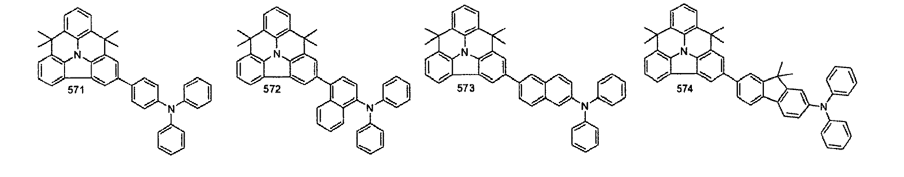

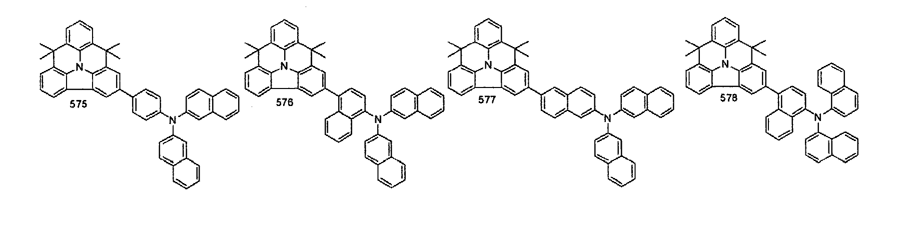

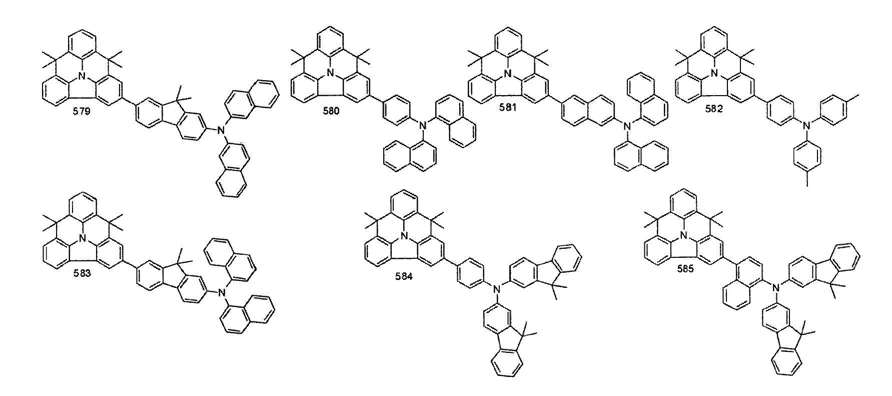

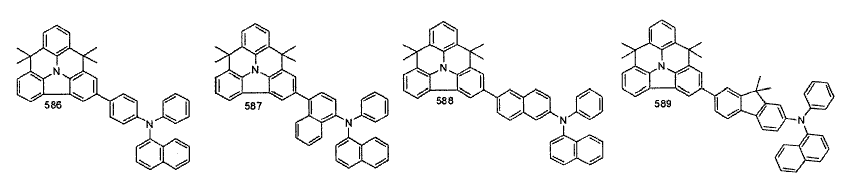

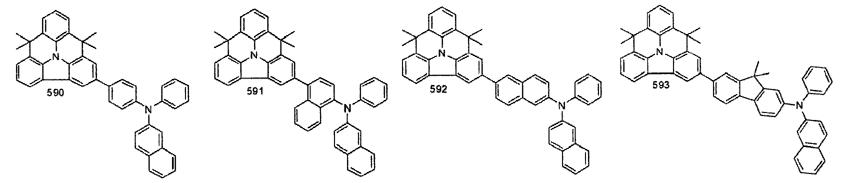

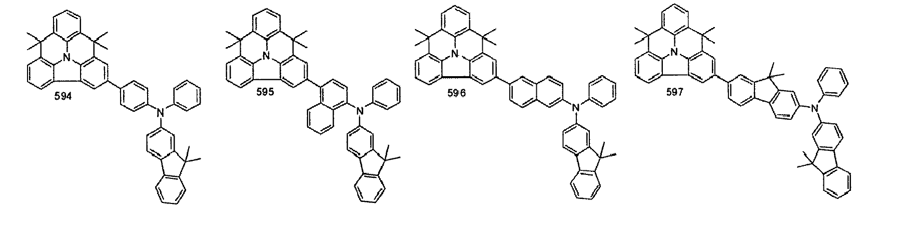

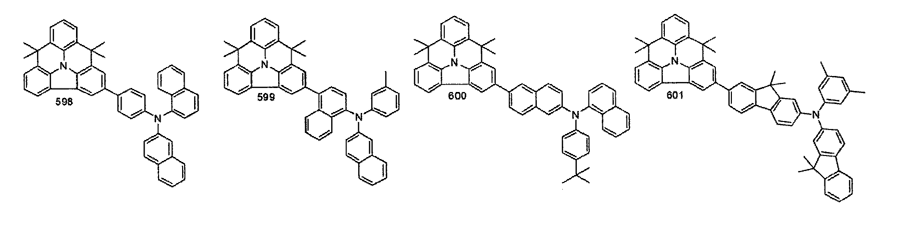

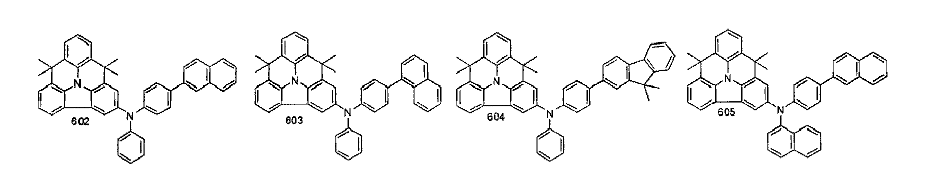

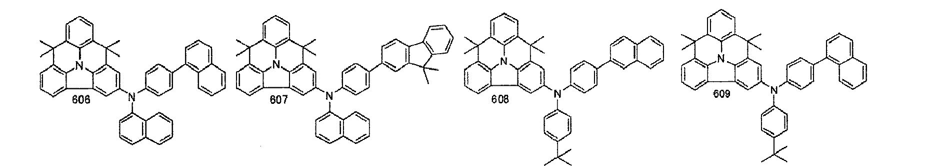

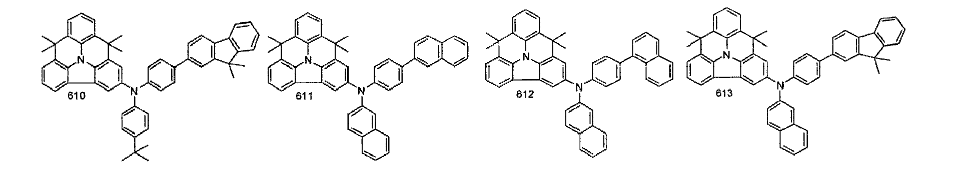

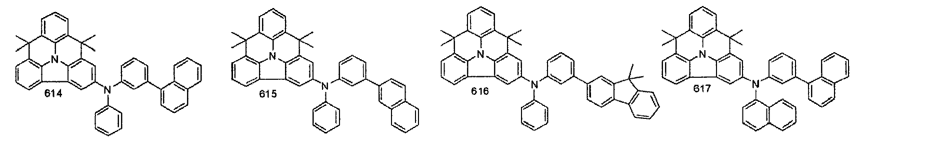

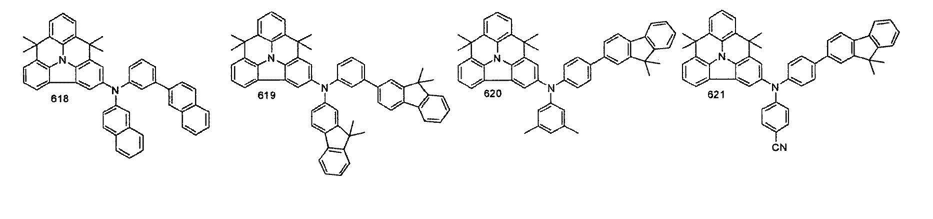

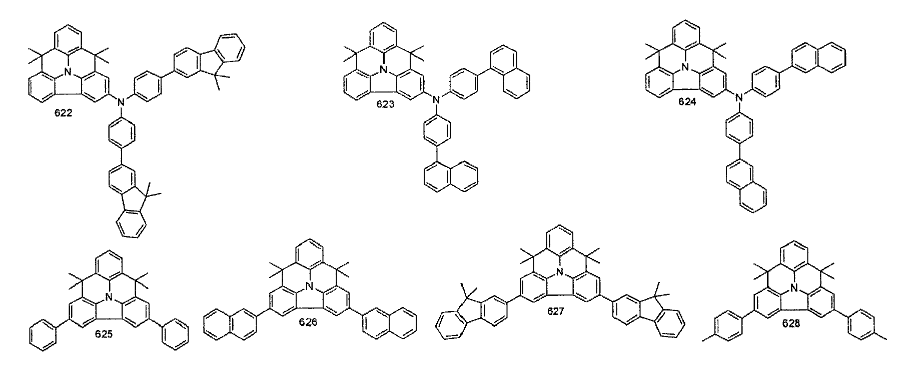

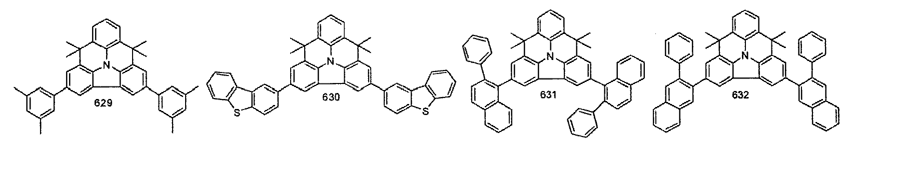

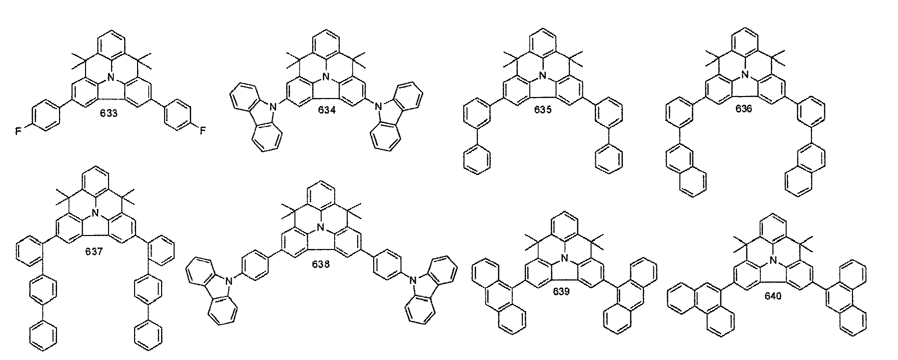

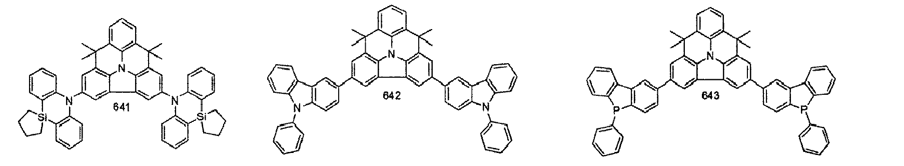

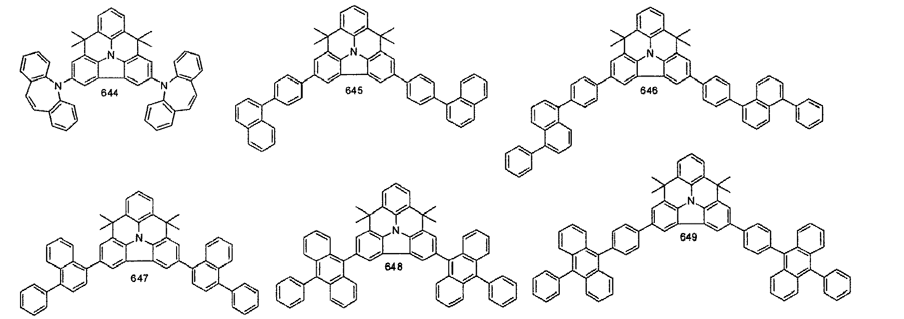

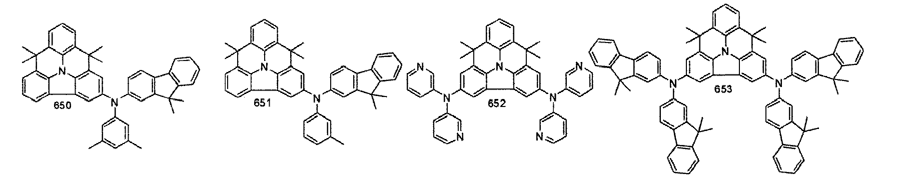

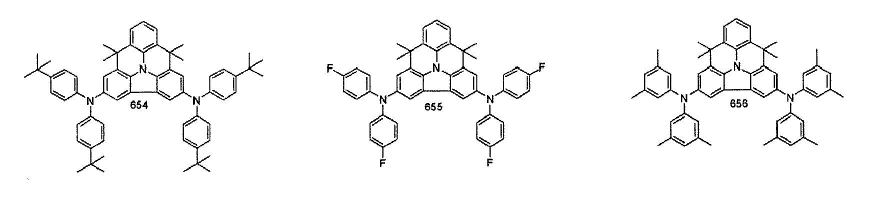

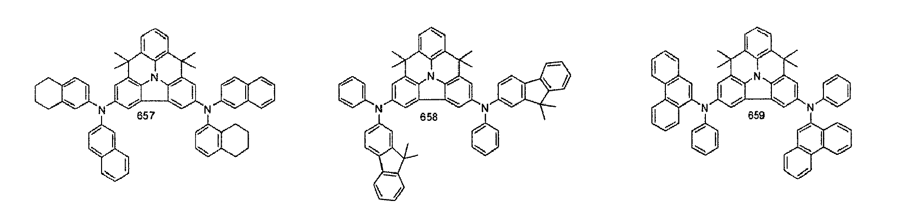

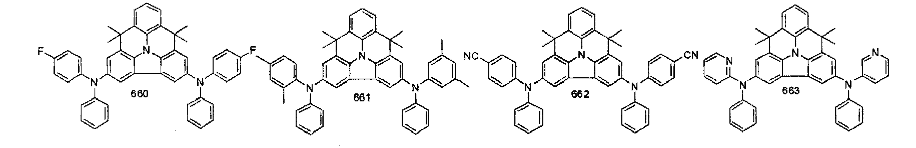

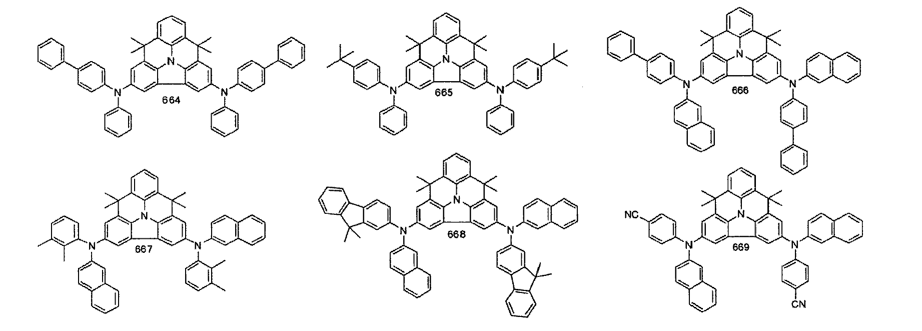

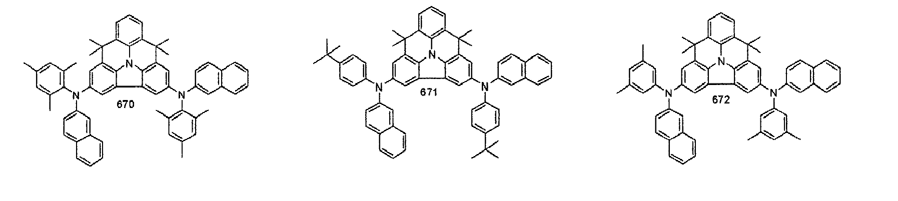

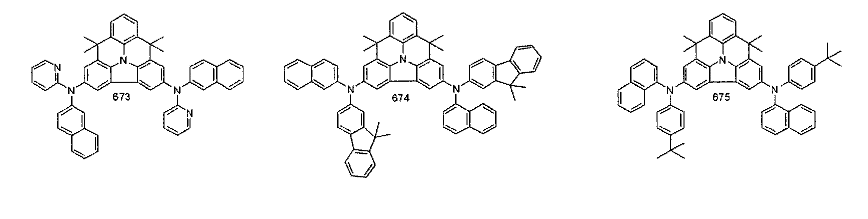

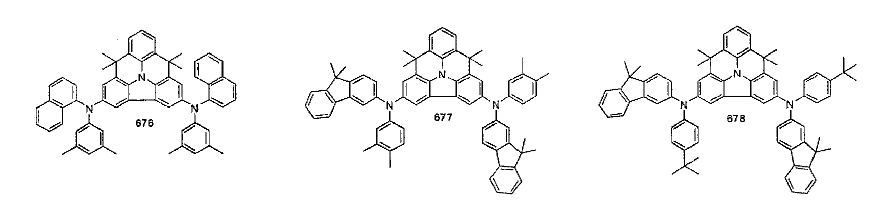

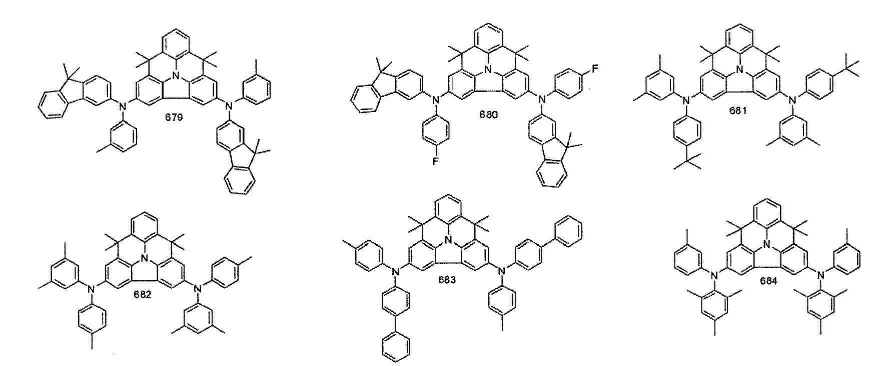

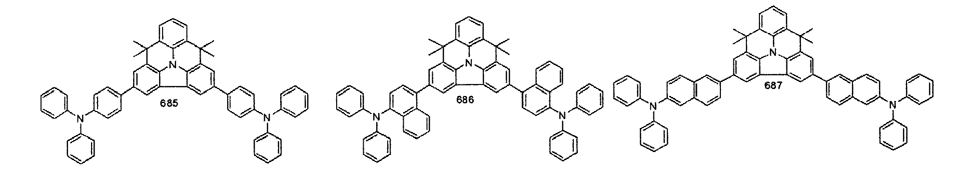

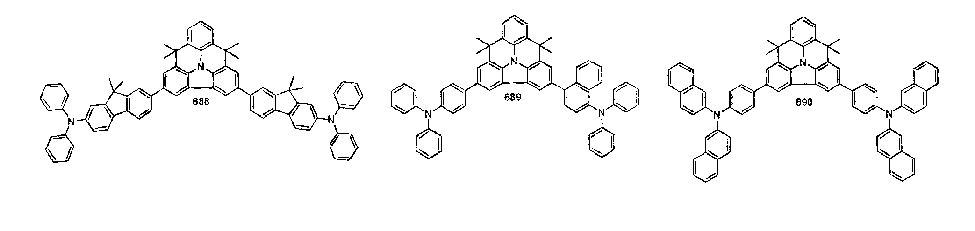

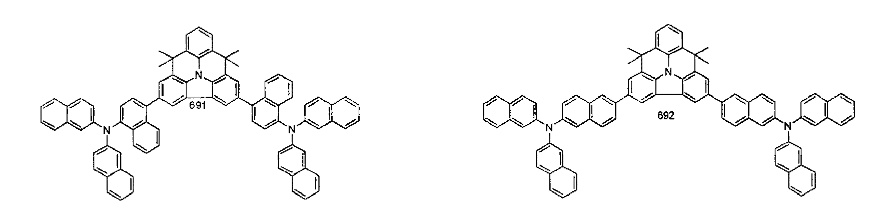

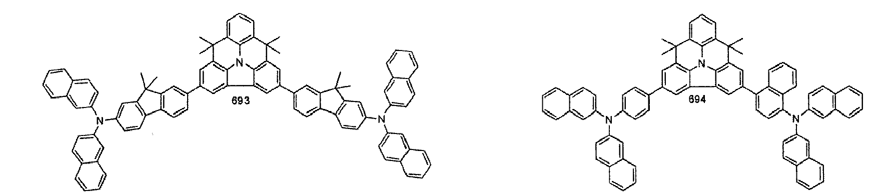

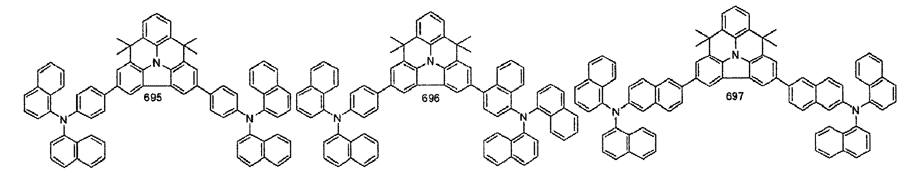

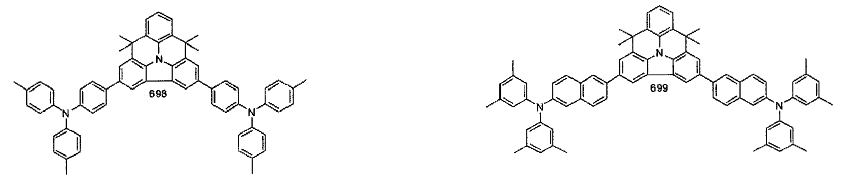

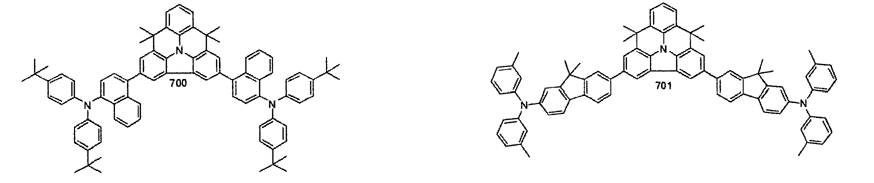

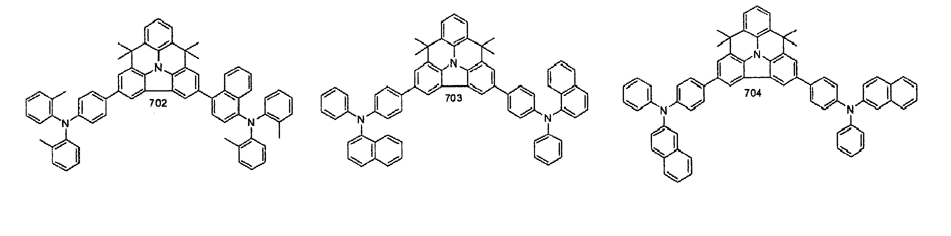

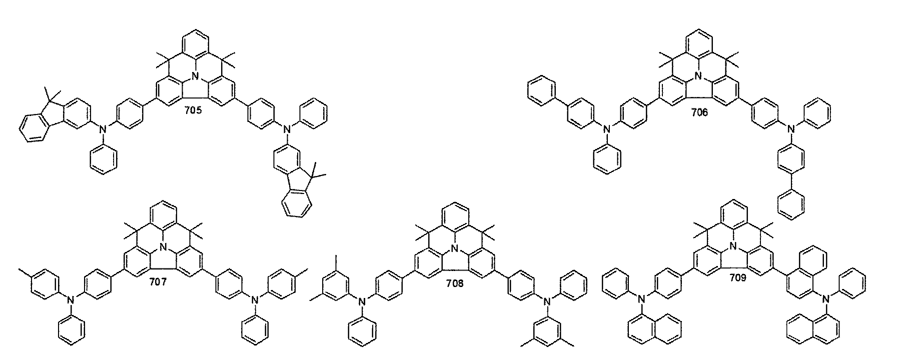

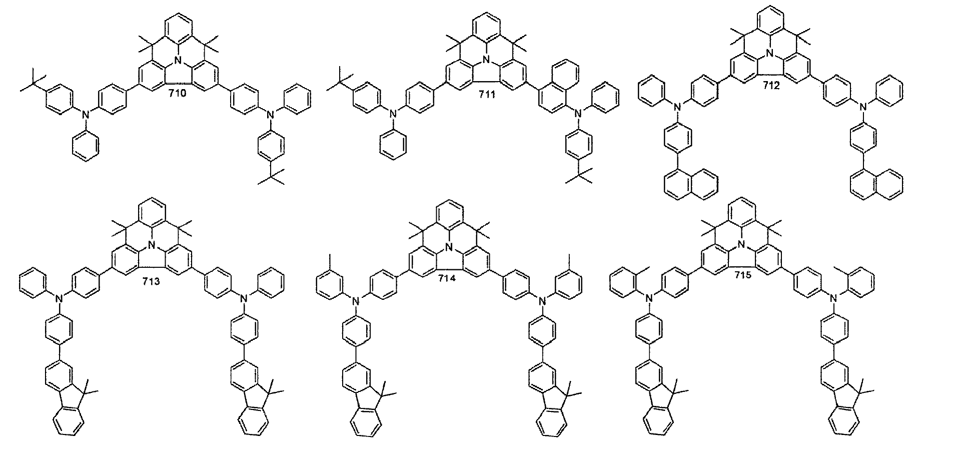

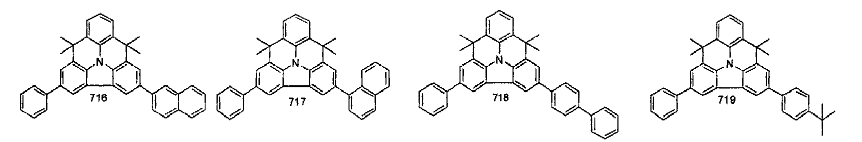

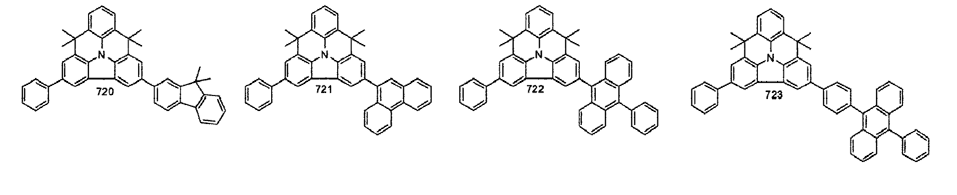

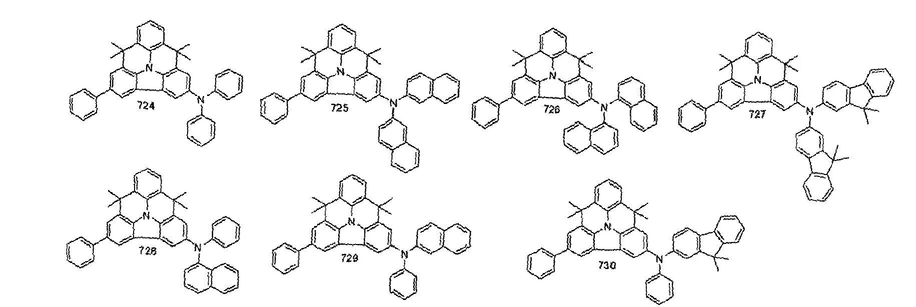

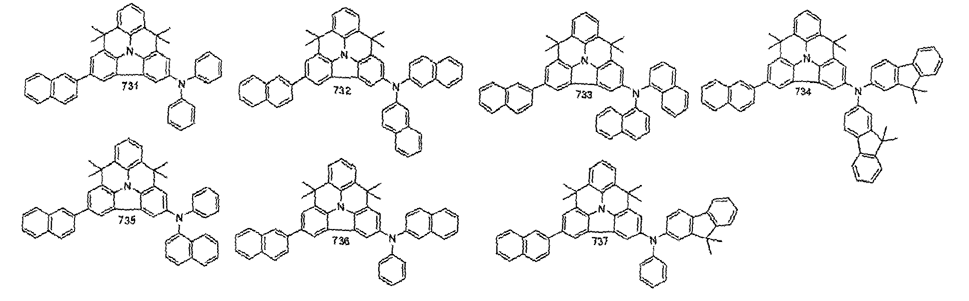

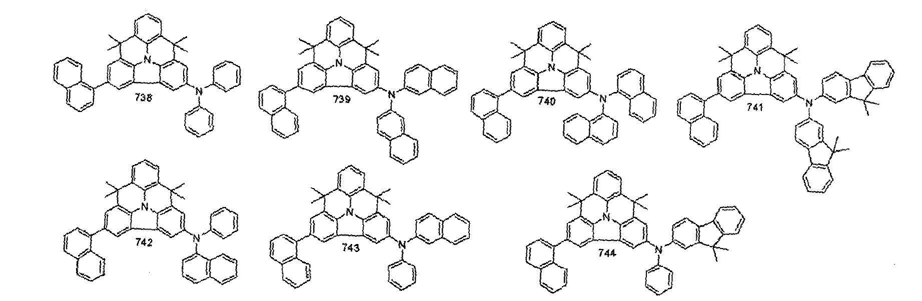

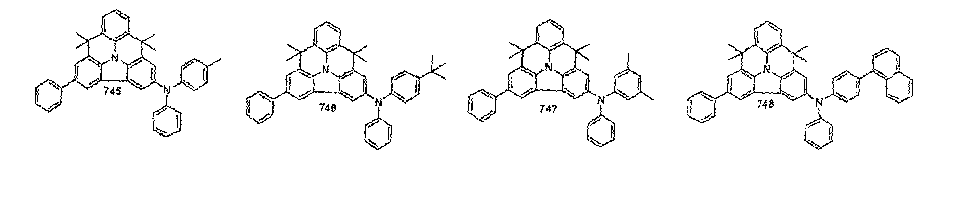

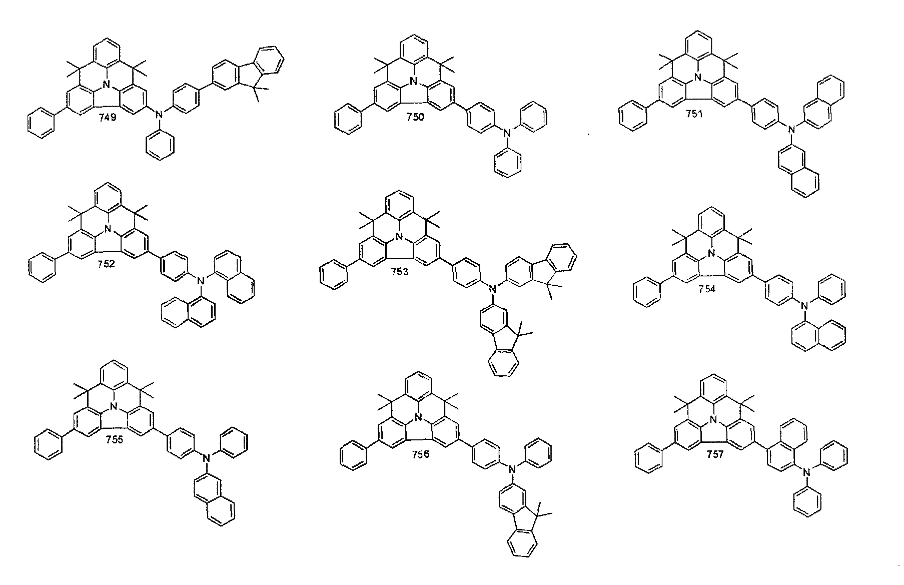

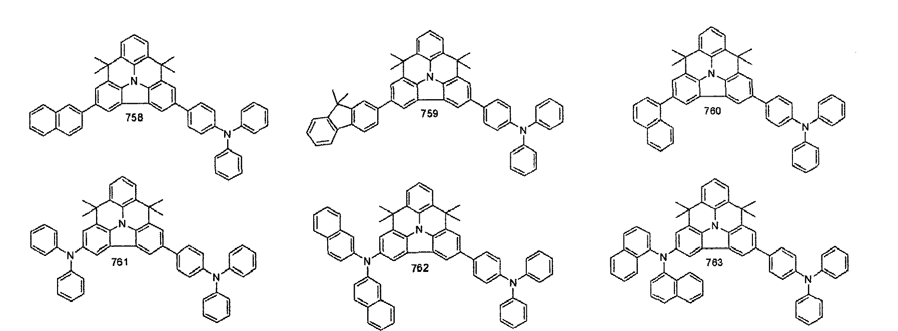

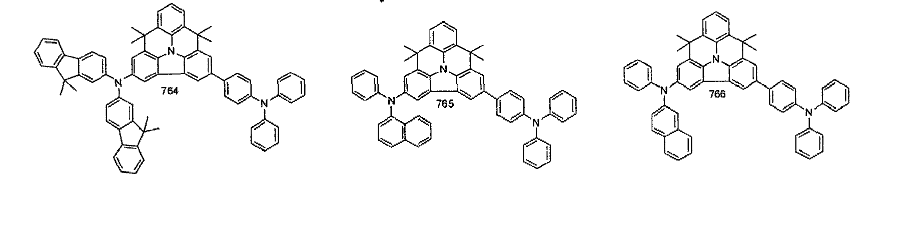

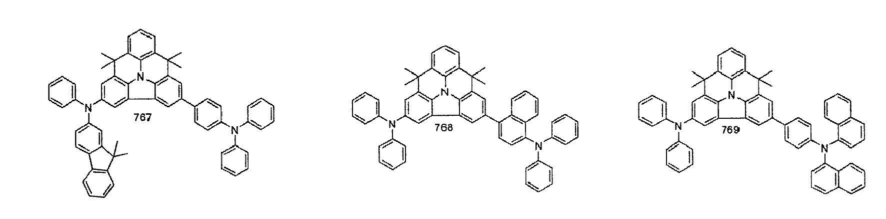

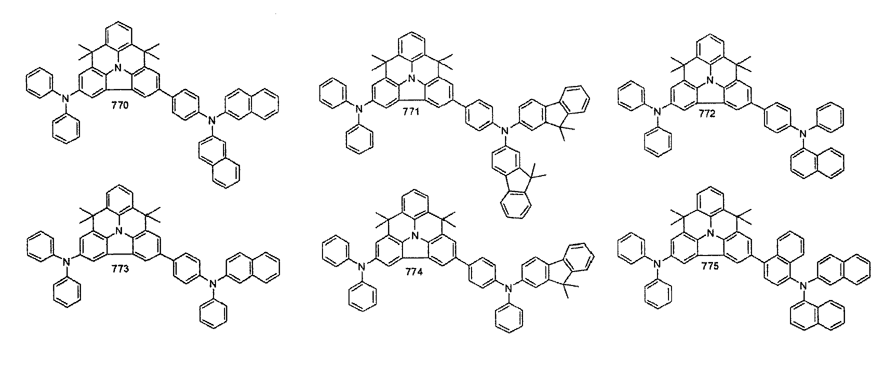

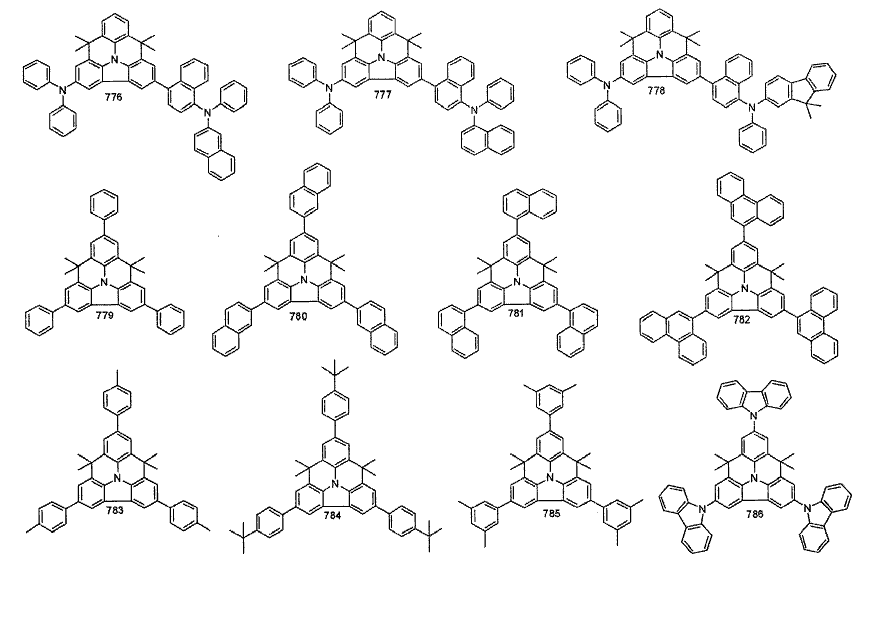

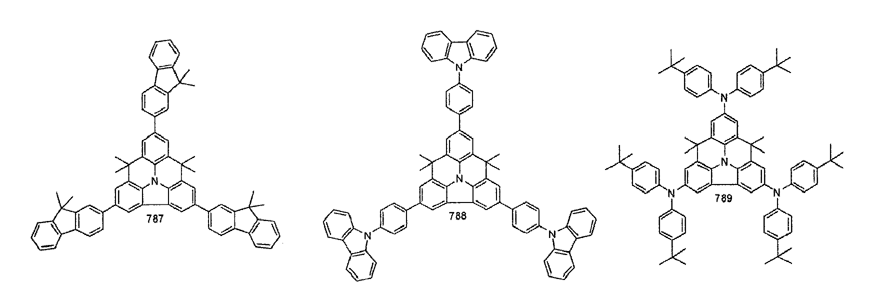

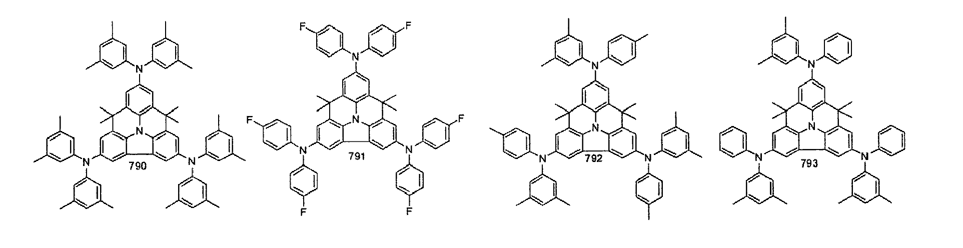

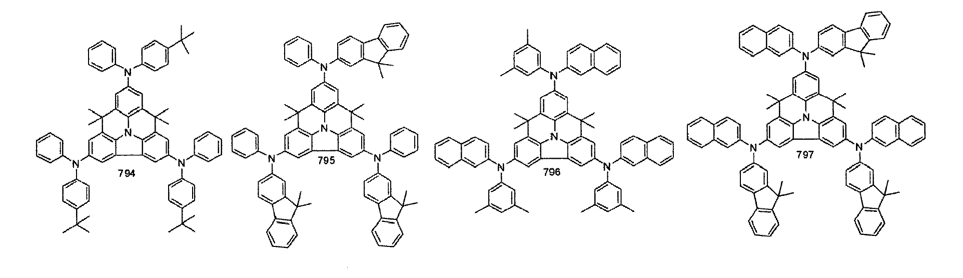

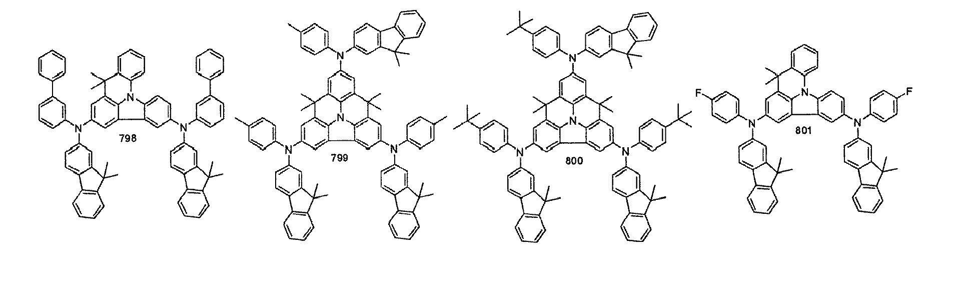

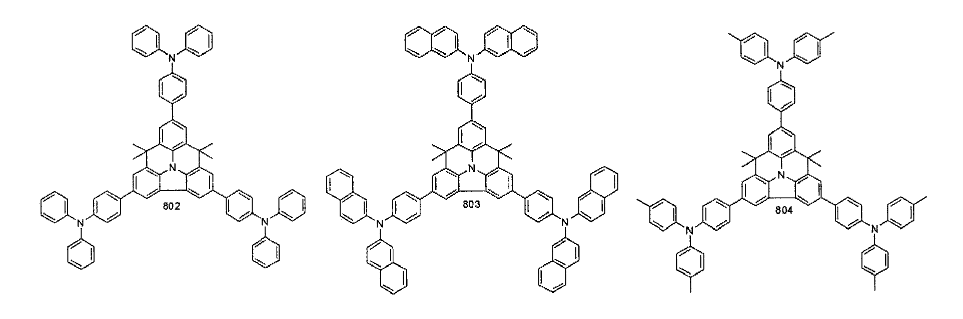

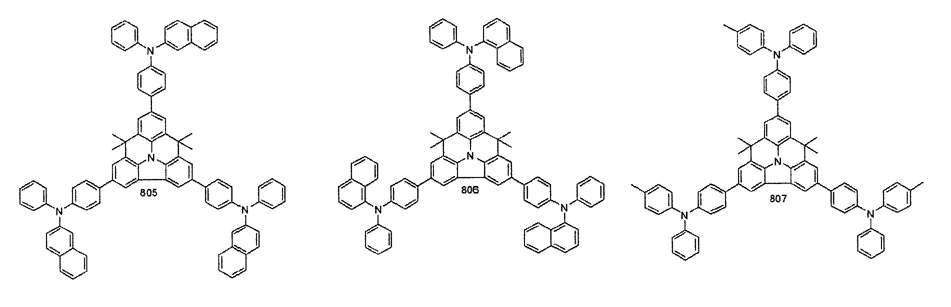

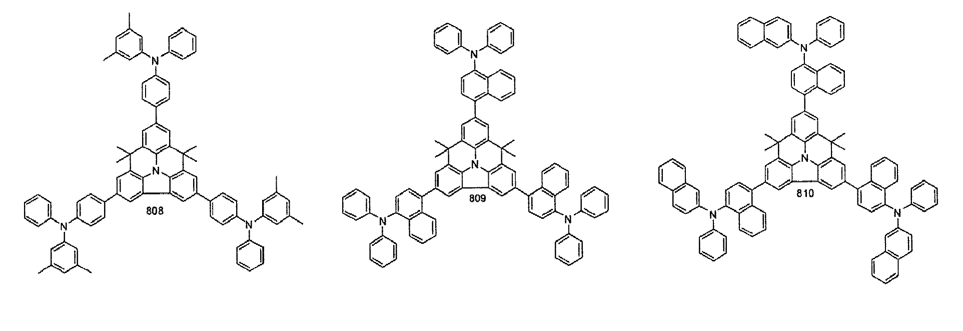

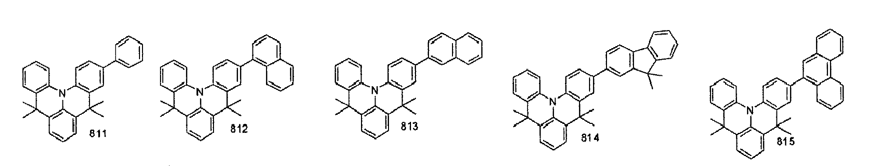

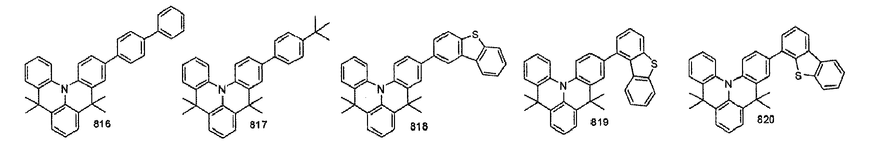

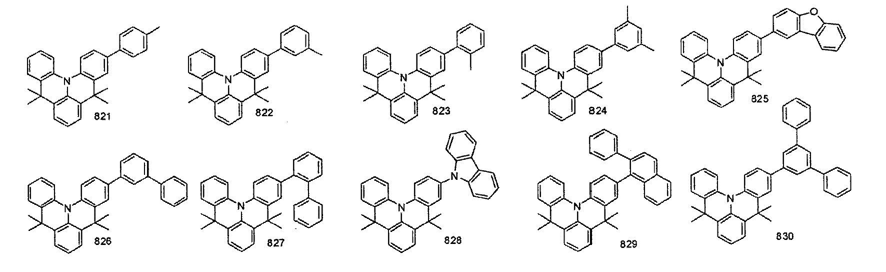

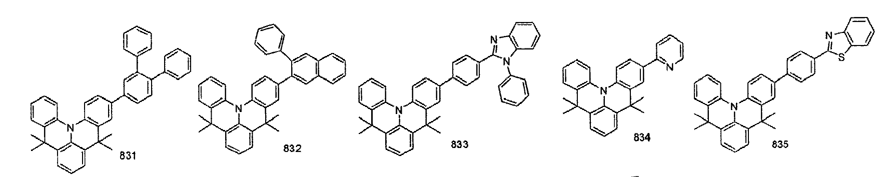

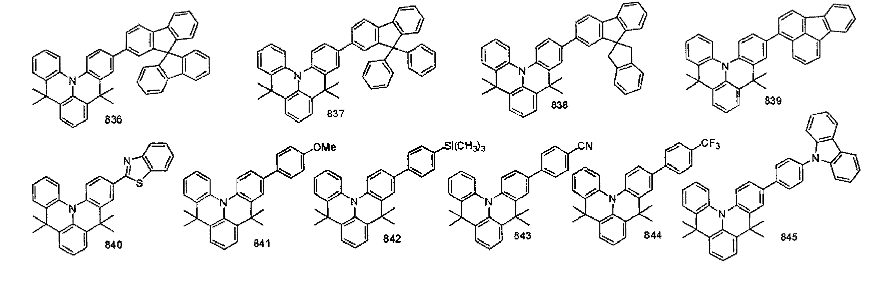

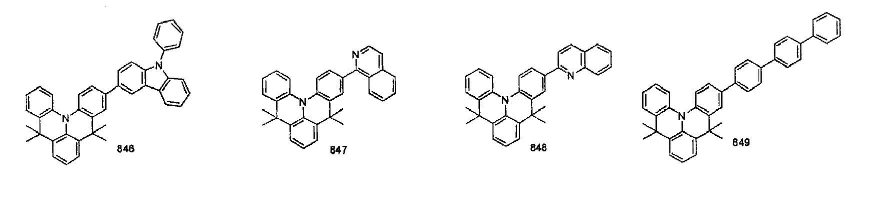

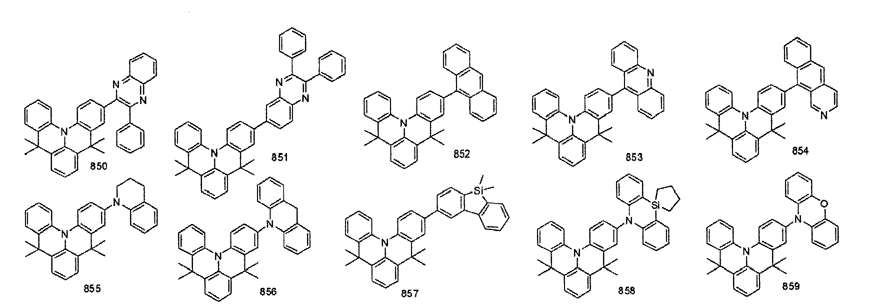

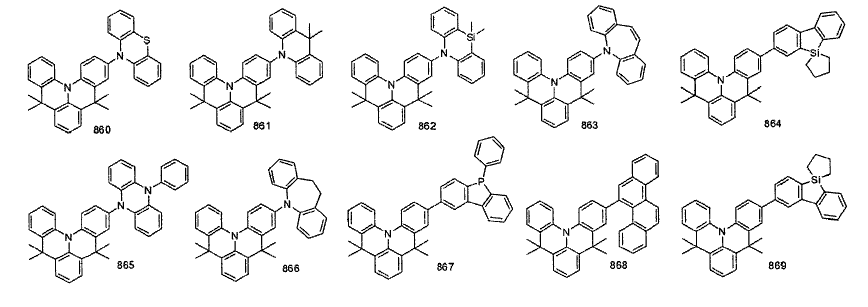

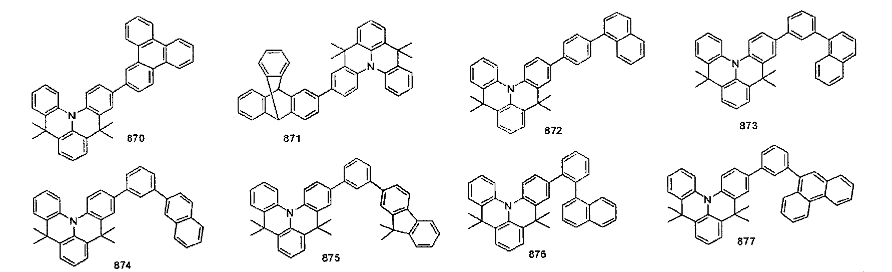

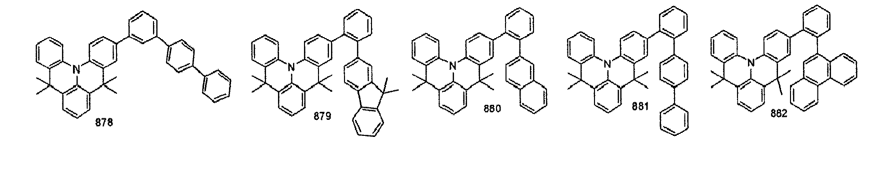

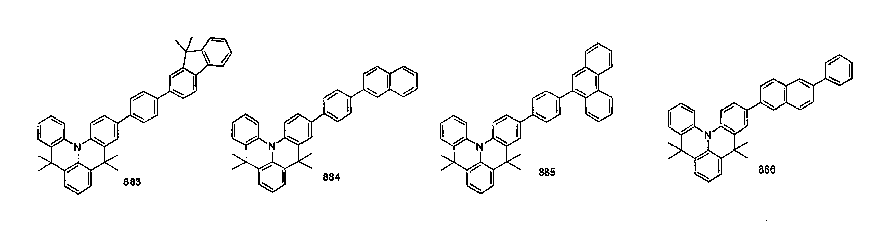

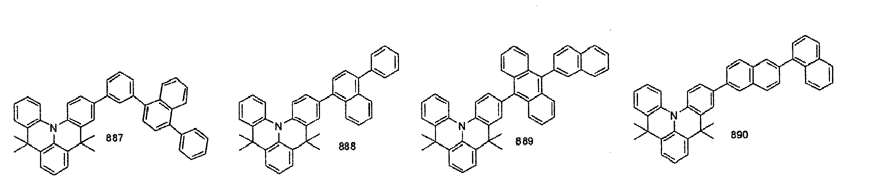

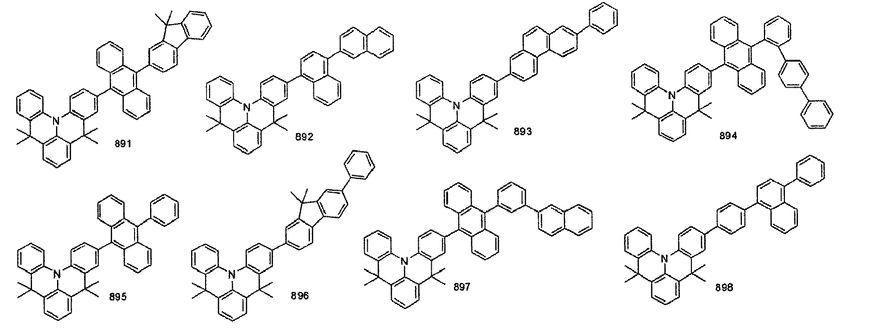

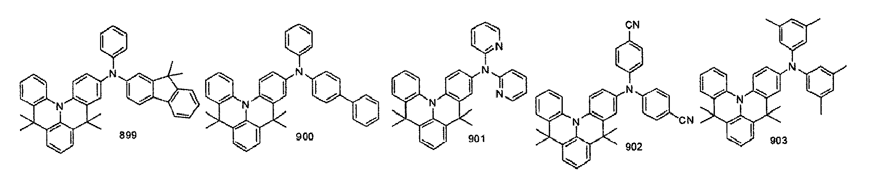

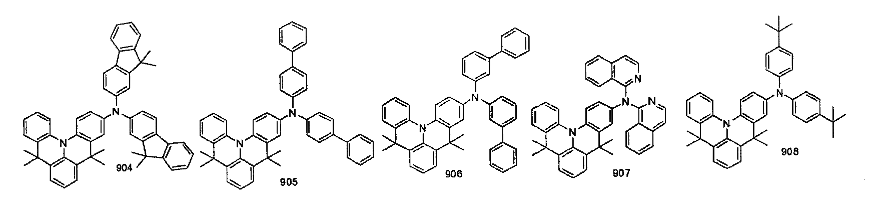

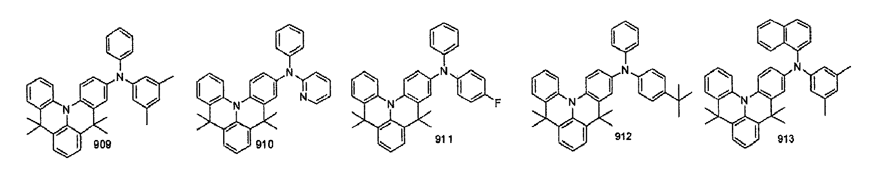

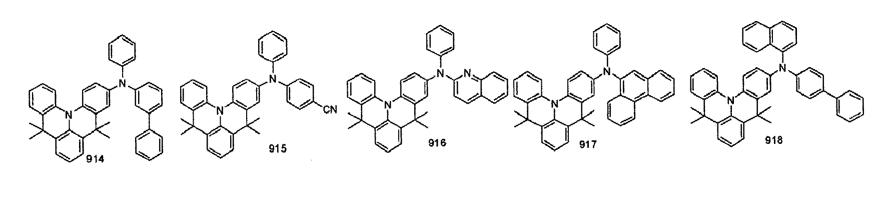

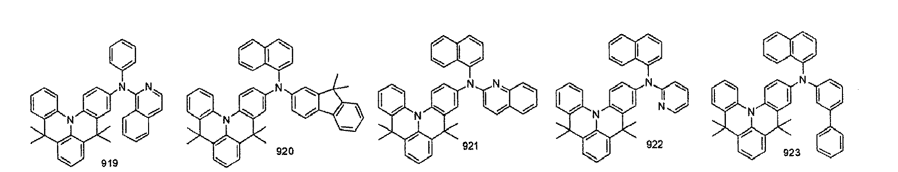

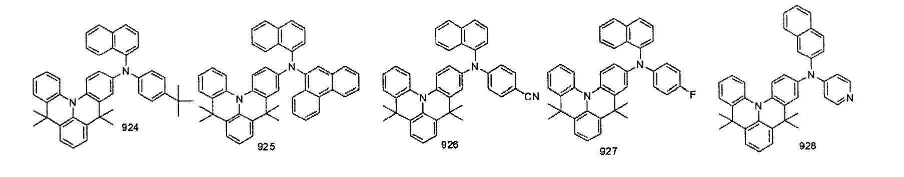

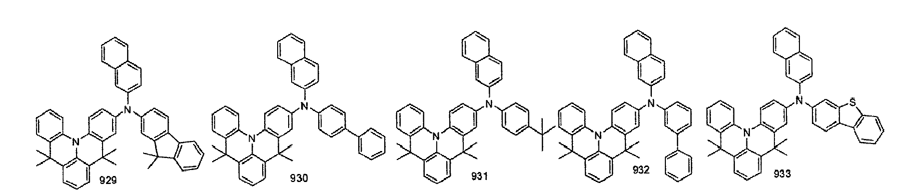

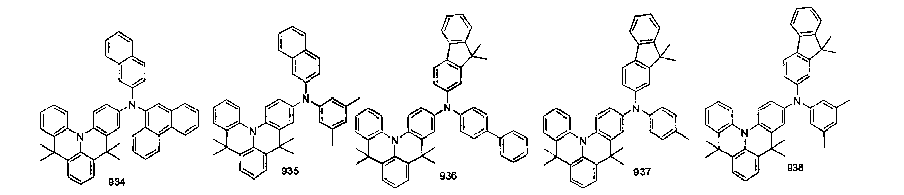

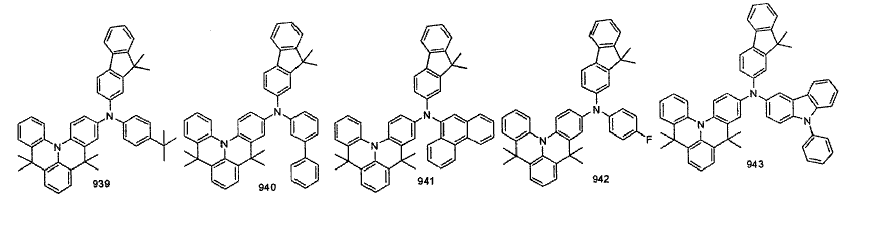

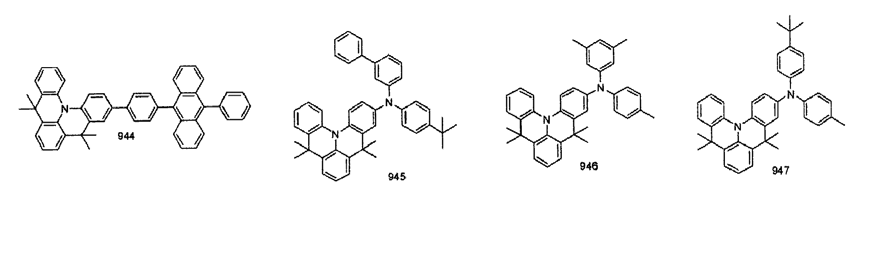

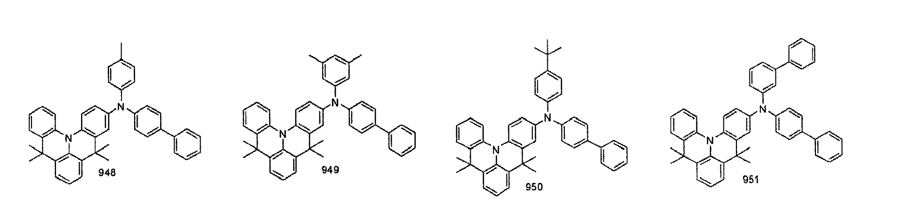

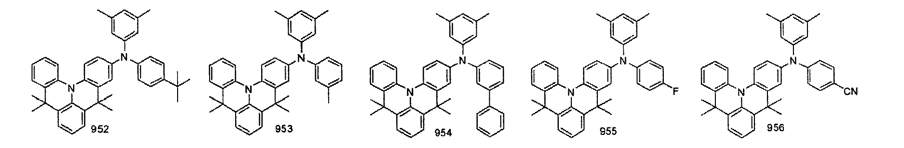

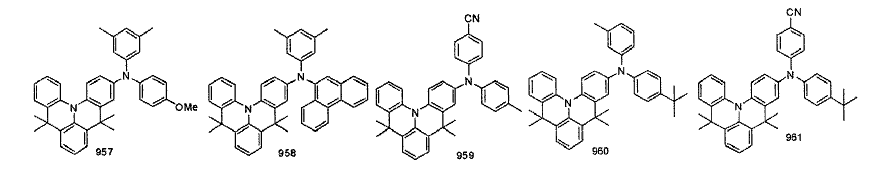

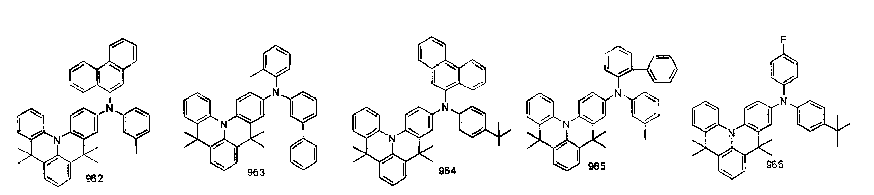

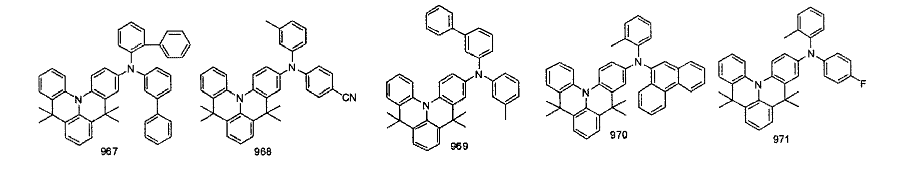

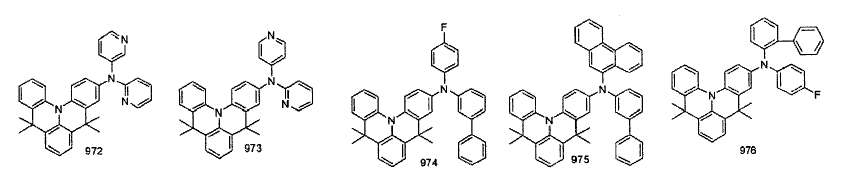

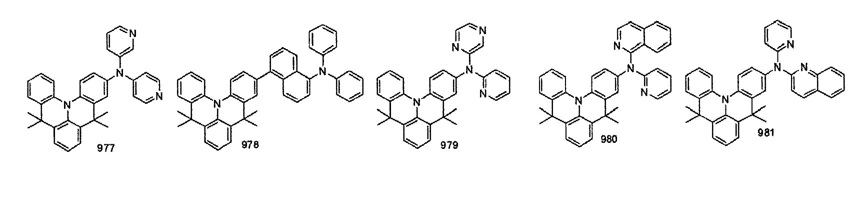

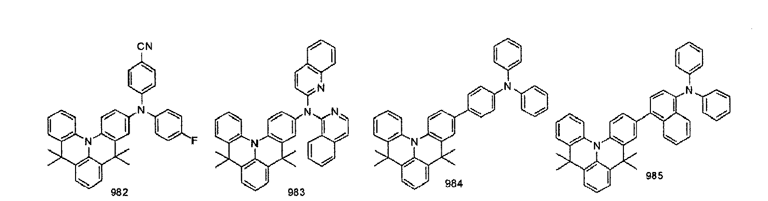

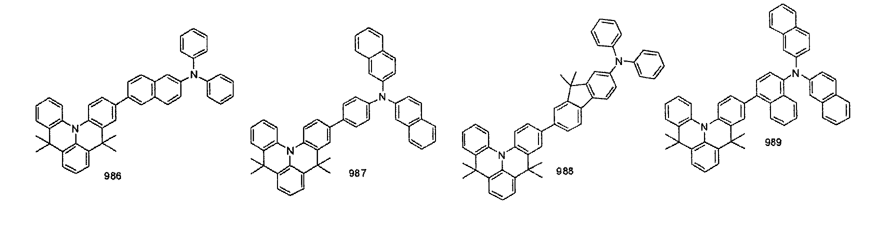

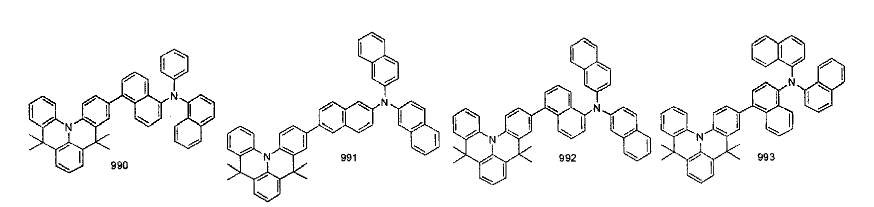

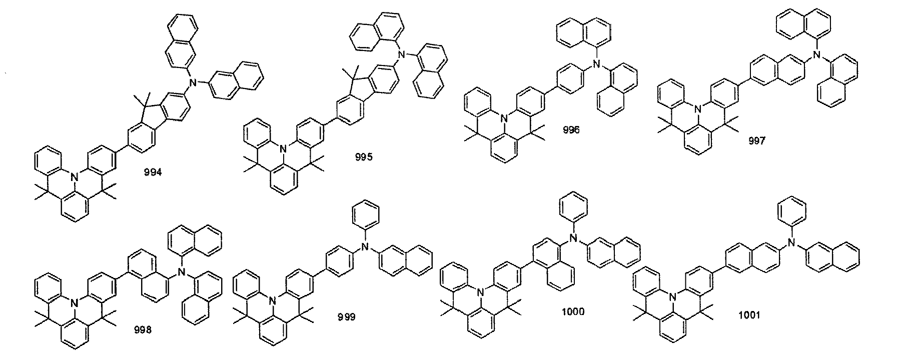

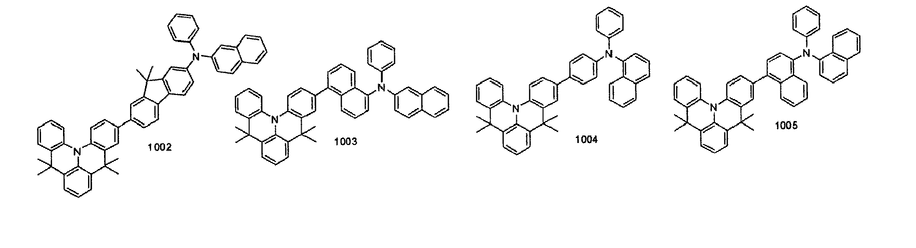

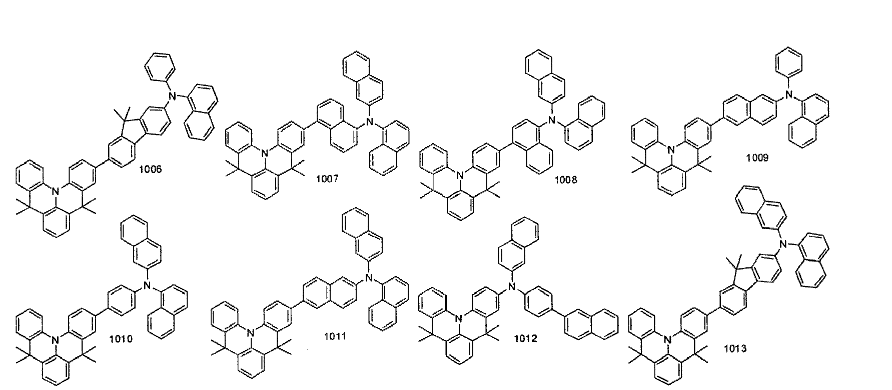

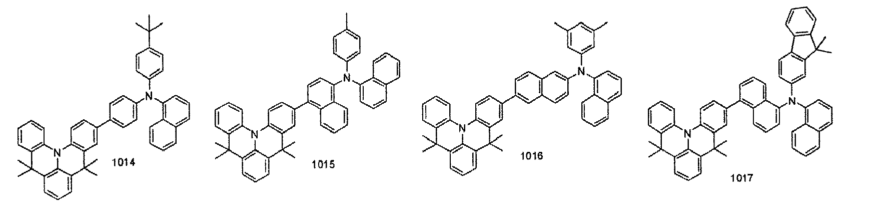

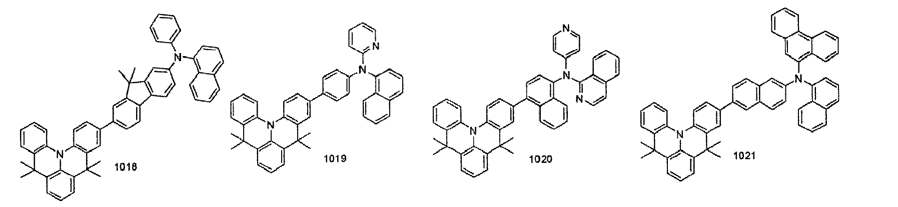

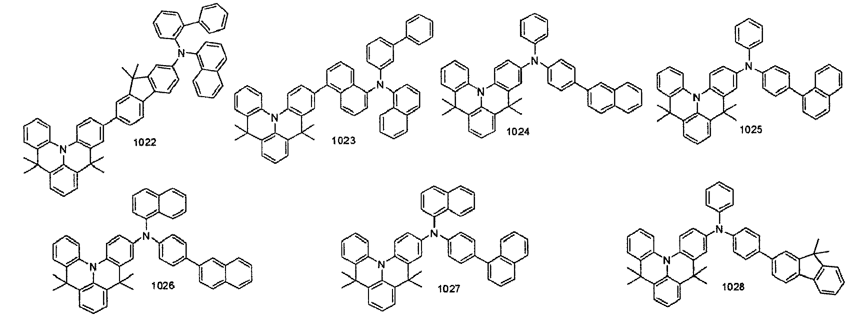

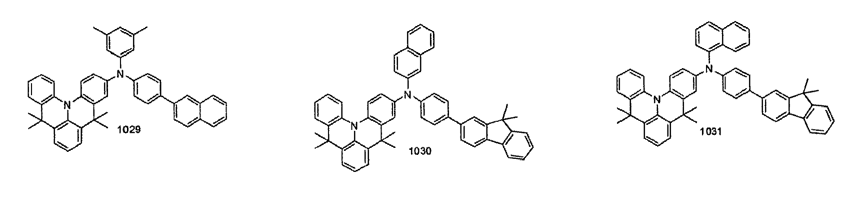

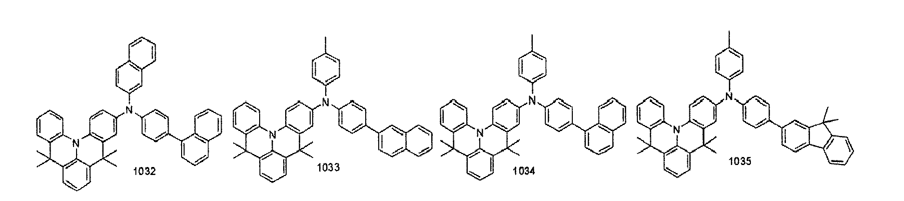

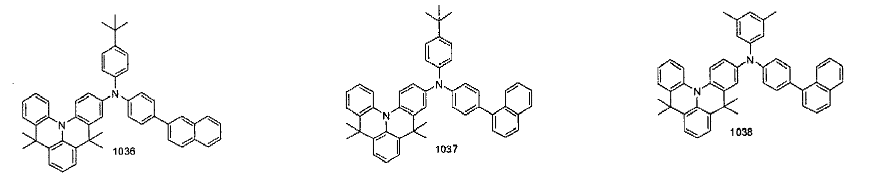

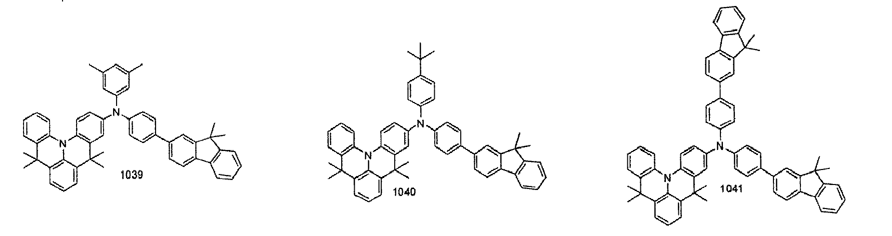

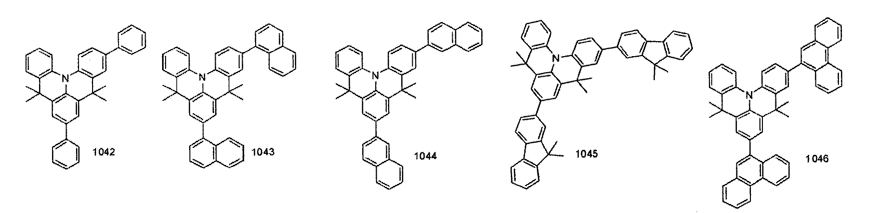

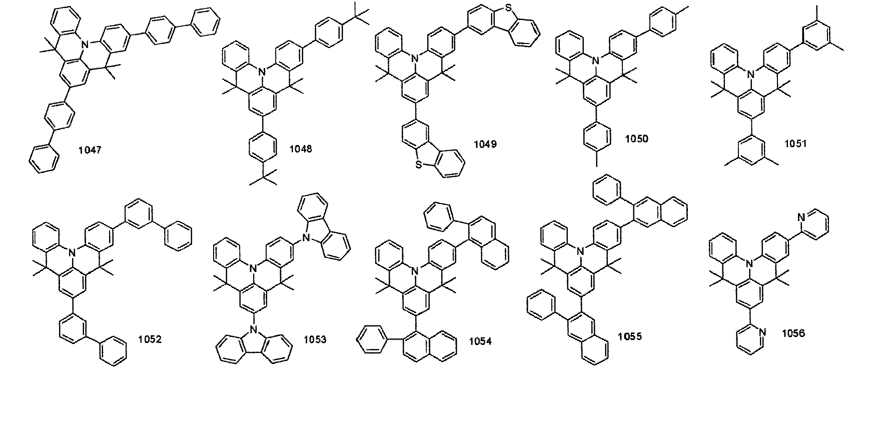

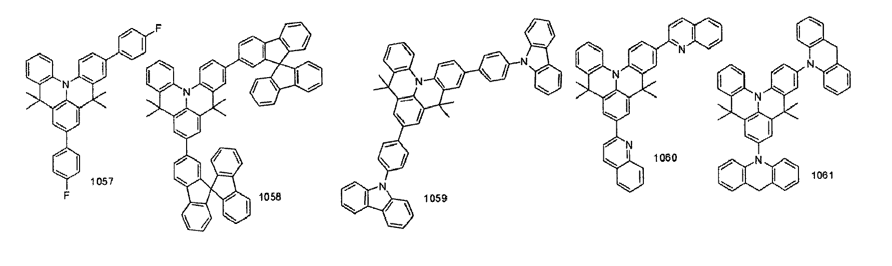

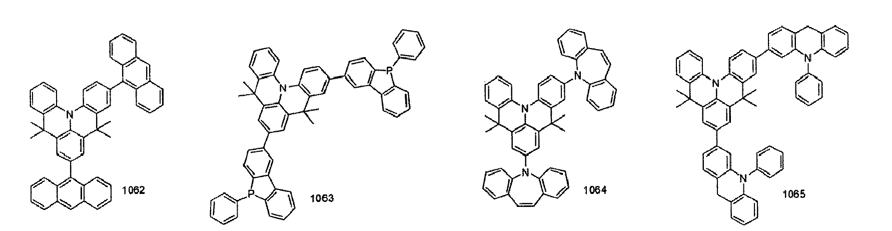

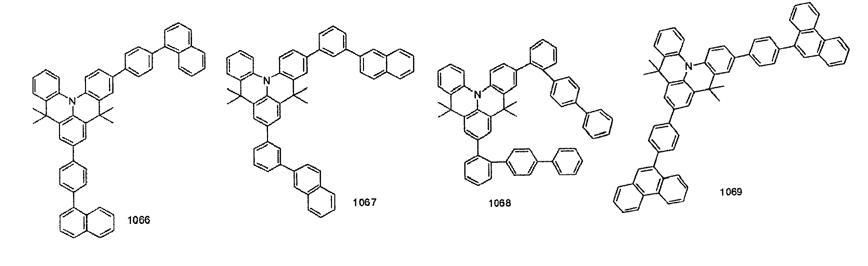

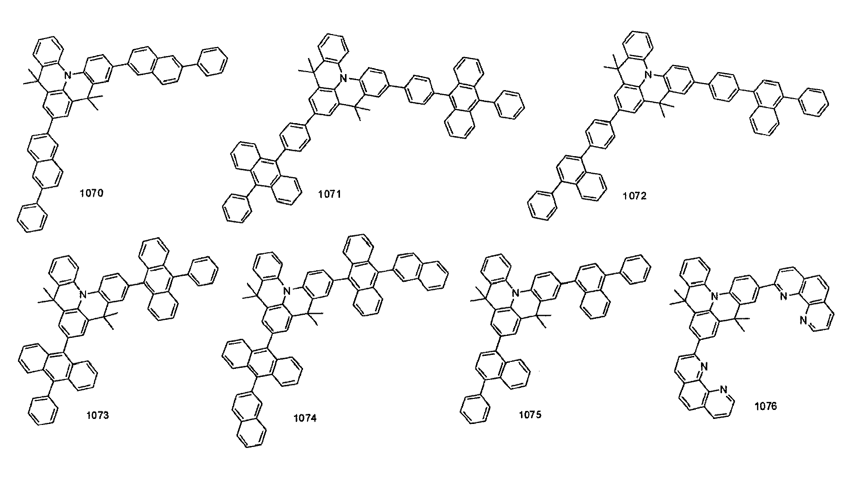

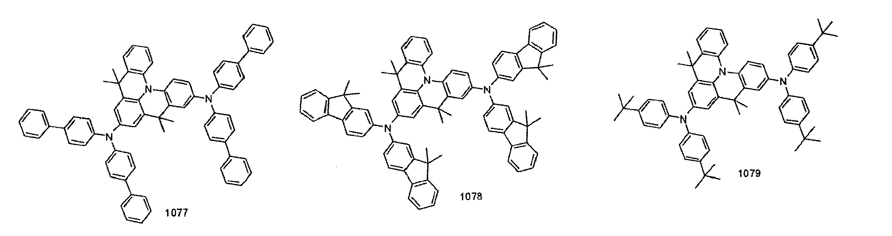

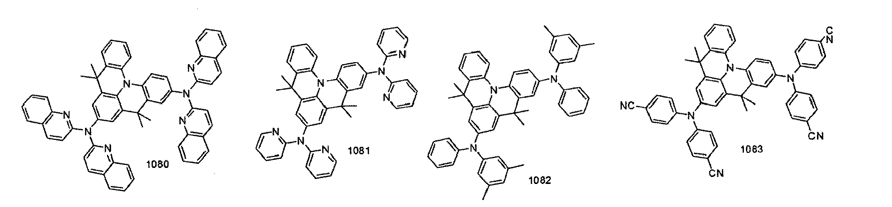

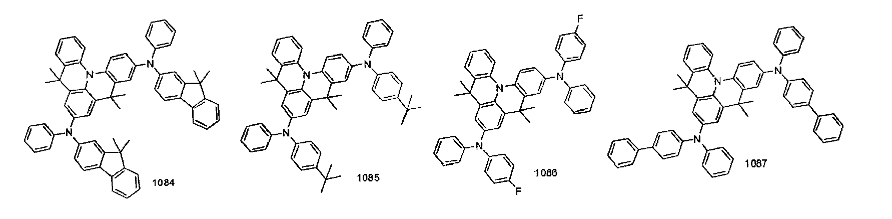

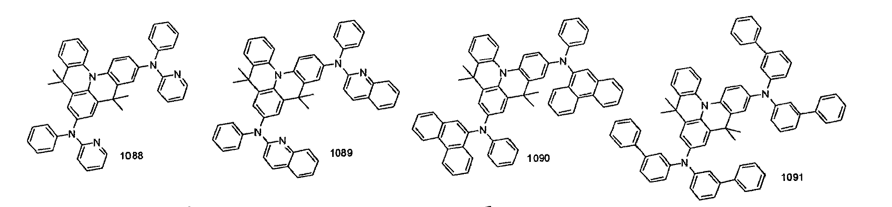

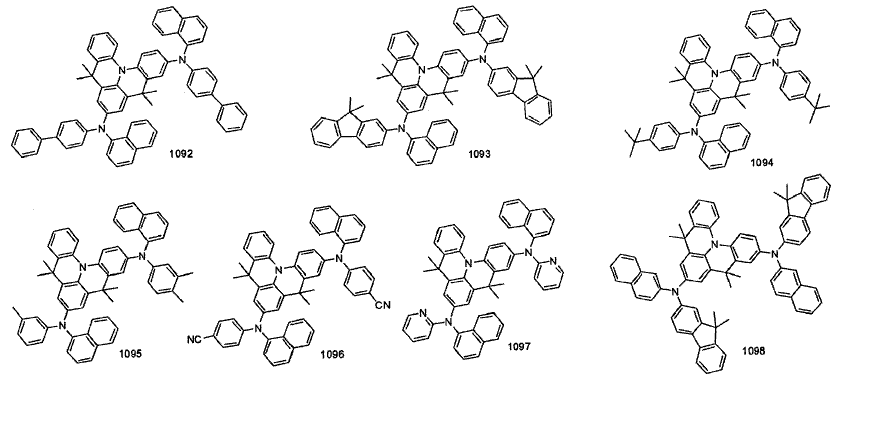

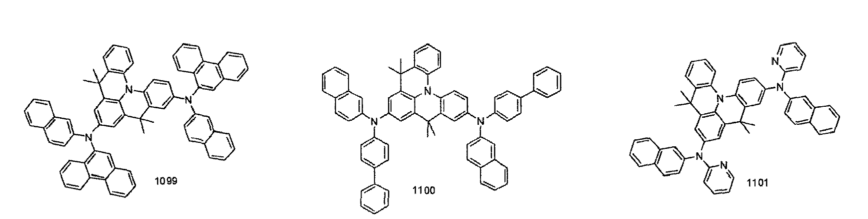

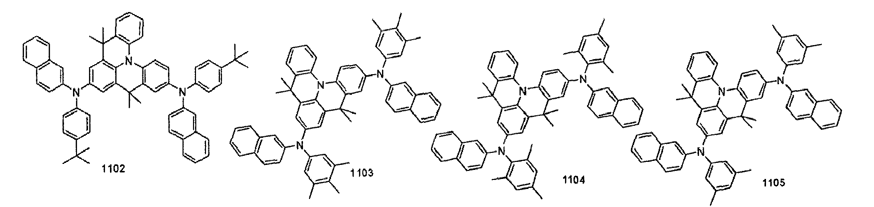

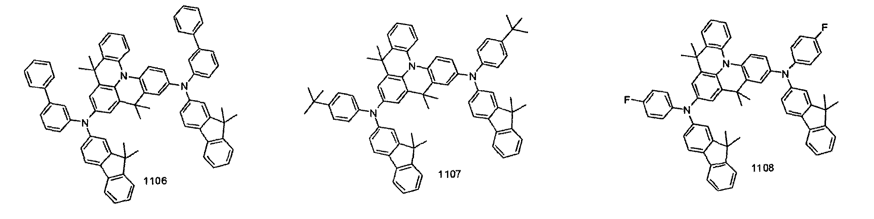

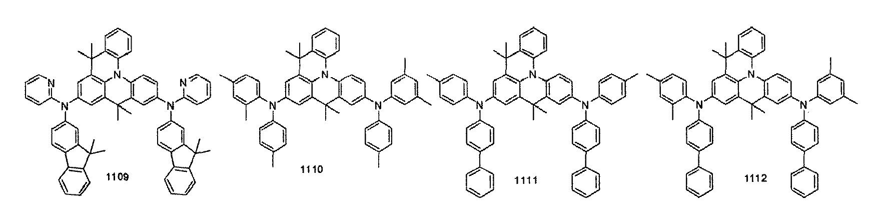

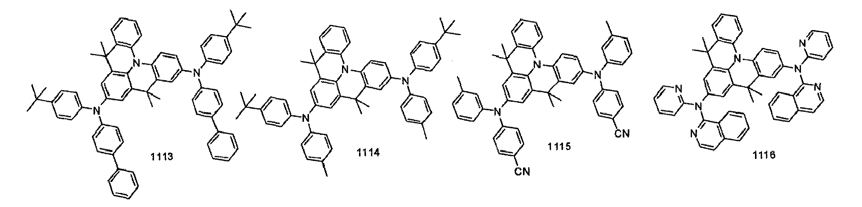

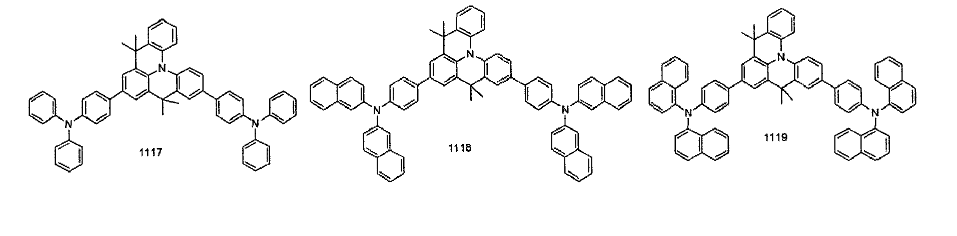

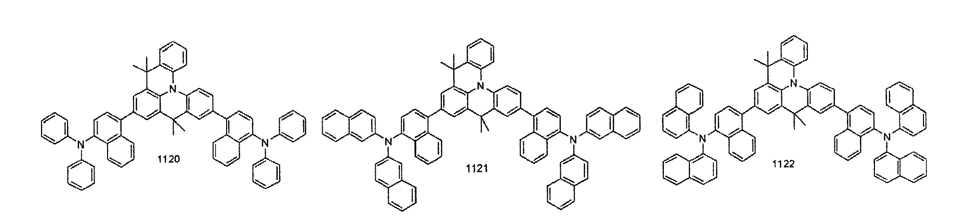

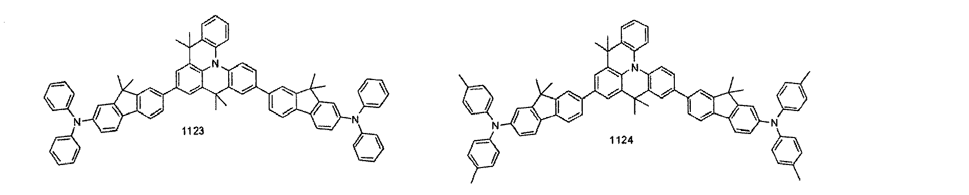

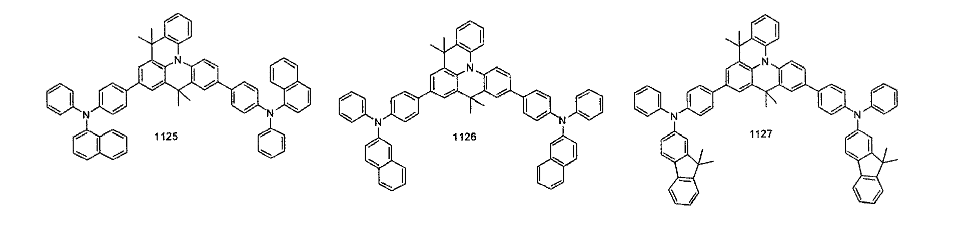

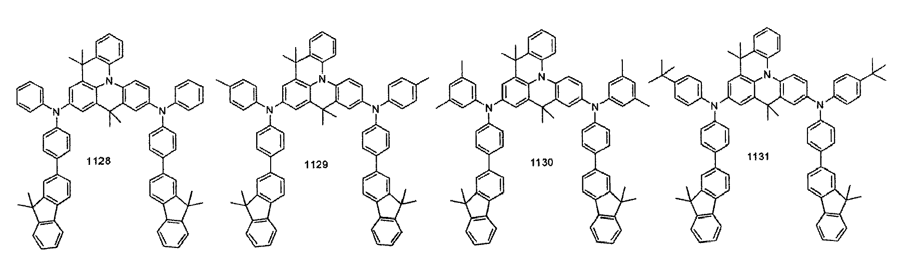

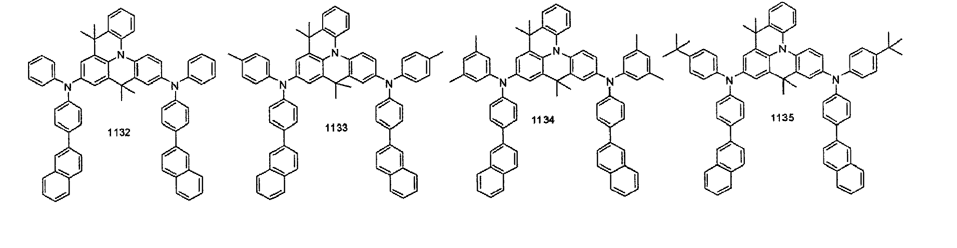

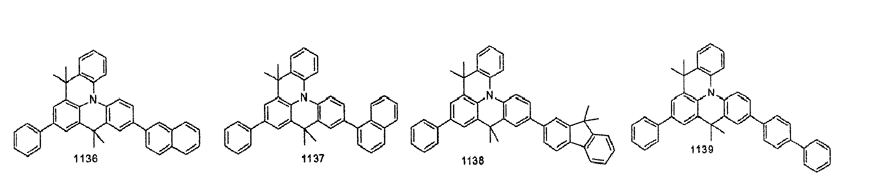

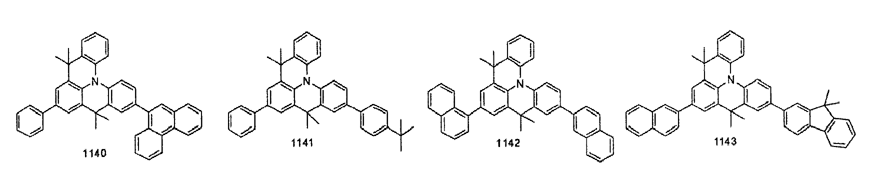

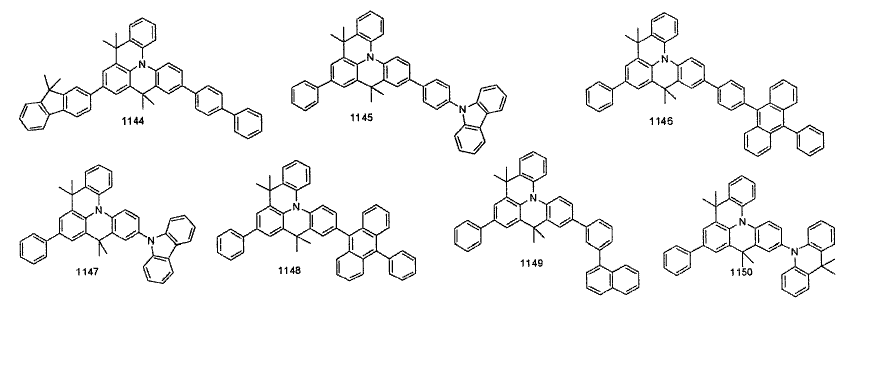

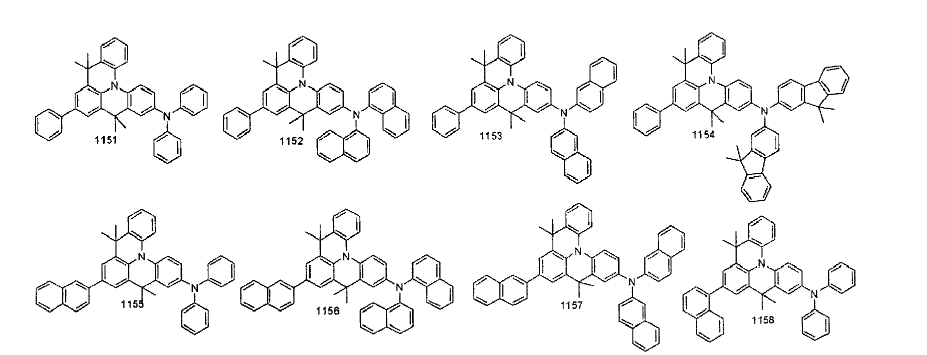

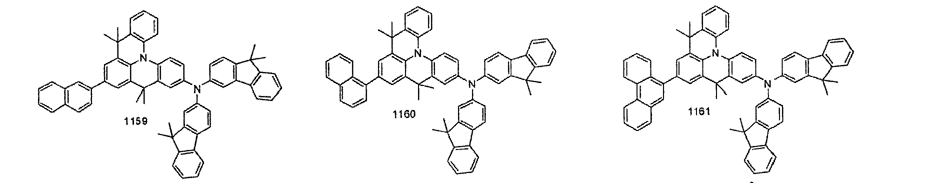

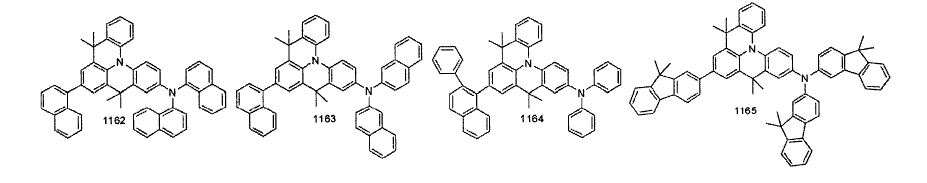

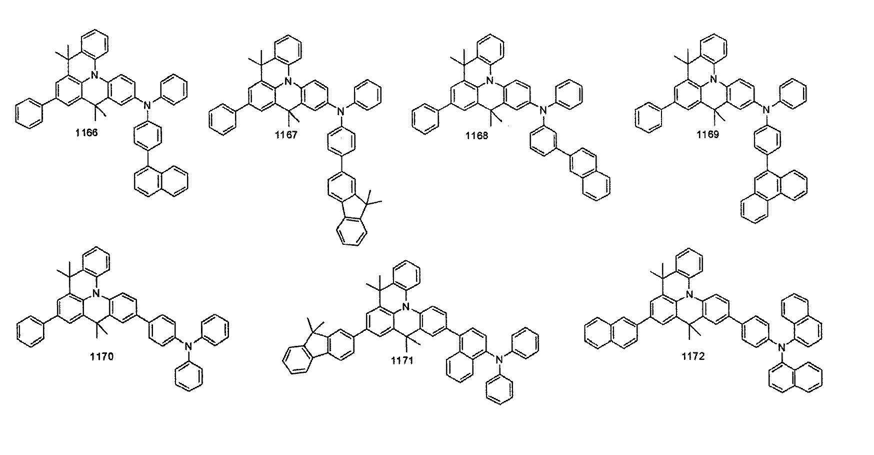

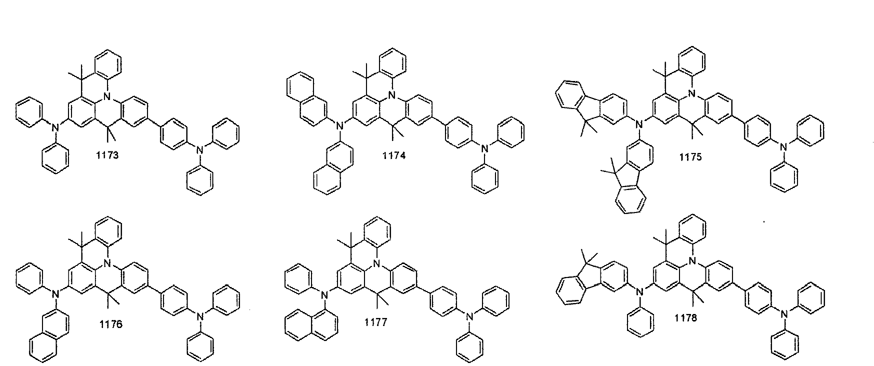

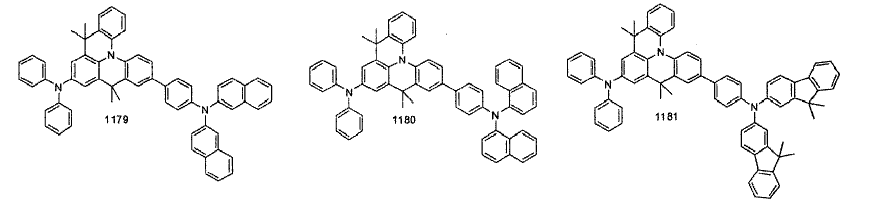

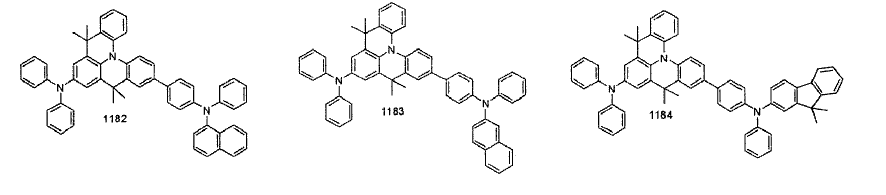

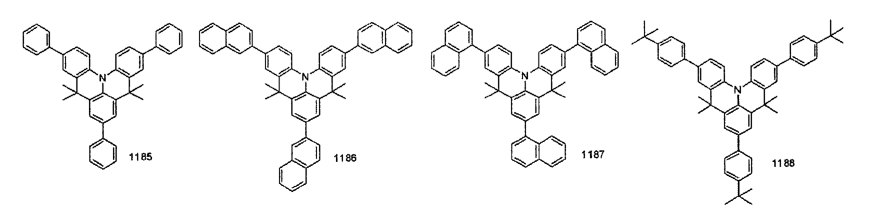

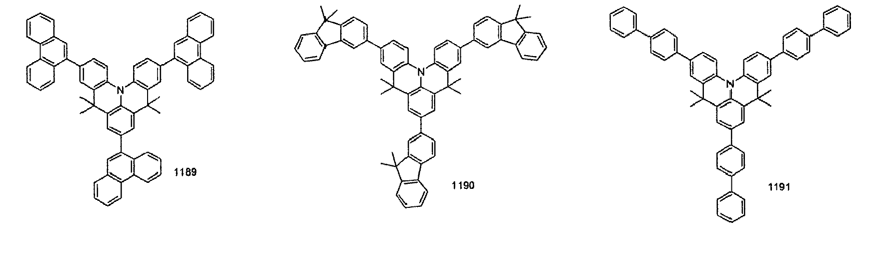

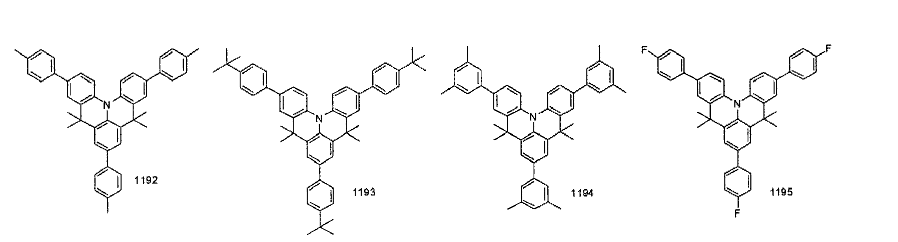

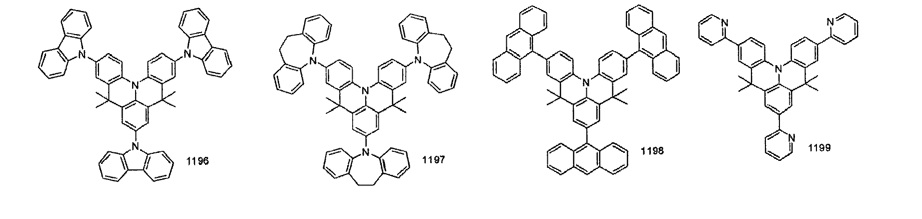

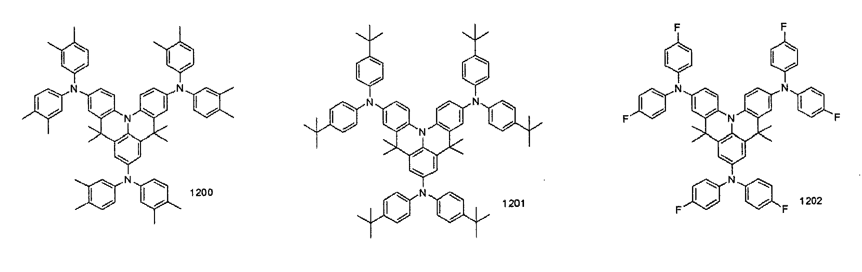

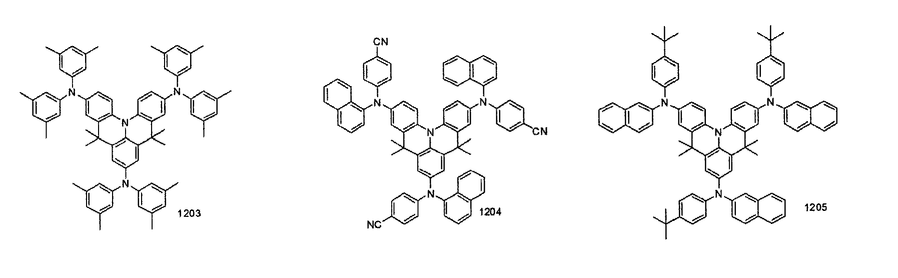

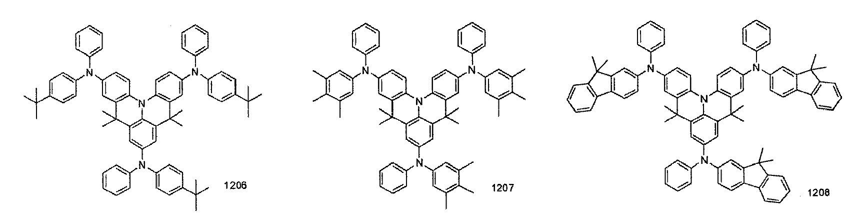

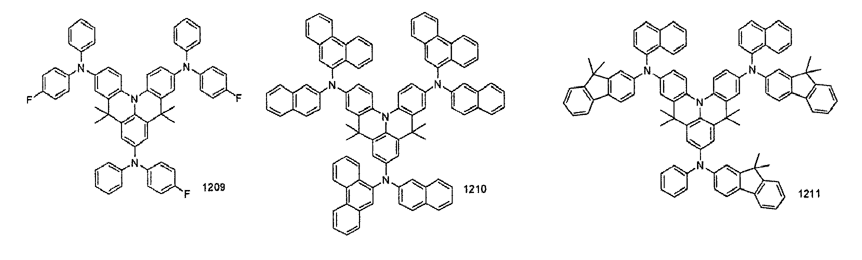

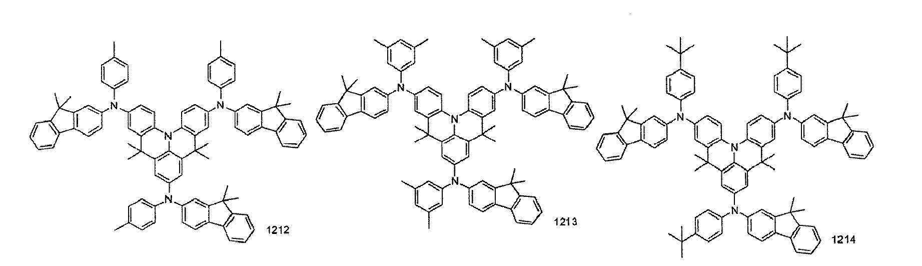

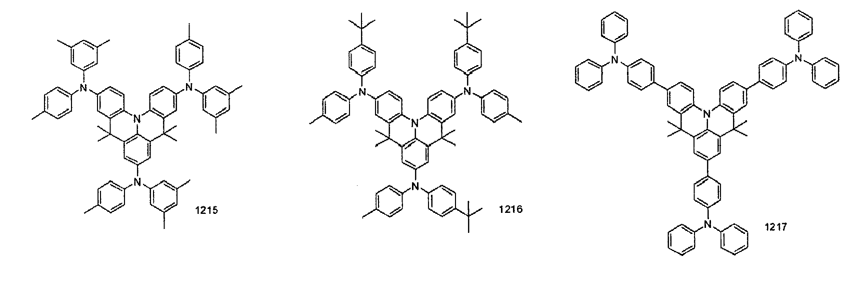

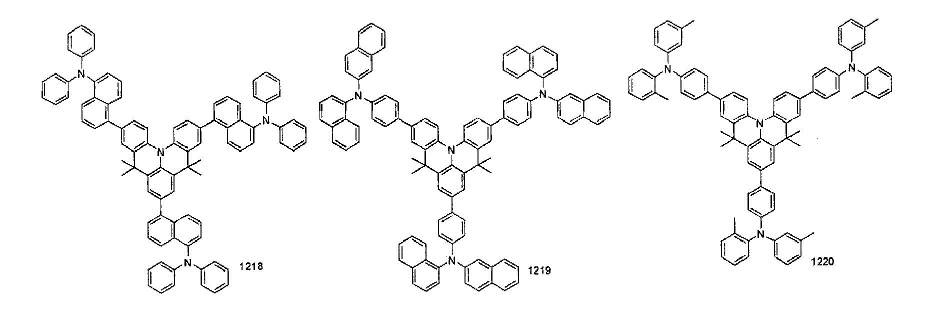

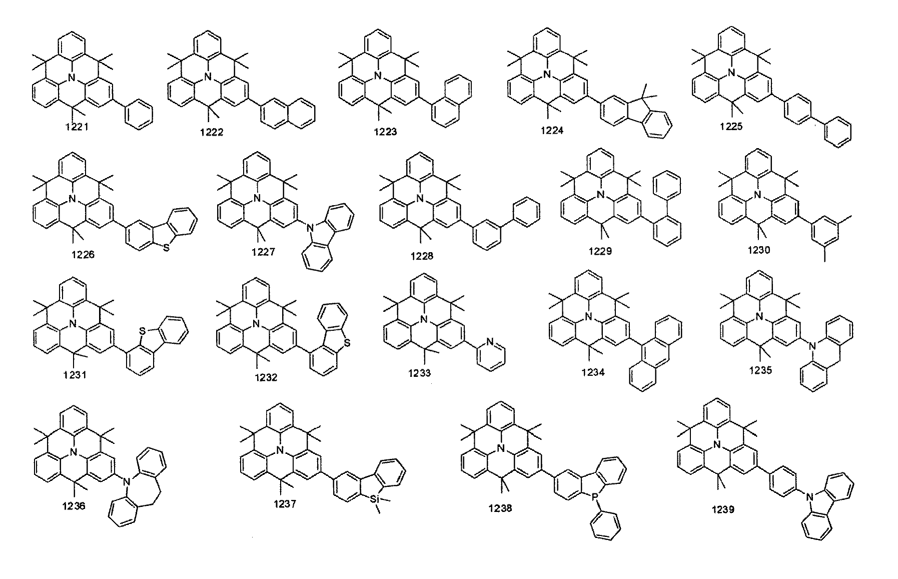

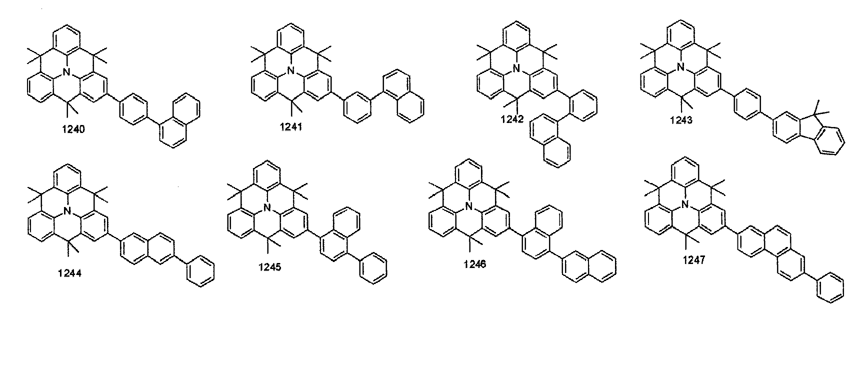

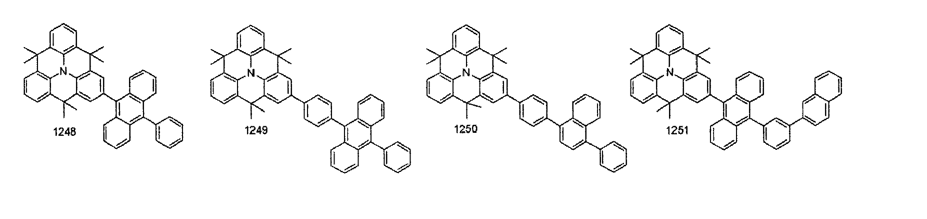

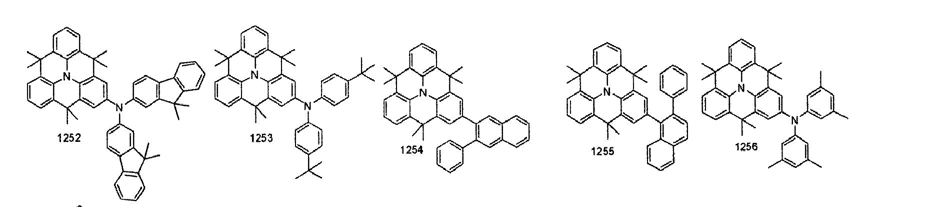

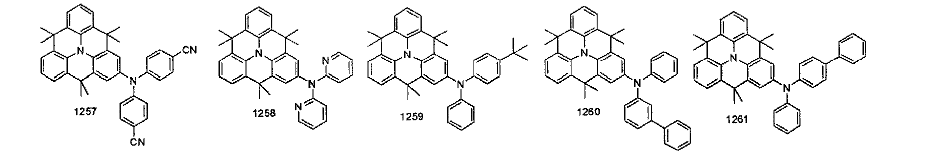

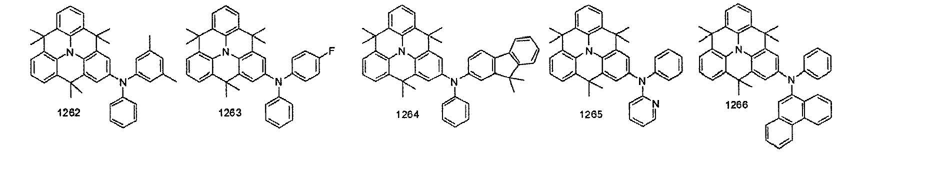

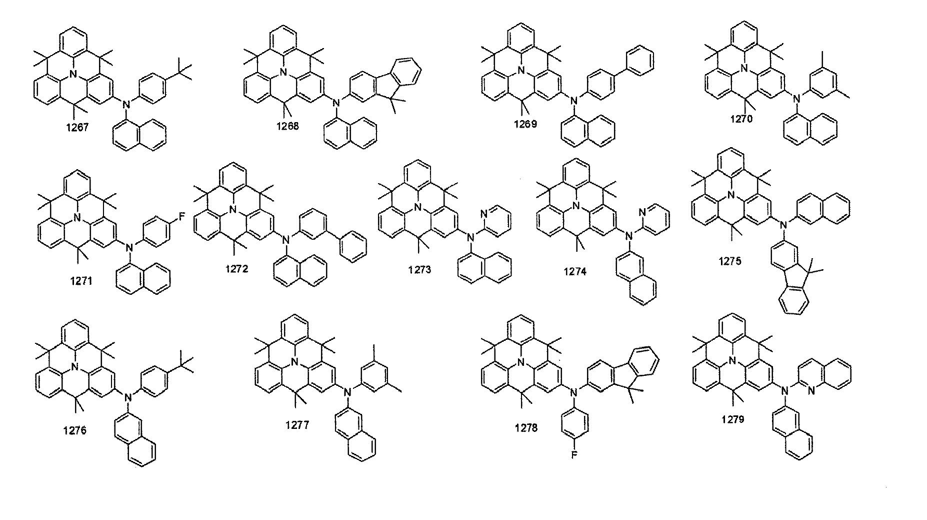

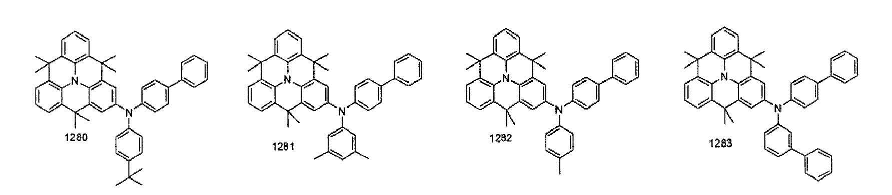

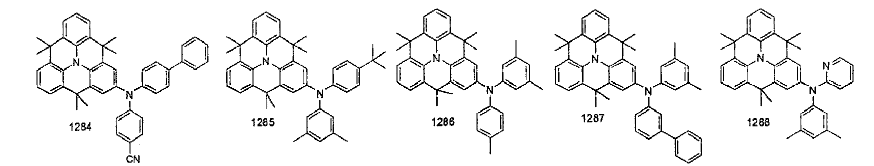

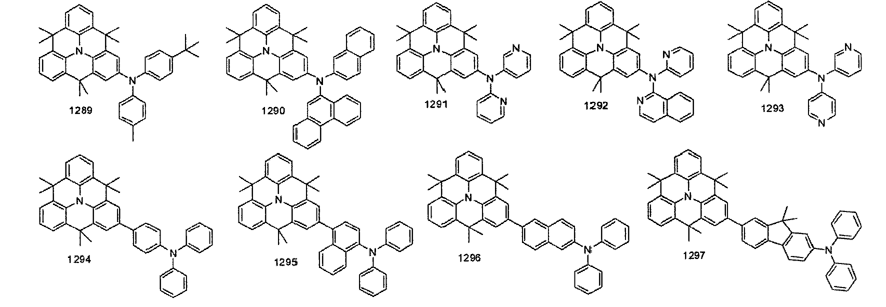

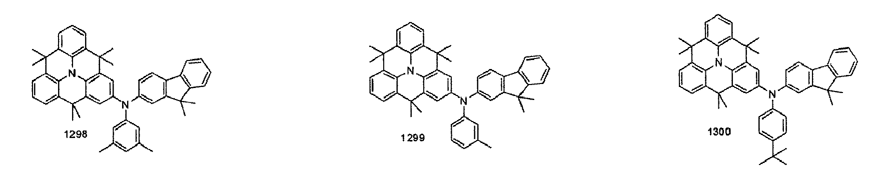

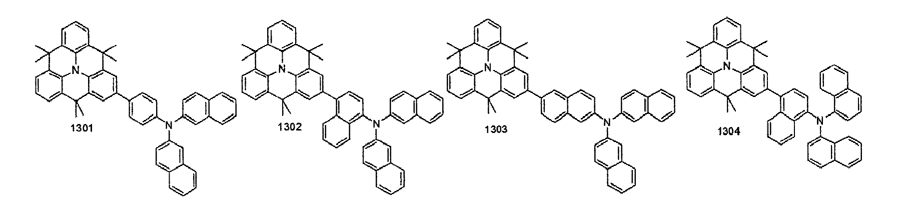

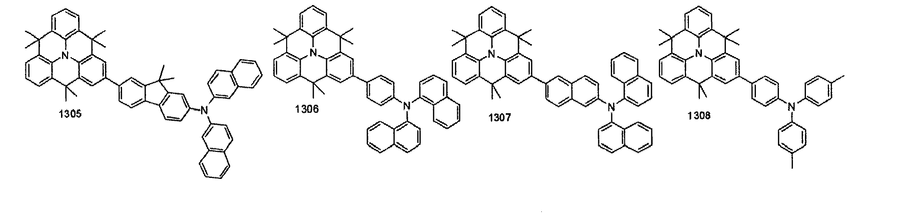

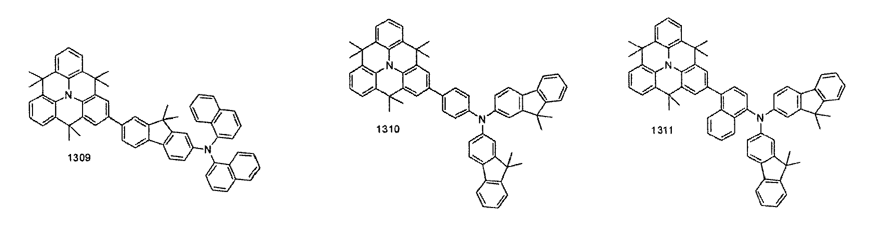

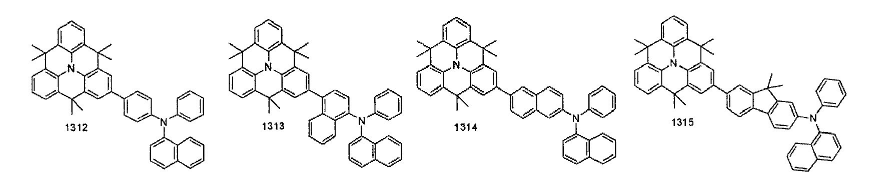

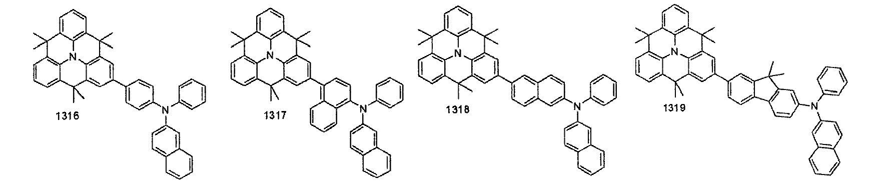

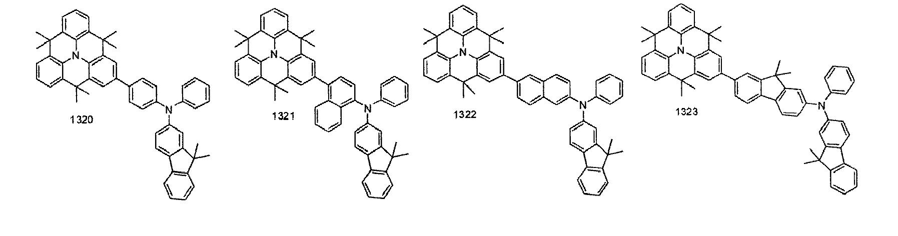

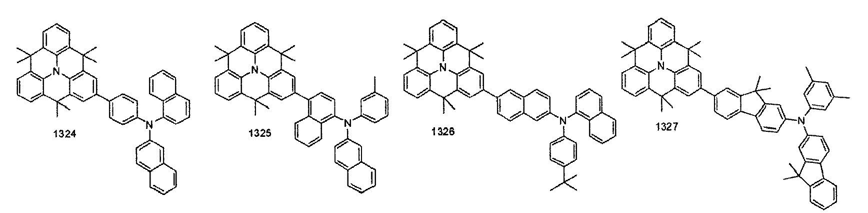

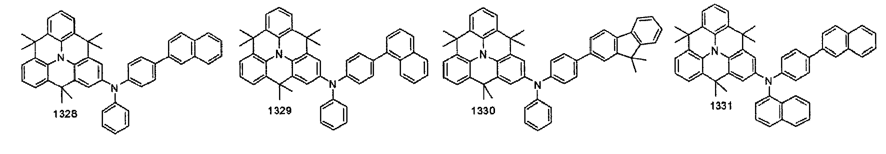

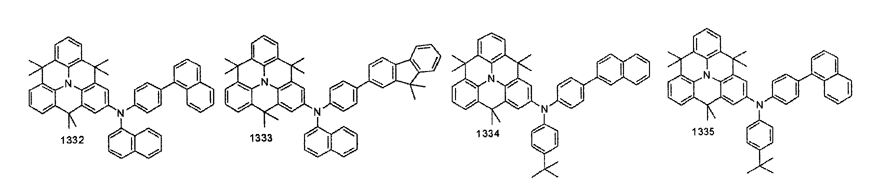

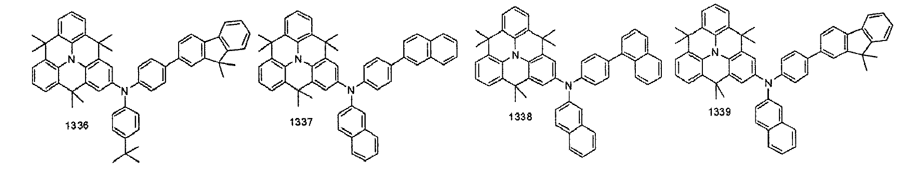

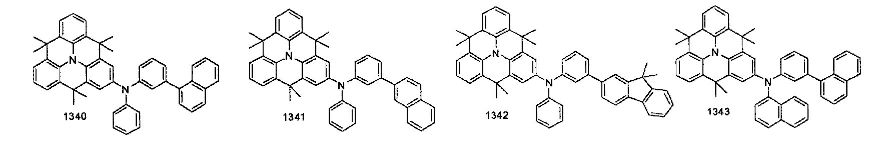

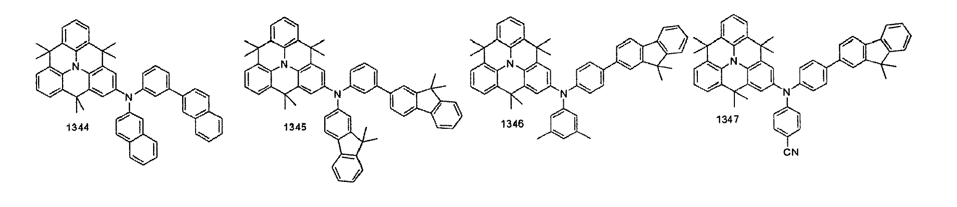

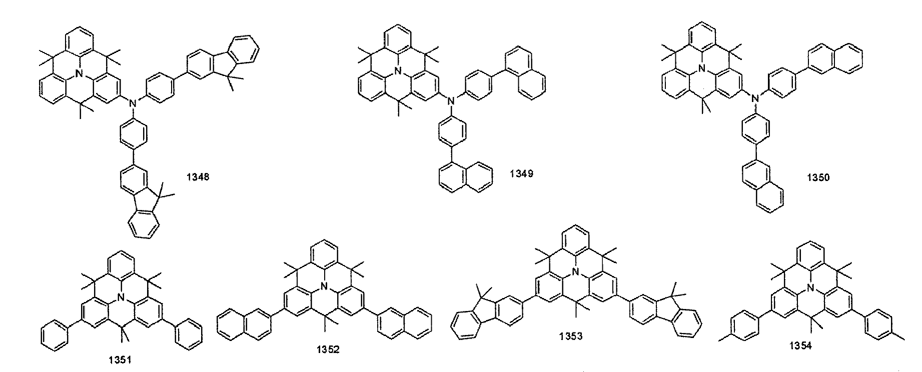

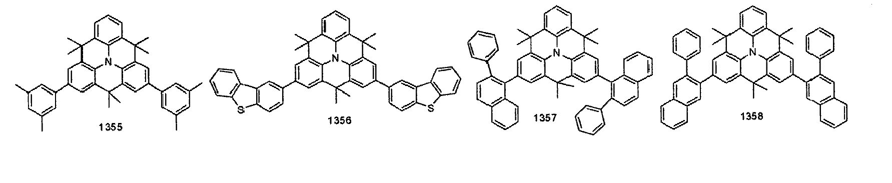

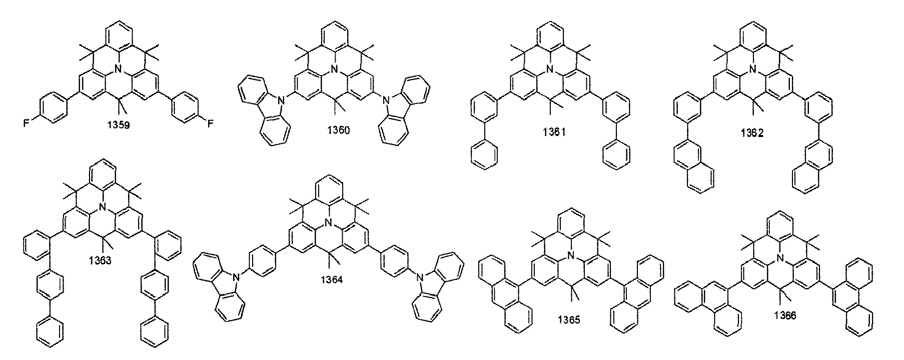

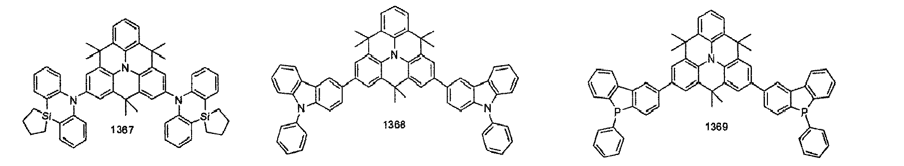

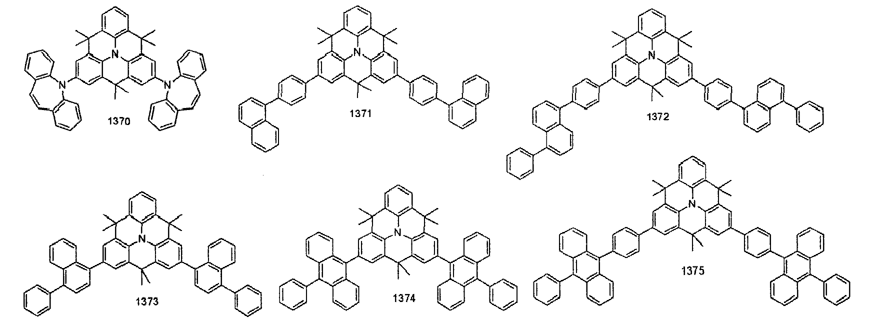

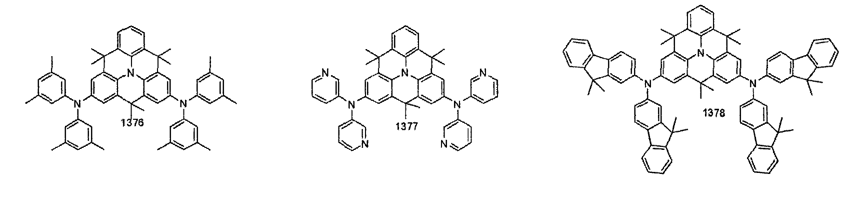

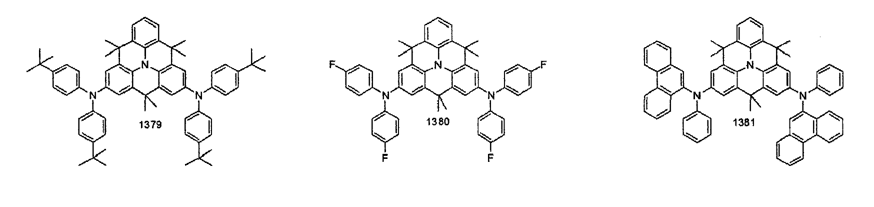

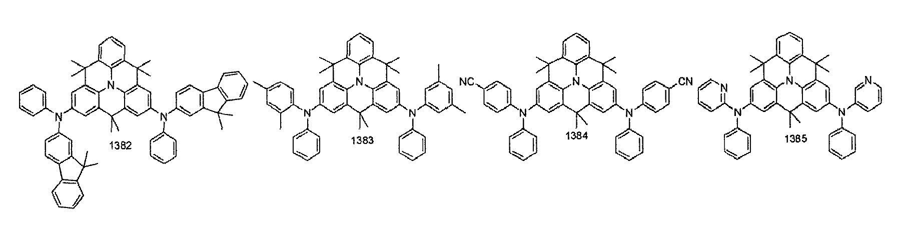

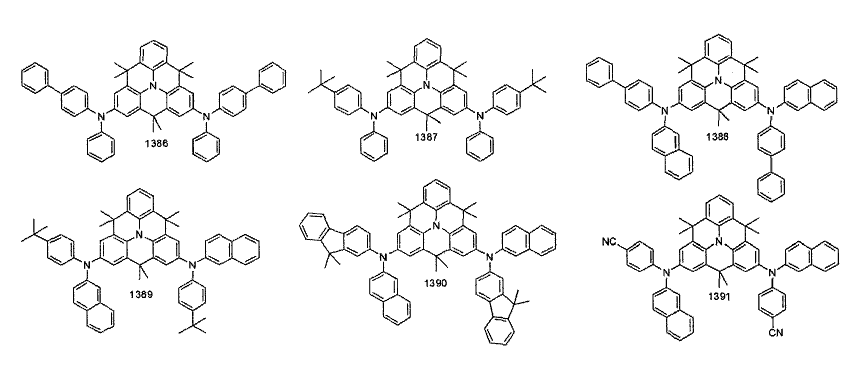

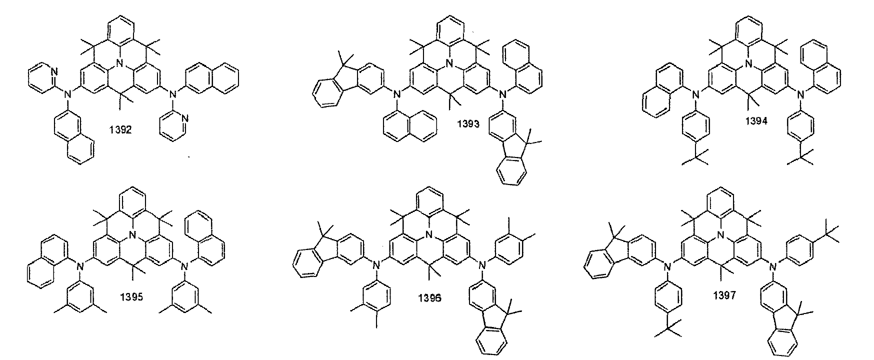

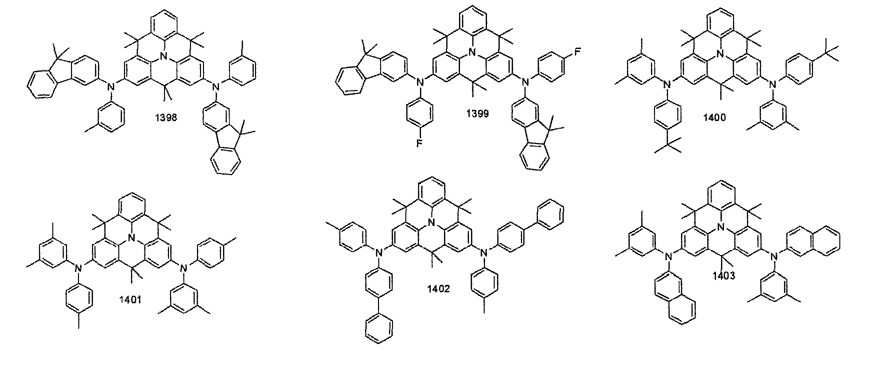

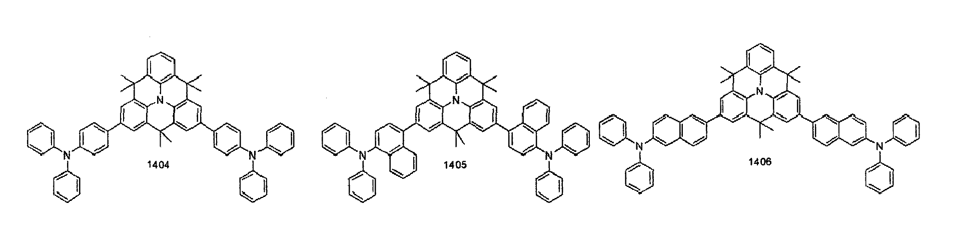

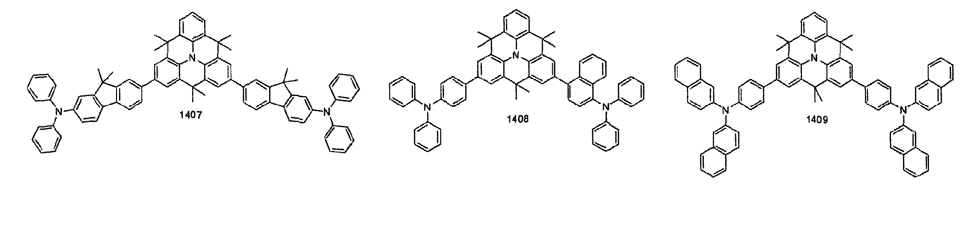

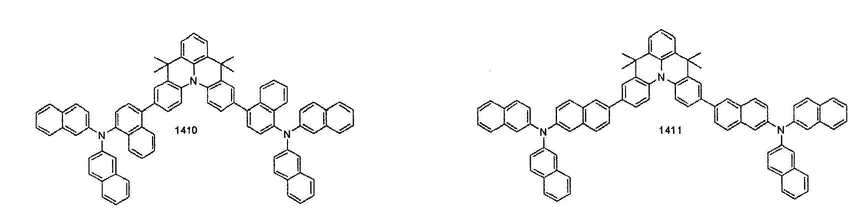

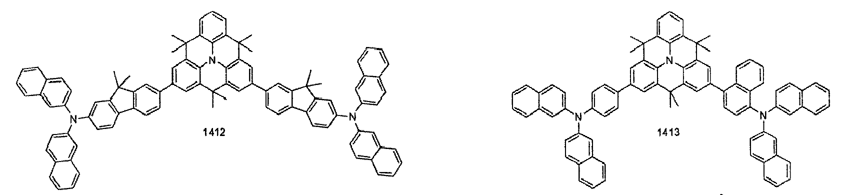

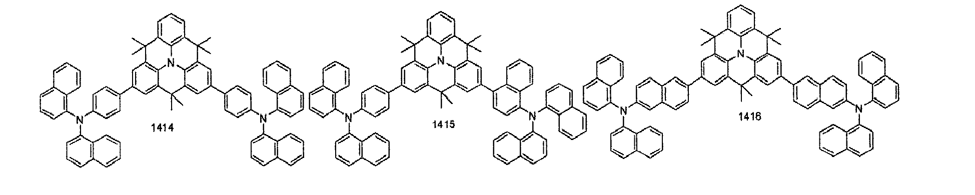

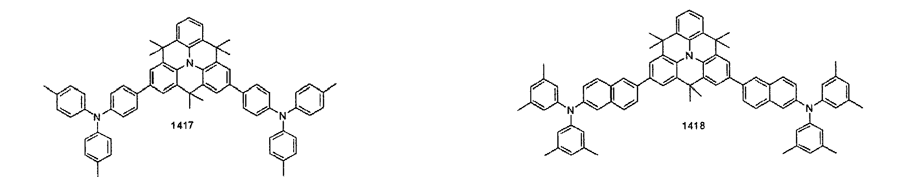

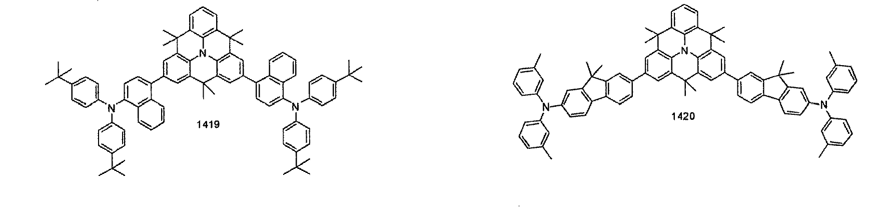

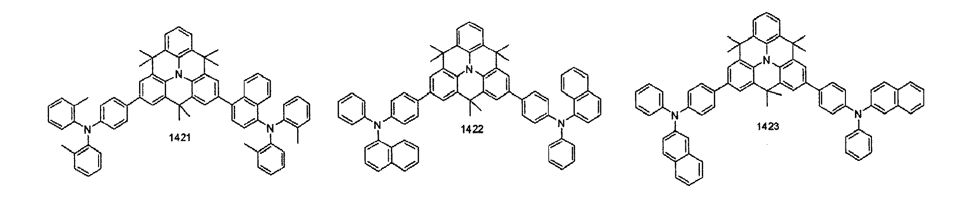

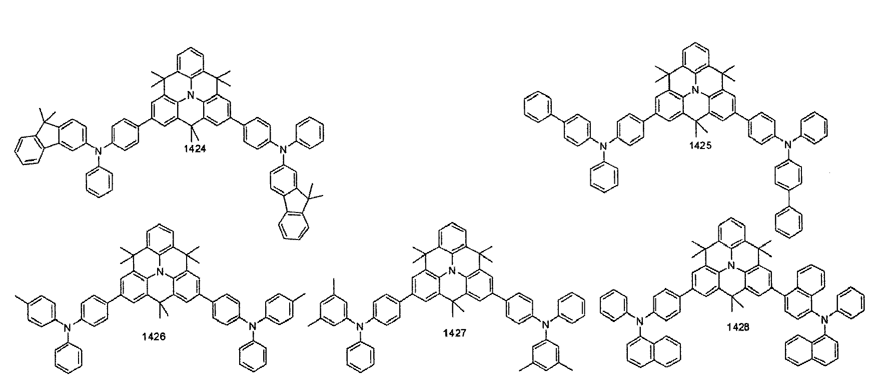

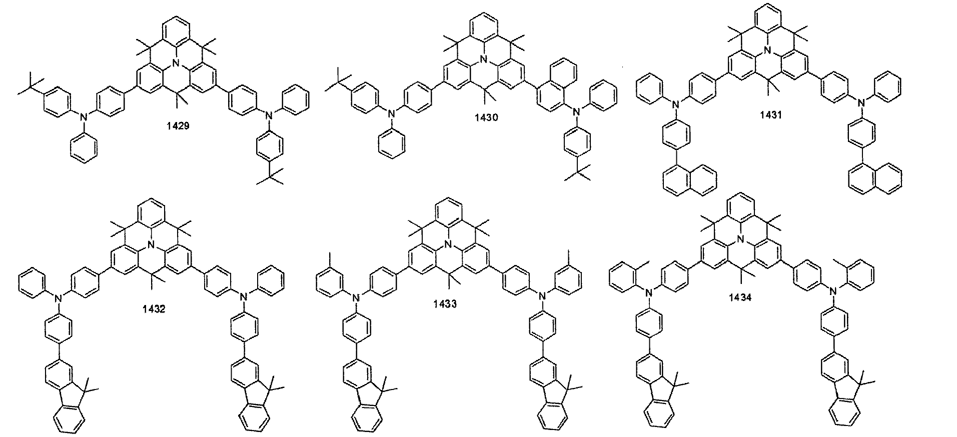

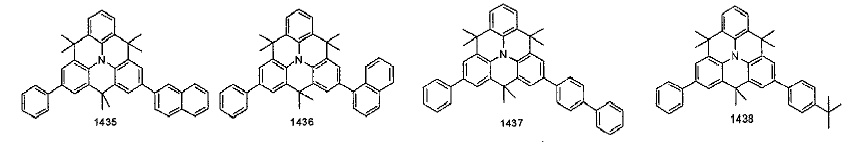

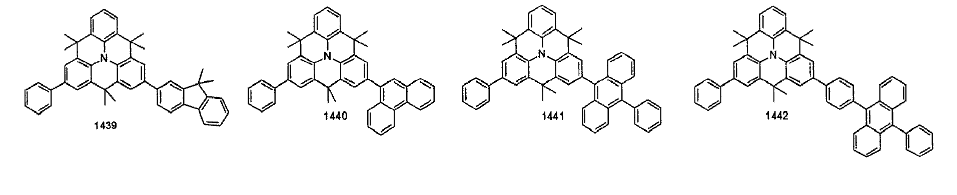

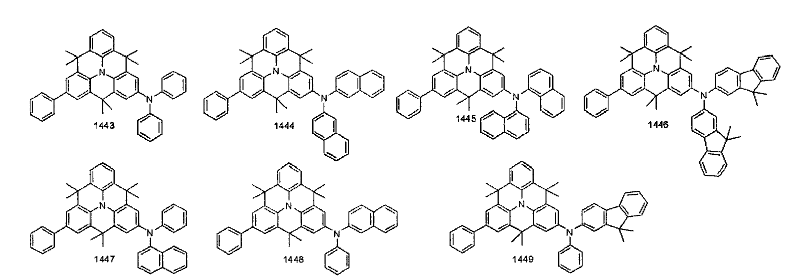

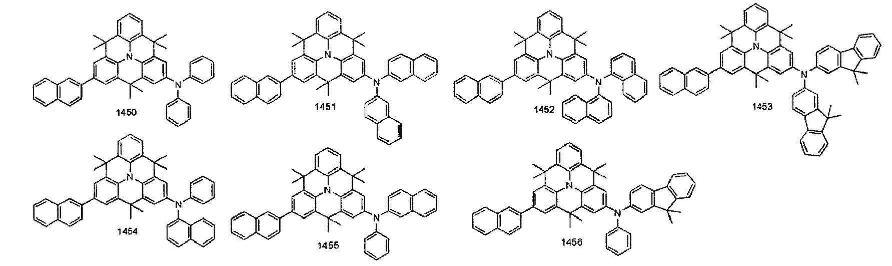

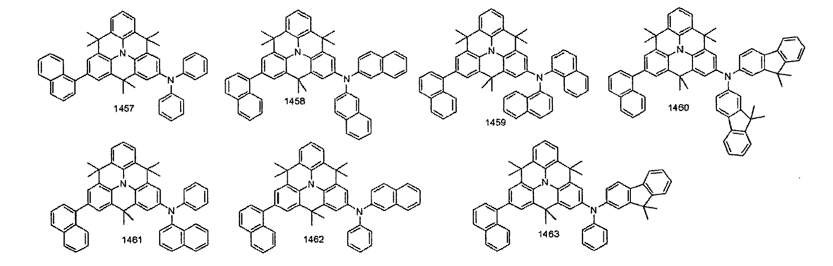

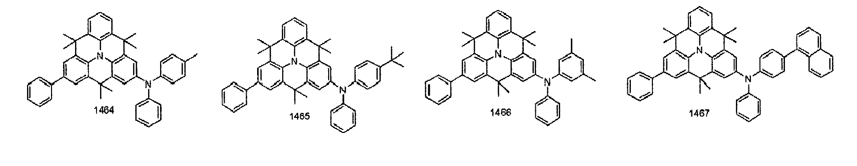

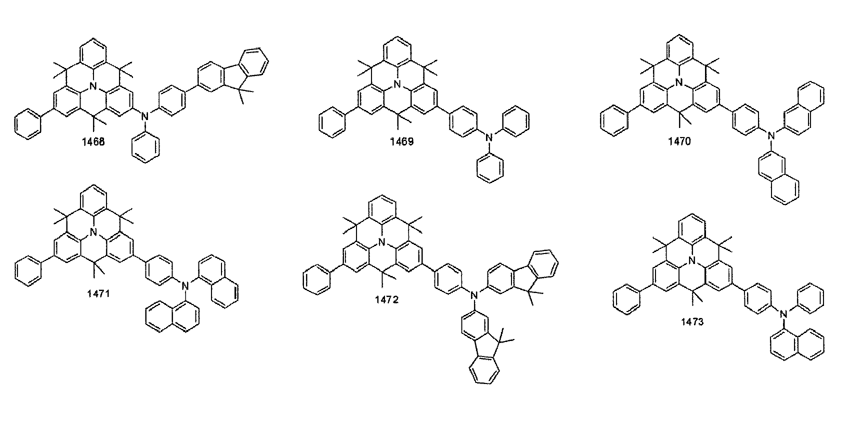

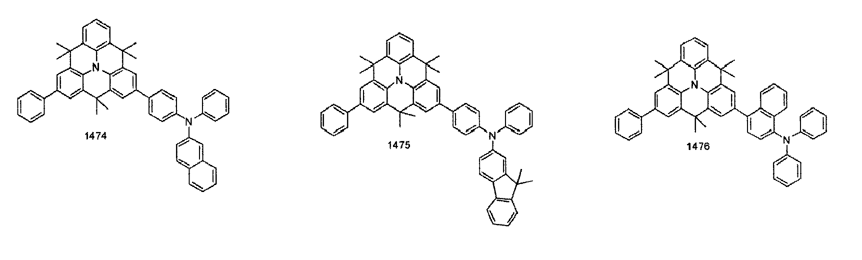

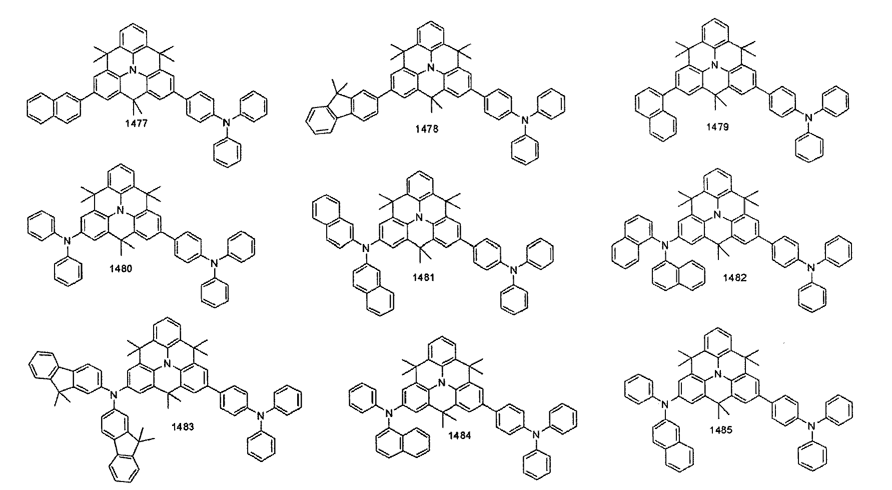

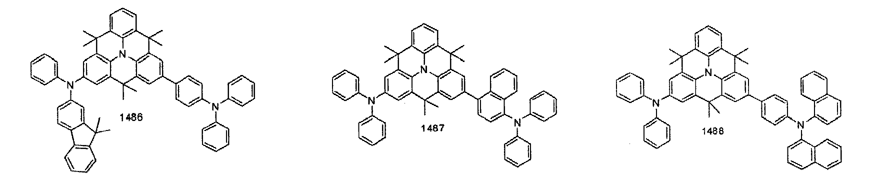

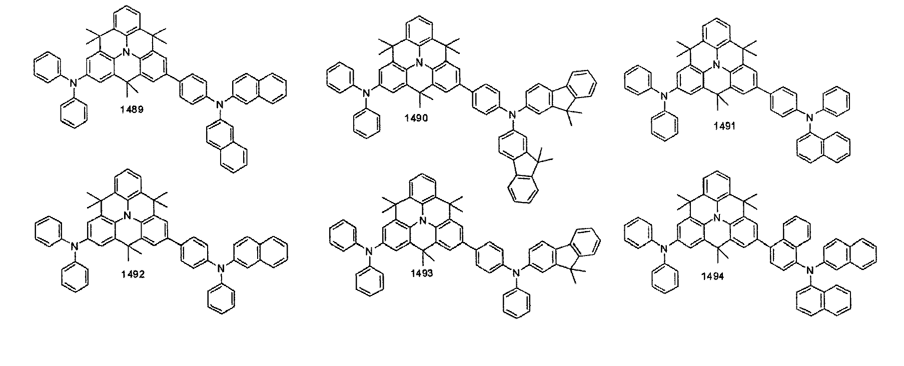

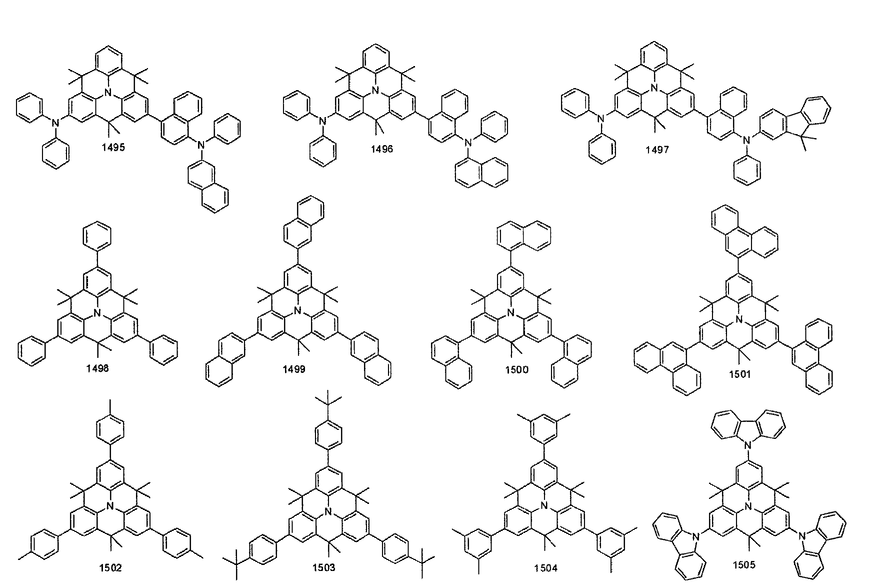

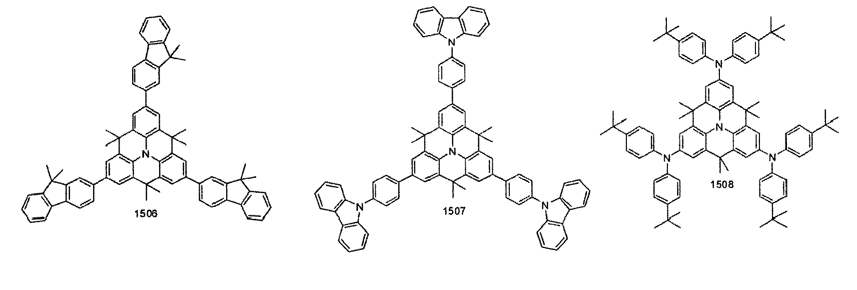

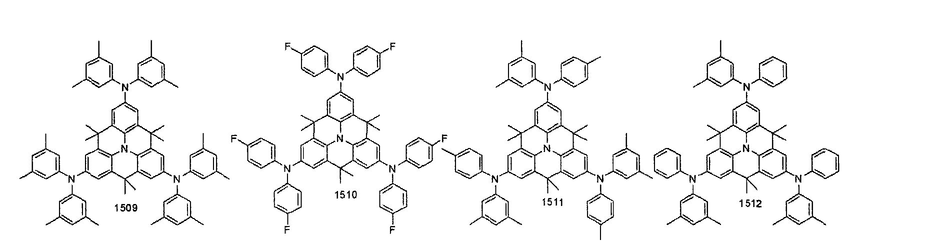

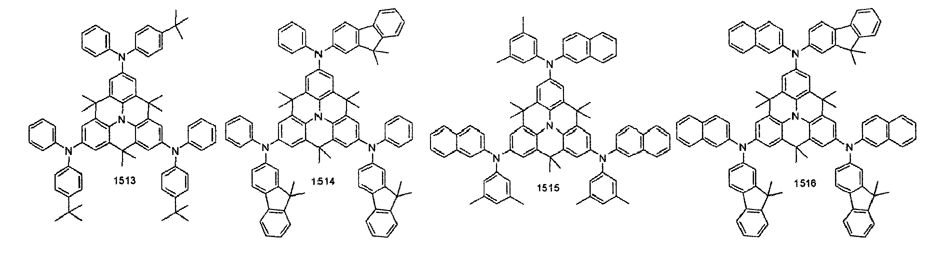

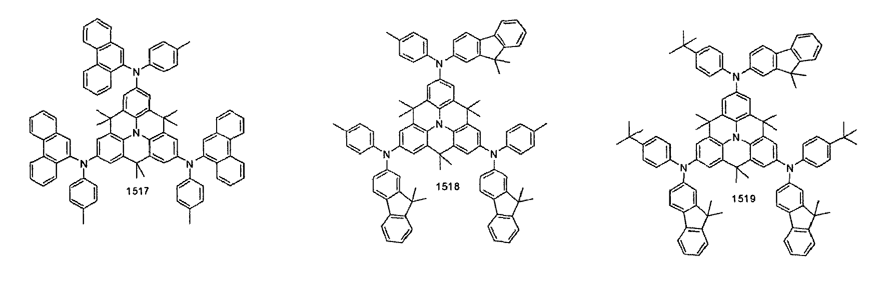

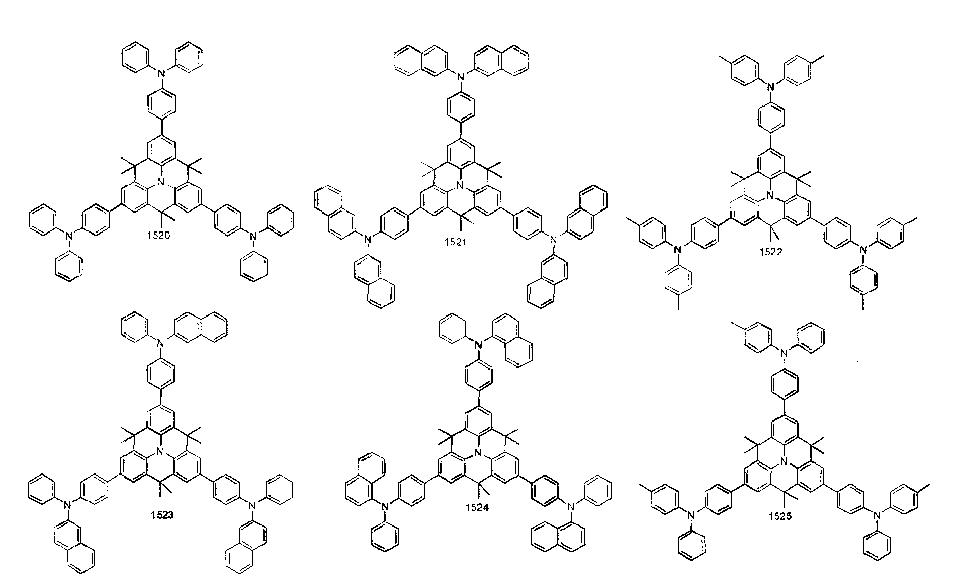

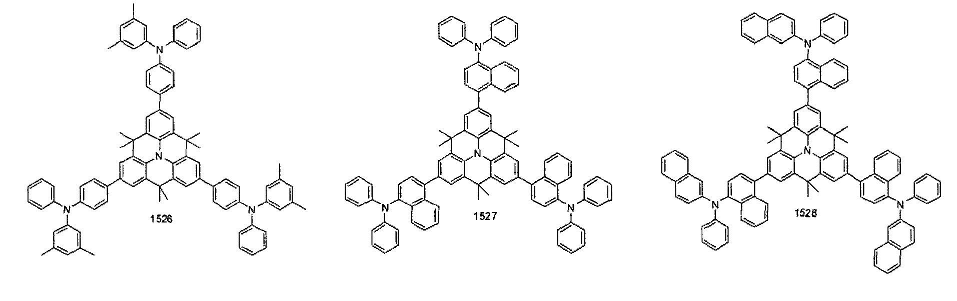

The compounds for organic electronic material according to the present invention can be prepared by a procedure as illustrated by Reaction Schemes (1): 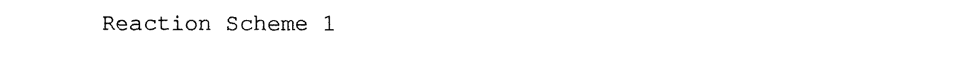 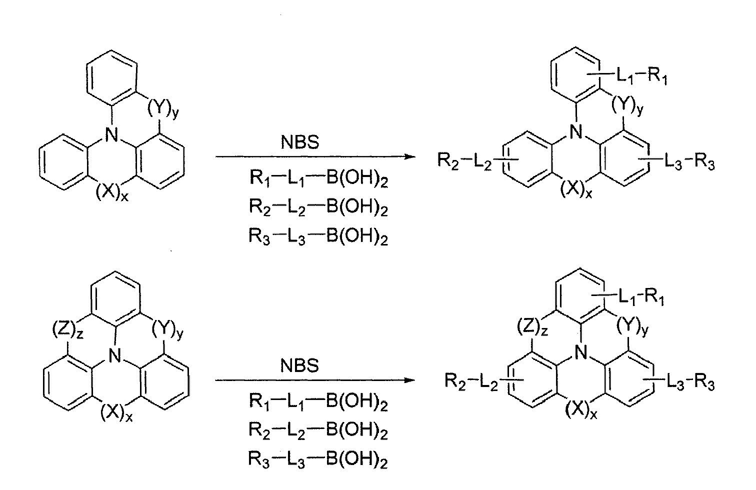 wherein, L₁, L₂, L₃, R₁, R₂, R₃, R₃₁ and R₃₂ are defined as in Chemical Formula (1) or (2).

The present invention also provides organic solar cells, which comprises one or more compound(s) for organic electronic material represented by Chemical Formula (1) or (2).

The present invention also provides an organic electronic device which is comprised of a first electrode; a second electrode; and at least one organic layer(s) interposed between the first electrode and the second electrode; wherein the organic layer comprises one or more compound(s) for organic electronic material represented by Chemical Formula (1) or (2). The compounds for organic electronic material may be used as material for forming a hole injecting layer or a hole transport layer, or host material for an electroluminescent layer.

The organic electronic device according to the present invention is **characterized in that** the organic layer comprises an electroluminescent layer, which comprises one or more dopant(s), in addition to one or more compound(s) for organic electronic material represented by Chemical Formula (1) or (2). The dopant to be applied to the organic electronic device according to the invention is not particularly restricted, but preferably selected from compounds represented by Chemical Formula (7):

Chemical Formula 7 **M¹L¹⁰¹L¹⁰²L¹⁰³**

wherein, M¹ is selected from a group consisting of metals of Group 7, 8, 9, 10, 11, 13, 14, 15 and 16 in the Periodic Table of Elements, and ligands L¹⁰¹, L¹⁰² and L¹⁰³ are independently selected from the following structures: 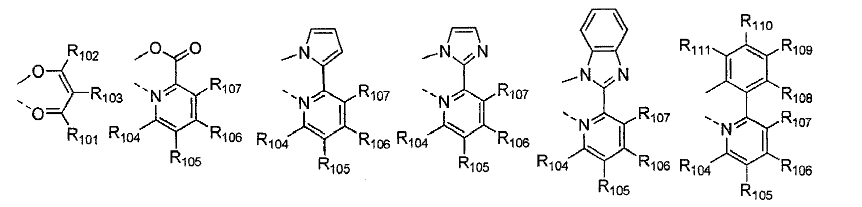 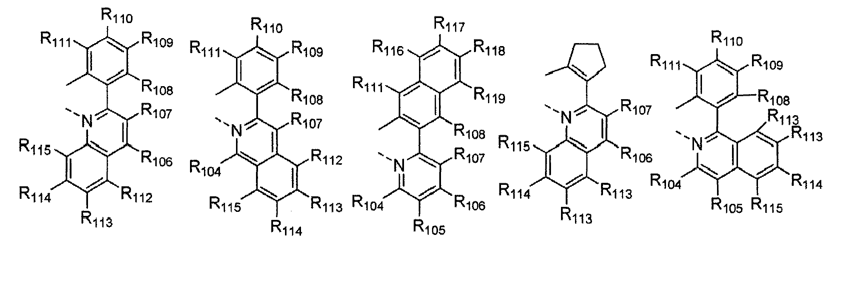 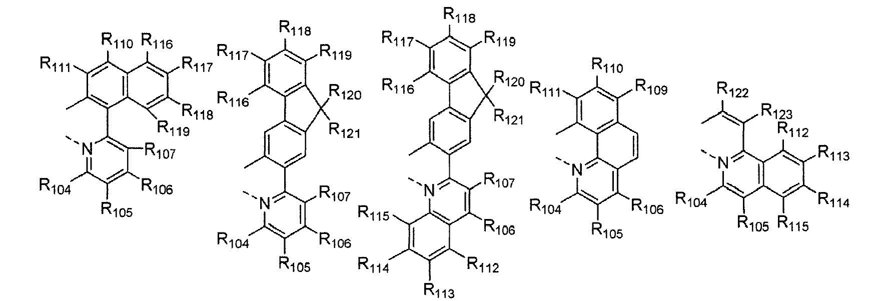 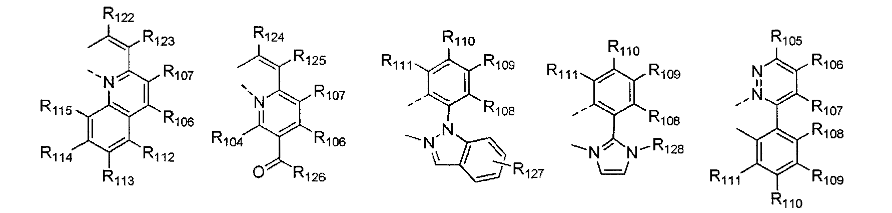 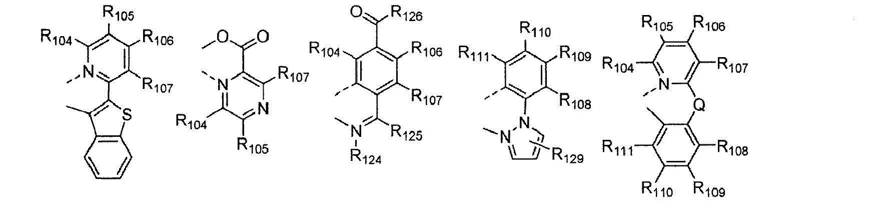
wherein, R₁₀₁ through R₁₀₃ independently represent hydrogen, deuterium, (C1-C60)alkyl with or without halogen substituent(s), (C6-C60)aryl with or without (C1-C60)alkyl substituent(s), or halogen;
R₁₀₄ through R₁₁₉ independently represent hydrogen, deuterium, (C1-C60)alkyl, (C1-C30)alkyloxy, (C3-C60)cycloalkyl, (C2-C30)alkenyl, (C6-C60)aryl, mono or di(C1-C30)alkylamino, mono or di(C6-C30)arylamino, SF₅, tri(C1-C30)alkylsilyl, di(C1-C30)alkyl(C6-C30)arylsilyl, tri(C6-C30)arylsilyl, cyano or halogen; the alkyl, cycloalkyl, alkenyl or aryl of R₁₀₄ through R₁₁₉ may be further substituted by one or more substituent(s) selected from deuterium, (C1-C60)alkyl, (C6-C60) aryl and halogen;
R₁₂₀ through R₁₂₃ independently represent hydrogen, deuterium, (C1-C60)alkyl with or without halogen substituent(s), or (C6-C60)aryl with or without (C1-C60)alkyl substituent(s);
R₁₂₄ and R₁₂₅ independently represent hydrogen, deuterium, (C1-C60)alkyl, (C6-C60)aryl or halogen, or R₁₂₄ and R₁₂₅ may be linked via (C3-C12)alkylene or (C3-C12)alkenylene with or without a fused ring to form an alicyclic ring, or a monocyclic or polycyclic aromatic ring; the alkyl or aryl of R₁₂₄ and R₁₂₅, or the alicyclic ring or the monocyclic or polycyclic aromatic ring formed therefrom by linkage via (C3-C12)alkylene or (C3-C12)alkenylene with or without a fused ring may be further substituted by one or more substituent(s) selected from deuterium, (C1-C60)alkyl with or without halogen substituent(s), (C1-C30)alkyloxy, halogen, tri(C1-C30)alkylsilyl, tri(C6-C30)arylsilyl and (C6-C60)aryl;
R₁₂₆ represents (C1-C60)alkyl, (C6-C60)aryl, (C5-C60)heteroaryl or halogen;
R₁₂₇ through R₁₂₉ independently represent hydrogen, deuterium, (C1-C60)alkyl, (C6-C60)aryl or halogen; the alkyl or aryl of R₁₂₆ through R₁₂₉ may be further substituted by halogen or (C1-C60)alkyl; and
Q represents 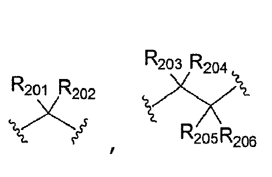 or 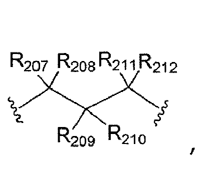 wherein R₂₀₁ through R₂₁₂ independently represent hydrogen, deuterium, (C1-C60)alkyl with or without halogen substituent(s), (C1-C30)alkyloxy, halogen, (C6-C60)aryl, cyano or (C5-C60)cycloalkyl, or each of R₂₀₁ through R₂₁₂ may be linked to an adjacent substituent via alkylene or alkenylene to form a (C5-C7)spiro ring or a (C5-C9)fused ring, or linked to R₁₀₇ or R₁₀₈ via alkylene or alkenylene to form a (C5-C7)fused ring.

The dopant compounds of Chemical Formula (7) can be exemplified by the following compounds, without restriction:

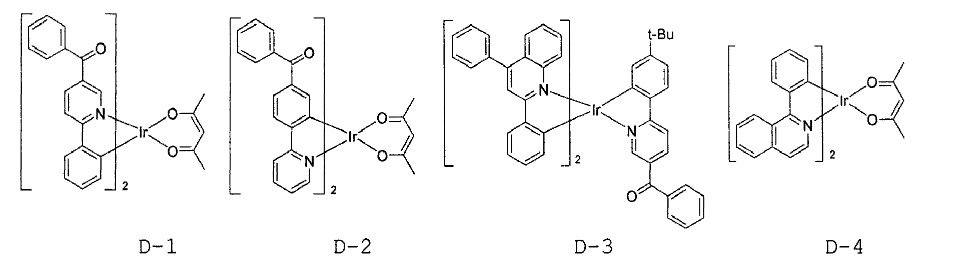

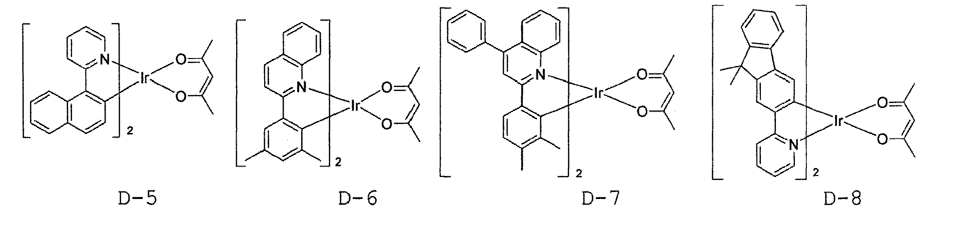

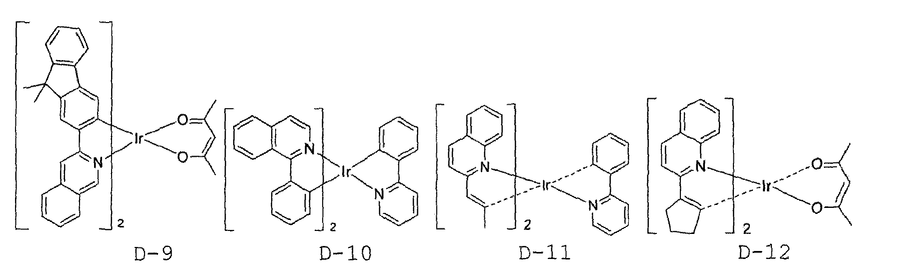

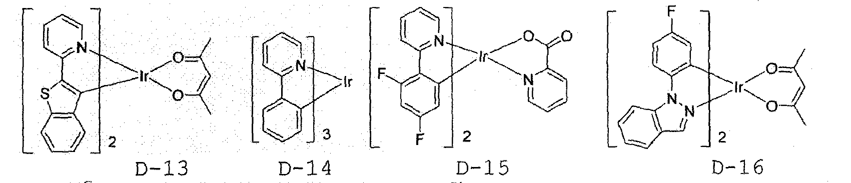

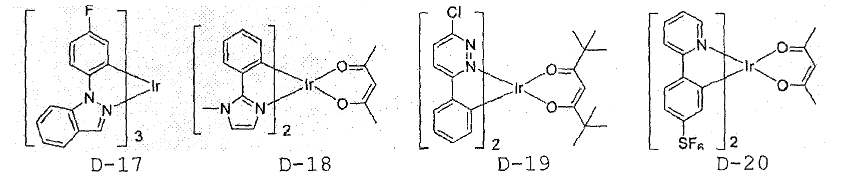

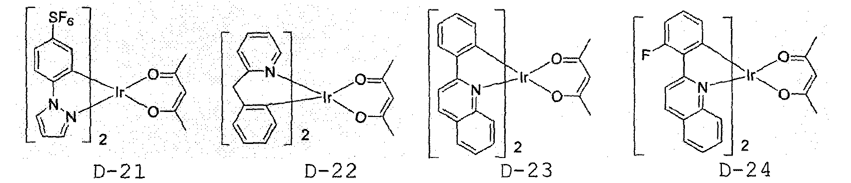

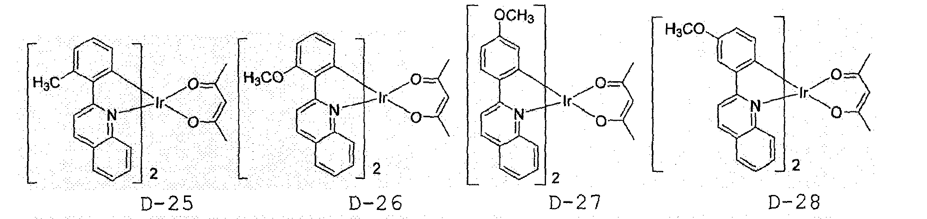

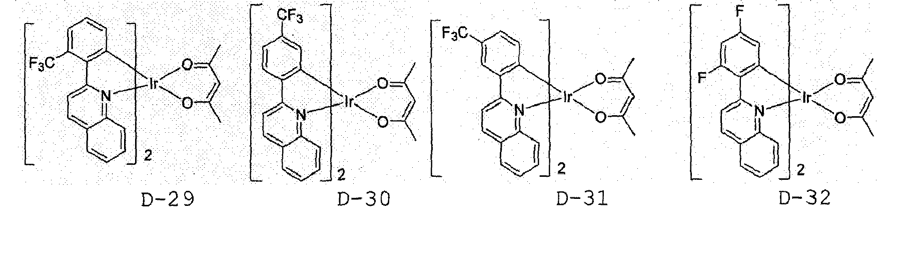

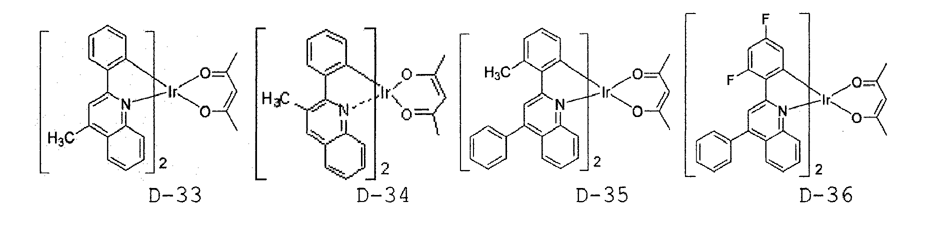

The organic electronic device according to the invention may further comprise one or more compound(s) selected from a group consisting of arylamine compounds and styrylarylamine compounds, in addition to the compound for organic electronic material represented by Chemical Formula (1) or (2). Examples of the arylamine or styrylarylamine compounds include the compounds represented by Chemical Formula (8), but they are not restricted thereto: 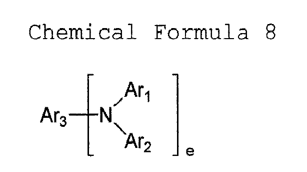
wherein, Ar₁ and Ar₂ independently represent (C1-C60)alkyl, (C6-C60)aryl, (C4-C60)heteroaryl, (C6-C60)arylamino, (C1-C60)alkylamino, morpholino, thiomorpholino, 5- or 6-membered heterocycloalkyl containing one or more heteroatom(s) selected from N, O and S, or (C3-C60)cycloalkyl, or Ar₁ and Ar₂ may be linked via (C3-C60)alkylene or (C3-C60)alkenylene with or without a fused ring to form an alicyclic ring, or a monocyclic or polycyclic aromatic ring;
the aryl, heteroaryl, arylamino or heterocycloalkyl of Ar₁ and Ar₂ may be further substituted by one or more substituent(s) selected from deuterium, halogen, (C1-C60)alkyl, (C2-C60)alkenyl, (C2-C60)alkynyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl;
when e is 1, Ar₃ represents (C6-C60)aryl, (C4-C60)heteroaryl, or a substituent selected from the following structures: 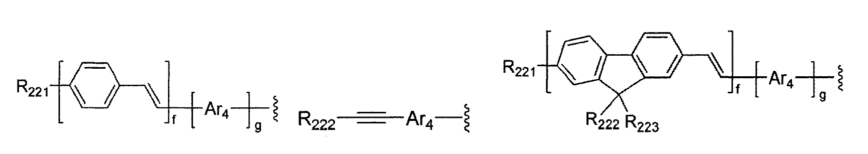
when e is 2, Ar₃ represents (C6-C60)arylene, (C4-C60)heteroarylene, or a substituent selected from the following structures: 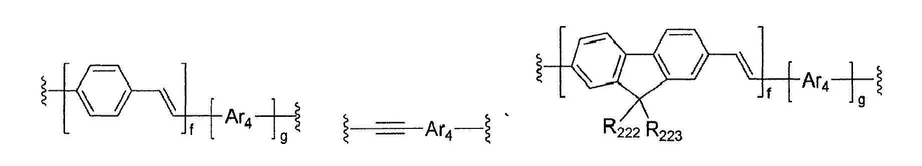
wherein Ar₄ represents (C6-C60)arylene or (C4-C60)heteroarylene;
R₂₂₁, R₂₂₂ and R₂₂₃ independently represent hydrogen, deuterium, (C1-C60)alkyl or (C6-C60)aryl;
f is an integer from 1 to 4, g is an integer of 0 or 1; and
the alkyl, aryl, heteroaryl, arylamino, alkylamino, cycloalkyl or heterocycloalkyl of Ar₁ and Ar₂; the aryl, heteroaryl, arylene or heteroarylene of Ar₃; the arylene or heteroarylene of Ar₄ and Ar₅; or the alkyl or aryl of R₂₂₁ through R₂₂₃ may be further substituted by one or more substituent(s) selected from a group consisting of deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

The arylamine compounds and styrylarylamine compounds can be more specifically exemplified by the following compounds, but are not restricted thereto.

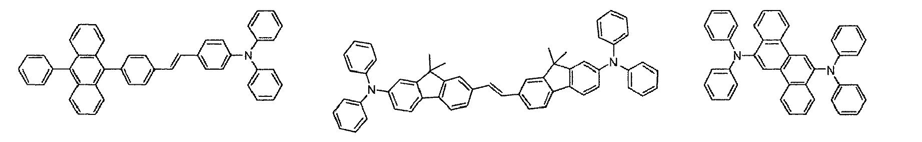

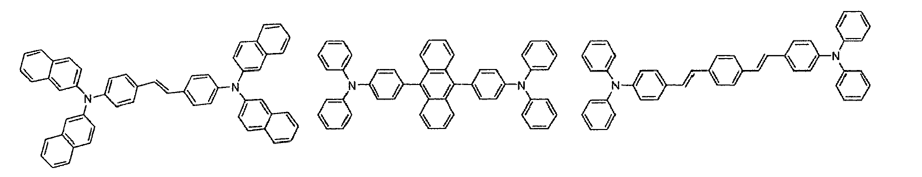

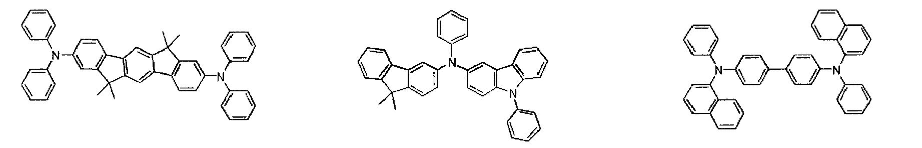

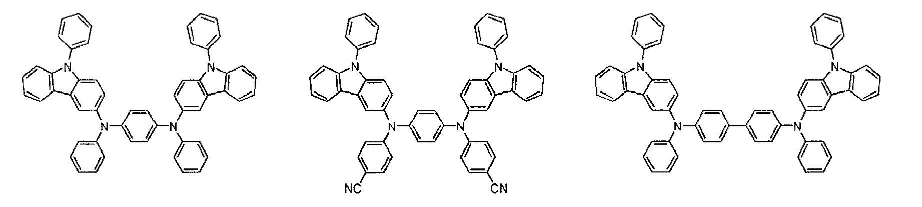

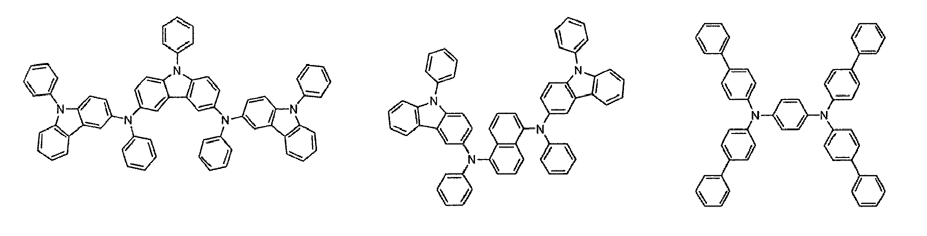

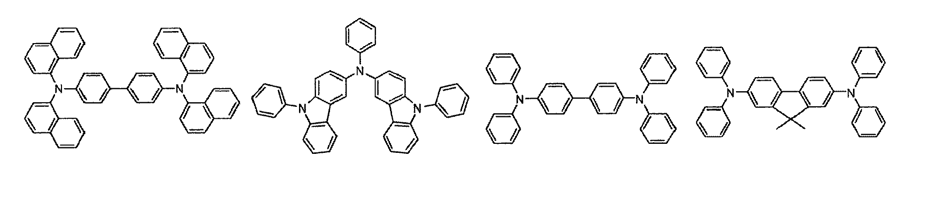

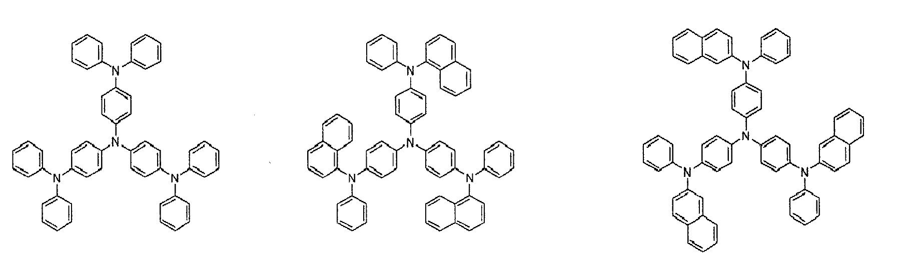

In an organic electronic device according to the present invention, the organic layer may further comprise one or more metal(s) selected from a group consisting of organometals of Group 1, Group 2, 4^{th} period and 5^{th} period transition metals, lanthanide metals and d-transition elements in the Periodic Table of Elements, in addition to the compounds for organic electronic material represented by Chemical Formula (1) or (2). The organic layer may comprise an electroluminescent layer and a charge generating layer at the same time.

The present invention can realize an organic electronic device having a pixel structure of independent light-emitting mode, which comprises an organic electronic device containing the compound for organic electronic material of Chemical Formula (1) or (2) as a sub-pixel and one or more sub-pixel(s) comprising one or more metallic compound(s) selected from a group consisting of Ir, Pt, Pd, Rh, Re, Os, Tl, Pb, Bi, In, Sn, Sb, Te, Au and Ag, patterned in parallel at the same time.

The organic electronic device may comprise one or more compound(s) selected from compounds having electroluminescent peak of wavelength of blue, green or red in addition to the organic electroluminescent compound in the organic layer to form a white electroluminescent device. The compounds having electroluminescent peak of wavelength of blue, green or red can be exemplified by those represented by one of Chemical Formulas (9) to (12), but they are not restricted thereto.

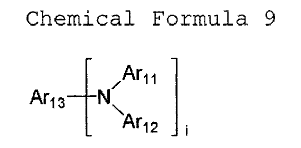

In Chemical Formula (9), Ar₁₁ and Ar₁₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or Ar₁₁ and Ar₁₂ may be linked via (C3-C60)alkylene or (C3-C60)alkenylene with or without a fused ring to form an alicyclic ring, or a monocyclic or polycyclic aromatic ring;
when i is 1, Ar₁₃ represents (C6-C60)aryl, (C4-C60)heteroaryl, or a substituent selected from the following structures: 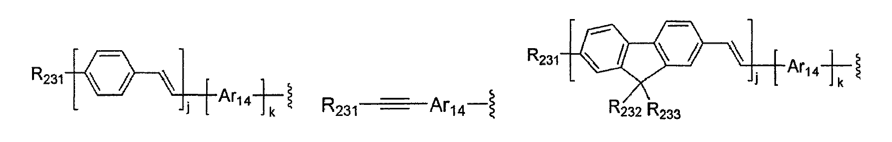
when i is 2, Ar₁₃ represents (C6-C60)arylene, (C4-C60)heteroarylene, or a substituent selected from the following structures: 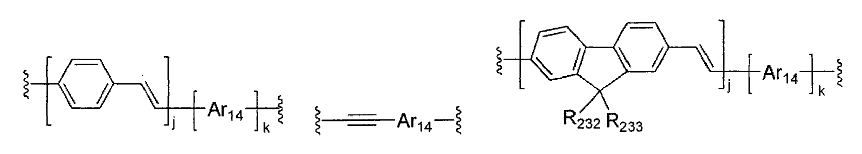
wherein Ar₁₄ represents (C6-C60)arylene or (C4-C60)heteroarylene;
R₂₃₁ through R₂₃₃ independently represent hydrogen, deuterium, (C1-C60)alkyl or (C6-C60)aryl;
j is an integer from 1 to 4, k is an integer of 0 or 1; and
the alkyl, aryl, heteroaryl, arylamino, alkylamino, cycloalkyl or heterocycloalkyl of Ar₁₁ and Ar₁₂; the aryl, heteroaryl, arylene or heteroarylene of Ar₁₃; the arylene or heteroarylene of Ar₁₄; or the alkyl or aryl of R₂₃₁ through R₂₃₃ may be further substituted by one or more substituent(s) selected from a group consisting of deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

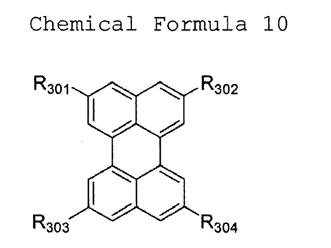

In Chemical Formula (10), R₃₀₁ through R₃₀₄ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₃₀₁ through R₃₀₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino or arylamino of R₃₀₁ through R₃₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

Chemical Formula 11 (Ar₃₁)ₚ-L₁₁-(Ar₃₂)_{q}

Chemical Formula 12 (Ar₃₃)ᵣ-L₁₂-(Ar₃₄)ₛ

In Chemical Formulas (11) and (12),
L₁₁ represents (C6-C60)arylene or (C4-C60)heteroarylene;
L₁₂ represents anthracenylene;
Ar₃₁ through Ar₃₄ are independently selected from hydrogen, deuterium, (C1-C60)alkyl, (C1-C60)alkoxy, halogen, (C4-C60)heteroaryl, (C5-C60)cycloalkyl and (C6-C60)aryl; the cycloalkyl, aryl or heteroaryl of Ar₃₁ through Ar₃₄ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsily di(C1-C60)alkyl(C6-C60)arylsilyl and tri(C6-C60)arylsilyl; and
p, q, r and s independently represent an integer from 0 to 4.

The compounds represented by Chemical Formula (11) or (12) can be exemplified by anthracene derivatives or benz[a]anthracene derivatives represented by one of Chemical Formulas (13) to (16).

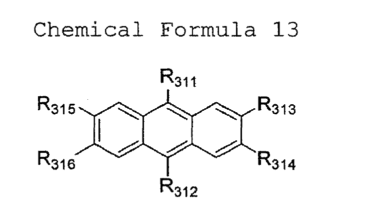

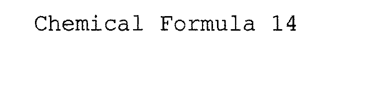

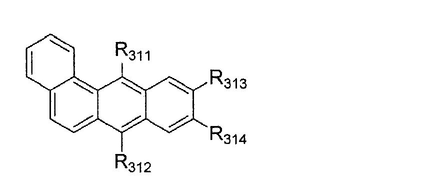

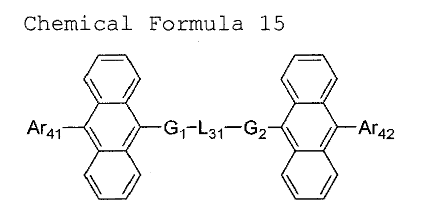

In Chemical Formulas (13) through (15),
R₃₁₁ and R₃₁₂ independently represent (C6-C60)aryl, (C4-C60)heteroaryl, 5- or 6-membered heterocycloalkyl containing one or more heteroatom(s) selected from N, O and S, or (C3-C60)cycloalkyl; the aryl or heteroaryl of R₃₁₁ and R₃₁₂ may be further substituted by one or more substituent(s) selected from a group consisting of deuterium, (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₃₁₃ through R₃₁₆ independently represent hydrogen, deuterium, (C1-C60)alkyl, (C1-C60)alkoxy, halogen, (C4-C60)heteroaryl, (C5-C60)cycloalkyl or (C6-C60)aryl, and the heteroaryl, cycloalkyl or aryl of R₃₁₃ through R₃₁₆ may be further substituted by one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
G₁ and G₂ independently represent a chemical bond, or (C6-C60)arylene with or without one or more substituent(s) selected from (C1-C60)alkyl, (C1-C60)alkoxy, (C6-C60)aryl, (C4-C60)heteroaryl and halogen;
Ar₄₁ and Ar₄₂ represent (C4-C60)heteroaryl or aryl selected from the following structures: 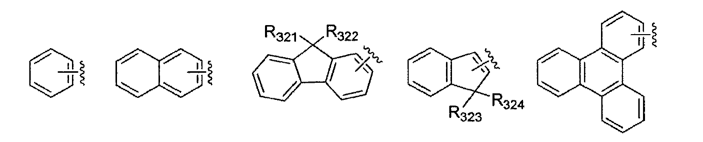 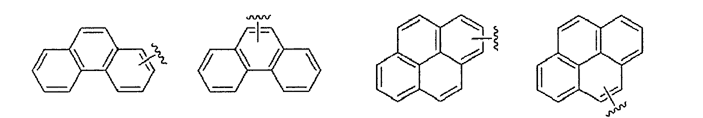 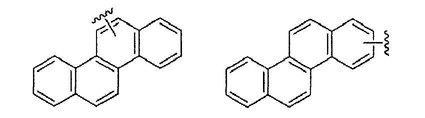
the aryl or heteroaryl of Ar₄₁ and Ar₄₂ may be substituted by one or more substituent(s) selected from deuterium, (C1-C60)alkyl, (C1-C60)alkoxy, (C6-C60)aryl and (C4-C60)heteroaryl;
L₃₁ represents (C6-C60)arylene, (C4-C60)heteroarylene or a group represented by the following structural formula: 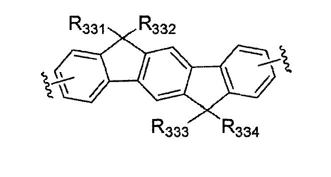
the arylene or heteroarylene of L₃₁ may be substituted by one or more substituent(s) selected from deuterium, (C1-C60)alkyl, (C1-C60)alkoxy, (C6-C60)aryl, (C4-C60)heteroaryl and halogen;
R₃₂₁ through R₃₂₄ independently represent hydrogen, deuterium, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and
R₃₃₁ through R₃₃₄ independently represent hydrogen, deuterium, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

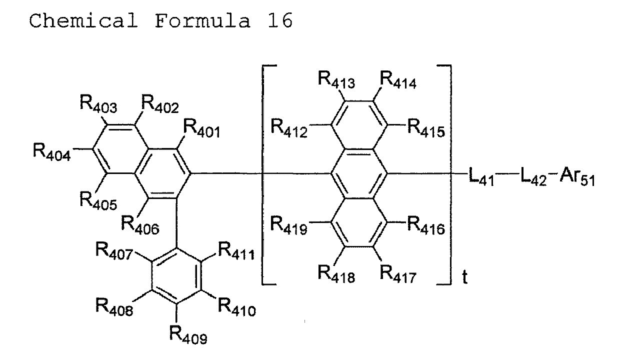

In Chemical Formula (16), L₄₁ and L₄₂ independently represent a chemical bond, (C6-C60)arylene, (C3-C60)heteroarylene; the arylene or heteroarylene of L₄₁ and L₄₂ may be further substituted by one or more substituent(s) selected from deuterium, (C1-C60)alkyl, halogen, cyano, (C1-C60)alkoxy, (C3-C60)cycloalkyl, (C6-C60)aryl, (C3-C60)heteroaryl, tri(C1-C30)alkylsilyl, di(C1-C30)alkyl(C6-C30)arylsilyl and tri(C6-C30)arylsilyl;
R₄₀₁ through R₄₁₉ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamzio, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₄₀₁ through R₄₁₉ may be linked to an adjacent substituent via (C3-C60)alkylene or (C3-C60)alkenylene with or without a fused ring to form an alicyclic ring, or a monocyclic or polycyclic aromatic ring;
Ar₅₁ represents (C6-C60)aryl, (C4-C60)heteroaryl, 5- or 6-membered heterocycloalkyl containing one or more heteroatom(s) selected from N, O and S, (C3-C60)cycloalkyl, adamantyl, (C7-C60)bicycloalkyl, or a substituent selected from the following structures: 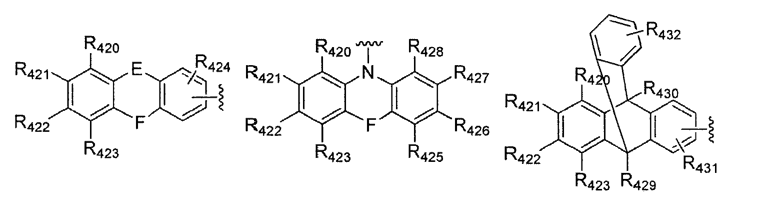
wherein, R₄₂₀ through R₄₃₂ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-c60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
E and F independently represent a chemical bond, - (CR₄₃₃R₄₃₄)_{w}-, -N(R₄₃₅)-, -S-, -O-, -Si(R₄₃₆) (R₄₃₇)-, -P(R₄₃₈)-, - C(=O)-, -B(R₄₃₉)-, -In(R₄₄₀)-, -Se-, -Ge(R₄₄₁) (R₄₄₂)-, - Sn(R₄₄₃)(R₄₄₄)-, -Ga(R₄₄₅)- or -(R₄₄₆)C=C(R₄₄₇)-;
R₄₃₃ through R₄₄₅ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-c60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; or each of R₄₃₃ through R₄₄₅ may be linked to an adjacent substituent via (C3-C60)alkylene or (C3-C60)alkenylene with or without a fused ring to form an alicyclic ring, or a monocyclic or polycyclic aromatic ring;
the aryl, heteroaryl, heterocycloalkyl, adamantyl or bicycloalkyl of Ar₅₁; or the alkyl, alkenyl, alkynyl, cycloalkyl, heterocycloalkyl, aryl, heteroaryl, arylsilyl, alkylsilyl, alkylamino or arylamino of R₄₀₁ through R₄₄₅ may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60) alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
t and w independently represent an integer from 1 to 4.

The compounds having electroluminescent peak of wavelength of blue, green or red color, to be contained in the electroluminescent layer can be exemplified by the following compounds, without restriction.

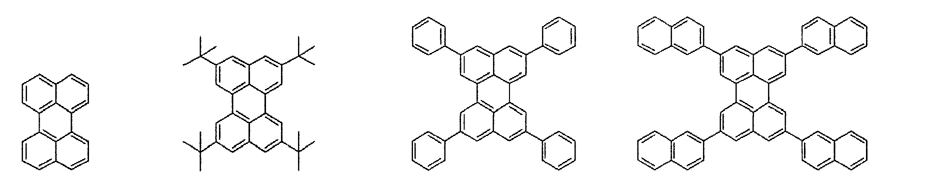

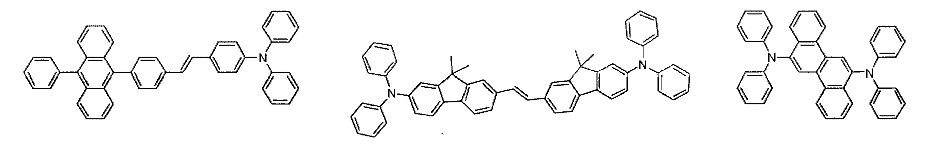

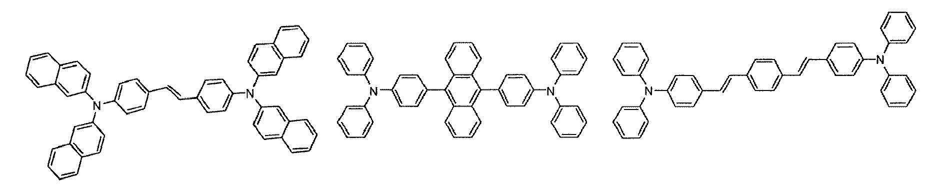

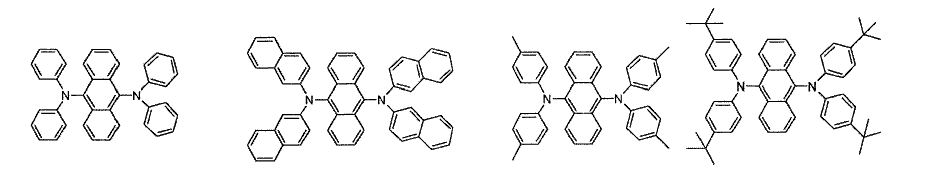

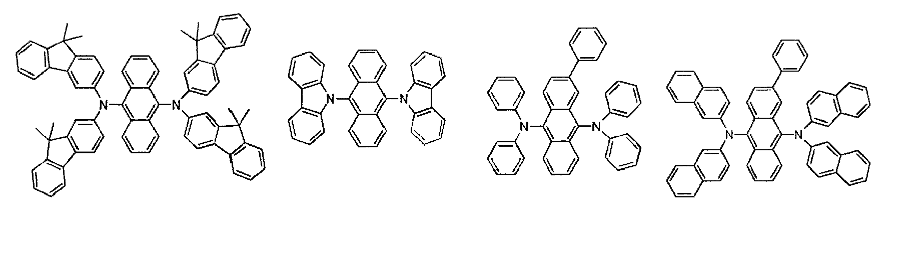

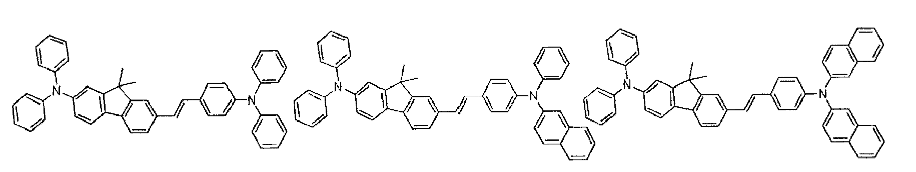

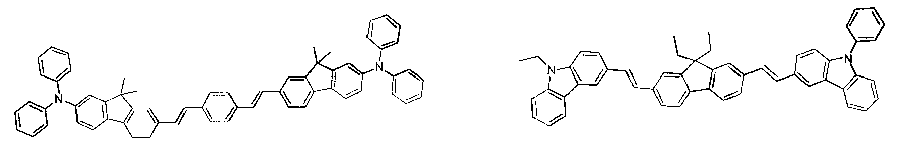

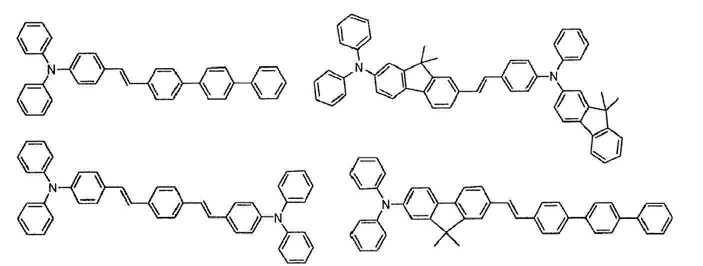

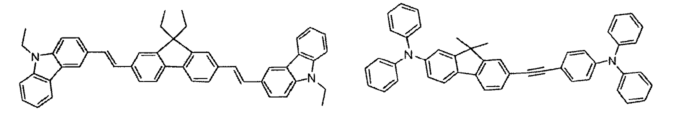

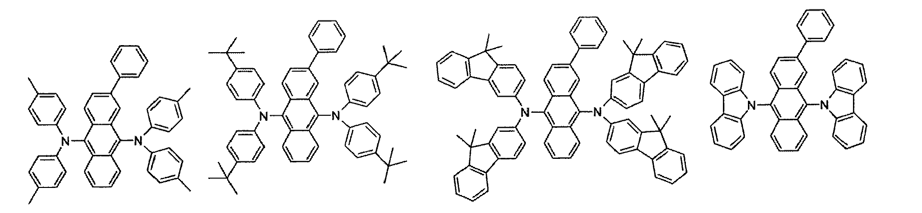

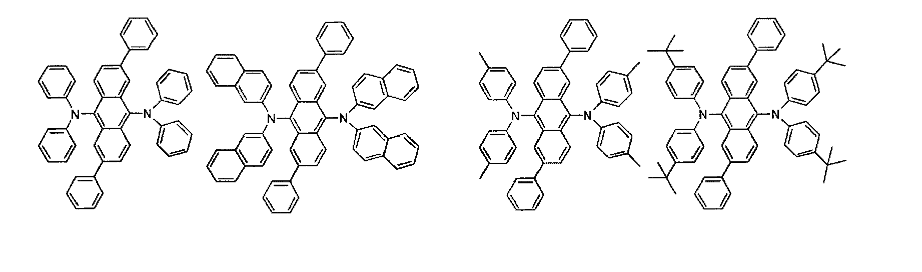

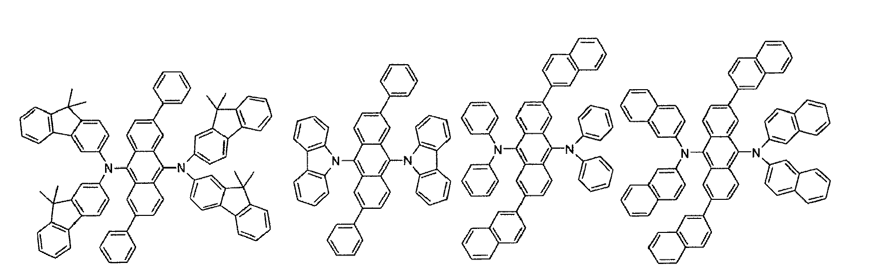

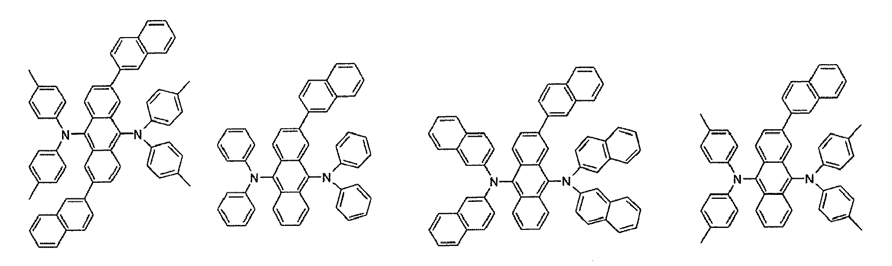

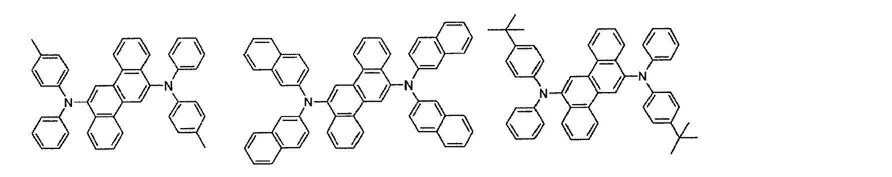

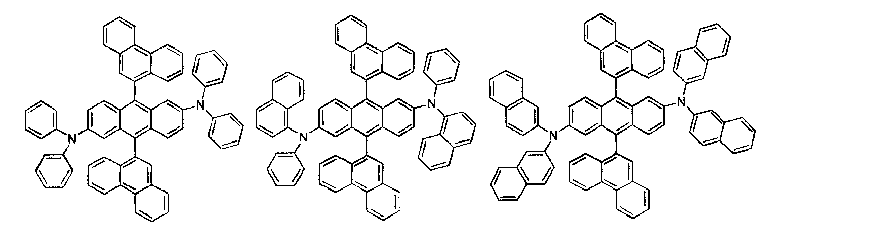

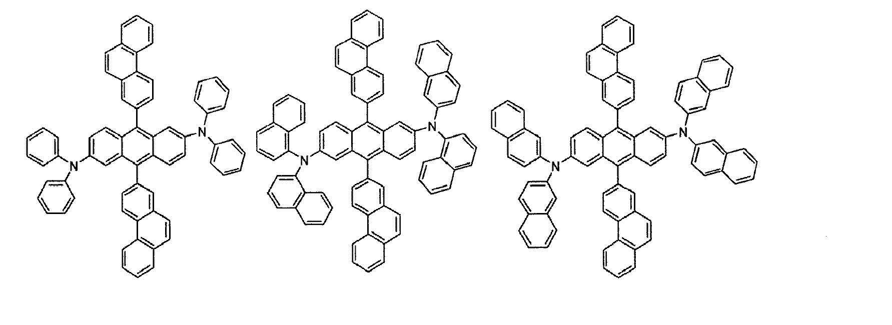

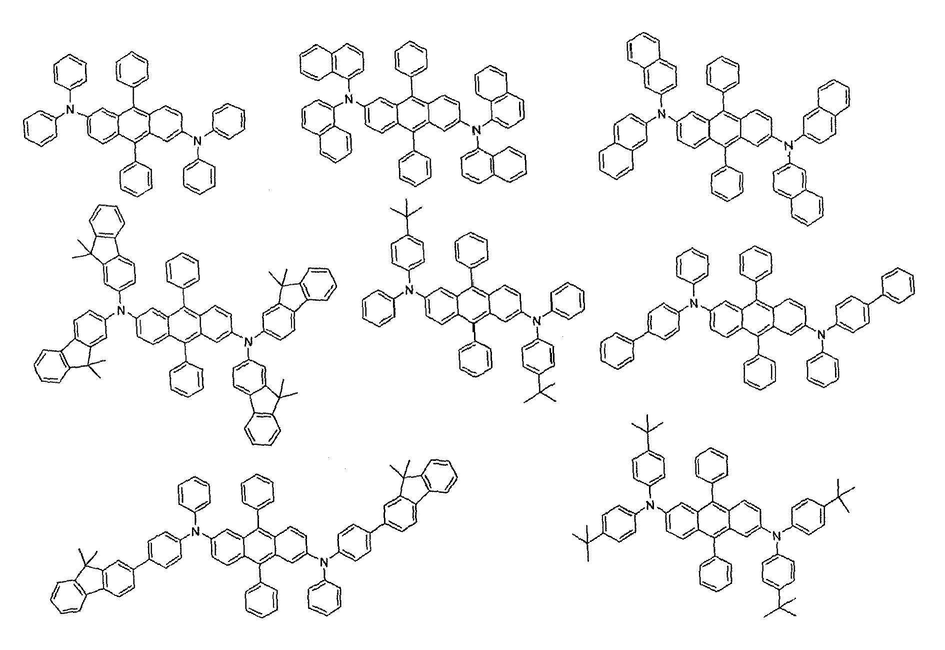

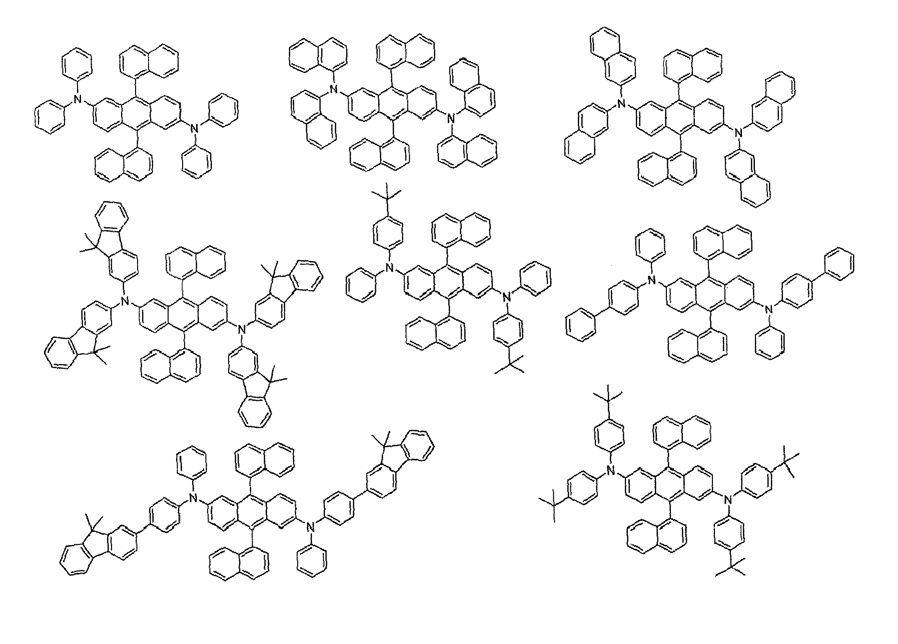

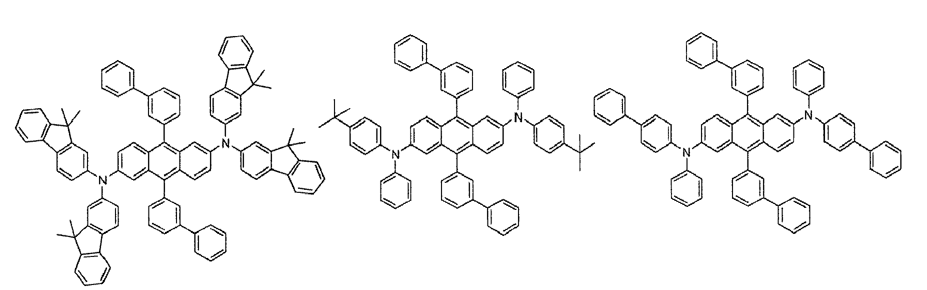

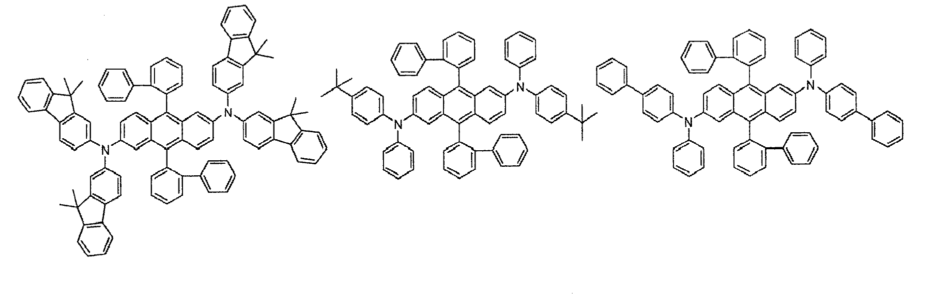

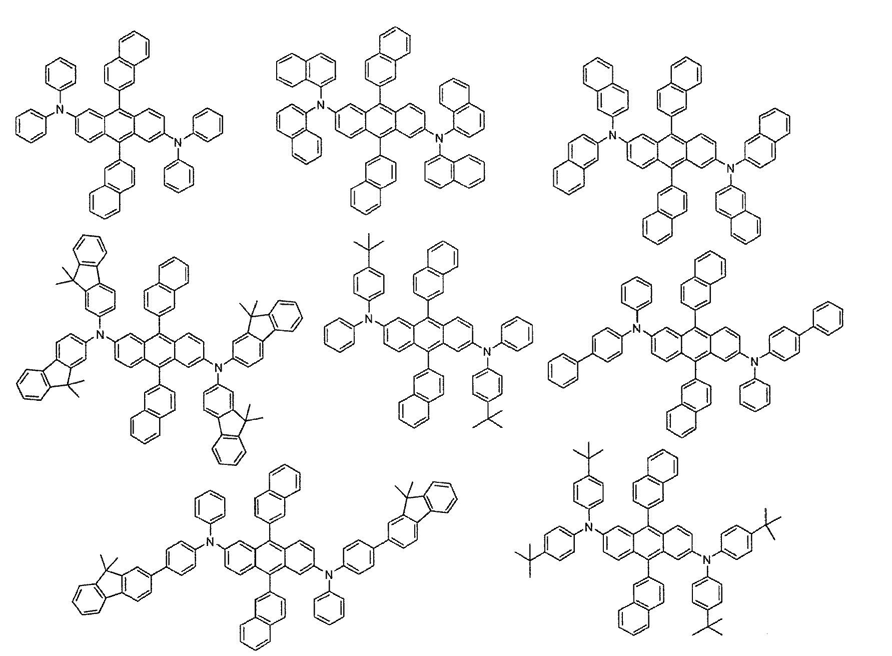

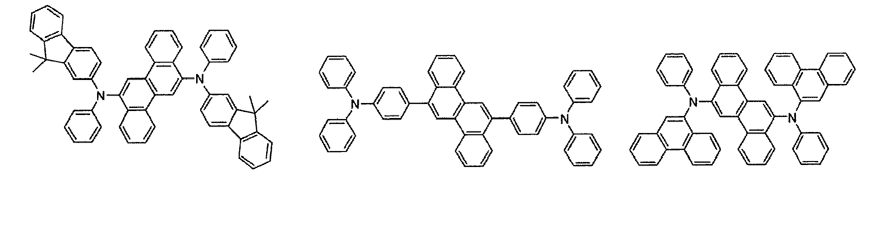

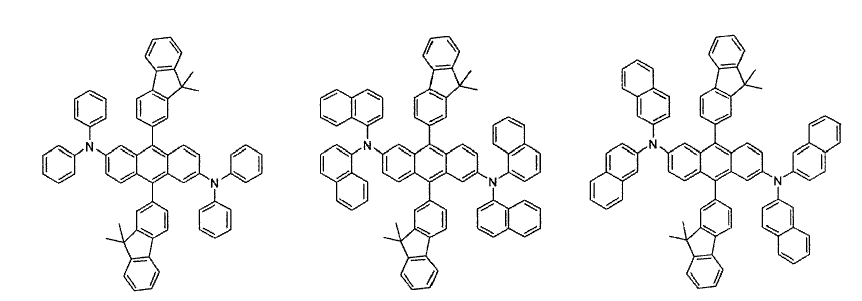

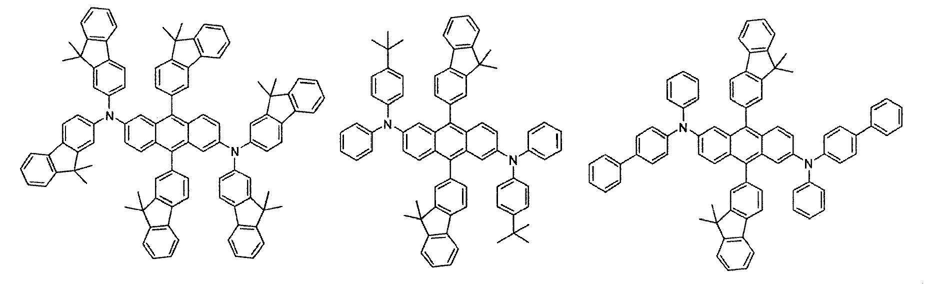

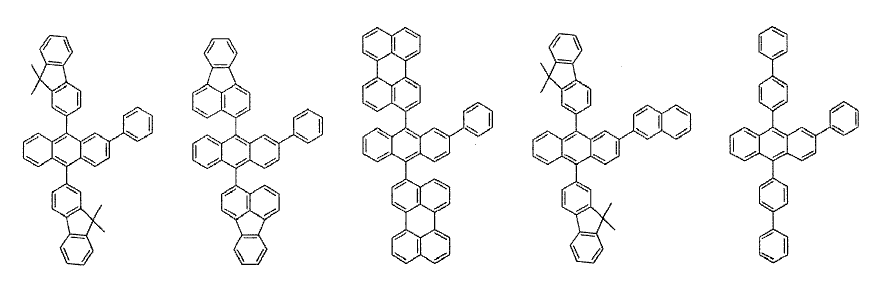

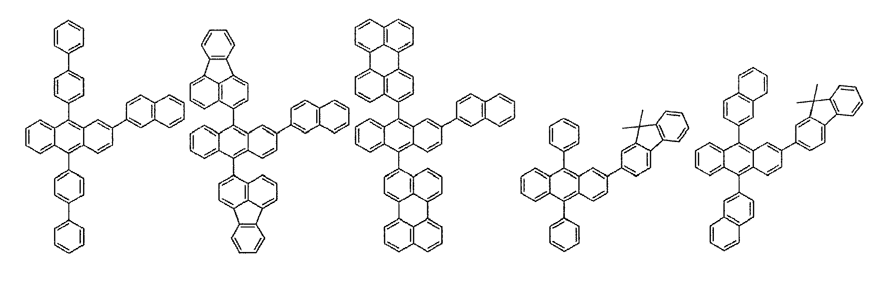

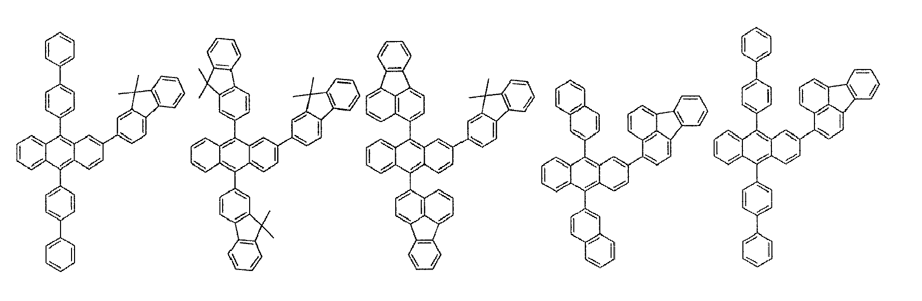

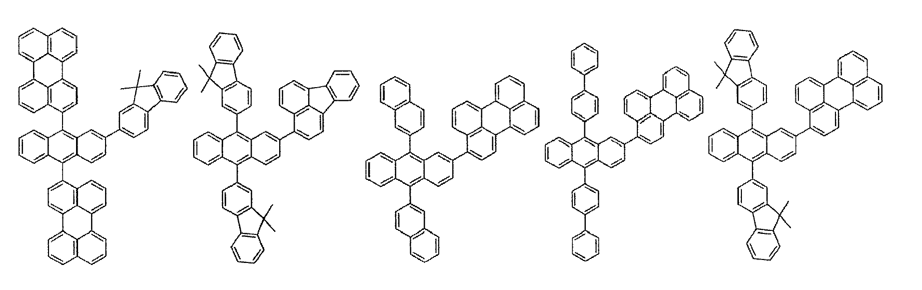

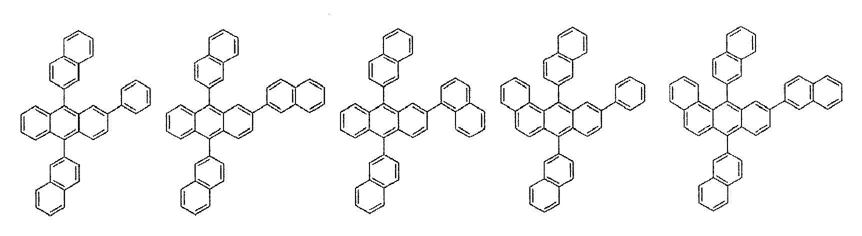

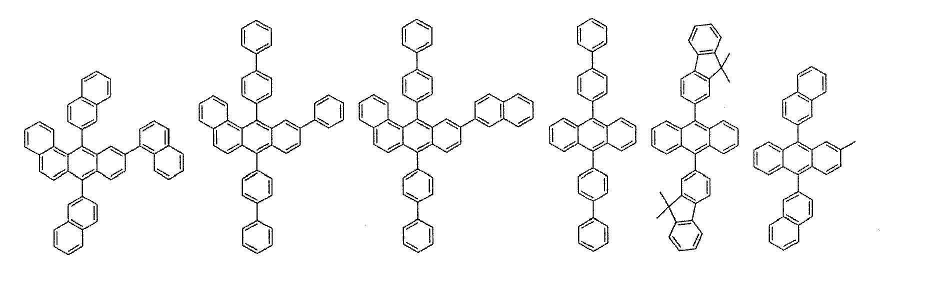

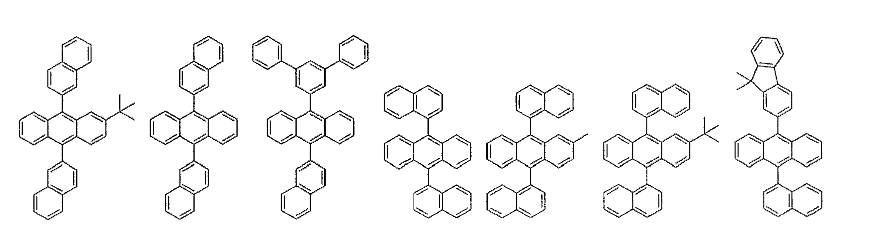

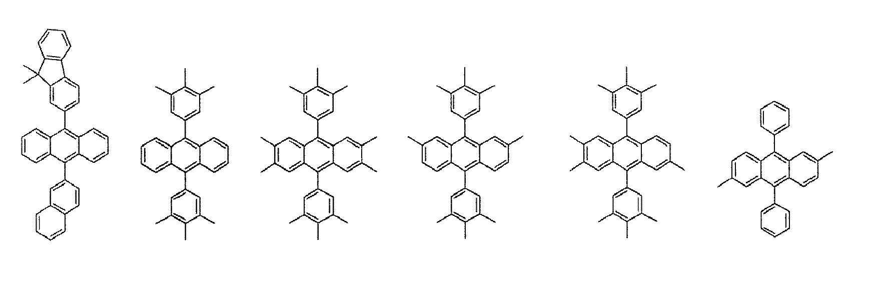

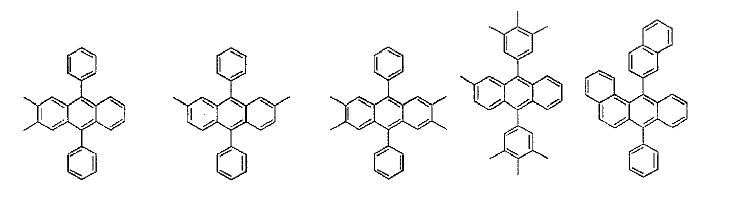

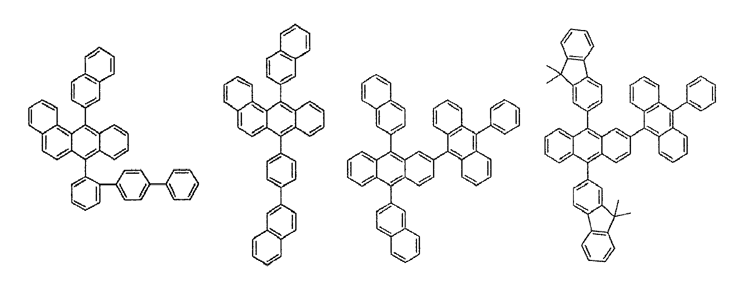

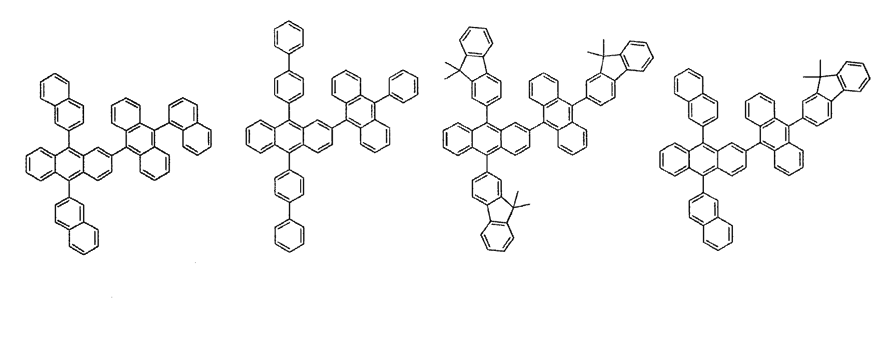

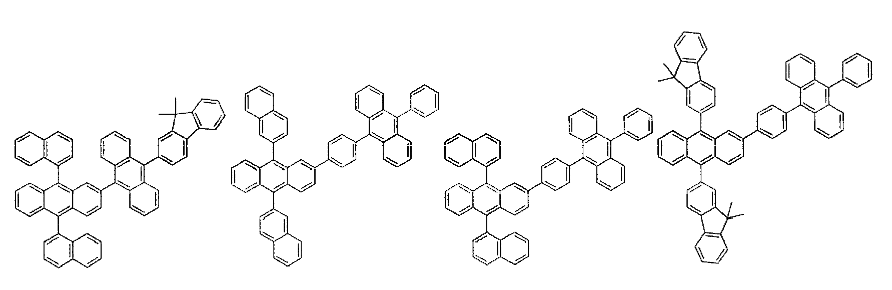

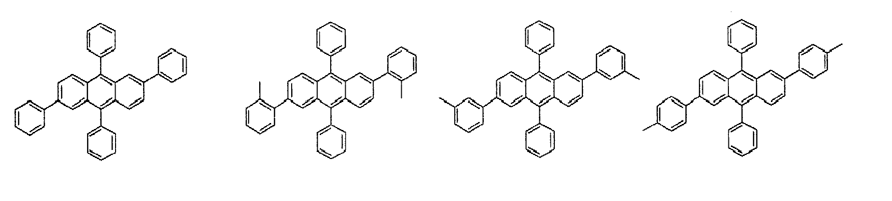

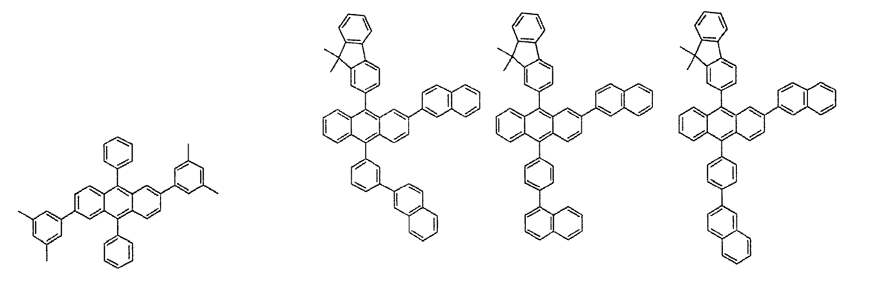

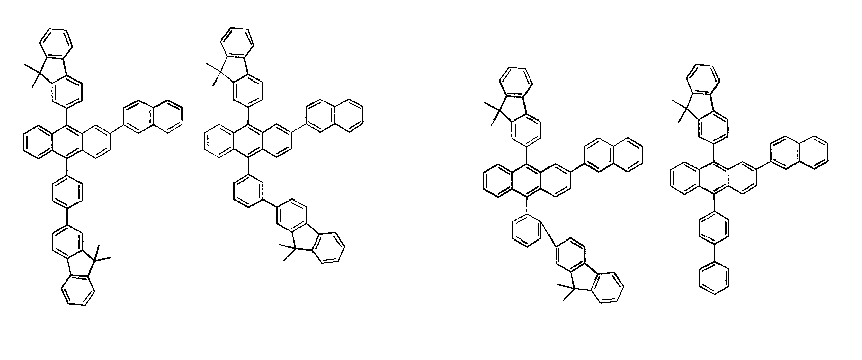

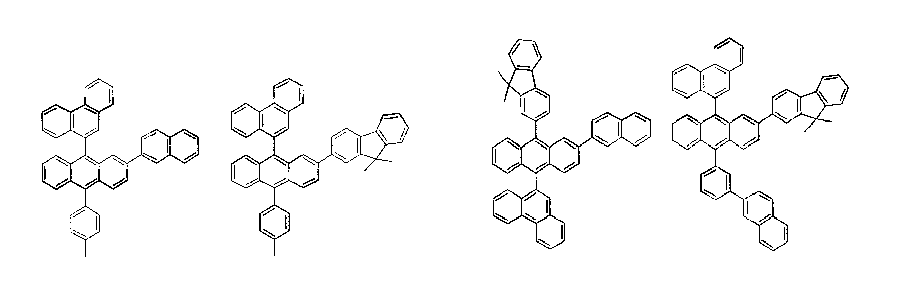

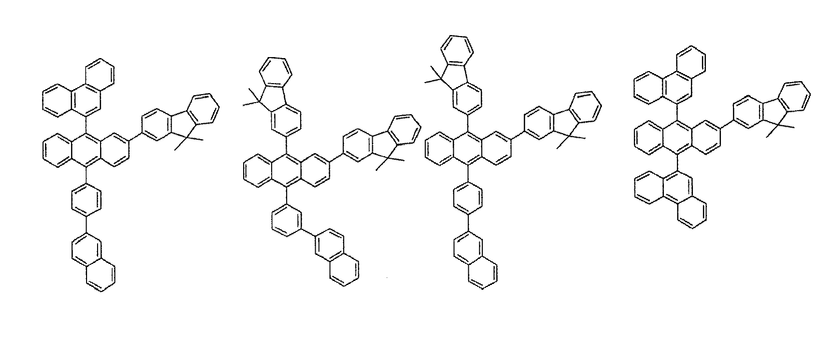

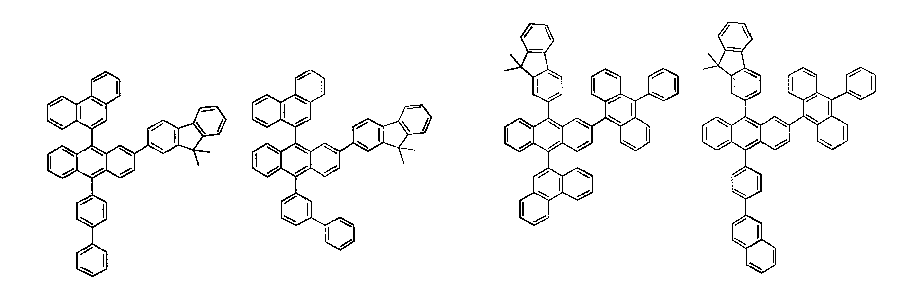

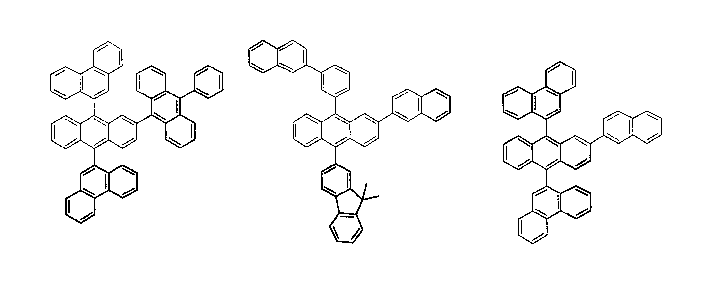

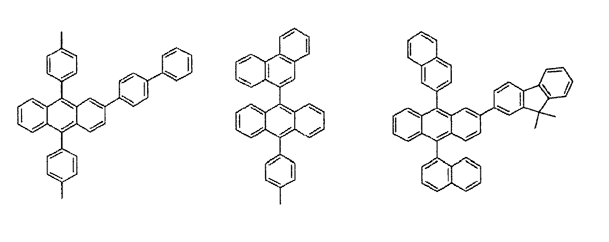

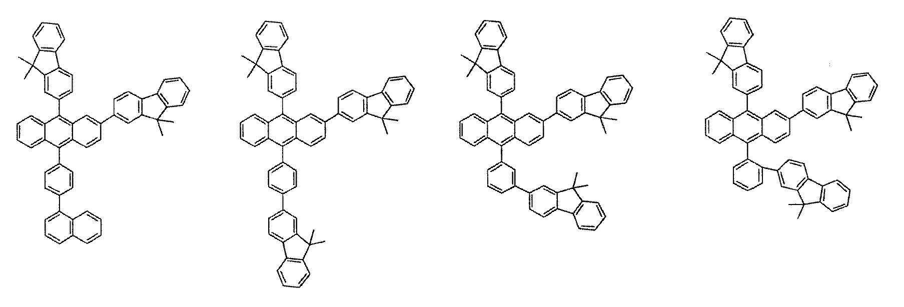

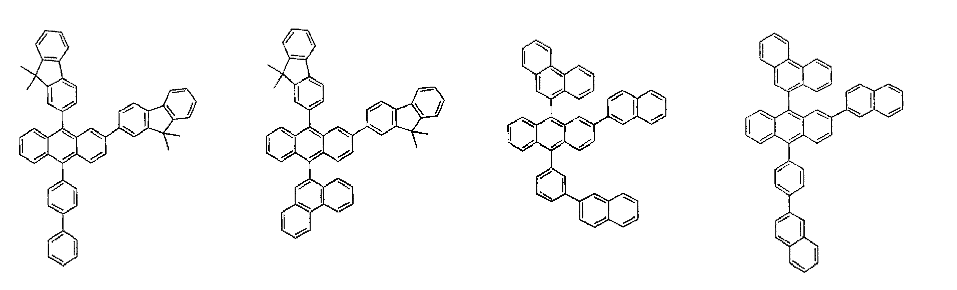

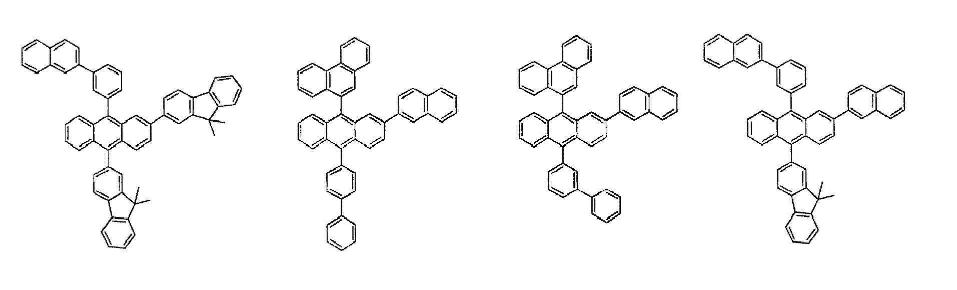

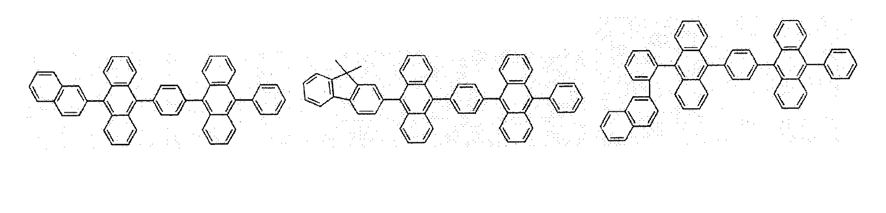

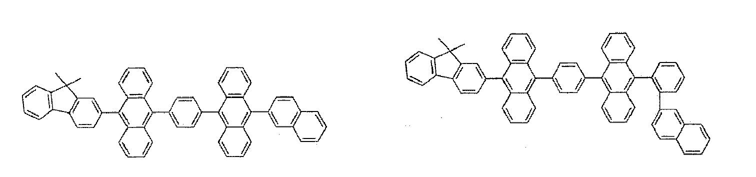

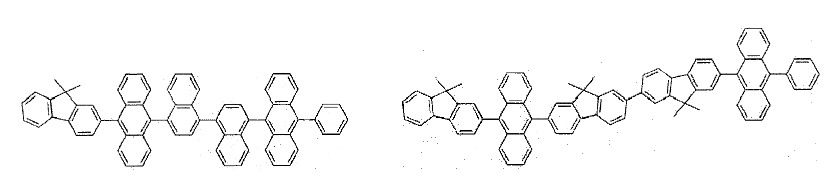

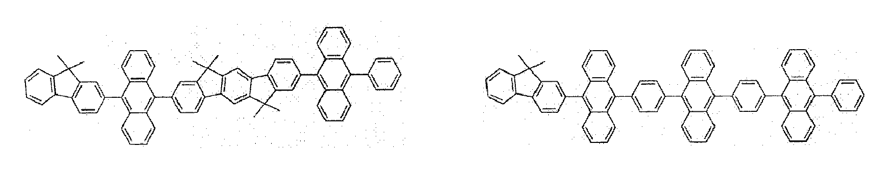

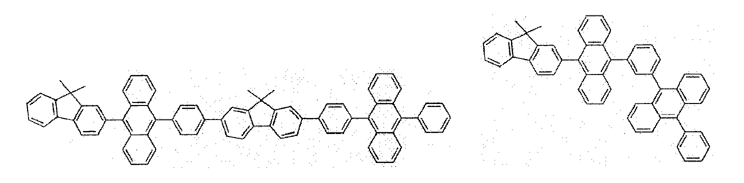

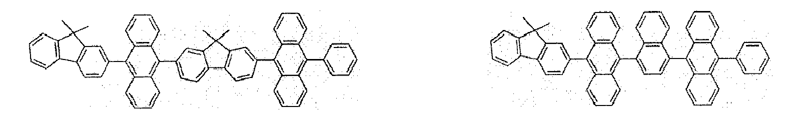

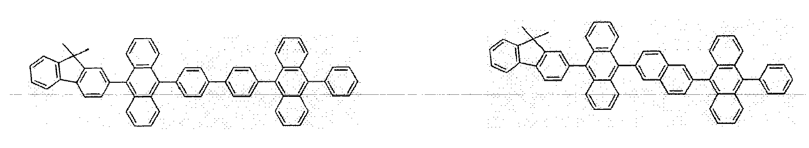

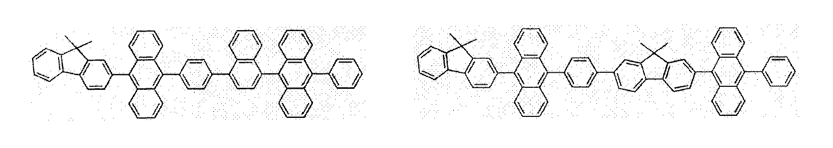

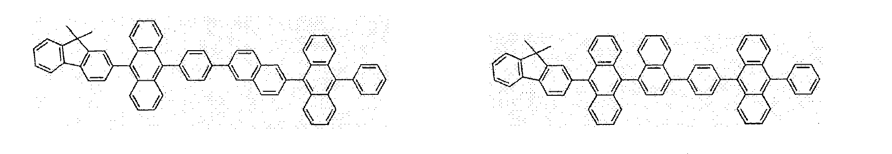

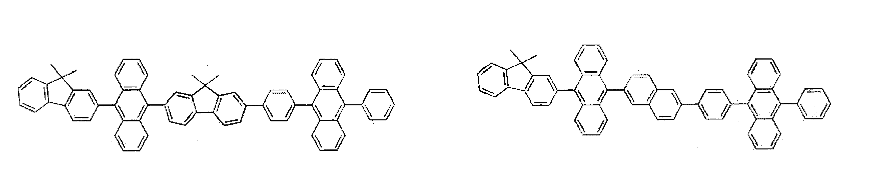

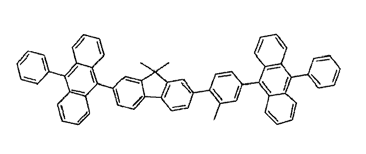

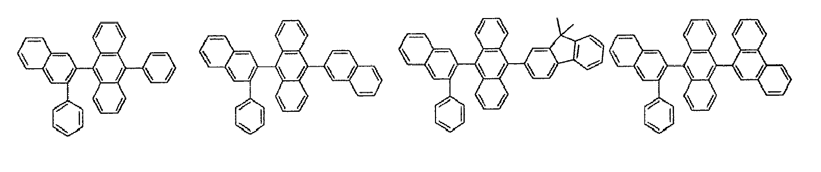

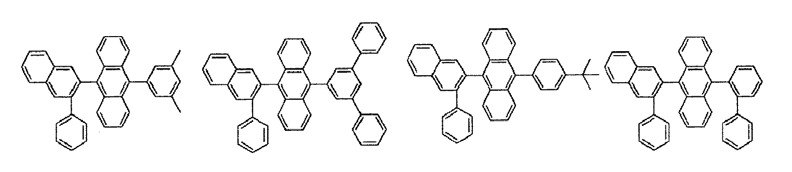

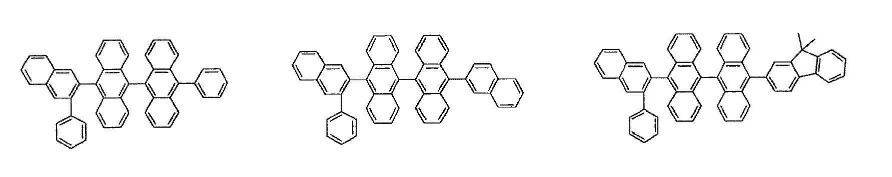

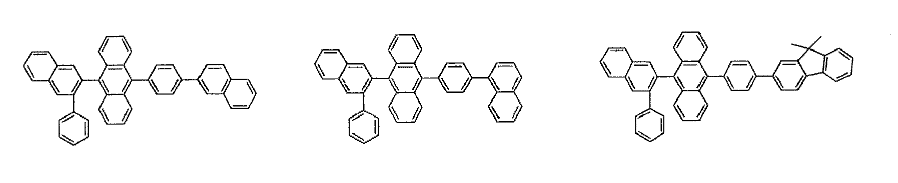

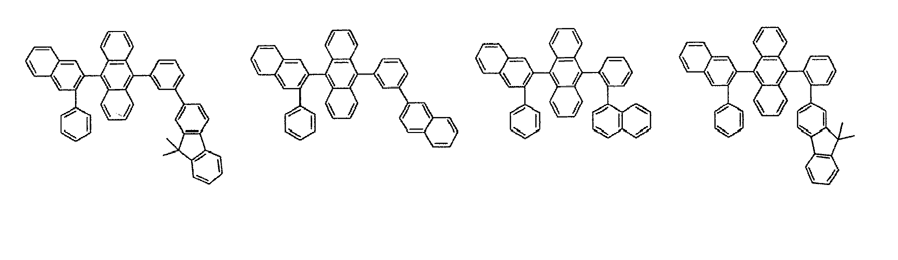

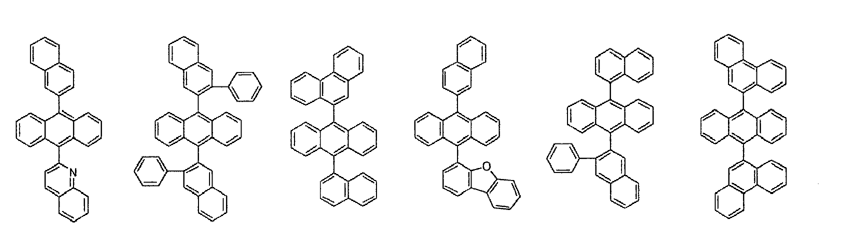

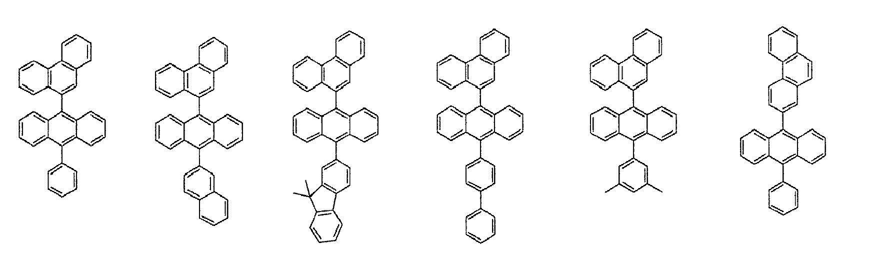

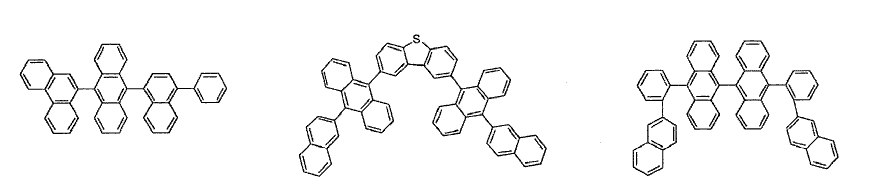

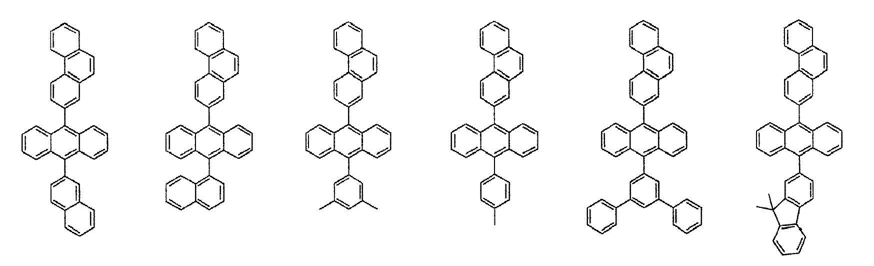

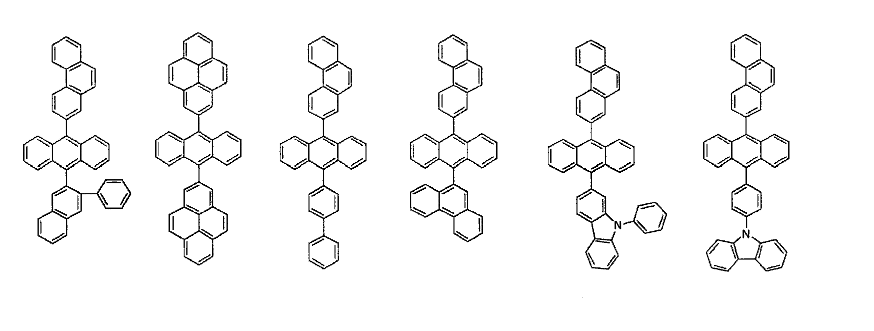

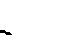

In an organic electronic device according to the present invention, it is preferable to place one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal, or the like. Examples of metal oxides preferably include Cs₂O, Li₂O, MgO, SrO, BaO, CaO, or the like.

In an organic electronic device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The compounds for organic electronic material according to the invention exhibit high luminous efficiency and excellent life property of material, so that OLED's having very good operation life of device can be advantageously manufactured therefrom. The compounds for organic electronic material according to the invention can be contained in a hole transport layer or a hole injecting layer, or employed as host for phosphor to result in lowered operation voltage, thus providing advantages of noticeably decreased power consumption while exhibiting at least comparable luminous efficiency.

### Best Mode

The present invention is further described with respect to the representative compounds of the invention, by describing the compounds for organic electronic material, the processes for preparing the same, and electroluminescent properties of the device manufactured therefrom in the Examples below, which are provided for illustration of the embodiments only but are not intended to limit the scope of the invention by any means.

### Preparation Examples

### [Preparation Example 1] Preparation of Compound (1)

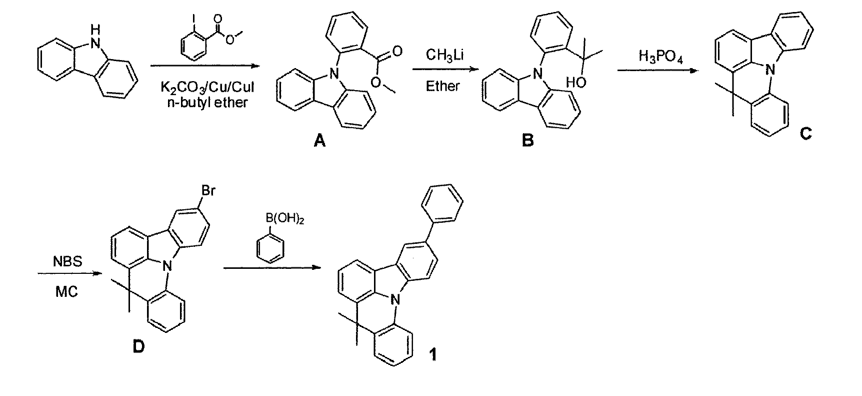

### Preparation of Compound (A)

Carbazole (20 g, 119.6 mmmol), methyl-2-iodobenzoate (26.4 mL, 179.4 mmol), K₂CO₃ (21.5 g, 155.5 mmol), Cu (1.52 g, 23.9 mmol) and CuI (1.14 g, 5.98 mmol) were dissolved in dibutyl ether (500 mL), and the solution was stirred under reflux for 48 hours in the presence of argon atmosphere. When the reaction was completed, the reaction mixture was cooled to room temperature, and extracted with water (800 mL). The organic layer was evaporated under reduced pressure. The residue was purified via column chromatography by using hexane and ethyl acetate (4:1), and recrystallized from ethanol (300 mL) to obtain Compound (A) (24.5 g, 68%).

### Preparation of Compound (B)

Compound (A) (15.0 g, 49.8 mmol) was dissolved in ether (100 mL), and the solution was chilled to -78°C. Methyl lithium (1.6 M in ether, 78 mL, 124.4 mmol) was added thereto, and the mixture stirred for 1 hour. The reaction mixture was slowly warmed to room temperature, and further stirred for 4 hours. When the reaction was completed, the mixture was extracted with water (200 mL), and the organic layer was evaporated under reduced pressure. Recrystallization from ethanol/acetone (1:1 v/v) gave Compound (B) (10.1 g, 67%).

### Preparation of Compound (C)

Compound (B) (14.0 g, 46.5 mmol) was dissolved in concentrated H₃PO₄ (150 mL), and the solution was stirred for 5.5 hours. The reaction mixture was extracted with water (200 mL) and ethyl acetate (200 mL), and the organic layer was evaporated under reduced pressure. Recrystallization from ethanol (150 mL) gave Compound (C) (9.48 g, 72%).

### Preparation of Compound (D)

Compound (C) (7 g, 24.7 mmol) was dissolved in dichloromethane (100 mL), and N-bromosuccinimide (5.28 g, 29.6 mmol) was added thereto at 0°C. The solution was stirred for 4 hours, while maintaining the temperature. Then the reaction was quenched by adding distilled water (150 mL), and the mixture was extracted with dichloromethane (100 mL), and the extract was evaporated under reduced pressure. Recrystallization from ethanol/acetone (1:2 v/v) gave Compound (D) (6.4 g, 72%).

### Preparation of Compound (1)

Compound (D) (5 g, 13.8 mmol), phenylboronic acid (1.9 g, 15.18 mmol) and Pd(PPh₃)₄ (0.8 g, 0.52 mmol) were dissolved in toluene (100 mL) and ethanol (50 mL), and aqueous 2 M sodium carbonate solution (50 mL) was added thereto. After stirring under reflux at 120°C for 4 hours, the reaction mixture was cooled to 25°C, and the reaction was quenched by adding distilled water (200 mL). The mixture was extracted with ethyl acetate (150 mL), and the extract was evaporated under reduced pressure. Purification via column chromatography gave the target compound (Compound 1) (1.6 g, 3.33 mmol).

### [Preparation Example 2] Preparation of Compound (320)

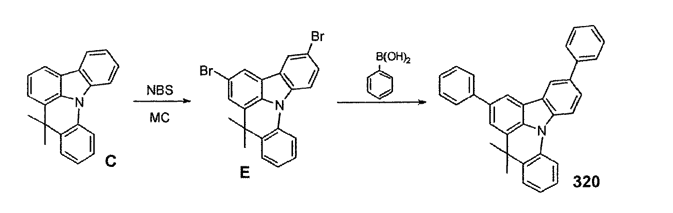

### Preparation of Compound (E)

Compound (C) (7 g, 24.7 mmol) was dissolved in dichloromethane (100 mL), and N-bromosuccinimide (10.5 g, 59.2 mmol) was added thereto at 0°C. The solution was stirred for 5 hours, while maintaining the temperature. Then the reaction was quenched by adding distilled water (150 mL), and the mixture was extracted with dichloromethane (100 mL), and the extract was evaporated under reduced pressure. Recrystallization from ethanol/acetone (1:2 v/v) gave Compound (E) (7.4 g, 68%).

### Preparation of Compound (320)

Compound (E) (5 g, 13.8 mmol), phenylboronic acid (3.8 g, 30.36 mmol) and Pd(PPh₃)₄ (1.6 g, 1.04 mmol) were dissolved in toluene (100 mL) and ethanol (50 mL), and aqueous 2 M sodium carbonate solution (50 mL) was added thereto. After stirring under reflux at 120°C for 4 hours, the reaction mixture was cooled to 25°C, and the reaction was quenched by adding distilled water (200 mL). The mixture was extracted with ethyl acetate (150 mL), and the extract was evaporated under reduced pressure. Purification via column chromatography gave the target compound (Compound 320) (3.9 g, 8.95 mmol).

### [Preparation Example 3] Preparation of Compound (642)

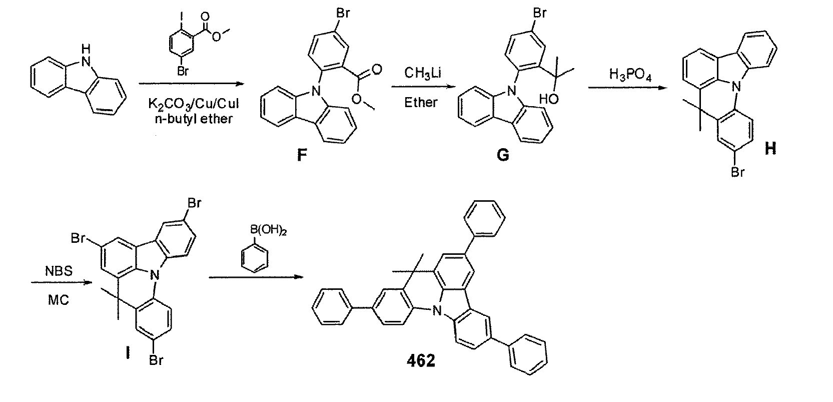

### Preparation of Compound (F)

Carbazole (20 g, 119.6 mmmol), 5-bromomethyl-2-iodobenzoate (26.4 mL, 179.4 mmol), K₂CO₃ (21.5 g, 155.5 mmol), Cu (1.52 g, 23.9 mmol) and CuI (1.14 g, 5.98 mmol) were dissolved in dibutyl ether (500 mL), and the solution was stirred under reflux for 48 hours in the presence of argon atmosphere. When the reaction was completed, the reaction mixture was cooled to room temperature, and extracted with water (800 mL). The organic layer was evaporated under reduced pressure. The residue was purified via column chromatography by using hexane and ethyl acetate (4:1), and recrystallized from ethanol (300 mL) to obtain Compound (F) (24.5 g, 58%).

### Preparation of Compound (G)

Compound (F) (15.0 g, 49.8 mmol) was dissolved in ether (100 mL), and the solution was chilled to -78°C. Methyl lithium (1.6 M in ether, 78 mL, 124.4 mmol) was added thereto, and the mixture stirred for 1 hour. The reaction mixture was slowly warmed to room temperature, and further stirred for 4 hours. When the reaction was completed, the mixture was extracted with water (200 mL), and the organic layer was evaporated under reduced pressure. Recrystallization from ethanol/acetone (1:1 v/v) gave Compound (G) (10.1 g, 57%).

### Preparation of Compound (H)

Compound (G) (14.0 g, 46.5 mmol) was dissolved in concentrated H₃PO₄ (150 mL), and the solution was stirred for 5.5 hours. The reaction mixture was extracted with water (200 mL) and ethyl acetate (200 mL), and the organic layer was evaporated under reduced pressure. Recrystallization from ethanol (150 mL) gave Compound (H) (9.48 g, 62%).

### Preparation of Compound (I)

Compound (H) (7 g, 24.7 mmol) was dissolved in dichloromethane (100 mL), and N-bromosuccinimide (5.28 g, 29.6 mmol) was added thereto at 0°C. The solution was stirred for 4 hours, while maintaining the temperature. Then the reaction was quenched by adding distilled water (150 mL), and the mixture was extracted with dichloromethane (100 mL), and the extract was evaporated under reduced pressure. Recrystallization from ethanol/acetone (1:2 v/v) gave Compound (I) (6.4 g, 62%).

### Preparation of Compound (462)

Compound (I) (5 g, 13.8 mmol), phenylboronic acid (5.7 g, 45.54 mmol) and Pd(PPh₃)₄ (2.4 g, 1.56 mmol) were dissolved in toluene (100 mL) and ethanol (50 mL), and aqueous 2 M sodium carbonate solution (50 mL) was added thereto. After stirring under reflux at 120°C for 4 hours, the reaction mixture was cooled to 25°C, and the reaction was quenched by adding distilled water (200 mL). The mixture was extracted with ethyl acetate (150 mL), and the extract was evaporated under reduced pressure. Purification via column chromatography gave the target compound (Compound 462) (1.6 g, 3.12 mmol).

### [Preparation Example 4] Preparation of Compound (498)

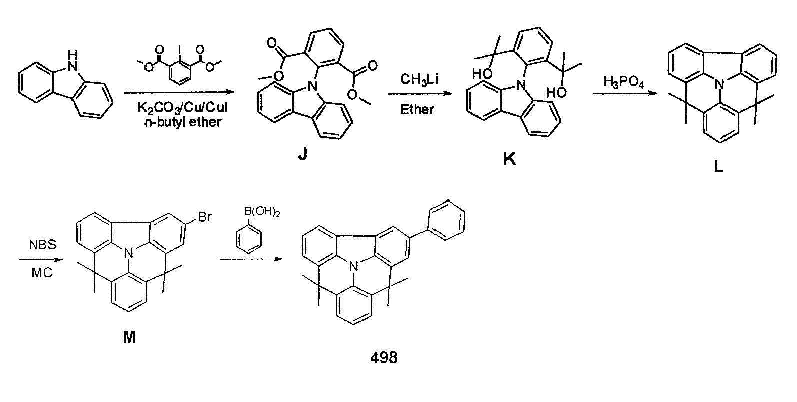

### Preparation of Compound (J)

Carbazole (20 g, 119.6 mmmol), dimethyl-2-iodoisophthalate (49.8 g, 155.5 mmol), K₂CO₃ (21.5 g, 155.5 mmol), Cu (1.52 g, 23.9 mmol) and CuI (1.14 g, 5.98 mmol) were dissolved in dibutyl ether (800 mL), and the solution was stirred under reflux for 48 hours in the presence of argon atmosphere. When the reaction was completed, the reaction mixture was cooled to room temperature, and extracted with water (800 mL). The organic layer was evaporated under reduced pressure. The residue was purified via column chromatography by using hexane and ethyl acetate (4:1), and recrystallized from ethanol (300 mL) to obtain Compound (J) (27.1 g, 63%).

### Preparation of Compound (K)

Compound (J) (15.0 g, 41.7 mmol) was dissolved in ether (300 mL), and the solution was chilled to -78°C. Methyl lithium (1.6 M in ether, 120 mL, 187.8 mmol) was added thereto, and the mixture stirred for 1 hour. The reaction mixture was slowly warmed to room temperature, and further stirred for 4 hours. When the reaction was completed, the mixture was extracted with water (200 mL), and the organic layer was evaporated under reduced pressure. Recrystallization from ethanol/acetone (1:1 v/v) gave Compound (K) (8.7 g, 58%).

### Preparation of Compound (L)

Compound (K) (14.0 g, 38.9 mmol) was dissolved in concentrated H₃PO₄ (150 mL), and the solution was stirred for 5.5 hours. The reaction mixture was extracted with water (200 mL) and ethyl acetate (200 mL), and the organic layer was evaporated under reduced pressure. Recrystallization from ethanol (150 mL) gave Compound (L) (7.68 g, 61%).

### Preparation of Compound (M)

Compound (L) (7 g, 21.6 mmol) was dissolved in dichloromethane (100 mL), and N-bromosuccinimide (5.28 g, 28.1 mmol) was added thereto at 0°C. The solution was stirred for 4 hours, while maintaining the temperature. Then the reaction was quenched by adding distilled water (150 mL), and the mixture was extracted with dichloromethane (100 mL), and the extract was evaporated under reduced pressure. Recrystallization from ethanol/acetone (1:2 v/v) gave Compound (M) (5.7 g, 66%).

### Preparation of Compound (498)

Compound (M) (5 g, 12.4 mmol), phenylboronic acid (1.9 g, 15.18 mmol) and Pd(PPh₃)₄ (0.8 g, 0.52 mmol) were dissolved in toluene (100 mL) and ethanol (50 mL), and aqueous 2 M sodium carbonate solution (50 mL) was added thereto. After stirring under reflux at 120°C for 4 hours, the reaction mixture was cooled to 25°C, and the reaction was quenched by adding distilled water (200 mL). The mixture was extracted with ethyl acetate (150 mL), and the extract was evaporated under reduced pressure. Purification via column chromatography gave the target compound (Compound 498) (1.5 g, 3.75 mmol).

### [Preparation Example 5] Preparation of Compound (811)

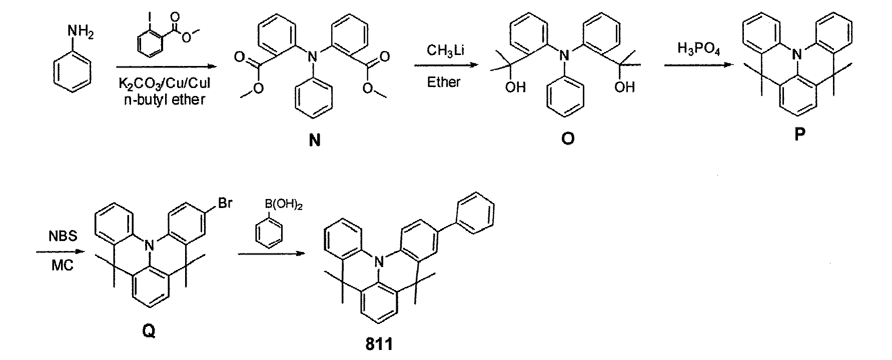

### Preparation of Compound (N)

A reaction vessel was charged with aniline (4.89 mL, 53.7 mmol), methyl-2-iodobenzoate (23.7 mL, 161 mmol), K₂CO₃ (15.6 g, 113 mmol), Cu (0.68 g, 10.7 mmol), CuI (0.51 g, 2.69 mmol) and di-n-butyl ether (50 mL), and the mixture was stirred under reflux for 48 hours in the presence of argon atmosphere. The organic product was purified via column chromatography by using hexane and ethyl acetate (4:1), and recrystallized from ethanol to obtain Compound (N) (15.7 g, 81%).

### Preparation of Compound (O)

Compound (N) (15.0 g, 41.5 mmol) was dissolved in ether (80 mL), and methyl lithium (in ether, 45 mmol) was added thereto at -78°C. The mixture was slowly warmed to ambient temperature, and reacted for 4 hours. Recrystallization from ethanol/acetone (1:1 v/v) gave Compound (O) (7.68 g, 61%).

### Preparation of Compound (P)

Compound (O) (14.0 g, 38.7 mmol) was added to concentrated H₃PO₄ (150 mL), and the mixture was stirred for 5.5 hours. The reaction mixture was extracted with water/ethyl acetate, and the organic product was recrystallized from ethanol to obtain Compound (P) (7.8 g, 68%).

### Preparation of Compound (Q)

Compound (P) (7 g, 21.5 mmol) was dissolved in methylene chloride, and N-bromosuccinimide (NBS) (4.98 g, 27.95 mmol) was added thereto at 0°C. After 5 hours, the reaction mixture was extracted and recrystallized from ethanol/acetone (1:2 v/v) to obtain Compound (Q) (6 g, 75%).

### Preparation of Compound (811)

Compound (Q) (5 g, 12.4 mmol), phenylboronic acid (1.9 g, 15.18 mmol) and Pd(PPh₃)₄ (0.8 g, 0.52 mmol) were dissolved in toluene (100 mL) and ethanol (50 mL), and aqueous 2 M sodium carbonate solution (50 mL) was added thereto. After stirring under reflux at 120°C for 4 hours, the reaction mixture was cooled to 25°C, and the reaction was quenched by adding distilled water (200 mL). The mixture was extracted with ethyl acetate (150 mL), and the extract was evaporated under reduced pressure. Purification via column chromatography gave the target compound (Compound 811) (1.5 g, 3.75 mmol).

### [Preparation Example 6] Preparation of Compound (1221)

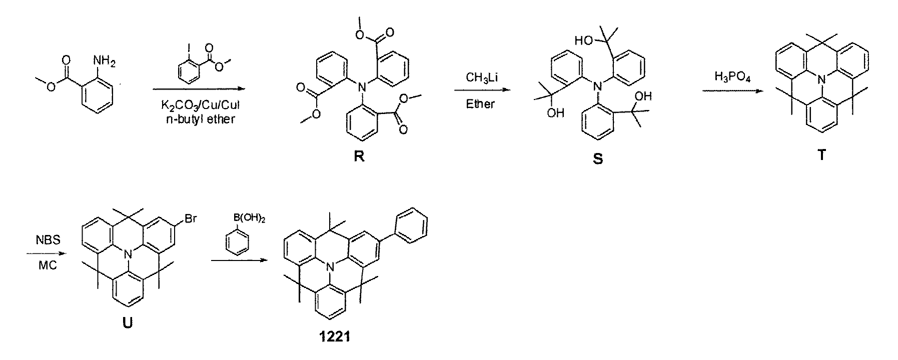

### Preparation of Compound (R)

A reaction vessel was charged with anthranilic acid methyl ester (10 g, 66.2 mmol), methyl-2-iodobenzoate (28.2 mL, 191.98 mmol), K₂CO₃ (19.2 g, 139.02 mmol), Cu (0.84 g, 13.24 mmol), CuI (0.63 g, 3.31 mmol) and di-n-butyl ether (120 mL), and the mixture was stirred under reflux for 48 hours in the presence of argon atmosphere. The organic product was purified via column chromatography by using hexane and ethyl acetate (4:1), and recrystallized from ethanol to obtain Compound (R) (21.7 g, 78%).

### Preparation of Compound (S)

Compound (R) (15.0 g, 35.8 mmol) was dissolved in ether (100 mL), and methyl lithium (1.6 M in ether, 145 mL, 232.46 mmol) was added thereto at -78°C. The mixture was slowly warmed to room temperature, and continuously reacted for 4 hours. Recrystallization from ethanol/acetone (1:1 v/v) gave Compound (S) (10.4 g, 69%).

### Preparation of Compound (T)

Compound (S) (14.0 g, 33.4 mmol) was added to concentrated H₃PO₄ (150 mL), and the mixture was stirred for 5.5 hours. The reaction mixture was extracted with water/ethyl acetate, and the organic product was recrystallized from ethanol to obtain Compound (T) (9.15 g, 75%).

### Preparation of Compound (U)

Compound (T) (7 g, 19.2 mmol) was dissolved in methylene chloride, and N-bromosuccinimide (NBS) (4.09 g, 23.0 mmol) was added thereto at 0°C. After 5 hours, the reaction mixture was extracted by using water and ethyl acetate, and the organic product was recrystallized from ethanol/acetone (1:2 v/v) to obtain Compound (U) (6.4 g, 75%).

### Preparation of Compound (1221)

Compound (U) (5 g, 11.3 mmol), phenylboronic acid (1.9 g, 15.18 mmol) and Pd(PPh₃)₄ (0.8 g, 0.52 mmol) were dissolved in toluene (100 mL) and ethanol (50 mL), and aqueous 2 M sodium carbonate solution (50 mL) was added thereto. After stirring under reflux at 120°C for 4 hours, the reaction mixture was cooled to 25°C, and the reaction was quenched by adding distilled water (200 mL). The mixture was extracted with ethyl acetate (150 mL), and the extract was evaporated under reduced pressure. Purification via column chromatography gave the target compound (Compound 1221) (1.5 g, 3.75 mmol).

Organic electroluminescent compounds (Compounds 1 to 1528) were prepared according to the procedure described in Preparation Examples 1 to 6, and the ¹H NMR and MS/FAB data of those compounds are shown in Table 1.

**Table 1**

| Compound No. | ¹H NMR(CDCl₃, 200 MHz) | MS/FAB | |
|---|---|---|---|
| | | found | calculated |
| **1** | δ = 1.72(6H, s), 6.95(1H, m), 7.17(1H, m), 7.26∼7.3(2H, m), 7.37∼7.41(3H, m), 7.51∼7.52(4H, m), 7.69(1H, m), 7.77(1H, m), 7.87(1H, m), 8.37(1H, m) | 359.46 | 359.17 |
| **4** | δ = 1.72(12H, s), 6.95(1H, m), 7.17(1H, m), 7.26∼7.3(3H, m), 7.37∼7.4(3H, m), 7.55(1H, m), 7.63(1H, m), 7.69(1H, m), 7.77(2H, m), 7.87∼7.93(3H, m), 8.37(1H, m) | 475.62 | 475.23 |
| **16** | δ = 1.72(6H, s), 6.95(1H, m), 7.17(1H, m), 7.26∼7.3(2H, m), 7.37∼7.41(3H, m), 7.48∼7.57(7H, m), 7.69∼7.7(2H, m), 7.77(1H, m), 7.87(1H, m), 8.37(1H, m) | 435.56 | 435.20 |
| **28** | δ = 1.72(6H, s), 6.95(1H, m), 7.17(1H, m), 7.26∼7.3(2H, m), 7.37∼7.41(3H, m), 7.51(2H, m), 7.59(2H, m), 7.69(1H, m), 7.77∼7.79(3H, m), 7.87(1H, m), 8(2H, m), 8.37∼8.4(3H, m) | 485.62 | 485.21 |
| **35** | δ = 1.72(6H, s), 6.95(1H, m), 7.17∼7.3(7H, m), 7.37∼7.5(5H, m), 7.58∼7.59(3H, m), 7.69(1H, m), 7.77(1H, m), 7.85∼7.87(3H, m), 8.37(1H, m), 8.56(1H, m) | 551.68 | 551.24 |
| **38** | δ = 1.72(6H, s), 6.95(1H, m), 7.17(1H, m), 7.25∼7.3(4H, m), 7.37∼7.4(2H, m), 7.53(2H, m), 7.69(1H, m), 7.77(1H, m), 7.85∼7.87(3H, m), 8.01(1H, m), 8.18(1H, m), 8.37(1H, m) | 492.63 | 492.17 |
| **49** | δ = 1.72(6H, s), 6.95(1H, m), 7.17(1H, m), 7.25∼7.4(7H, m), 7.5(1H, m), 7.63∼7.69(4H, m), 7.77∼7.79(3H, m), 7.87(1H, m), 7.94(1H, m), 8.12(1H, m), 8.37(1H, m), 8.55(1H, m) | 524.65 | 524.23 |
| **50** | δ = 1.72(6H, s), 6.95(1H, m), 7.17(1H, m), 7.25∼7.33(9H, m), 7.58(2H, m), 7.69(2H, m), 7.77(2H, m), 7.87(2H, m), 7.94(1H, m), 8.37(1H, m), 8.55(1H, m) | 524.65 | 524.23 |
| **72** | δ = 1.72(6H, s), 6.95(1H, m), 7.17(1H, m), 7.26∼7.3(2H, m), 7.36∼7.42(5H, m), 7.48(1H, m), 7.69∼7.87(8H, m), 8.03∼8.12(3H, m), 8.37(1H, m) | 541.62 | 541.20 |
| **91** | δ = 1.72(6H, s), 6.95(1H, m), 7.17(1H, m), 7.25∼7.3(6H, m), 7.37∼7.4(2H, m), 7.55(2H, m), 7.61(1H, m), 7.69(1H, m), 7.77(1H, m), 7.87(1H, m), 8.04∼8.08(2H, m), 8.37∼8.42(2H, m), 8.55(1H, m) | 485.62 | 485.21 |
| **94** | δ = 1.72(6H, s), 6.95(1H, m), 7.17(1H, m), 7.26∼7.3(2H, m), 7.37∼7.4(2H, m), 7.48(2H, m), 7.57∼7.59(4H, m), 7.69∼7.77(4H, m), 7.87∼7.92(2H, m), 8(2H, m), 8.37(1H, m) | 485.62 | 485.21 |
| **104** | δ = 1.72(6H, s), 6.95(1H, m), 7.17(1H, m), 7.26∼7.3{2H, m), 7.37∼7.41(3H, m), 7.51∼7.52(4H, m), 7.58(2H, m), 7.69∼7.77(4H, m), 7.87∼7.92(3H, m), 8.37(1H, m) | 485.62 | 485.21 |
| **106** | δ = 1.72(6H, s), 6.95(1H, m), 7.17(1H, m), 7.26∼7.3(2H, m), 7.37∼7.41(3H, m), 7.51∼7.55(4H, m), 7.69(1H, m), 7.77∼7.79(3H, m), 7.87(1H, m), 8.01(2H, m), 8.37(1H, m), 8.55(2H, m) | 485.62 | 485.21 |
| **112** | δ = 1.72(6H, s), 6.95(1H, m), 7.17(1H, m), 7.26∼7.3(2H, m), 7.37∼7.4(6H, m), 7.58∼7.59(3H, m), 7.69∼7.77(3H, m), 7.87∼7.92(6H, m), 8(2H, m), 8.37(1H, m) | 585.73 | 585.25 |
| **119** | δ = 1.72(6H, s), 2.34(12H, s), 6.36(4H, m), 6.71∼6.77(4H, m), 6.95(1H, m), 7.17(1H, m), 7.26∼7.3(2H, m), 7.37∼7.4(3H, m), 8.37(1H, m) | 506.68 | 506.27 |
| **132** | δ = 1.72(12H, s), 6.58∼6.63(3H, m), 6.75∼6.81(4H, m), 6.95(1H, m), 7.17∼7.3(6H, m), 7.37∼7.4(4H, m), 7.55(1H, m), 7.62(1H, m), 7.87(1H, m), 8.37(1H, m) | 566.73 | 566.27 |
| **146** | δ = 1.72(6H, s), 6.69∼6.77(4H, m), 6.95∼6.98(2H, m), 7.17(1H, m), 7.26∼7.3(2H, m), 7.37∼7.41(5H, m), 7.51∼7.57(9H, m), 8.02∼8.07(2H, m), 8.37(1H, m) | 576.73 | 576.26 |
| **158** | δ = 1.72(12H, s), 6.58(1H, m), 6.75∼6.77(3H, m), 6.95(1H, m), 7.17(1H, m), 7.26∼7.3(3H, m), 7.36∼7.4(5H, m), 7.49∼7.55(3H, m), 7.62(1H, m), 7.74∼7.77(2H, m), 7.84∼7.88(3H, m), 8.37(1H, m) | 616.79 | 616.29 |
| **171** | δ = 1.35(9H, s), 1.72(12H, s), 6.55∼6.58(3H, m), 6.75∼6.77(3H, m), 6.95∼7.01(3H, m), 7.17(1H, m), 7.26∼7.3(3H, m), 7.37∼7.4(4H, m), 7.55(1H, m), 7.62(1H, m), 7.87(1H, m), 8.37(1H, m) | 622.84 | 622.33 |
| **235** | δ = 1.72(6H, s), 6.62(1H, m), 6.7∼6.77(3H, m), 6.95∼6.99(3H, m), 7.17(1H, m), 7.26∼7.3(2H, m), 7.37∼7.4(3H, m), 7.55(1H, m), 8.07(1H, m), 8.37(1H, m), 8.46(2H, m) | 452.55 | 452.20 |
| **261** | δ = 1.72(6H, s), 6.63(4H, m), 6.69(2H, m), 6.81(2H, m), 6.95(1H, m), 7.17∼7.3(7H, m), 7.37∼7.4(2H, m), 7.54(2H, m), 7.69(1H, m), 7.77(1H, m), 7.87(1H, m), 8.37(1H, m) | 526.67 | 526.24 |
| **263** | δ = 1.72(6H, s), 6.63(4H, m), 6.81(2H, m), 6.95∼6.98(2H, m), 7.17∼7.3(7H, m), 7.37∼7.4(3H, m), 7.6(1H, m), 7.69(1H, m), 7.77(1H, m), 7.87(1H, m), 8.03∼8.04(2H, m), 8.37∼8.4(2H, m) | 576.73 | 576.26 |
| **266** | δ = 1.72(12H, s), 6.58∼6.63(5H, m), 6.75∼6.81(3H, m), 6.95(1H, m), 7.17∼7.3(7H, m), 7.37∼7.4(2H, m), 7.62∼7.63(2H, m), 7.69(1H, m), 7.77(2H, m), 7.87∼7.93(2H, m), 8.37(1H, m) | 642.83 | 642.30 |
| **271** | δ = 1.72(12H, s), 6.58(1H, m), 6.75(1H, m), 6.95(1H, m), 7.17(1H, m), 7.26∼7.3(2H, m), 7.36∼7.4(4H, m), 7.49∼7.5(4H, m), 7.62∼7.63(2H, m), 7.69∼7.77(7H, m), 7.84∼7.93(6H, m), 8.37(1H, m) | 742.95 | 742.33 |
| **278** | δ = 1.72(6H, s), 6.63(2H, m), 6.81(1H, m), 6.95∼6.98(2H, m), 7.17∼7.3(5H, m), 7.37∼7.46(8H, m), 7.64∼7.77(5H, m), 7.84∼7.87(2H, m), 8.02∼8.07(2H, m), 8.37(1H, m) | 626.79 | 626.27 |
| **301** | δ = 1.72(6H, s), 6.63(2H, m), 6.69∼6.81(5H, m), 6.95(1H, m), 7.17∼7.3(5H, m), 7.37∼7.4(3H, m), 7.54∼7.59(5H, m), 7.73(1H, m), 7.92(1H, m), 8(2H, m), 8.37(1H, m) | 576.73 | 576.26 |
| **320** | δ = 1.72(6H, s), 7.26∼7.3(2H, m), 7.37∼7.41(4H, m), 7.51∼7.52(8H, m), 7.59(1H, m), 7.69(1H, m), 7.77(1H, m), 7.87(1H, m), 8.54(1H, m) | 435.56 | 435.20 |
| **341** | δ = 1.72(6H, s), 7.25∼7.4(10H, m), 7.5(2H, m), 7.59∼7.69(8H, m), 7.77∼7.79(5H, m), 7.87(1H, m), 7.94(2H, m), 8.12(2H, m), 8.54∼8.55(3H, m) | 765.94 | 765.31 |
| **360** | δ = 1.72(30H, s), 6.15(1H, m), 6.58(4H, m), 6.75∼6.77(6H, m), 7.26∼7.3(6H, m), 7.37∼7.45(8H, m), 7.55(4H, m), 7.62(4H, m), 7.87(4H, m) | 1082.42 | 1081.53 |
| **374** | δ = 1.72(18H, s), 6.15(1H, m), 6.58(2H, m), 6.75∼6.77(4H, m), 6.98(2H, m), 7.26∼7.3(4H, m), 7.37∼7.45(8H, m), 7.53∼7.62(10H, m), 7.87(2H, m), 8.02∼8.07(4H, m) | 950.22 | 949.44 |
| **394** | δ = 1.72(6H, s), 6.63(8H, m), 6.69(4H, m), 6.81(4H, m), 7.2∼7.3(10H, m), 7.37∼7.4(2H, m), 7.54∼7.59(5H, m), 7.69(1H, m), 7.77(1H, m), 7.87(1H, m), 8.54(1H, m) | 769.97 | 769.35 |
| **402** | δ = 1.72(6H, s), 6.63(4H, m), 6.69(4H, m), 6.81(2H, m), 6.98(2H, m), 7.2∼7.3(6H, m), 7.37∼7.4(4H, m), 7.53∼7.59(11H, m), 7.69(1H, m), 7.77(1H, m), 7.87(1H, m), 8.02∼8.07(4H, m), 8.54(1H, m) | 870.09 | 869.38 |
| **415** | δ = 1.72(12H, s), 7.26∼7.3(3H, m), 7.37∼7.41(4H, m), 7.51∼7.63(7H, m), 7.69(1H, m), 7.77(2H, m), 7.87∼7.93(3H, m), 8.54(1H, m) | 551.72 | 551.26 |
| **428** | δ = 1.72(6H, s), 6.63(4H, m), 6.75∼6.81(4H, m), 7.2∼7.3(6H, m), 7.37∼7.41(4H, m), 7.51∼7.52(4H, m), 7.59(1H, m), 8.54(1H, m) | 526.67 | 526.24 |
| **432** | δ = 1.72(6H, s), 6.63(4H, m), 6.75∼6.81(4H, m), 7.2∼7.3(6H, m), 7.37∼7.4(3H, m), 7.58∼7.59(4H, m), 7.73(1H, m), 7.92(1H, m), 8(2H, m), 8.54(1H, m) | 576.73 | 576.26 |
| **447** | δ = 1.72(6H, s), 6.63(4H, m), 6.69(2H, m), 6.81(2H, m), 7.2∼7.3(6H, m), 7.37∼7.41(3H, m), 7.51∼7.59(7H, m), 7.69(1H, m), 7.77(1H, m), 7.87(1H, m), 8.54(1H, m) | 602.76 | 602.27 |
| **454** | δ = 1.72(6H, s), 6.15(1H, m), 6.63(6H, m), 6.69(2H, m), 6.81(3H, m), 6.98(1H, m), 7.2∼7.3(8H, m), 7.37∼7.45(4H, m), 7.53∼7.57(5H, m), 7.69(1H, m), 7.77(1H, m), 7.87(1H, m), 8.02∼8.07(2H, m) | 743.93 | 743.33 |
| **462** | δ = 1.72(6H, s), 7.41(3H, m), 7.51∼7.52(12H, m), 7.59∼7.61(3H, m), 7.69(1H, m), 7.77(1H, m), 7.86∼7.87(2H, m), 8.54(1H, m) | 511.65 | 511.23 |
| **473** | δ = 1.72(6H, s), 7.25∼7.33(10H, m), 7.44∼7.54(5H, m), 7.63(5H, m), 7.8(1H, m), 7.94∼8.01(5H, m), 8.12(3H, m), 8.55(3H, m) | 778.94 | 778.31 |
| **480** | δ = 1.72(6H, s), 2.34(36H, s), 6.15(1H, m), 6.36(12H, m), 6.45(1H, m), 6.62(1H, m), 6.71∼6.77(8H, m), 7.29(1H, m), 7.38(1H, m), 7.45(1H, m) | 953.31 | 952.54 |
| **494** | δ = 1.72(6H, s), 6.63(12H, m), 6.69(6H, m), 6.81(6H, m), 7.2(12H, m), 7.54∼7.61(9H, m), 7.69(1H, m), 7.77(1H, m), 7.86∼7.87(2H, m), 8.54(1H, m) | 1013.27 | 1012.45 |
| **496** | δ = 1.72(6H, s), 6.69(6H, m), 6.98(3H, m), 7.36∼7.35(6H, m), 7.49∼7.54(24H, m), 7.69∼7.77(8H, m), 7.84∼7.88(8H, m), 8.02∼8.07(6H, m), 8.54(1H, m) | 1313.63 | 1312.54 |
| **516** | δ = 1.72(12H, s), 6.95(1H, m), 7.12∼7.17(3H, m), 7.25∼7.33(3H, m), 7.5∼7.51(2H, m), 7.59∼7.68(4H, m), 7.79∼7.81(3H, m), 7.94(1H, m), 8.12(1H, m), 8.37(1H, m), 8.55(1H, m) | 564.72 | 564.26 |
| **538** | δ = 1.72(12H, s), 6.15(1H, m), 6.57∼6.63(3H, m), 6.69(2H, m), 6.81(1H, m), 6.95(1H, m), 7.12∼7.2(5H, m), 7.41(1H, m), 7.51∼7.54(7H, m), 8.37(1H, m) | 566.73 | 566.27 |
| **571** | δ = 1.72(12H, s), 6.63(4H, m), 6.69(2H, m), 6.81(2H, m), 6.95(1H, m), 7.12∼7.2(7H, m), 7.51∼7.59(4H, m), 7.81(1H, m), 8.37(1H, m) | 566.73 | 566.27 |
| **586** | δ = 1.72(12H, s), 6.63(2H, m), 6.69(2H, m), 6.81(1H, m), 6.95∼6.98(2H, m), 7.12∼7.2(5H, m), 7.38(1H, m), 7,51∼7.59(7H, m), 7.81(1H, m), 8.02∼8.07(2H, m), 8.37(1H, m) | 616.79 | 616.29 |
| **600** | δ = 1.35(9H, s), 1.72(12H, s), 6.55(2H, m), 6.95∼7.01(4H, m), 7.12∼7.17(3H, m), 7.38(1H, m), 7.46(1H, m), 7.49(1H, m), 7.51∼7.57(8H, m), 7.81∼7.84(2H, m), 8.02∼8.07(2H, m), 8.37(1H, m) | 722.96 | 722.37 |
| **602** | δ = 1.72(12H, s), 6.15(1H, m), 6.57∼6.63(3H, m), 6.69(2H, m), 6.81(1H, m), 6.95(1H, m), 7.12∼7.2(5H, m), 7.51∼7.59(6H, m), 7.73(1H, m), 7.92(1H, m), 8(2H, m), 8.37(1H, m) | 616.79 | 616.29 |
| **624** | δ = 1.72(12H, s), 6.15(1H, m), 6.57(1H, m), 6.69(4H, m), 6.95(1H, m), 7.12∼7.17(3H, m), 7.51∼7.59(11H, m), 7.73(2H, m), 7.92(2H, m), 8(4H, m), 8.37(1H, m) | 742.95 | 742.33 |
| **648** | δ = 1.72(12H, s), 7.12(2H, m), 7.39∼7.41(10H, m), 7.51∼7.52(9H, m), 7.59(2H, m), 7.81(1H, m), 7.91(8H, m), 8.54(1H, m) | 828.05 | 827.36 |
| **685** | δ = 1.72(12H, s), 6.63(8H, m), 6.69(4H, m), 6.81(4H, m), 7.12(2H, m), 7.2(8H, m), 7.51∼7.59(7H, m), 7.81(1H, m), 8.54(1H, m) | 810.04 | 809.38 |
| **689** | δ = 1.72(12H, s), 6.63(8H, m), 6.69(2H, m), 6.81 (4H, m), 7.04(1H, m) , 7.12(2H, m), 7.2(8H, m), 7.51∼7.59(7H, m), 7.78∼7.81(2H, m), 8.07(1H, m), 8.49∼8.54(2H, m) | 860.09 | 859.39 |
| **698** | δ = 1.72(12H, s), 2.34(12H, s), 6.51(8H, m), 6.69(4H, m), 6.98(8H, m), 7.12(2H, m), 7.51∼7.59(7H, m), 7.81(1H, m), 8.54(1H, m) | 866.14 | 865.44 |
| **712** | δ = 1.72(12H, s), 6.63(4H, m), 6.69(8H, m), 6.81(2H, m), 7.12(2H, m), 7.2(4H, m), 7.51∼7.61(17H, m), 7.81(1H, m), 8.04∼8.08(4H, m), 8.42(2H, m), 8.54∼8.55(3H, m) | 1062.34 | 1061.47 |
| **724** | δ = 1.72(12H, s), 6.15(1H, m), 6.57∼6.63(5H, m), 6.81(2H, m), 7.12(2H, m), 7.2(4H, m), 7.41(1H, m), 7.51∼7.32(5H, m), 7.59(1H, m), 8.54(1H, m) | 566.73 | 566.27 |
| **736** | δ = 1.72(12H, s), 6.15(1H, m), 6.57∼6.63(3H, m), 6.81(1H, m), 7.12(2H, m), 7.2(2H, m), 7.36(1H, m), 7.49∼7.51(3H, m), 7.58∼7.59(4H, m), 7.73∼7.77(3H, m), 7.84∼7.92(3H, m), 8(2H, m), 8.54(1H, m) | 666.85 | 666.30 |
| **758** | δ = 1.72(12H, s), 6.63(4H, m), 6.69(2H, m), 6.81(2H, m), 7.12(2H, m), 7.2(4H, m), 7.51∼7.59(8H, m), 7.73(1H, m), 7.81(1H, m), 7.92(1H, m), 8(2H, m), 8.54(1H, m) | 692.89 | 692.32 |
| **779** | δ = 1.72(12H, s), 7.41(3H, m), 7.51∼7.52(12H, m), 7.59(2H, m), 7.68(2H, m), 7.81(1H, m), 8.54(1H, m) | 551.72 | 551.26 |
| **802** | δ = 1.72(12H, s), 6.63(12H, m), 6.69(6H, m), 6.81(6H, m), 7.2(12H, m), 7.54∼7.59(8H, m), 7.68(2H, m), 7.81(1H, m), 8.54(1H, m) | 1053.34 | 1052.48 |
| **805** | δ = 1.72(12H, s), 6.63(6H, m), 6.69(6H, m), 6.81(3H, m), 7.2(6H, m), 7.36(3H, m), 7.49∼7.59(14H, m), 7.68(2H, m), 7.74∼7.88(13H, m), 8.54(1H, m) | 1203.51 | 1202.53 |
| **830** | δ = 1.72(12H, s), 6.55(1H, m), 6.61(1H, m), 6.73(1H, m), 6.87(3H, m), 7.02∼7.05(2H, m), 7.36∼7.41(3H, m), 7.51∼7.52(8H, m), 7.61∼7.66(4H, m) | 553.73 | 553.28 |
| **872** | δ = 1.72(12H, s), 6.55(1H, m), 6.61(1H, m), 6.73(1H, m), 6.87(3H, m), 7.02∼7.05(2H, m), 7.25(4H, m), 7.36(1H, m), 7.55(2H, m), 7.61(2H, m), 8.04∼8.08(2H, m), 8.42(1H, m), 8.55(1H, m) | 527.70 | 527.26 |
| **925** | δ = 1.72(12H, s), 6.2(1H, m), 6.3(1H, m), 6.37(1H, m), 6.55(1H, m), 6.73(1H, m), 6.87∼6.91(4H, m), 6.98∼7.05(3H, m), 7.38(1H, m), 7.53∼7.57(3H, m), 7.82∼7.88(4H, m), 8.02∼8.12(4H, m), 8.93(2H, m) | 642.83 | 642.30 |
| **978** | δ = 1.72(12H, s), 6.55(1H, m), 6.61∼6.63(5H, m), 6.73(1H, m), 6.81(2H, m), 6.87(3H, m), 6.98∼7.05(3H, m), 7.2(4H, m), 7.36∼7.38(2H, m), 7.6∼7.61(2H, m), 8.03∼8.04(2H, m), 8.4(1H, m) | 618.81 | 618.30 |
| **984** | δ = 1.72(12H, s), 6.55(1H, m), 6.61∼6.63(5H, m), 6.69∼6.73(3H, m), 6.81(2H, m), 6.87(3H, m), 7.02∼7.05(2H, m), 7.2(4H, m), 7.36(1H, m), 7.54(2H, m), 7.61(1H, m) | 568.75 | 568.29 |
| **1002** | δ = 1.72(18H, s), 6.55∼6.63(5H, m), 6.73∼6.81(3H, m), 6.87(3H, m), 7.02∼7.05(2H, m), 7.2(2H, m), 7.36(2H, m), 7.49∼7.5(2H, m), 7.61∼7.63(3H, m), 7.74∼7.77(3H, m), 7.84∼7.93(3H, m) | 734.97 | 734.37 |
| **1004** | δ = 1.72(12H, s), 6.55(1H, m), 6.61∼6.63(3H, m), 6.69∼6.73(3H, m), 6.81(1H, m), 6.87(3H, m), 6.98∼7.05(3H, m), 7.2(2H, m), 7.36∼7.38(2H, m), 7.53∼7.61(6H, m), 8.02∼8.07(2H, m) | 618.81 | 618.30 |
| **1026** | δ = 1.72(12H, s), 6.2(1H, m), 6.3(1H, m), 6.37(1H, m), 6.55(1H, m), 6.69∼6.73(3H, m), 6.87(3H, m), 6.98∼7.05(3H, m), 7.38(1H, m), 7.53∼7.59(8H, m), 7.73(1H, m), 7.92(1H, m), 8∼8.07(4H, m) | 668.87 | 668.32 |
| **1042** | δ = 1.72(12H, s), 6.55(1H, m), 6.61(1H, m), 6.73(1H, m), 7.02∼7.05(2H, m), 7.36∼7.43(5H, m), 7.51∼7.52(8H, m), 7.61(1H, m) | 477.64 | 477.25 |
| **1048** | δ = 1.35(18H, s), 1.72(12H, s), 6.55(1H, m), 6.61(1H, m), 6.73(1H, m), 7.02∼7.05(2H, m), 7.36∼7.43(11H, m), 7.61(1H, m) | 589.85 | 589.37 |
| **1073** | δ = 1.72(12H, s), 6.55(1H, m), 6.61(1H, m), 6.73(1H, m), 7.02∼7.05(2H, m), 7.36∼7.43(13H, m), 7.51∼7.52(8H, m), 7.61(1H, m), 7.91(8H, m) | 830.06 | 829.37 |
| **1117** | δ = 1.72(12H, s), 6.55(1H, m), 6.61∼6.63(9H, m), 6.69∼6.73(5H, m), 6.81(4H, m), 7.02∼7.05(2H, m), 7.2(8H, m), 7.36(1H, m), 7.43(2H, m), 7.54(4H, m), 7.61(1H, m) | 812.05 | 811.39 |
| **1123** | δ = 1.72(24H, s), 6.55∼6.63(12H, m), 6.73∼6.81(7H, m), 7.02∼7.05(2H, m), 7.2(8H, m), 7.36(1H, m), 7.43(2H, m), 7.61∼7.63(5H, m), 7.77(2H, m), 7.93(2H, m) | 1044.37 | 1043.52 |
| **1138** | δ = 1.72(18H, s), 6.55(1H, m), 6.61(1H, m), 6.73(1H, m), 7.02∼7.05(2H, m), 7.28(1H, m), 7.36∼7.43(5H, m), 7.51∼7.55(5H, m), 7.61∼7.63(2H, m), 7.77(1H, m), 7.87∼7.93(2H, m) | 593.80 | 593.31 |
| **1155** | δ = 1.72(12H, s), 6.2(1H, m), 6.3(1H, m), 6.37(1H, m), 6.55(1H, m), 6.63(4H, m), 6.73(1H, m), 6.81(2H, m), 7.02∼7.05(2H, m), 7.2(4H, m), 7.43(2H, m), 7.58∼7.59(3H, m), 7.73(1H, m), 7.92(1H, m), 8(2H, m) | 618.81 | 618.30 |
| **1166** | δ = 1.72(12H, s), 6.2(1H, m), 6.3(1H, m), 6.37(1H, m), 6.55(1H, m), 6.63(2H, m), 6.69∼6.73(3H, m), 6.81(1H, m), 7.02∼7.05(2H, m), 7.2(2H, m), 7.41∼7.43(3H, m), 7.51∼7.55(8H, m), 7.61(1H, m), 8.04∼8.08(2H, m), 8.42(1H, m), 8.55(1H, m) | 694.90 | 694.33 |
| **1173** | δ = 1.72(12H, s), 6.19(2H, m), 6.55(1H, m), 6.61∼6.63(9H, m), 6.69∼6.73(3H, m), 6.81(4H, m), 7.02∼7.05(2H, m), 7.2(8H, m), 7.36(1H, m), 7.54(2H, m), 7.61(1H, m) | 735.96 | 735.36 |
| **1177** | δ = 1.72(12H, s), 6.19(2H, m), 6.55(1H, m), 6.61∼6.63(7H, m), 6.69∼6.73(3H, m), 6.81(3H, m), 6.98∼7.05(3H, m), 7.2(6H, m), 7.36∼7.38(2H, m), 7.53∼7.61(6H, m), 8.02∼8.07(2H, m) | 786.01 | 785.38 |
| **1183** | δ = 1.72(12H, s), 6.19(2H, m), 6.55(1H, m), 6.61∼6.63(7H, m), 6.69∼6.73(3H, m), 6.81(3H, m), 7.02∼7.05(2H, m), 7.2(6H, m), 7.36(2H, m), 7.49∼7.54(4H, m), 7.61(1H, m), 7.74∼7.77(2H, m), 7.84∼7.88(2H, m) | 786.01 | 785.38 |
| **1196** | δ = 1.72(12H, s), 6.55(2H, m), 7.19∼7.38(15H, m), 7.5(3H, m), 7.63(3H, m), 7.94(3H, m), 8.12(3H, m), 8.55(3H, m) | 821.02 | 820.36 |
| **1201** | δ = 1.35(54H, s), 1.72(12H, s), 6.19∼6.2(4H, m), 6.3(2H, m), 6.37(2H, m), 6.55(12H, m), 7.01(12H, m) | 1163.70 | 1162.78 |
| **1208** | δ = 1.72(30H, s), 6.19∼6.2(4H, m), 6.3(2H, m), 6.37(2H, m), 6.58∼6.63(9H, m), 6.75∼6.81(6H, m), 7.2(6H, m), 7.28(3H, m), 7.38(3H, m), 7.55(3H, m), 7.62(3H, m), 7.87(3H, m) | 1175.55 | 1174.59 |
| **1217** | δ = 1.72(12H, s), 6.61∼6.63(14H, m), 6.69(6H, m), 6.81(6H, m), 7.2(12H, m), 7.36(2H, m), 7.43(2H, m), 7.54(6H, m), 7.61(2H, m) | 1055.35 | 1054.50 |
| **1219** | δ = 1.72(12H, s), 6.61(2H, m), 6.69(6H, m), 6.98(3H, m), 7.36∼7.43(10H, m), 7.49∼7.61(23H, m), 7.74∼7.77(6H, m), 7.84∼7.88(6H, m), 8.02∼8.07(6H, m) | 1355.71 | 1354.59 |
| **1243** | δ = 1.72(24H, s), 6.87(6H, m), 7.25∼7.28(5H, m), 7.38∼7.43(3H, m), 7.55(1H, m), 7.63(1H, m), 7.77(1H, m), 7.87∼7.93(2H, m) | 633.86 | 633.34 |
| **1249** | δ = 1.72(18H, s), 6.87(6H, m), 7.25(4H, m), 7.39∼7.43(7H, m), 7.51∼7.52(4H, m), 7.91(4H, m) | 693.92 | 693.34 |
| **1264** | δ = 1.72(24H, s), 6.19(2H, m), 6.58∼6.63(3H, m), 6.75∼6.81(2H, m), 6.87(6H, m), 7.2(2H, m), 7.28(1H, m), 7.38(1H, m), 7.55(1H, m), 7.62(1H, m), 7.87(1H, m) | 648.88 | 648.35 |
| **1295** | δ = 1.72(18H, s), 6.63(4H, m), 6.81(2H, m), 6.87(6H, m), 7.04(1H, m), 7.2(4H, m), 7.43(2H, m), 7.53∼7.54(2H, m), 7.78(1H, m), 8.07(1H, m), 8.49(1H, m) | 658.87 | 658.33 |
| **1316** | δ = 1.72(18H, s), 6.63(2H, m), 6.69(2H, m), 6.81(1H, m), 6.87(6H, m), 7.2(2H, m), 7.36(1H, m), 7.43(2H, m), 7.49∼7.54(4H, m), 7.74∼7.77(2H, m), 7.84∼7.88(2H, m) | 658.87 | 658.33 |
| **1364** | δ = 1.72(18H, s), 6.87(3H, m), 7.25∼7.33(6H, m), 7.43(4H, m), 7.5(2H, m), 7.63∼7.68(6H, m), 7.79(4H, m), 7.94(2H, m), 8.12(2H, m), 8.55(2H, m) | 848.08 | 847.39 |
| **1404** | δ = 1.72(18H, s), 6.63(8H, m), 6.69(4H, m), 6.81(4H, m), 6.87(3H, m), 7.2(8H, m), 7.43(4H, m), 7.54(4H, m) | 852.12 | 851.42 |
| **1409** | δ = 1.72(18H, s), 6.69(4H, m), 6.87(3H, m), 7.36(4H, m), 7.43(4H, m), 7.49∼7.54(12H, m), 7.74∼7.77(8H, m), 7.84∼7.88(8H, m) | 1052.35 | 1051.49 |
| **1417** | δ = 1.72(18H, s), 2.34(12H, s), 6.51(8H, m), 6.69(4H, m), 6.87(3H, m), 6.98(8H, m), 7.43(4H, m), 7.54(4H, m) | 908.22 | 907.49 |
| **1428** | δ = 1.72(18H, s), 6.63(4H, m), 6.69(2H, m), 6.81(2H, m), 6.87(3H, m), 6.98∼7.04(3H, m), 7.2(4H, m), 7.38∼7.43(6H, m), 7.53∼7.57(10H, m), 7.78(1H, m), 8.02∼8.07(5H, m), 8.49(1H, m) | 1002.29 | 1001.47 |
| **1454** | δ = 1.72(18H, s), 6.19(2H, m), 6.63(2H, m), 6.81(1H, m), 6.87(3H, m), 6.98(1H, m), 7.2(2H, m), 7.38∼7.43(3H, m), 7.53∼7.59(6H, m), 7.73(1H, m), 7.92(1H, m), 8∼8.07(4H, m) | 708.93 | 708.35 |
| **1469** | δ = 1.72(18H, s), 6.63(4H, m), 6.69(2H, m), 6.81(2H, m), 6.87(3H, m), 7.2(4H, m), 7.41∼7.43(5H, m), 7.51∼7.54(6H, m) | 684.91 | 684.35 |
| **1473** | δ = 1.72(18H, s), 6.63(2H, m), 6.69(2H, m), 6.81(1H, m), 6.87(3H, m), 6.98(1H, m), 7.2(2H, m), 7.38∼7.43(6H, m), 7.51∼7.57(9H, m), 8.02∼8.07(2H, m) | 734.97 | 734.37 |
| **1484** | δ = 1.72(18H, s), 6.19(2H, m), 6.63(6H, m), 6.69(2H, m), 6.81(3H, m), 6.87(3H, m), 6.98(1H, m), 7.2(6H, m), 7.38∼7.43(3H, m), 7.53∼7.57(5H, m), 8.02∼8.07(2H, m) | 826.08 | 825.41 |
| **1505** | δ = 1.72(18H, s), 7.2∼7.33(15H, m), 7.5(3H, m), 7.63(3H, m), 7.94(3H, m), 8.12(3H, m), 8.55(3H, m) | 861.08 | 860.39 |
| **1507** | δ = 1.72(18H, s), 7.25∼7.33(9H, m), 7.43(6H, m), 7.5(3H, m), 7.63∼7.68(9H, m), 7.79(6H, m), 7.94(3H, m), 8.12(3H, m), 8.55(3H, m) | 1089.37 | 1088.48 |
| **1513** | δ = 1.35(27H, s), 1.72(18H, s), 6.19(6H, m), 6.55(6H, m), 6.63(6H, m), 6.81(3H, m), 7.01(6H, m), 7.2(6H, m) | 1035.45 | 1034.62 |
| **1520** | δ = 1.72(18H, s), 6.63(12H, m), 6.69(6H, m), 6.81(6H, m), 7.2(12H, m), 7.43(6H, m), 7.54(6H, m) | 1095.42 | 1094.53 |
| **1524** | δ = 1.72(18H, s), 6.63(6H, m), 6.69(6H, m), 6.81(3H, m), 6.98(3H, m), 7.2(6H, m), 7.38∼7.43(9H, m), 7.53∼7.57(15H, m), 8.02∼8.07(6H, m) | 1245.59 | 1244.58 |
| **1528** | δ = 1.72(18H, s), 6.63(6H, m), 6.81(3H, m), 7.04(3H, m), 7.2(6H, m), 7.36(3H, m), 7.43(6H, m), 7.49∼7.54(12H, m), 7.74∼7.88(15H, m), 8.07(3H, m), 8.49(3H, m) | 1395.77 | 1394.62 |

### [Example 1] Manufacture of an OLED by using compounds for organic electronic material according to the invention (I)

An OLED device was manufactured by using a compound for electronic material according to the invention.

First, a transparent electrode ITO thin film (15 Ω/□) (2) prepared from glass for OLED (produced by Samsung Corning) (1) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) (of which the structure is shown below) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injecting layer (3) having 60 nm thickness on the ITO substrate.

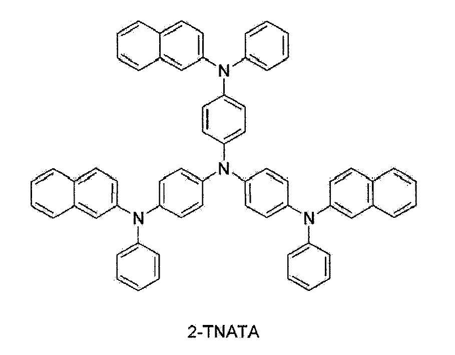

Then, to another cell of the vacuum vapor-deposit device, charged was N,N'-bis(α-naphthyl)-N,N'-diphenyl-4,4'-diamine (NPB) (of which the structure is shown below), and electric current was applied to the cell to evaporate NPB, thereby providing vapor-deposit of a hole transport layer (4) of 20 nm thickness on the hole injecting layer.

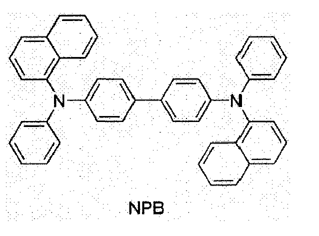

After forming a hole injecting layer and a hole transport layer, an electroluminescent layer was vapor-deposited as follows. To one cell of said vacuum vapor-deposit device, charged was a compound according to the invention (e.g., Compound 279) as host material (which had been purified via sublimation in vacuo at 10⁻⁶ torr), and an electroluminescent dopant compound (e.g., (piq)₂Ir(acac)) was charged to another cell. The two substances were evaporated at different rates to carry out doping at a concentration of 4 to 10% by weight, thereby vapor-depositing an electroluminescent layer (5) with 30 nm thickness on the hole transport layer.

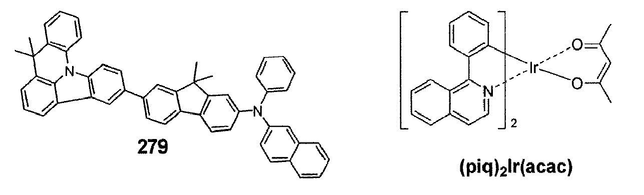

Then, on the electroluminescent layer, bis(2-methyl-8-quinolinato)(p-phenylphenolato)aluminum (III) (BAlq) was vapor-deposited as a hole blocking layer with a thickness of 5 nm, tris(8-hydroxyquinoline)aluminum (III) (Alq) was vapor-deposited as an electron transport layer (6) with a thickness of 20 nm, and then lithium quinolate (Liq) was vapor-deposited as an electron injecting layer (7) with a thickness of 1 to 2 nm. Thereafter, an Al cathode (8) was vapor-deposited with a thickness of 150 nm by using another vacuum vapor-deposit device to manufacture an OLED.

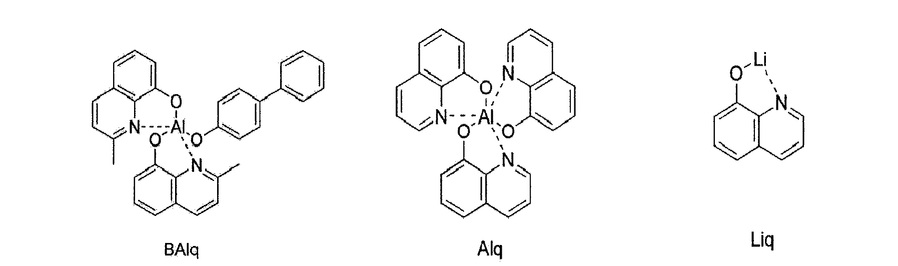

### [Comparative Example 1] Electroluminescent properties of OLED employing conventional electroluminescent material

An OLED device was manufactured according to the same procedure as in Example 1, but using bis(2-methyl-8-quinolinato)(p-phenylphenolato)aluminum (III) (BAlq) as electroluminescent host material in another cell of the vacuum deposit device, instead of the electroluminescent compound according to the present invention.

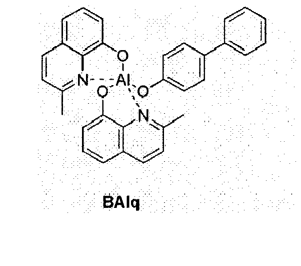

The luminous efficiencies and power efficiencies of the OLED's comprising the organic electroluminescent compound according to the present invention (Example 1) or conventional electroluminescent compound (Comparative Example 1) were measured at 1,000 cd/m², respectively, and the results are shown in Table 2.

**Table 2**

| No. | Host material | EL material | Hole blocking layer | Operation voltage (V) @1,000 cd/m² | Power efficiency (lm/W) @1,000 cd/m² | EL color |
|---|---|---|---|---|---|---|
| 1 | **279** | (piq)₂Ir(acac) | BAlq | 6.5 | 4.4 | Red |
| 2 | **298** | (piq)₂Ir(acac) | BAlq | 6.7 | 4.7 | Red |
| 3 | **592** | (piq)₂Ir(acac) | BAlq | 6.5 | 4.8 | Red |
| 4 | **600** | (piq)₂Ir(acac) | BAlq | 6.8 | 4.4 | Red |
| 5 | **990** | (piq)₂Ir(acac) | BAlq | 6.8 | 4.0 | Red |
| 6 | **1005** | (piq)₂Ir(acac) | BAlq | 7.0 | 4.4 | Red |
| 7 | **1301** | (piq)₂Ir(acac) | BAlq | 6.9 | 4.3 | Red |
| 8 | **1306** | (piq)₂Ir(acac) | BAlq | 6.5 | 4.3 | Red |
| 9 | **1315** | (piq)₂Ir(acac) | BAlq | 6.8 | 4.7 | Red |
| 10 | **1473** | (piq)₂Ir(acac) | BAlq | 6.9 | 4.2 | Red |
| Comp.1 | BAlq | (piq)₂Ir(acac) | - | 7.5 | 2.6 | Red |

As can be seen from Table 2, the complexes developed according to the present invention showed better electroluminescent properties than conventional material.

Thus, the devices employing the electroluminescent compounds as host material exhibit excellent electroluminescent properties with lowered operation voltage to induce increase of power efficiency by 0.8 ∼ 2.2 lm/W with improved power consumption.

### [Example 2] Electroluminescent properties of OLED using compounds for organic electronic material according to the invention (II)

After forming a hole injecting layer (3) according to the same procedure as in Example 1, Compound (394) (of which the structure is shown below) was charged to another cell of the vacuum vapor-deposit device, and electric current was applied to the cell to evaporate the material to vapor-deposit a hole transport layer (4) with a thickness of 20 nm on the hole injecting layer.

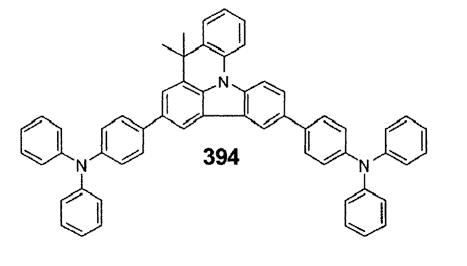

An OLED was manufactured according to the same procedure as in Example 1, otherwise.

The luminous efficiencies of the OLED's comprising the compound for electronic material according to the present invention (Example 2) or conventional electroluminescent compound (Comparative Example 1) were measured at 5,000 cd/m², respectively, and the results are shown in Table 3.

**Table 3**

| No. | Material of hole transport layer | Operation voltage (V) @1,000 cd/m² | Luminous efficiency (cd/A) @1,000 cd/m² | Color |
|---|---|---|---|---|
| 1 | Compound 394 | 5.5 | 5.4 | Red |
| 2 | Compound 765 | 5.4 | 5.6 | Red |
| Comp.1 | NPB | 6 | 4.5 | Red |

It is confirmed that the compounds developed by the present invention exhibit better properties than conventional materials in view of performances.

### [Example 3] Electroluminescent properties of OLED employing compounds for organic electronic material according to the invention (III)

An ITO substrate was equipped in a substrate folder of a vacuum vapor-deposit device according to the same procedure as in Example 1. Compound (494) (of which the structure is shown below) was placed in a cell of the vacuum vapor-deposit device, which was then ventilated up to 10⁻⁶ torr of vacuum in the chamber. Electric current was applied to the cell to evaporate Compound (494), thereby providing vapor-deposit of a hole injecting layer (3) having 60 nm thickness on the ITO substrate.

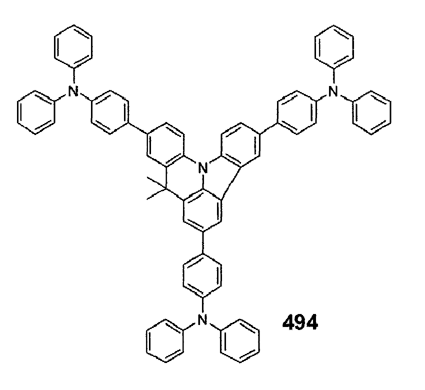

An OLED was manufactured according to the same procedure as in Example 1, otherwise.

The luminous efficiencies of the OLED's comprising organic electroluminescent compounds according to the present invention (Example 3) or conventional electroluminescent compound (Comparative Example 1) were measured at 5,000 cd/m², respectively, and the results are shown in Table 4.

**Table 4**

| No. | Material of hole injecting layer | Operation voltage (V) @1,000 cd/m² | Luminous efficiency (cd/A) @1,000 cd/m² | Color |
|---|---|---|---|---|
| 1 | Compound 494 | 5.2 | 5.4 | Red |
| 2 | Compound 805 | 5.0 | 5.6 | Red |
| Comp.1 | 2-TNATA | 6 | 4.5 | Red |

It is confirmed that the compounds developed by the present invention exhibit better properties than conventional materials in view of performances.

## Claims

1. A compound for organic electronic material represented by Chemical Formula (1) or (2): 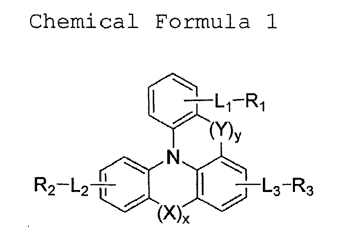 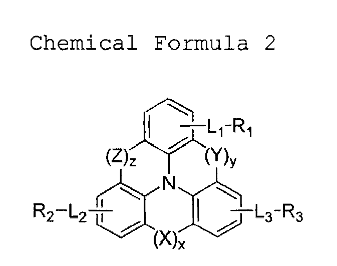 wherein,
L₁, L₂ and L₃ independently represent a chemical bond, (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C6-C60)ar(C1-C60)alkylene, (C1-C60)alkyenethio, (C1-C60)alkylenoxy, (C6-C60)arylenoxy or (C6-C60)arylenethio;
R₁, R₂ and R₃ independently represent hydrogen, deuterium, (C6-C60)aryl, (C3-C60)heteroaryl, 5- or 6-membered heterocycloalkyl containing one or more heteroatom(s) selected from N, O and S, (C3-C60)cycloalkyl, adamantyl, (C7-C60)bicycloalkyl, (C2-C60)alkenyl, (C2-C60)alkynyl, (C1-C60)alkylthio, (C6-C60)aryloxy, (C6-C60)arylthio, NR₁₁R₁₂, or a substituent selected from the following structures (excluding the case wherein R₁, R₂ and R₃ are hydrogen all at the same time): 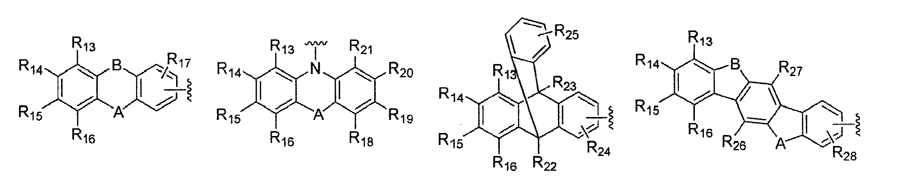 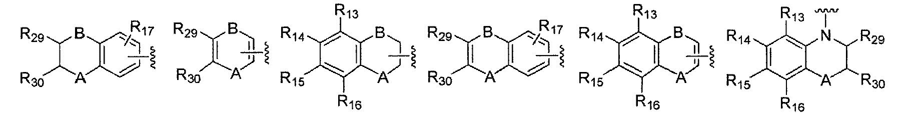 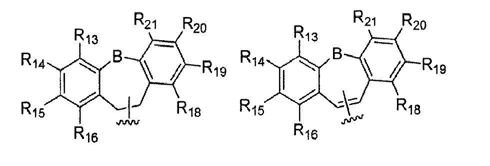
R₁₁ and R₁₂ independently represent (C6-C60)aryl or (C3-C60)heteroaryl, or may be linked via (C3-C60)alkylene or (C3-C60)alkenylene with or without a fused ring to form an alicyclic ring, or a monocyclic or polycyclic aromatic ring;
R₁₃ through R₃₀ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsil tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbon (C1-C60)alkylcarbonyl, (C6-C60)arylcarbonyl, carboxyl, nitro or hydroxyl, or each of R₁₃ through R₂₅ may be linked to an adjacent substituent via (C3-C60)alkylene or (C3-C60)alkenylene with or without a fused ring to form an alicyclic ring, or a monocyclic or polycyclic aromatic ring;
A, B, X, Y and Z independently represent a chemical bond, or -(CR₃₁R₃₂)ₐ-, -N(R₃₃)-, -S-, -O-, -Si(R₃₄)(R₃₅)-, -P(R₃₆)-, - C(=O)-, -B(R₃₇)-, -In(R₃₈)-, -Se-, -Ge(R₃₉)(R₄₀)-, -Sn(R₄₁)(R₄₂)-, -Ga(R₄₃)- or -(R₄₄)C=C(R₄₅)-;
R₃₁ through R₄₅ independently represent hydrogen, deuterium, halogen, (C1-C60)alkyl, (C6-C60) aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenoxy or arylenethio of L₁, L₂ and L₃; the aryl or heteroaryl of R₁, R₂, R₃, R₁₁ and R₁₂; or the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, aralkyl, alkyloxy, alkylthio, aryloxy, arylthio, alkylamino, arylamino, alkoxycarbonyl, alkylcarbonyl or arylcarbonyl of R₁₃ through R₃₀ and R₃₁ through R₄₅ may be further substituted by one or more substituent(s) selected from a group consisting of deuterium, halogen, (C1-C60)alkyl with or without halogen substituent(s), (C6-C60)aryl, (C3-C60)heteroaryl with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsil di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
a, x, y and z independently represent an integer from 0 to 4;
but excluding the case wherein R₁-L₁-, R₂-L₂- or R₃-L₃-independently represents diphenylamino group (-NPh₂).

2. The compound for organic electronic material according to claim 1, which is selected from the compounds represented by Chemical Formulas (3) to (6): 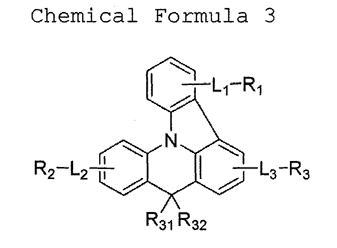 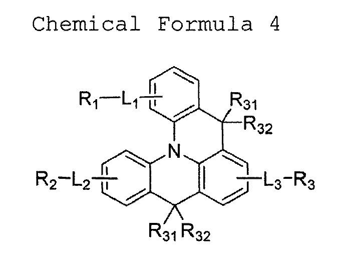 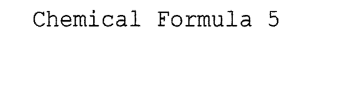 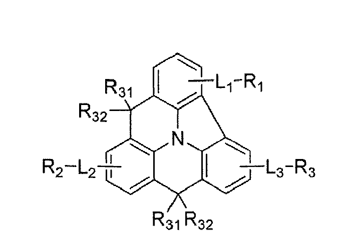 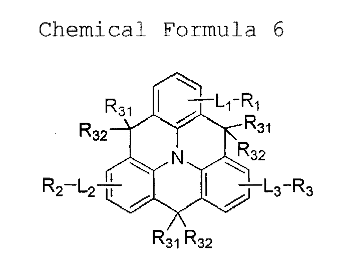 wherein, L₁, L₂, L₃, R₁, R₂, R₃, R₃₁ and R₃₂ are defined as in claim 1.

3. The compound for organic electronic material according to claim 2, wherein R₁, R₂ and R₃ independently represent hydrogen, deuterium or NR₁₁R₁₂, or a substituent selected from the following structures: 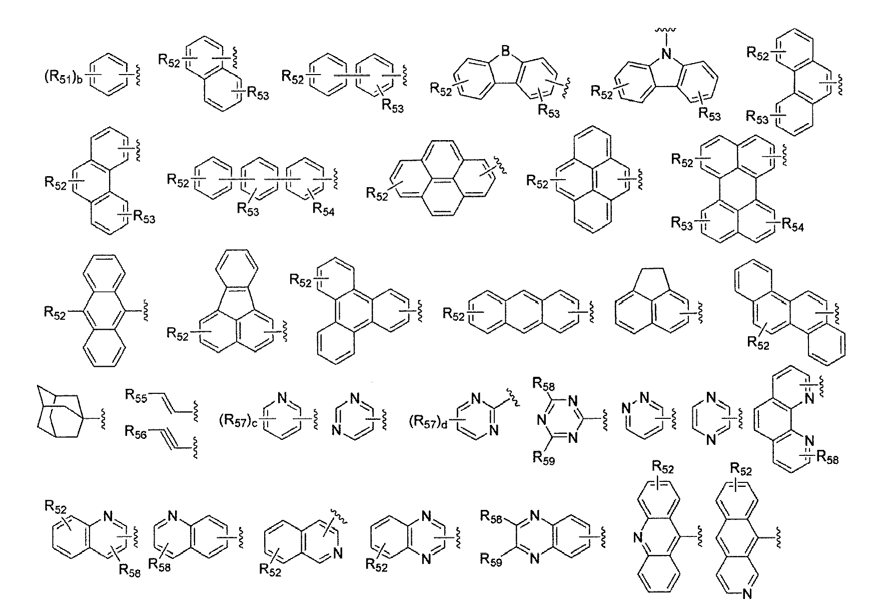 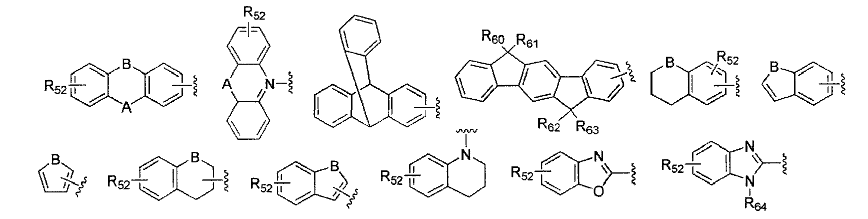 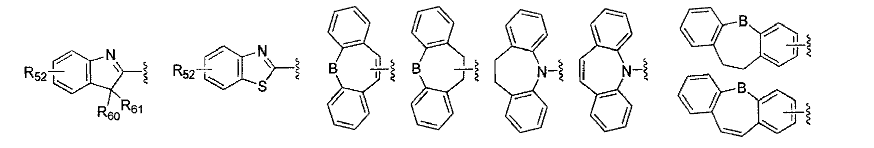
wherein, R₁₁ and R₁₂ are defined as in claim 1;
R₅₁ through R₆₄ independently represent hydrogen, deuterium, halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl;
A and B independently represent -C(R₃₁)(R₃₂)-, -N(R₃₃)-, - S-, -O-, -Si(R₃₄)(R₃₅)-, -P(R₃₆)-, -C(=O)-, -B(R₃₇)-, -In(R₃₈)-, - Se-, -Ge(R₃₉)(R₄₀)-, -Sn(R₄₁)(R₄₂)-, -Ga(R₄₃)- or -(R₄₄)C=C(R₄₅)-;
R₃₁ through R₄₅ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₃₁ through R₄₅ and R₅₁ through R₆₄ may be further substituted by deuterium, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl;
b represents an integer from 1 to 5; c represents an integer from 1 to 4; and d represents an integer from 1 to 3.

4. An organic electronic device which is comprised of a first electrode, a second electrode, and at least one organic layer(s) interposed between the first electrode and the second electrode;
wherein the organic layer comprises an organic electronic material represented by Chemical Formula (1) or (2): 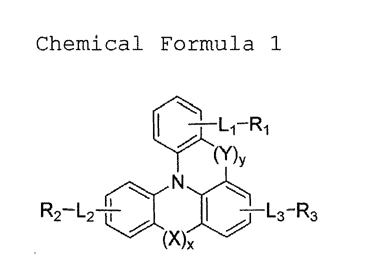 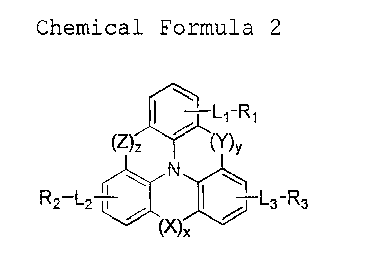 wherein,
L₁, L₂ and L₃ independently represent a chemical bond, (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C6-C60)ar(C1-C60)alkylen (C1-C60)alkyenethio, (C1-C60)alkylenoxy, (C6-C60)arylenoxy or (C6-C60)arylenethio;
R₁, R₂ and R₃ independently represent hydrogen, deuterium, (C6-C60)aryl, (C3-C60)heteroaryl, 5- or 6-membered heterocycloalkyl containing one or more heteroatom(s) selected from N, O and S, (C3-C60)cycloalkyl, adamantyl, (C7-C60)bicycloalkyl, (C2-C60)alkenyl, (C2-C60)alkynyl, (C1-C60)alkylthio, (C6-C60)aryloxy, (C6-C60)arylthio, NR₁₁R₁₂, or a substituent selected from the following structures (excluding the case wherein R₁, R₂ and R₃ are hydrogen all at the same time): 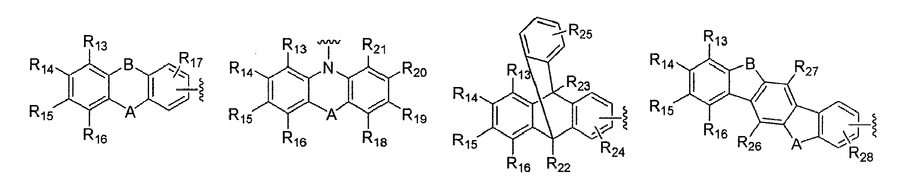 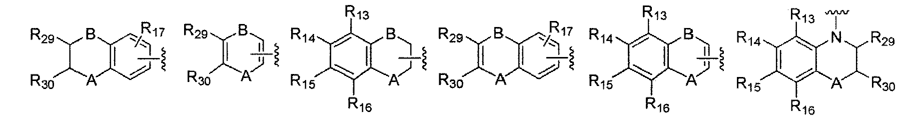 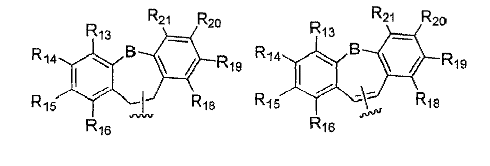
R₁₁ and R₁₂ independently represent (C6-C60)aryl or (C3-C60)heteroaryl, or may be linked via (C3-C60)alkylene or (C3-C60)alkenylene with or without a fused ring to form an alicyclic ring, or a monocyclic or polycyclic aromatic ring;
R₁₃ through R₃₀ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbo (C6-C60)arylcarbonyl, carboxyl, nitro or hydroxyl, or each of R₁₃ through R₂₅ may be linked to an adjacent substituent via (C3-C60)alkylene or (C3-C60)alkenylene with or without a fused ring to form an alicyclic ring, or a monocyclic or polycyclic aromatic ring;
A, B, X, Y and Z independently represent a chemical bond, or -(CR₃₁R₃₂)ₐ-, -N(R₃₃)-, -S-, -O-, -Si(R₃₄)(R₃₅)-, -P(R₃₆)-, - C(=O)-, -B(R₃₇)-, -In(R₃₈)-, -Se-, -Ge(R₃₉)(R₄₀)-, -Sn(R₄₁)(R₄₂)-, -Ga(R₄₃)- or -(R₄₄)C=C(R₄₅)-;
R₃₁ through R₄₅ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsiyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenoxy or arylenethio of L₁, L₂ and L₃; the aryl or heteroaryl of R₁, R₂, R₃, R₁₁ and R₁₂; or the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, aralkyl, alkyloxy, alkylthio, aryloxy, arylthio, alkylamino, arylamino, alkoxycarbonyl, alkylcarbonyl or arylcarbonyl of R₁₃ through R₃₀ and R₃₁ through R₄₅ may be further substituted by one or more substituent(s) selected from a group consisting of deuterium, halogen, (C1-C60)alkyl with or without halogen substituent(s), (C6-C60)aryl, (C3-C60)heteroaryl with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
a, x, y and z independently represent an integer from 0 to 4;
but excluding the case wherein R₁-L₁-, R₂-L₂- or R₃-L₃-independently represents diphenylamino group (-NPh₂) and one or more dopant(s) represented by Chemical Formula (7):
Chemical Formula 7 **M¹L¹⁰¹L¹⁰²L¹⁰³**
wherein, M¹ is selected from a group consisting of metals of Group 7, 8, 9, 10, 11, 13, 14, 15 and 16 in the Periodic Table of Elements, and ligands L¹⁰¹, L¹⁰² and L¹⁰³ are independently selected from the following structures: 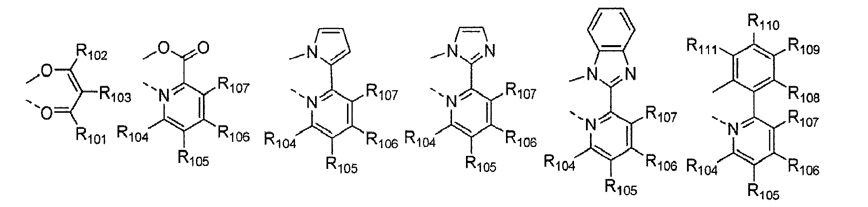 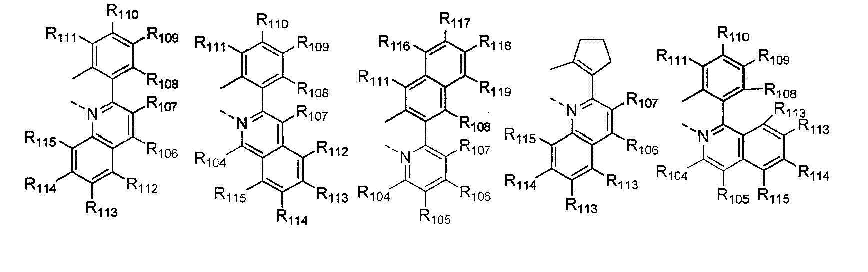 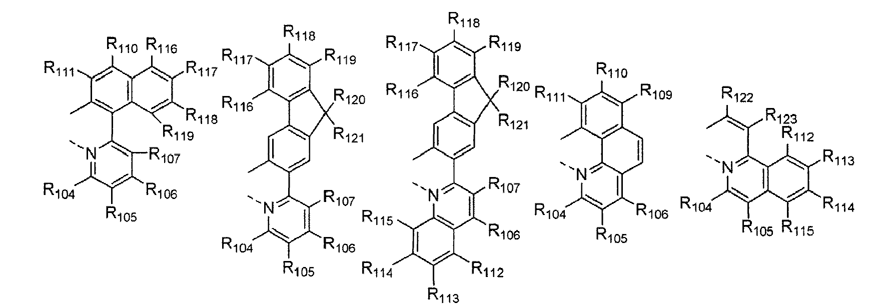 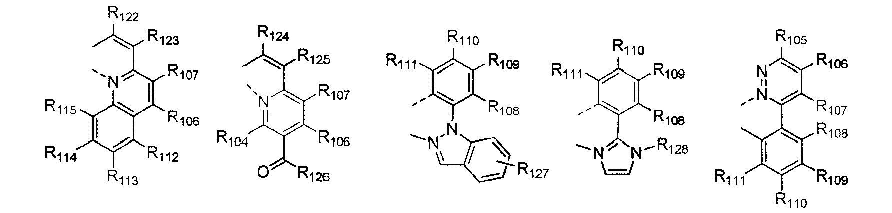 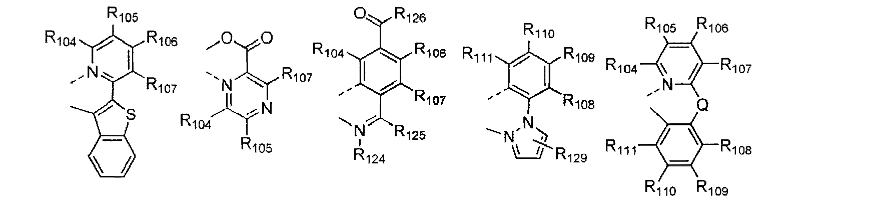
wherein, R₁₀₁ through R₁₀₃ independently represent hydrogen, deuterium, (C1-C60)alkyl with or without halogen substituent(s), (C6-C60)aryl with or without (C1-C60)alkyl substituent(s), or halogen;
R₁₀₄ through R₁₁₉ independently represent hydrogen, deuterium, (C1-C60)alkyl, (C1-C30)alkyloxy, (C3-C60)cycloalkyl, (C2-C30)alkenyl, (C6-C60)aryl, mono or di(C1-C30)alkylamino, mono or di(C6-C30)arylamino, SF₅, tri(C1-C30)alkylsilyl, di(C1-C30)alkyl(C6-C30)arylsilyl, tri(C6-C30)arylsilyl, cyano or halogen; the alkyl, cycloalkyl, alkenyl or aryl of R₁₀₄ through R₁₁₉ may be further substituted by one or more substituent(s) selected from deuterium, (C1-C60)alkyl, (C6-C60)aryl and halogen;
R₁₂₀ through R₁₂₃ independently represent hydrogen, deuterium, (C1-C60)alkyl with or without halogen substituent(s), or (C6-C60)aryl with or without (C1-C60)alkyl substituent(s);
R₁₂₄ and R₁₂₅ independently represent hydrogen, deuterium, (C1-C60)alkyl, (C6-C60) aryl or halogen, or R₁₂₄ and R₁₂₅ may be linked via (C3-C12)alkylene or (C3-C12)alkenylene with or without a fused ring to form an alicyclic ring, or a monocyclic or polycyclic aromatic ring; the alkyl or aryl of R₁₂₄ and R₁₂₅, or the alicyclic ring or the monocyclic or polycyclic aromatic ring formed therefrom by linkage via (C3-C12)alkylene or (C3-C12)alkenylene with or without a fused ring may be further substituted by one or more substituent(s) selected from deuterium, (C1-C60)alkyl with or without halogen substituent(s), (C1-C30)alkyloxy, halogen, tri(C1-C30)alkylsilyl, tri(C6-C30)arylsilyl and (C6-C60)aryl;
R₁₂₆ represents (C1-C60)alkyl, (C6-C60)aryl, (C5-C60)heteroaryl or halogen;
R₁₂₇ through R₁₂₉ independently represent hydrogen, deuterium, (C1-C60)alkyl, (C6-C60)aryl or halogen; the alkyl or aryl of R₁₂₆ through R₁₂₉ may be further substituted by halogen or (C1-C60)alkyl; and
Q represents 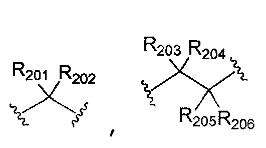 or 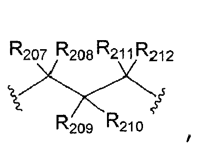 wherein R₂₀₁ through R₂₁₂ independently represent hydrogen, deuterium, (C1-C60)alkyl with or without halogen substituent(s), (C1-C30)alkyloxy, halogen, (C6-C60)aryl, cyano or (C5-C60)cycloalkyl, or each of R₂₀₁ through R₂₁₂ may be linked to an adjacent substituent via alkylene or alkenylene to form a (C5-C7)spiro ring or a (C5-C9) fused ring, or linked to R₁₀₇ or R₁₀₈ via alkylene or alkenylene to form a (C5-C7)fused ring.

5. The organic electronic device according to claim 4, wherein the organic layer comprises one or more compound(s) selected from a group consisting of arylamine compounds and styrylarylamine compounds.

6. The organic electronic device according to claim 4, which comprises one or more metal(s) selected from a group consisting of organometals of Group 1, Group 2, 4^{th} period and 5^{th} period transition metals, lanthanide metals and d-transition elements in the Periodic Table of Elements.

7. The organic electronic device according to claim 4, wherein the organic layer comprises an electroluminescent layer and a charge generating layer.

8. A white electroluminescent device comprising an organic electroluminescent compound represented by Chemical Formula (1) or (2): 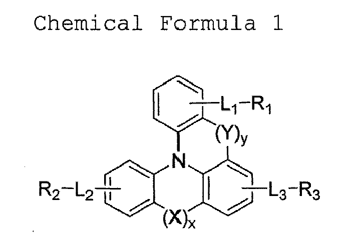 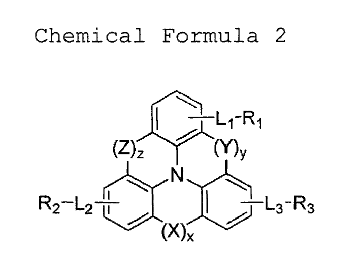 wherein,
L₁, L₂ and L₃ independently represent a chemical bond, (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C6-C60)ar(C1-C60)alkylene, (C1-C60)alkyenethio, (C1-C60)alkylenoxy, (C6-C60)arylenoxy or (C6-C60)arylenethio;
R₁, R₂ and R₃ independently represent hydrogen, deuterium, (C6-C60)aryl, (C3-C60)heteroaryl, 5- or 6-membered heterocycloalkyl containing one or more heteroatom(s) selected from N, O and S, (C3-C60)cycloalkyl, adamantyl, (C7-C60)bicycloalkyl, (C2-C60)alkenyl, (C2-C60)alkynyl, (C1-C60)alkylthio, (C6-C60)aryloxy, (C6-C60)arylthio, NR₁₁R₁₂, or a substituent selected from the following structures (excluding the case wherein R₁, R₂ and R₃ are hydrogen all at the same time) : 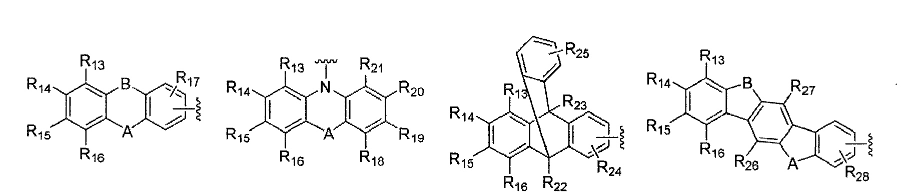 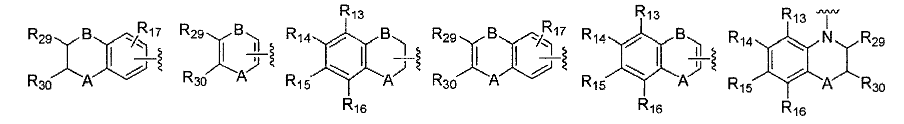 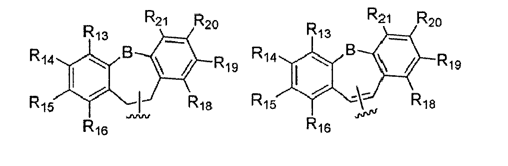
R₁₁ and R₁₂ independently represent (C6-C60)aryl or (C3-C60)heteroaryl, or may be linked via (C3-C60)alkylene or (C3-C60)alkenylene with or without a fused ring to form an alicyclic ring, or a monocyclic or polycyclic aromatic ring;
R₁₃ through R₃₀ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₁₃ through R₂₅ may be linked to an adjacent substituent via (C3-C60)alkylene or (C3-C60)alkenylene with or without a fused ring to form an alicyclic ring, or a monocyclic or polycyclic aromatic ring;
A, B, X, Y and Z independently represent a chemical bond, or -(CR₃₁R₃₂)ₐ-, -N(R₃₃)-, -S-, -O-, -Si(R₃₄)(R₃₅)-, -P(R₃₆)-, - C(=O)-, -B(R₃₇)-, -In(R₃₈)-, -Se-, -Ge(R₃₉)(R₄₀)-, -Sn(R₄₁)(R₄₂)-, -Ga(R₄₃)- or -(R₄₄)C=C(R₄₅)-;
R₃₁ through R₄₅ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenoxy or arylenethio of L₁, L₂ and L₃; the aryl or heteroaryl of R₁, R₂, R₃, R₁₁ and R₁₂; or the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, aralkyl, alkyloxy, alkylthio, aryloxy, arylthio, alkylamino, arylamino, alkoxycarbonyl, alkylcarbonyl or arylcarbonyl of R₁₃ through R₃₀ and R₃₁ through R₄₅ may be further substituted by one or more substituent(s) selected from a group consisting of deuterium, halogen, (C1-C60)alkyl with or without halogen substituent(s), (C6-C60) aryl, (C3-C60)heteroaryl with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
a, x, y and z independently represent an integer from 0 to 4;
but excluding the case wherein R₁-L₁-, R₂-L₂- or R₃-L₃-independently represents diphenylamino group (-NPh₂).

9. An organic solar cell comprising an compound for organic electronic material represented by Chemical Formula (1) or (2) : 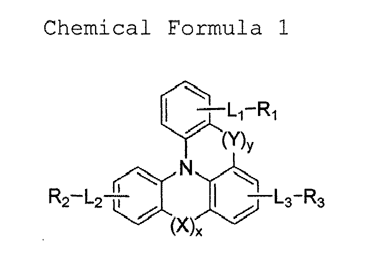 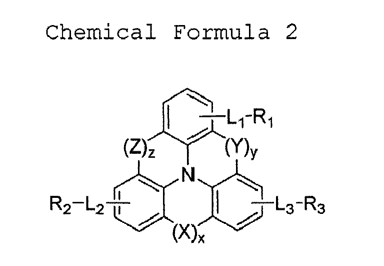 wherein,
L₁, L₂ and L₃ independently represent a chemical bond, (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C6-C60)ar(C1-C60)alkylene, (C1-C60)alkyenethio, (C1-C60)alkylenoxy, (C6-C60)arylenoxy or (C6-C60)arylenethio;
R₁, R₂ and R₃ independently represent hydrogen, deuterium, (C6-C60)aryl, (C3-C60)heteroaryl, 5- or 6-membered heterocycloalkyl containing one or more heteroatom(s) selected from N, O and S, (C3-C60)cycloalkyl, adamantyl, (C7-C60)bicycloalkyl, (C2-C60)alkenyl, (C2-C60)alkynyl, (C1-C60)alkylthio, (C6-C60)aryloxy, (C6-C60)arylthio, NR₁₁R₁₂, or a substituent selected from the following structures (excluding the case wherein R₁, R₂ and R₃ are hydrogen all at the same time) : 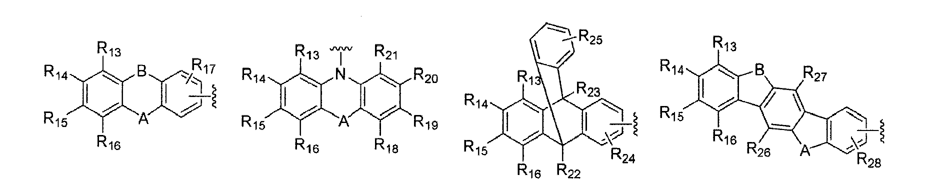 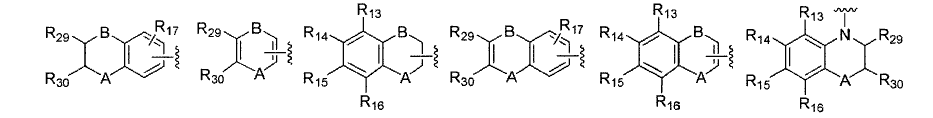 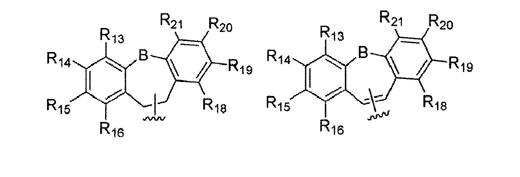
R₁₁ and R₁₂ independently represent (C6-C60)aryl or (C3-C60)heteroaryl, or may be linked via (C3-C60)alkylene or (C3-C60)alkenylene with or without a fused ring to form an alicyclic ring, or a monocyclic or polycyclic aromatic ring;
R₁₃ through R₃₀ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (Cl-C60)alkoxycarbonyl, (C1-C60)alkylcarbonyl, (C6-C60)arylcarbonyl, carboxyl, nitro or hydroxyl, or each of R₁₃ through R₂₅ may be linked to an adjacent substituent via (C3-C60)alkylene or (C3-C60)alkenylene with or without a fused ring to form an alicyclic ring, or a monocyclic or polycyclic aromatic ring;
A, B, X, Y and Z independently represent a chemical bond, or -(CR₃₁R₃₂)ₐ-, -N(R₃₃)-, -S-, -O-, -Si(R₃₄)(R₃₅)-, -P(R₃₆)-, - C(=O)-, -B(R₃₇)-, -In(R₃₈)-, -Se-, -Ge(R₃₉)(R₄₀)-, -Sn(R₄₁)(R₄₂)-, -Ga(R₄₃)- or -(R₄₄)C=C(R₄₅)-;
R₃₁ through R₄₅ independently represent hydrogen, deuterium, halogen, (C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenoxy or arylenethio of L₁, L₂ and L₃; the aryl or heteroaryl of R₁, R₂, R₃, R₁₁ and R₁₂; or the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, aralkyl, alkyloxy, alkylthio, aryloxy, arylthio, alkylamino, arylamino, alkoxycarbonyl, alkylcarbonyl or arylcarbonyl of R₁₃ through R₃₀ and R₃₁ through R₄₅ may be further substituted by one or more substituent(s) selected from a group consisting of deuterium, halogen, (C1-C60)alkyl with or without halogen substituent(s), (C6-C60)aryl, (C3-C60)heteroaryl with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsil di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
a, x, y and z independently represent an integer from 0 to 4;
but excluding the case wherein R₁-L₁-, R₂-L₂- or R₃-L₃-independently represents diphenylamino group (-NPh₂).
